(19) Europäisches Patentamt
European Patent Office
Office européen des brevets

(11) Veröffentlichungsnummer: **0 192 060**
**A1**

(12) # EUROPÄISCHE PATENTANMELDUNG

(21) Anmeldenummer: **86100708.6**

(22) Anmeldetag: **17.01.86**

(51) Int. Cl.[4]: **C 07 D 401/06**
C 07 D 401/14, C 07 D 403/06
C 07 D 403/14, C 07 D 413/06
C 07 D 413/14, C 07 D 417/06
C 07 D 417/14, C 07 D 405/06
C 07 D 409/06, C 07 D 419/06

(30) Priorität: **04.02.85 JP 18627/85**
**04.02.85 JP 18628/85**
**12.02.85 JP 23683/85**
**21.05.85 JP 106853/85**
**21.05.85 JP 106854/85**
**03.10.85 JP 219082/85**

(43) Veröffentlichungstag der Anmeldung:
**27.08.86 Patentblatt 86/35**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Shiokawa, Kozo**
**210-6, Shukugawara Tama-ku**
**Kawasaki-shi Kanagawa-ken(JP)**

(72) Erfinder: **Tsuboi, Shinichi**
**3-26-1, Hirayama**
**Hino-shi Tokyo(JP)**

(72) Erfinder: **Kagabu, Shinzo**
**432-131-105, Terada-machi**
**Hachioji-shi Tokyo(JP)**

(72) Erfinder: **Moriya, Koichi**
**39-15, Namiki-cho**
**Hachioji-shi Tokyo(JP)**

(74) Vertreter: **Ernst, Hilmar, Dr. et al,**
**Bayer AG Konzernverwaltung RP Patentabteilung**
**D-5090 Leverkusen, Bayerwerk(DE)**

(54) **Neue heterocyclische Verbindungen.**

(57) Die vorliegende Erfindung betrifft neue heterocyclische Verbindungen der Formel (I)

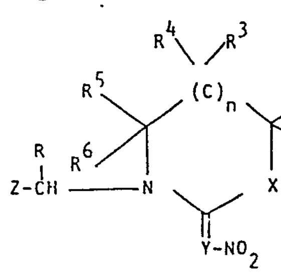

(I)

worin

N 0 oder 1,

$R^1$, $R^2$, $R^5$ und $R^6$ unabhängig voneinander ein Wasserstoffatom oder eine Alkylgruppe bedeuten,

$R^3$ und $R^4$ unabhängig voneinander ein Wasserstoffatom, eine Hydroxygruppe oder eine Alkylgruppe bezeichnen, wenn n 1 bezeichnet, $R^2$ eine Einfachbindung zusammen mit $R^5$ zu bilden vermag,

X ein Schwefelatom, ein Sauerstoffatom oder die folgenden Gruppen bedeutet:

$-N(-)-R^7$ oder $-CH(-)-R^8$

worin $R^7$ und $R^8$ die im Anmeldetext angegebene Bedeutung haben,

R ein Wasserstoffatom oder eine Alkylgruppe bedeutet,

Y ein Stickstoffatom oder die Gruppe

$=C(-)-R^9$

bedeutet, worin $R^9$ die im Anmeldungstext angegebene Bedeutung hat und

Z die im Anmeldungstext angegebene Bedeutung hat.

EP 0 192 060 A1

## Neue heterocyclische Verbindungen

Die vorliegende Erfindung betrifft neue heterocyclische Verbindungen, Verfahren zu ihrer Herstellung und ihre Verwendung als Insektizide.

Es wurde bereits offenbart, daß nicht nur bestimmte Nitromethylen-Derivate insektizide Funktion aufweisen, beispielsweise 1-Benzyl-2-nitromethylen-tetrahydropyrimidin (siehe die DE-OS 2 514 402), sondern daß bestimmte Triazolidin-Derivate Anti-Tumor-Wirkung gegen Tumoren des Magen-Darm-Traktes besitzen (siehe die JP-OS 196 877/1984).

Weiterhin ist 1-Benzyl-2-nitroiminoimidazolidin in Can. J. Chem., Band 39, Seiten 1787-1796, beschrieben.

Nunmehr wurden neue heterocyclische Verbindungen der Formel (I)

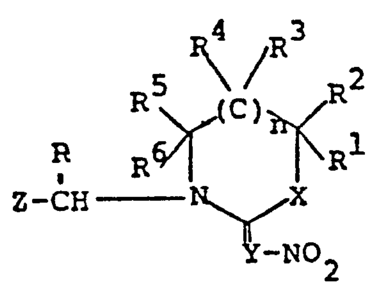

(I)

gefunden, in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

X ein Schwefel-Atom, ein Sauerstoff-Atom oder eine der folgenden Gruppen $-\overset{|}{N}-R^7$ oder $-\overset{|}{C}H-R^8$ bezeichnet, worin

$R^7$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Alkenylcarbonyl-Gruppe, eine Alkylcarbonyl-Gruppe, die durch wenigstens einen Substituenten substituiert sein kann, der

ausgewählt ist aus der aus Alkoxy-Gruppen, einer Phenoxy-Gruppe, Alkylthio-Gruppen und Halogen-Atomen bestehenden Klasse, eine Benzoyl-Gruppe, die durch wenigstens einen Substituenten substituiert sein kann, der ausgewählt ist aus der aus Halogen-Atomen, Alkyl-Gruppen, Halogenomethyl-Gruppen, Alkoxy-Gruppen, Halogenoalkoxy-Gruppen und einer Nitro-Gruppe bestehenden Klasse, eine Benzylcarbonyl-Gruppe, die durch eine Alkyl-Gruppe und/oder ein Halogen-Atom substituiert sein kann, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus einer Methyl-Gruppe, einer Methoxy-Gruppe, Halogenomethyl-Gruppen, Halogenomethoxy-Gruppen, Halogen-Atomen und einer Nitro-Gruppe bestehenden Klasse, eine Phenylthiocarbonyl-Gruppe, die durch ein Halogen-Atom und/oder eine Alkyl-Gruppe substituiert sein kann, eine Benzyloxycarbonyl-Gruppe, eine Monoalkyl- oder Dialkylaminocarbonyl-Gruppe, eine Phenylaminocarbonyl-Gruppe, die durch wenigstens einen Substituenten substituiert sein kann, der ausgewählt ist aus der aus Alkyl-Gruppen, Halogenoalkyl-Gruppen und Halogen-Atomen bestehenden Klasse, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine

Alkyl-Gruppe und/oder ein Halogen-Atom substituiert sein kann, eine Phenylthio-Gruppe, die durch eine Alkyl-Gruppe und/oder ein Halogen-Atom substituiert sein kann, eine Alkylsulfonyl-Gruppe, die durch ein Halogen-Atom substituiert sein kann, eine Phenylsulfonyl-Gruppe, die durch wenigstens einen Substituenten substituiert sein kann, der ausgewählt ist aus der aus Alkyl-Gruppen, Halogen-Atomen und einer Nitro-Gruppe bestehenden Klasse, eine Alkylcarbonylmethyl-Gruppe, eine Phenacyl-Gruppe, die durch ein Halogen-Atom und/oder eine Alkyl-Gruppe substituiert sein kann, eine Organophosphono-Gruppe, eine Organothiophosphono-Gruppe, die folgenden Gruppen $-CH_2-W$ oder -CO-W, worin W eine 5- bis 6-gliedrige heterocyclische Gruppe bezeichnet, die wenigstens ein aus der aus Sauerstoff-Atomen, Schwefel-Atomen und Stickstoff-Atomen bestehenden Klasse ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen bestehenden Klasse, darstellt,

$R^8$ ein Wasserstoff-Atom, eine Alkyl-Gruppe, eine Aryl-Gruppe oder eine Benzyl-Gruppe darstellt,

Y ein Stickstoff-Atom oder die nachstehende Gruppe $=\overset{|}{C}-R^9$ bezeichnet, worin

$R^9$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert

sein kann, der ausgewählt ist aus der aus Halogen-Atomen, einer Hydroxy-Gruppe, Alkoxy-Gruppen, Alkylthio-Gruppen, einer Cyano-Gruppe, Mono- oder Dialkylamino-Gruppen, Alkylcarbonyl-Gruppen, Alkoxycarbonyl-Gruppen und Phenoxycarbonyl-Gruppen bestehenden Klasse, eine Alkenyl-Gruppe, die durch ein Halogen-Atom substituiert sein kann, eine Alkinyl-Gruppe, eine Phenyl-Gruppe, die durch eine Alkyl-Gruppe und/oder ein Halogen-Atom substituiert sein kann, eine Alkylcarbonyl-Gruppe, die durch ein Halogen-Atom substituiert sein kann, eine Alkenylcarbonyl-Gruppe, eine Benzoyl-Gruppe, die durch wenigstens einen Substituenten substituiert sein kann, der ausgewählt ist aus der aus Halogen-Atomen, Alkyl-Gruppen und Alkoxy-Gruppen bestehenden Klasse, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus Halogen-Atomen, Alkyl-Gruppen, Alkoxy-Gruppen und einer Nitro-Gruppe bestehenden Klasse, eine Phenylthiocarbonyl-Gruppe, die durch eine Alkyl-Gruppe und/oder ein Halogen-Atom substituiert sein kann, eine Benzyloxycarbonyl-Gruppe, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert

sein kann, eine Alkylsulfonylaminocarbonyl-Gruppe, eine Alkylthio-Gruppe, eine Alkylsulfonyl-Gruppe, die durch ein Halogen-Atom substituiert sein kann, eine Phenylthio-Gruppe, die durch eine Alkyl-Gruppe und/oder ein Halogen-Atom substituiert sein kann, eine Phenylsulfonyl-Gruppe, die durch eine Alkyl-Gruppe und/oder ein Halogen-Atom substituiert sein kann, und außerdem

$R^9$ eine Bis-Form der Formel (I) über eine Methylen-Gruppe zu bilden vermag,

R ein Wasserstoff-Atom oder eine Alkyl-Gruppe darstellt und

Z eine 5- bis 6-gliedrige heterocyclische Gruppe bezeichnet, die wenigstens ein aus der aus Sauerstoff-Atomen, Schwefel-Atomen und Stickstoff-Atomen bestehenden Klasse ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen, einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer Thioxo-Gruppe, Halogenoalkenylthio-Gruppen,

Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die gegebenenfalls durch einen der gleichen Substituenten substituiert sind, wie sie für die Aryl-Gruppen angegeben ist, bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf.

In dem Fall, in dem Verbindungen der Formel (I) die nachstehende Formel (Ia)

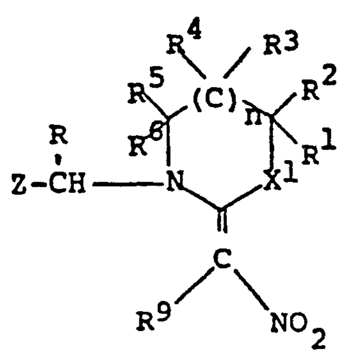

(Ia)

Nit 189

haben, in der

n, $R^1$, $R^2$, $R^3$, $R^4$, $R^5$, $R^6$, R, $R^9$ und Z die im Vorstehenden angegebenen Bedeutungen haben,

$X^1$ ein Schwefel-Atom, ein Sauerstoff-Atom oder die folgende Gruppe $-\overset{|}{N}-R^{10}$ bezeichnet,

$R^{10}$ in der Definition von $R^7$ andere Gruppen als Acyl-Gruppen einschließlich Sulfonyl-Gruppen und Phosphono-Gruppen bezeichnet,

werden die Verbindungen der Formel (Ia) erhalten, wenn

(a) die Verbindungen der Formel (II)

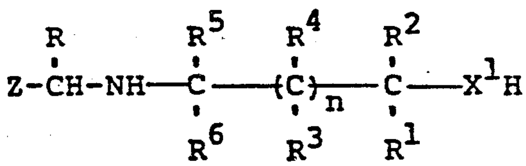

(II)

,

in der n, $R^1$, $R^2$, $R^3$, $R^4$, $R^5$, $R^6$, R, $X^1$ und Z die im Vorstehenden angegebenen Bedeutungen haben, mit den Verbindungen der Formel (III)

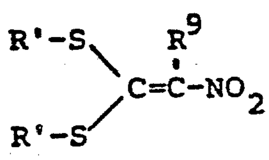

(III)

umgesetzt werden, in der R' eine Niederalkyl- oder Benzyl-Gruppe bezeichnet oder die beiden R' zusammen mit den zwei Schwefel-Atomen, an die sie gebunden sind, einen Ring bilden können, und $R^9$ die gleiche Bedeutung hat, wie sie oben angegeben ist, gegebenfalls in Anwesenheit inerter Lösungsmittel,

(b) die Verbindungen der vorstehenden Formel (II) mit Verbindungen der Formel (IV)

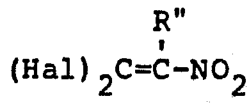

(IV)

umgesetzt werden, in der Hal ein Halogen-Atom bezeichnet und R" für ein Wasserstoff-Atom, ein Halogen-Atom oder eine Niederalkyl-Gruppe steht, gegebenfalls in Anwesenheit inerter Lösungsmittel und in Anwesenheit von Säure-Acceptoren, oder

(c) die Verbindungen der vorstehenden Formel (II) mit Verbindungen der Formel (V)

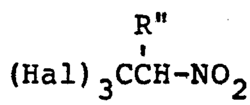

(V)

umgesetzt werden, in der Hal und R" die gleichen Bedeutungen haben, wie sie oben angegeben sind, gegebenfalls in Anwesenheit inerter Lösungsmittel und in Anwesenheit von Säure-Acceptoren.

In dem Fall, in dem Verbindungen der Formel (I) die nachstehende Formel (Ib)

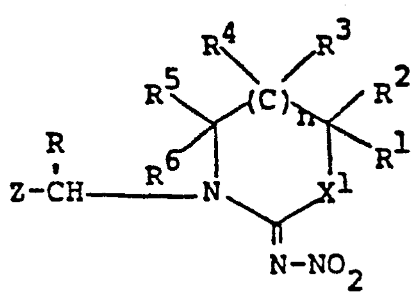

(Ib)

haben, in der

n, $R^1$, $R^2$, $R^3$, $R^4$, $R^5$, $R^6$, R, $X^1$ und Z die im Vorstehenden angegebenen Bedeutungen haben,

werden die Verbindungen der Formel (Ib) erhalten, wenn

(d) die Verbindungen der vorstehenden Formel (II) mit Nitroguanidin der folgenden Formel

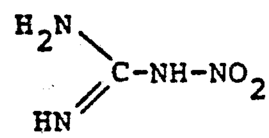

umgesetzt werden, gegebenfalls in Anwesenheit inerter Lösungsmittel.

In dem Fall, in dem Verbindungen der Formel (I) die nachstehende Formel (Ic)

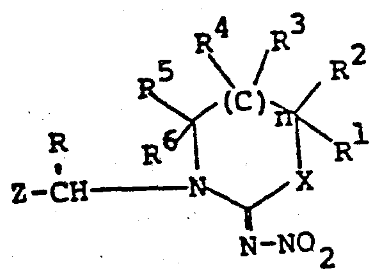

(Ic)

haben, in der

n, $R^1$, $R^2$, $R^3$, $R^4$, $R^5$, $R^6$, X, R und Z die im Vorstehenden angegebenen Bedeutungen haben,

werden die Verbindungen der Formel (Ic) erhalten, wenn

(e) die Verbindungen der vorstehenden Formel (VI)

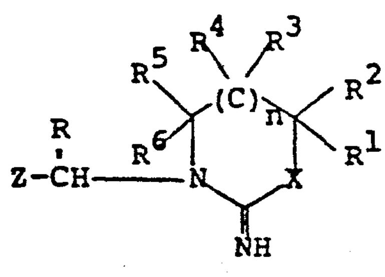

(VI)

,

in der
n, $R^1$, $R^2$, $R^3$, $R^4$, $R^5$, $R^6$, X, R und Z die im Vorstehenden angegebenen Bedeutungen haben, mit rauchender Salpetersäure umgesetzt werden, gegebenenfalls in Anwesenheit inerter Lösungsmittel.

Die Verbindungen der Formel (I) werden erhalten, wenn
(f) die Verbindungen der Formel (VII)

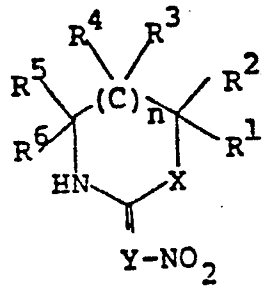

(VII)

,

in der
n, $R^1$, $R^2$, $R^3$, $R^4$, $R^5$, $R^6$, X und Y die im Vorstehenden angegebenen Bedeutungen haben, mit den Verbindungen der Formel (VIII)

$$\begin{array}{c} R \\ | \\ Z\text{-}CH\text{-}M \end{array} \qquad (VIII)$$

umgesetzt werden, in der R und Z die im Vorstehenden angegebenen Bedeutungen haben, M ein Halogen-Atom oder die folgende Gruppe $-OSO_2T$ bezeichnet und T für eine Niederalkyl-Gruppe, eine Phenyl-Gruppe oder eine Tolyl-Gruppe steht, gegebenenfalls in Anwesenheit inerter Lösungsmittel und in Anwesenheit von Säure-Acceptoren.

Die neuen heterocyclischen Verbindungen zeigen potente insektizide Eigenschaften.

Überraschenderweise zeigen die erfindungsgemäßen heterocyclischen Verbindungen eine wesentlich stärkere und bei weitem überragendere insektizide Wirkung als die am nächsten kommenden Verbindungen des oben genannten Standes der Technik.

Außerdem zeigen die erfindungsgemäßen heterocyclischen Verbindungen auch eine bemerkenswerte insektizide Wirkung gegen Schadinsekten, insbesondere saugende Insekten, wie sie typisch durch Insekten der Gattung Hemiptera repräsentiert werden, etwa Blattläuse, Laternenträger und Heuschrecken, die aufgrund der Langzeit-Verwendung von Insektiziden vom Typ organischer Phosphate und Carbamate Resistenz gegen diese Mittel erworben haben.

Unter den neuen heterocyclischen Verbindungen der Formel (I) gemäß der vorliegenden Erfindung sind bevorzugte Verbindungen diejenigen, in denen

n 0 oder 1 bezeichnet,

$R^1$, $R^2$, $R^5$ und $R^6$ unabhängig voneinander ein Wasserstoff-Atom oder eine Alkyl-Gruppe mit 1 bis 4 Kohlenstoff-Atomen bezeichnen,

$R^3$ und $R^4$ unabhängig voneinander ein Wasserstoff-Atom, eine Hydroxy-Gruppe oder eine Alkyl-Gruppe mit 1 bis 4 Kohlenstoff-Atomen bezeichnen,

X ein Schwefel-Atom, ein Sauerstoff-Atom oder eine der folgenden Gruppen $-\overset{|}{N}-R^7$ oder $-\overset{|}{C}H-R^8$ bezeichnet,

$R^7$ ein Wasserstoff-Atom, ein Fluor-Atom, ein Chlor-Atom, ein Brom-Atom, eine Hydroxy-Gruppe, eine Alkoxy-Gruppe mit 1 bis 4 Kohlenstoff-Atomen, eine Benzyloxy-Gruppe, eine Alkyl-Gruppe mit 1 bis 4

Kohlenstoff-Atomen, die durch wenigstens einen Substituenten substituiert sein kann, der ausgewählt ist aus der aus Alkoxy-Gruppen mit 1 bis 4 Kohlenstoff-Atomen, Alkylthio-Gruppen mit 1 bis 4 Kohlenstoff-Atomen, einer Cyano-Gruppe, einem Fluor-Atom, einem Chlor-Atom, einem Brom-Atom, einer Dimethylamino-Gruppe und Trimethylsilyl bestehenden Klasse, eine Alkenyl-Gruppe mit 2 bis 3 Kohlenstoff-Atomen, die durch ein Chlor-Atom substituiert sein kann, eine Alkinyl-Gruppe mit 2 bis 3 Kohlenstoff-Atomen, eine Benzyl-Gruppe, die durch wenigstens einen Substituenten substituiert sein kann, der ausgewählt ist aus der aus einer Methyl-Gruppe, einer Methoxy-Gruppe, einem Fluor-Atom, einem Chlor-Atom, einem Brom-Atom und einer Nitro-Gruppe bestehenden Klasse, eine Formyl-Gruppe, eine Alkenylcarbonyl-Gruppe mit einem Alkenyl-Teil mit 2 bis 3 Kohlenstoff-Atomen, eine Alkyl-Gruppe mit 1 bis 5 Kohlenstoff-Atomen, die durch wenigstens einen Substituenten substituiert sein kann, der ausgewählt ist aus der aus einer Methoxy-Gruppe, einer Phenoxy-Gruppe, einem Fluor-Atom, einem Chlor-Atom und einem Brom-Atom bestehenden Klasse, eine Benzoyl-Gruppe, die durch wenigstens einen Substituenten substituiert sein kann, der ausgewählt ist aus der aus einem Fluor-Atom, einem Chlor-Atom, einem Brom-Atom, einer Methyl-Gruppe, einer Trifluoromethyl-Gruppe, einer Methoxy-Gruppe, einer Difluoromethoxy-Gruppe, einer Trifluoromethoxy-Gruppe und einer Nitro-Gruppe bestehenden Klasse, eine Benzylcarbonyl-Gruppe, die durch wenigstens einen Substituenten substituiert sein kann, der ausgewählt ist aus der aus einem Fluor-Atom, einem Chlor-Atom und einem

Brom-Atom bestehenden Klasse, eine Alkoxycarbonyl-Gruppe mit einem Alkyl mit 1 bis 4 Kohlenstoff-Atomen, die durch ein Fluor-Atom und/oder ein Chlor-Atom substituiert sein kann, eine Alkylthiocarbonyl-Gruppe mit einem Alkyl mit 1 bis 4 Kohlenstoff-Atomen, eine Phenoxycarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Phenylthiocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Benzyloxycarbonyl-Gruppe, eine Dimethylaminocarbonyl-Gruppe, eine Phenylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Benzoylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Phenylsulfonylaminocarbonyl-Gruppe, die durch wenigstens einen Substituenten substituiert sein kann, der ausgewählt ist aus der aus einem Fluor-Atom, einem Chlor-Atom und einem Brom-Atom bestehenden Klasse, eine Phenylthio-Gruppe, eine Alkylsulfonyl-Gruppe, die durch ein Fluor-Atom und/oder ein Chlor-Atom substituiert sein kann, eine Phenylsulfonyl-Gruppe, die durch wenigstens einen Substituenten substituiert sein

kann, der ausgewählt ist aus der aus einer Methyl-Gruppe, einem Fluor-Atom, einem Chlor-Atom, einem Brom-Atom und einer Nitro-Gruppe bestehenden Klasse, eine Methylcarbonylmethyl-Gruppe, eine Phenacyl-Gruppe, die durch ein Fluor-Atom und/oder ein Chlor-Atom substituiert sein kann, eine Organophosphono-Gruppe, eine Organothiophosphono-Gruppe, die folgenden Gruppen $-CH_2-W$ oder $-CO-W$,

W eine 5- bis 6-gliedrige heterocyclische Gruppe bezeichnet, die ein oder zwei aus der aus Sauerstoff-Atomen, Schwefel-Atomen und Stickstoff-Atomen bestehenden Klasse ausgewählte Hetero-Atome enthält und die durch wenigstens einen Substituenten substituiert sein kann, der ausgewählt ist aus der aus einem Fluor-Atom, einem Chlor-Atom, einem Brom-Atom und Alkyl-Gruppen mit 1 bis 4 Kohlenstoff-Atomen bestehenden Klasse,

$R^8$ ein Wasserstoff-Atom, eine Alkyl-Gruppe mit 1 bis 4 Kohlenstoff-Atomen, eine Phenyl-Gruppe oder eine Benzyl-Gruppe darstellt,

Y ein Stickstoff-Atom oder die nachstehende Gruppe $=\dot{C}-R^9$ bezeichnet,

$R^9$ ein Wasserstoff-Atom, ein Fluor-Atom, ein Chlor-Atom, ein Brom-Atom, eine Hydroxy-Gruppe, eine Alkoxy-Gruppe mit 1 bis 4 Kohlenstoff-Atomen, eine Benzyloxy-Gruppe, eine Alkyl-Gruppe mit 1 bis 4 Kohlenstoff-Atomen, die durch wenigstens einen Substituenten substituiert sein kann, der ausgewählt ist aus der aus einem Fluor-Atom, einem Chlor-Atom, einer Hydroxy-Gruppe, einer Alkoxy-Gruppe mit 1 bis 2 Kohlenstoff-Atomen, Alkylthio-Gruppen mit 1 bis 2 Kohlenstoff-Atomen, einer Cyano-Gruppe, einer Dimethylamino-Gruppe, Alkylcarbonyl-Gruppen mit einem Alkyl mit 1 bis 2 Kohlenstoff-Atomen und Alkoxycarbonyl-Gruppen mit einem Alkyl mit 1 bis 2 Kohlenstoff-Atomen bestehenden Klasse, eine Alkenyl-Gruppe mit 2 bis 3 Kohlenstoff-Atomen, eine Phenyl-Gruppe, eine Alkylcarbonyl-Gruppe mit einem Alkyl mit 1 bis 4 Kohlenstoff-Atomen, die durch wenigstens einen Substituenten substituiert sein kann, der ausgewählt ist aus der aus einer Methoxy-Gruppe, einem Chlor-Atom und einem Fluor-Atom bestehenden Klasse, eine Alkenylcarbonyl-Gruppe mit einem Alkenyl mit 2 bis 3 Kohlenstoff-Atomen, eine Benzoyl-Gruppe, die durch wenigstens einen Substituenten substituiert sein kann, der ausgewählt ist aus der aus einem Fluor-Atom, einem Chlor-Atom, einem Brom-Atom, einer Methoxy-Gruppe und einer Methyl-Gruppe bestehenden Klasse, eine Alkoxycarbonyl-Gruppe, die durch ein Fluor-Atom und/oder ein Chlor-Atom substituiert sein kann, eine Alkylthiocarbonyl-Gruppe mit mit einem Alkyl mit 1 bis 4 Kohlenstoff-Atomen, eine Phenoxycarbonyl-Gruppe, die durch wenigstens einen Substituenten substituiert sein kann, der ausgewählt ist aus der aus einem Fluor-Atom, einem Chlor-Atom, einem Brom-Atom, einer Methyl-Gruppe, einer Methoxy-Gruppe und einer Nitro-Gruppe bestehenden Klasse, eine Phenylthiocarbonyl-Gruppe, eine Benzyloxycarbonyl-Gruppe, eine Benzoylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Phenylsulfonylaminocarbonyl-Gruppe, die durch wenigstens einen Substituenten substituiert sein kann,

der ausgewählt ist aus der aus einer Methyl-Gruppe, einem Fluor-Atom, einem Chlor-Atom und einem Brom-Atom bestehenden Klasse, eine Alkylsulfonylaminocarbonyl-Gruppe mit einem Alkyl mit 1 bis 4 Kohlenstoff-Atomen, eine Alkylthio-Gruppe mit 1 bis 4 Kohlenstoff-Atomen, eine Alkylsulfonyl-Gruppe, die durch ein Fluor-Atom und/oder ein Chlor-Atom substituiert sein kann, eine Phenylthio-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, oder eine Phenylsulfonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, außerdem

$R^9$ eine Bis-Form der Formel (I) über eine Methylen-Gruppe zu bilden vermag,

R ein Wasserstoff-Atom oder eine Methyl-Gruppe darstellt und

Z eine 5- bis 6-gliedrige heterocyclische Gruppe bezeichnet, die ein bis drei Hetero-Atome ausgewählt aus der aus Sauerstoff-Atomen, Schwefel-Atomen und Stickstoff-Atom bestehenden Klasse enthält, von denen wenigstens eines ein Stickstoff-Atom ist, und die durch wenigstens einen Substituenten substituiert sein kann, der ausgewählt ist aus der aus einem Fluor-Atom, einem Chlor-Atom, einem Brom-Atom, Alkyl-Gruppen mit 1 bis 4 Kohlenstoff-Atomen, die durch ein Fluor-Atom und/oder ein Chlor-Atom substituiert sein können, einer Nitro-Gruppe, einer Cyano-Gruppe, Alkylsulfinyl-Gruppen mit 1 bis 4 Kohlenstoff-Atomen, Alkylsulfonyl-Gruppen mit 1 bis 4 Kohlenstoff-Atomen, Alkoxy-Gruppen mit 1 bis 4 Kohlenstoff-Atomen, die durch ein Fluor-Atom und/oder ein Chlor-Atom substituiert ein können, Alkylthio-Gruppen mit 1 bis 4 Kohlenstoff-Atomen, die durch ein Fluor-Atom und/oder ein Chlor-Atom substituiert ein können, Alkenyl-Gruppen mit 2 bis 3 Kohlenstoff-Atomen, die durch ein Chlor-Atom substituiert sein können, eine Acetamid-Gruppe, die durch ein Fluor-Atom und/oder ein Chlor-Atom substituiert ein kann, Alkoxycarbonyl-Gruppen mit einem Alkyl mit 1 bis 4 Kohlenstoff-Atomen, einer Thiocyanato-Gruppe, Alkinyl-Gruppen mit 2 bis 4 Kohlenstoff-Atomen, einer Amino-Gruppe, einer Methylamino-Gruppe, einer Dimethylamino-Gruppe, einer Acetyl-Gruppe, einer Formyl-Gruppe, einer Carboxy-Gruppe, einer Hydroxy-Gruppe, einer Mercapto-Gruppe, Cycloalkyl-Gruppen mit 3 bis 7 Kohlenstoff-Atomen, einer Oxo-Gruppe, einer Thioxo-Gruppe, Alkenylthio-Gruppen, die durch ein Fluor-Atom, ein Chlor-Atom und/oder ein Brom-Atom substituiert sind, Alkoxyalkyl-Gruppen mit insgesamt 2 bis 4 Kohlenstoff-Atomen, Alkylaminocarbonyl-Gruppen mit einem Alkyl mit 1 bis 2 Kohlenstoff-Atomen, Dialkylaminocarbonyl-Gruppen mit einem Alkyl mit 1 bis 2 Kohlenstoff-Atomen, einer Phenyl-Gruppe, einer Phenoxy-Gruppe und einer Benzyl-Gruppe bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf.

Nit 189

Ganz besonders bevorzugte heterocyclische Verbindungen der Formel (I) sind diejenigen, in denen

n 0 oder 1 bezeichnet,

$R^1$, $R^2$, $R^5$ und $R^6$ unabhängig voneinander ein Wasserstoff-Atom oder eine Methyl-Gruppe bezeichnen,

$R^3$ und $R^4$ unabhängig voneinander ein Wasserstoff-Atom oder eine Methyl-Gruppe bezeichnen,

X ein Schwefel-Atom, ein Sauerstoff-Atom oder eine der folgenden Gruppen $-\overset{|}{N}-R^7$ oder $-\overset{|}{C}H-R^8$ bezeichnet,

$R^7$ ein Wasserstoff-Atom, eine Alkyl-Gruppe, die durch wenigstens einen Substituenten substituiert sein kann, der ausgewählt ist aus der aus einer Methoxy-Gruppe, einer Ethoxy-Gruppe, einer Methylthio-Gruppe, einer Ethylthio-Gruppe, einer Cyano-Gruppe, einem Fluor-Atom, einem Chlor-Atom und einer Trimethylsilyl-Gruppe bestehenden Klasse,
eine Allyl-Gruppe, die durch ein Chlor-Atom substituiert sein kann,
eine Propargyl-Gruppe,
eine Benzyl-Gruppe, die durch eine Methyl-Gruppe und/oder ein Chlor-Atom substituiert sein kann,
eine Formyl-Gruppe, eine Vinylcarbonyl-Gruppe,
eine Alkylcarbonyl-Gruppe mit einem Alkyl mit 1 bis 3 Kohlenstoff-Atomen, die durch wenigstens einen Substituenten substituiert sein kann, der ausgewählt ist aus der aus einer Methoxy-Gruppe, einer Phenoxy-Gruppe und einem Chlor-Atom bestehenden Klasse,
eine Benzoyl-Gruppe, die durch wenigstens einen Substituenten substituiert sein kann, der ausgewählt ist aus der aus einem Chlor-Atom, einem Brom-Atom, einer Methyl-Gruppe, einer Trifluoromethyl-Gruppe, einer Methoxy-Gruppe und einer Nitro-Gruppe bestehenden Klasse,
eine Benzylcarbonyl-Gruppe, die durch ein Chlor-Atom substituiert sein kann,
eine Alkoxycarbonyl-Gruppe mit einem Alkyl mit 1 bis 2 Kohlenstoff-Atomen, die durch ein Fluor-Atom und/oder ein Chlor-Atom substituiert sein kann,
eine Alkylthiocarbonyl-Gruppe mit einem Alkyl mit 1 bis 2 Kohlenstoff-Atomen,
eine Phenoxycarbonyl-Gruppe, die durch eine Methyl-Gruppe und/oder ein Chlor-Atom substituiert sein kann,
eine Phenylthiocarbonyl-Gruppe, die durch ein Chlor-Atom substituiert sein kann,
eine Benzyloxycarbonyl-Gruppe, eine Dimethylaminocarbonyl-Gruppe, eine Phenylaminocarbonyl-Gruppe,
eine Benzoylaminocarbonyl-Gruppe,
eine Phenylsulfonylaminocarbonyl-Gruppe, die durch eine Methyl-Gruppe und/oder ein Chlor-Atom substituiert sein kann,
eine Phenylthio-Gruppe,
eine Methylsulfonyl-Gruppe, die durch ein Chlor-Atom substituiert sein kann,
eine Phenylsulfonyl-Gruppe, die durch eine Methyl-Gruppe substituiert sein kann,
eine Methylcarbonylmethyl-Gruppe,
eine Phenacyl-Gruppe, die durch ein Chlor-Atom substituiert sein kann,
eine O,O-Diethylthionophosphono-Gruppe,
eine O-Ethyl-S-n-propylthiolophosphono-Gruppe,
die folgenden Gruppen $-CH_2-W$ oder $-CO-W$,

W eine 5- bis 6-gliedrige heterocyclische Gruppe bezeichnet, die ein oder zwei aus der aus Sauerstoff-Atomen, Schwefel-Atomen und Stickstoff-Atomen bestehenden Klasse ausgewählte Hetero-Atome enthält und die durch ein Fluor-Atom, ein Chlor-Atom, ein Brom-Atom und eine Methyl-Gruppe substituiert sein kann,

$R^8$ ein Wasserstoff-Atom, eine Methyl-Gruppe, eine Phenyl-Gruppe oder eine Benzyl-Gruppe darstellt,

Y ein Stickstoff-Atom oder die nachstehende Gruppe $=\overset{|}{C}-R^9$ bezeichnet,

$R^9$ ein Wasserstoff-Atom, ein Chlor-Atom, ein Brom-Atom, eine Hydroxy-Gruppe, eine Methoxy-Gruppe, eine Benzyloxy-Gruppe, eine Alkyl-Gruppe, die durch wenigstens einen Substituenten substituiert sein kann, der ausgewählt ist aus der aus einem Fluor-Atom, einem Chlor-Atom, einer Hydroxy-Gruppe, einer Methoxy-Gruppe, einer Cyano-Gruppe, einer Dimethylamino-Gruppe, einer Acetyl-Gruppe und einer Methoxycarbonyl-Gruppe bestehenden Klasse,

eine Allyl-Gruppe, eine Phenyl-Gruppe,

eine Acetyl-Gruppe, die durch ein Chlor-Atom substituiert sein kann, eine Vinylcarbonyl-Gruppe, eine Allylcarbonyl-Gruppe, eine Benzoyl-Gruppe, eine Alkoxycarbonyl-Gruppe mit einem Alkyl mit 1 bis 2 Kohlenstoff-Atomen, die durch ein Fluor-Atom substituiert sein kann,

eine n-Butylthiocarbonyl-Gruppe, eine Phenoxycarbonyl-Gruppe, die durch ein Chlor-Atom und/oder eine Methyl-Gruppe substituiert sein kann, eine Phenylthiocarbonyl-Gruppe, eine Benzyloxycarbonyl-Gruppe, eine Benzoylaminocarbonyl-Gruppe, die durch ein Chlor-Atom substituiert sein kann, eine Phenylsulfonylaminocarbonyl-Gruppe, die durch eine Methyl-Gruppe substituiert sein kann, eine Methylsulfonylaminocarbonyl-Gruppe

eine Propylthio-Gruppe, eine Methylsulfonyl-Gruppe, die durch ein Fluor-Atom und/oder ein Chlor-Atom substituiert sein kann, eine Phenylthio-Gruppe, die durch ein Chlor-Atom substituiert sein kann, oder eine Phenylsulfonyl-Gruppe, zusätzlich

$R^9$ eine Bis-Form der Formel (I) über eine Methylen-Gruppe zu bilden vermag,

R ein Wasserstoff-Atom oder eine Methyl-Gruppe darstellt und

Z eine 5- bis 6-gliedrige heterocyclische Gruppe bezeichnet, die ein bis drei Hetero-Atome ausgewählt aus der aus Sauerstoff-Atomen, Schwefel-Atomen und Stickstoff-Atomen bestehenden Klasse enthält, von denen wenigstens eines ein Stickstoff-Atom ist, und die durch wenigstens einen Substituenten substituiert sein kann, der ausgewählt ist aus der aus einem Fluor-Atom, einem Chlor-Atom, einem Brom-Atom, einer Methyl-Gruppe, Fluoroalkyl-Gruppen mit 1 bis 2 Kohlenstoff-Atomen, einer Methoxy-Gruppe, einer Methylthio-Gruppe, einer Methylsulfinyl-Gruppe, einer Methylsulfonyl-Gruppe, einer Nitro-Gruppe, einer Cyano-Gruppe, einer Trifluoromethoxy-Gruppe, einer Trifluoromethylthio-Gruppe, einer Allyl-Gruppe, einer Acetamid-Gruppe, einer Methoxycarbonyl-Gruppe, einer Acetyl-Gruppe, einer Formyl-Gruppe und einer Carboxy-Gruppe bestehenden Klasse, mit der Maßgabe, daß, wenn

$R^1$, $R^2$, $R^3$, $R^4$, $R^5$ und $R^6$ gleichzeitig Wasserstoff-Atome darstellen,

X $-\overset{|}{N}H$ bezeichnet und

Y $=\overset{|}{C}H$ bezeichnet, dann

Z nicht für die Pyridyl-Gruppe stehen darf.

Speziell erwähnt seien die folgenden Verbindungen:

3-(2-Chloro-5-pyridylmethyl)-2-(nitromethylen)-tetrahydro-2H-1,3-thiazin,
3-(2-Chloro-5-pyridylmethyl)-2-(nitromethylen)-thiazolidin,
3-(2-Fluoro-5-pyridylmethyl)-2-(nitromethylen)-tetrahydro-2H-1,3-thiazin,
3-(2-Fluoro-5-pyridylmethyl)-2-(nitromethylen)-thiazolidin,
3-(2-Bromo-5-pyridylmethyl)-2-(nitromethylen)-tetrahydro-2H-1,3-thiazin,
3-(2-Bromo-5-pyridylmethyl)-2-(nitromethylen)-thiazolidin,
3-(2-Methyl-5-pyridylmethyl)-2-(nitromethylen)-thiazolidin,
3-(2-Methyl-5-pyridylmethyl)-2-(nitromethylen)-tetrahydro-2H-1,3-thiazin,
3-(2-Ethyl-5-pyridylmethyl)-2-(nitromethylen)-thiazolidin,
3-(2-Trifluoromethyl-5-pyridylmethyl)-2-(nitromethylen)-tetrahydro-2H-1,3-thiazin,
3-(3-Pyridylmethyl)-2-(nitromethylen)-thiazolidin,
3-(3-Pyridylmethyl)-2-(nitromethylen)-tetrahydro-2H-1,3-thiazin,
3-(2-Trifluoromethyl-5-pyridylmethyl)-2-(nitromethylen)-thiazolidin,
1-(5-Pyrazolylmethyl)-2-(nitromethylen)imidazolidin,
1-(5-Chloro-1-methyl-3-pyrazolylmethyl)-2-(nitromethylen)imidazolidin,
1-(1-Methyl-4-pyrazolylmethyl)-2-(nitromethylen)tetrahydropyrimidin,
1-(1-Methyl-4-pyrazolylmethyl)-2-(nitromethylen)imidazolidin,
1-(3-Trifluoromethyl-5-isoxazolylmethyl)-2-(nitromethylen)tetrahydropyrimidin,

1-(3-Methyl-5-isoxazolylmethyl)-2-(nitromethylen)imidazolidin,
1-(3-Methyl-5-isoxazolylmethyl)-2-(nitromethylen)tetrahydropyrimidin,
1-(5-Isoxazolylmethyl)-2-(nitromethylen)imidazolidin,
1-(2-Methyl-5-thiazolylmethyl)-2-(nitromethylen)imidazolidin,
1-(2-Chloro-5-thiazolylmethyl)-2-(nitromethylen)tetrahydropyrimidin,
1-(2-Trifluoromethyl-5-thiazolylmethyl)-2-(nitromethylen)imidazolidin,
1-(1,2,5-Thiadiazol-3-ylmethyl)-2-(nitromethylen)tetrahydropyrimidin,
1-(1,2,3-Thiadiazol-5-ylmethyl)-2-(nitromethylen)tetrahydropyrimidin,
1-(2-Methyl-5-thiazolylmethyl)-2-(nitromethylen)tetrahydropyrimidin,
1-(2-Chloro-5-thiazolylmethyl)-2-(nitromethylen)imidazolidin,
1-(1,2,5-Thiadiazol-3-ylmethyl)-2-(nitromethylen)imidazolidin,
1-(5-Thiazolylmethyl)-2-(nitromethylen)tetrahydropyrimidin,
1-(5-Pyrimidinylmethyl)-2-(nitromethylen)imidazolidin,
1-(2-Methyl-5-pyrimidinylmethyl)-2-(nitromethylen)imidazolidin,
1-(2-Pyrazinylmethyl)-2-(nitromethylen)imidazolidin,
1-(2-Methyl-5-pyrazinylmethyl)-2-(nitromethylen)imidazolidin,
1-(2-Chloro-5-pyrimidinylmethyl)-2-(nitromethylen)-tetrahydropyrimidin,
1-(2-Chloro-5-pyrimidinylmethyl)-2-(nitromethylen)imidazolidin,

1-(2-Fluoro-5-pyrimidinylmethyl)-2-(nitromethylen)imidazolidin,
1-(2-Trifluoromethyl-5-pyrimidinylmethyl)-2-(nitromethylen)imidazolidin,
1-(2-Chloro-5-pyrazinylmethyl)-2-(nitromethylen)imidazolidin,
1-/¯1-(2-Fluoro-5-pyrimidinyl)ethyl_7-2-(nitromethylen)imidazolidin,
1-(2-Chloro-5-pyridylmethyl)-2-(nitroimino)tetrahydropyrimidin,
1-(2-Chloro-5-pyridylmethyl)-2-(nitroimino)imidazolidin,
1-(2-Trifluoromethyl-5-pyridylmethyl)-2-(nitroimino)-tetrahydropyrimidin,
1-(2-Trifluoromethyl-5-pyridylmethyl)-2-(nitroimino)-imidazolidin,
1-(2-Fluoro-5-pyridylmethyl)-2-(nitroimino)imidazolidin,
1-(2-Bromo-5-pyridylmethyl)-2-(nitroimino)imidazolidin,
1-(2-Methyl-5-pyridylmethyl)-2-(nitroimino)imidazolidin,
1-(2-Methoxy-5-pyridylmethyl)-2-(nitroimino)imidazolidin,
1-(2-Chloro-5-pyridylmethyl)-2-(nitromethylen)pyrrolidin,
1-(2-Chloro-5-thiazolylmethyl)-2-(nitroimino)tetrahydropyrimidin,
1-(2-Chloro-5-pyrimidinylmethyl)-2-(nitroimino)imidazolidin,
1-(2-Chloro-5-pyridylmethyl)-4-methyl-2-(nitromethylen)imidazolidin,
1-(2-Chloro-5-pyridylmethyl)-3-(3-pyridylmethyl)-2-(nitromethylen)imidazolidin,

1-(2-Chloro-5-pyridylmethyl)-2-(bromonitromethylen)-imidazolidin,
1-(2-Chloro-5-pyridylmethyl)-2-(1-nitro-2-oxopentyl-iden)imidazolidin,
Ethyl-nitro/¯3-(2-chloro-5-pyridylmethyl)thiazolidin-2-yliden_7acetat,
1-Acetyl-3-(2-chloro-5-pyridylmethyl)-2-(nitroimino)-imidazolidin und
N-Phenylsulfonyl-nitro-/¯1-(2-chloro-5-pyridylmethyl)-imidazolidin-2-yliden_7acetamid.

Wenn in dem Verfahren (a) beispielsweise N-(1-Methyl-4-pyrazolylmethyl)trimethylendiamin und 1-Nitro-2,2-bis-(methylthio)ethylen als Ausgangsstoffe eingesetzt werden, läßt sich der Reaktionsverlauf durch die folgende Gleichung darstellen:

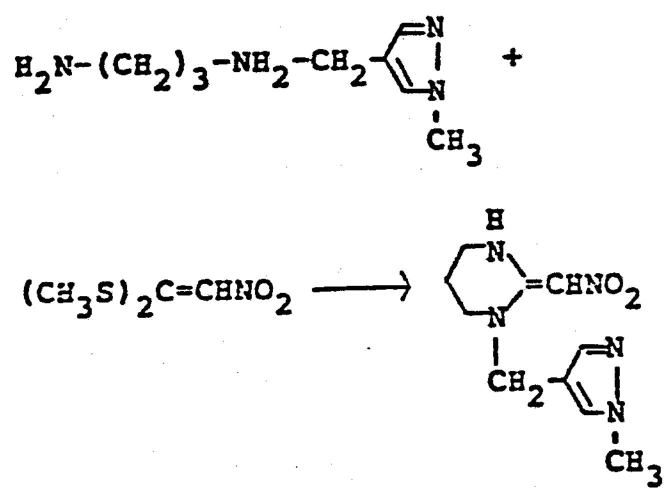

Wenn in dem Verfahren (b) beispielsweise 2-(2-Methyl-5-pyrazinylmethylamino)ethanthiol und 2,2-Dichloro-nitroethan als Ausgangsstoffe eingesetzt werden, läßt sich der Reaktionsverlauf durch die folgende Gleichung darstellen:

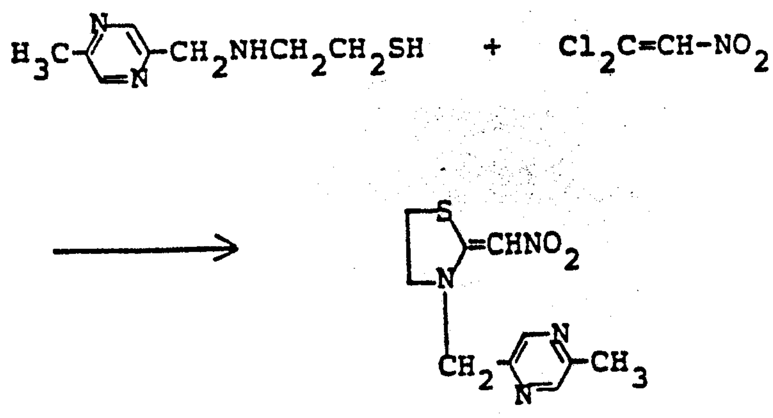

Wenn in dem Verfahren (c) beispielsweise 2-(2-Chloro-5-thiazolylmethylamino)ethanthiol und 1,2,2,2-Tetrachloro-1-nitroethan als Ausgangsstoffe eingesetzt werden, läßt sich der Reaktionsverlauf durch die folgende Gleichung darstellen:

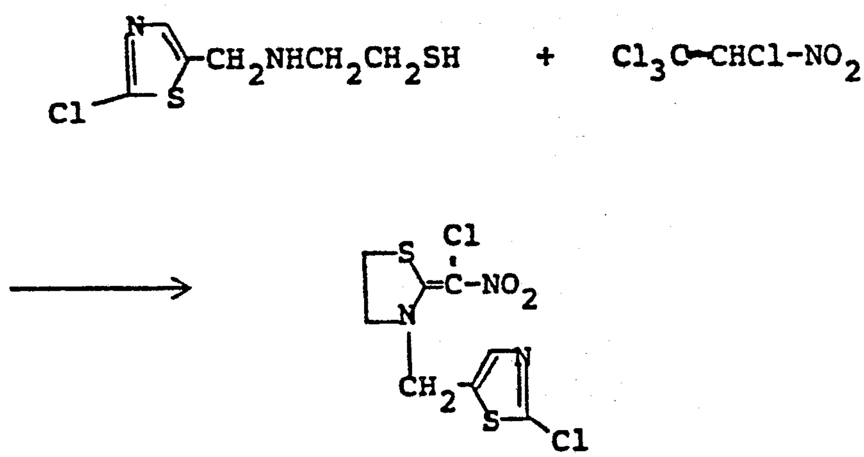

Wenn in dem Verfahren (d) beispielsweise N-(2-Chloro-5-pyridylmethyl)trimethylendiamin und Nitroguanidin als Ausgangsstoffe eingesetzt werden, läßt sich der Reaktionsverlauf durch die folgende Gleichung darstellen:

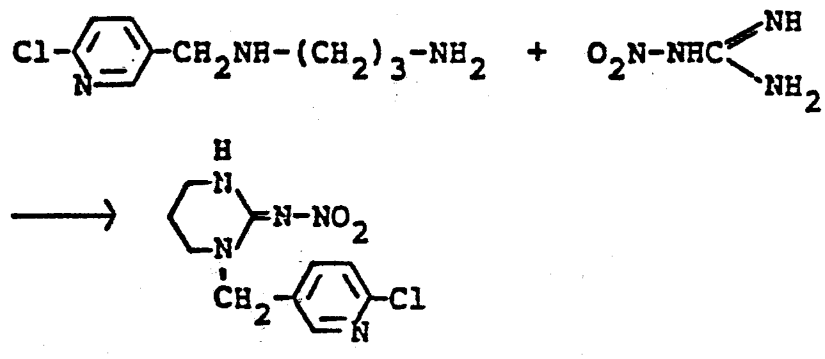

Wenn in dem Verfahren (e) beispielsweise 1-(2-Chloro-5-pyridylmethyl)-2-iminoimidazolidin und rauchende Salpetersäure als Ausgangsstoffe eingesetzt werden, läßt sich der Reaktionsverlauf durch die folgende Gleichung darstellen:

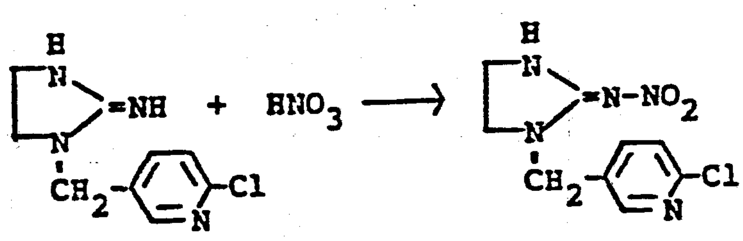

Wenn in dem Verfahren (f) beispielsweise 2-Nitromethylenthiazolidin und 2-Chloro-5-pyridylmethylchlorid als Ausgangsstoffe eingesetzt werden, läßt sich der Reaktionsverlauf durch die folgende Gleichung darstellen:

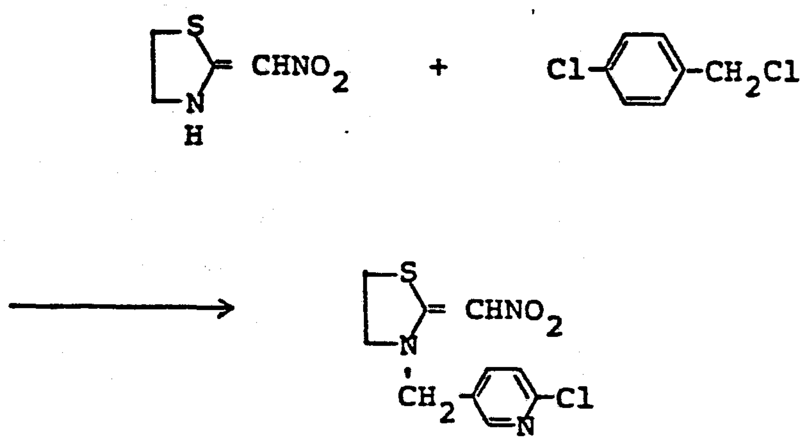

Die Formel (II) gibt eine allgemeine Definition der als Ausgangsstoffe in dem Verfahren (a) benötigten Verbindungen, wobei auf die im Vorstehenden angegebenen jeweiligen Bedeutungen von n, $R^1$, $R^2$, $R^3$, $R^4$, $R^5$, $R^6$, R, Z und $X^1$ Bezug genommen wird.

In der Formel (II) haben n, $R^1$, $R^2$, $R^3$, $R^4$, $R^5$, $R^6$, R, Z und $X^1$ vorzugsweise die bereits oben bezeichneten Bedeutungen.

Zu den erfindungsgemäß einsetzbaren Verbindungen der Formel (II) zählen sowohl bekannte als auch neue Verbindungen.

Die bekannten Beispiele sind bereits beschrieben in, beispielsweise, den JP-Patentanmeldungen 26 020/1984, 72 966/1984 und 132 943/1984, Z. Anorg. Allg. Chem. 312, S. 282-286, Khim. Geterotsikl. Soedin. 1974, No. 1, S. 122-123, Metody Poluch. Khim. Reactivon Prep. No. 17, S. 172-173, Issled. Obl. Geterotsikl. Soedin. 1971, S. 39-44, der US-PS 4 018 931, Arch. Pharm. 1982, Vol. 315, S. 212-221, Metody Poluch. Khim. Reactivon Prep. 1967, S. 133-134, und Zh. Obshch. Khim. 33, S. 1130-1135.

Als Beispiele seien die folgenden Verbindungen erwähnt:

N-(2-Chloro-5-pyridylmethyl)-2-aminoethanthiol,
N-(2-Chloro-5-pyridylmethyl-3-aminopropanthiol,
N-(2-Bromo-5-pyridylmethyl)-2-aminoethanthiol,
N-(2-Bromo-5-pyridylmethyl)-3-aminopropanthiol,
N-(2-Fluoro-5-pyridylmethyl)-2-aminoethanthiol,
N-(2-Fluoro-5-pyridylmethyl)-3-aminopropanthiol,
N-[1-(2-Chloro-5-pyridyl)ethyl]-2-aminoethanthiol,
N-(2,3-Dichloro-5-pyridylmethyl)-2-aminoethanthiol,

N-(3-Chloro-2-fluoro-5-pyridylmethyl)-2-aminoethanthiol,
N-(2-Chloro-4-pyridylmethyl)-2-aminoethanthiol,
N-(3-Chloro-2-pyridylmethyl)-2-aminoethanthiol,
N-(5-Chloro-2-pyridylmethyl)-3-aminopropanthiol,
N-(3,5-Dichloro-2-pyridylmethyl)-2-aminoethanthiol,
N-(5-Fluoro-2-pyridylmethyl)-3-aminopropanthiol,
N-(6-Bromo-2-pyridylmethyl)-2-aminoethanthiol,
N-(2-Chloro-3-pyridylmethyl)-3-aminopropanthiol,
N-(5-Chloro-3-pyridylmethyl)-3-aminopropanthiol,
N-(5-Bromo-3-pyridylmethyl)-2-aminoethanthiol,
N-(5-Fluoro-3-pyridylmethyl)-2-aminoethanthiol,
N-[1-(2-Fluoro-5-pyridyl)ethyl]-2-aminoethanthiol,
N-(2,4-Dichloro-5-pyridylmethyl)-3-aminopropanthiol,
N-(2,4-Dibromo-5-pyridylmethyl)-2-aminoethanthiol,
N-(2,6-Difluoro-4-pyridylmethyl)-2-aminoethanthiol,
N-(2-Fluoro-4-pyridylmethyl)-3-aminopropanthiol,
N-(2,6-Dibromo-4-pyridylmethyl)-2-aminoethanthiol,
N-(3-Bromo-2-fluoro-5-pyridylmethyl)-2-aminoethanthiol,
N-(2-Chloro-3-fluoro-5-pyridylmethyl)-2-aminoethanthiol,
N-[1-(2-Chloro-5-pyridyl)propyl]-2-aminoethanthiol,
N-(3-Pyridylmethyl)-2-aminoethanthiol,
N-(3-Pyridylmethyl)-2-aminopropanthiol,
N-(4-Pyridylmethyl)-2-aminoethanthiol,
N-(4-Pyridylmethyl)-2-aminopropanthiol,
N-(2-Methyl-5-pyridylmethyl)-2-aminoethanthiol,
N-(2-Methyl-5-pyridylmethyl)-3-aminopropanthiol,
N-(2-Ethyl-5-pyridylmethyl)-2-aminoethanthiol,
N-(2-Allyl-5-pyridylmethyl)-2-aminoethanthiol,
N-(2-Propargyl-5-pyridylmethyl)-2-aminoethanthiol,
N-(2-Methoxy-5-pyridylmethyl)-3-aminopropanthiol,
N-(2-Methylthio-5-pyridylmethyl)-2-aminopropanthiol,
N-(2-Methylsulfonyl-5-pyridylmethyl)-2-aminoethanthiol,

N-(2-Chloro-3-methyl-5-pyridylmethyl)-2-aminoethanthiol,
N-/¯1-(3-Pyridyl)ethyl_7-3-aminopropanthiol,
N-(2-Trifluoromethyl-5-pyridylmethyl)-2-aminoethanthiol,
N-(2-Trifluoromethyl-5-pyridylmethyl)-3-aminopropanthiol,
N-(2-Nitro-5-pyridylmethyl)-2-aminoethanthiol,
N-(2-Nitro-5-pyridylmethyl)-3-aminopropanthiol,
N-(2-Cyano-5-pyridylmethyl)-2-aminoethanthiol,
N-(2-Cyano-5-pyridylmethyl)-3-aminopropanthiol,
N-(2-Methylsulfinyl-5-pyridylmethyl)-2-aminoethanthiol,
N-(2-Phenyl-5-pyridylmethyl)-2-aminoethanthiol,
N-(2-Benzyl-5-pyridylmethyl)-2-aminoethanthiol,
N-(2-Phenoxy-5-pyridylmethyl)-3-aminopropanthiol,
N-(2-Trichloromethyl-5-pyridylmethyl)-2-aminoethanthiol,
N-/¯2-(2-Ethoxyethyl)-5-(pyridylmethyl_7-3-aminopropanthiol,
N-(2-Methoxymethyl-5-pyridylmethyl)-2-aminoethanthiol,
N-(2-Difluoromethoxy-5-pyridylmethyl)-2-aminoethanthiol,
N-(2-Trifluoromethoxy-5-pyridylmethyl)-2-aminoethanthiol,
N-/¯-2-(2,2,2-Trifluoroethoxy)-5-(pyridylmethyl_7-2-aminoethanthiol,
N-(2-Chlorodifluoromethylthio-5-pyridylmethyl)-3-aminopropanthiol,
N-(2-Trifluoromethylthio-5-pyridylmethyl)-2-aminoethanthiol,
N-(2-Difluoromethyl-5-pyridylmethyl)-2-aminoethanthiol,
N-(2-Trifluoromethylsulfonyl-5-pyridylmethyl)-2-aminoethanthiol,
N-(2-Trifluoromethylsulfinyl-5-pyridylmethyl)-2-aminoethanthiol,

N-/‾2-(2,2-Dichlorovinyl)-5-(pyridylmethyl_7-2-amino-ethanthiol,
N-(4-Pyrimidinylmethyl)ethylendiamin,
N-(2-Methyl-4-pyrimidinylmethyl)ethylendiamin,
N-(2-Methyl-6-oxo-1H,6H-dihydropyrimidin-4-ylmethyl)-trimethylendiamin,
N-(5-Pyrimidinylmethyl)ethylendiamin,
N-(2-Methyl-5-pyrimidinylmethyl)ethylendiamin,
N-(2-Dimethylamino-5-pyrimidinylmethyl)ethylendiamin,
N-(2-Dimethylamino-5-pyrimidinylmethyl)trimethylendiamin,
N-(2,4,6-Trichloro-5-pyrimidinylmethyl)ethylendiamin,
N-(4-Pyrazinylmethyl)ethylendiamin,
N-/‾1-(Pyrazinyl)ethyl_7ethylendiamin,
N-(2-Methyl-5-pyrazinylmethyl)ethylendiamin,
N-(3-Pyridazinylmethyl)ethylendiamin,
N-(2-Chloro-4-pyrimidinylmethyl)ethylendiamin,
N-(4-Chloro-6-pyrimidinylmethyl)ethylendiamin,
N-(4-Methyl-6-pyrimidinylmethyl)trimethylendiamin,
N-(2-Fluoro-5-pyrimidinylmethyl)ethylendiamin,
N-/‾1-(2-Fluoro-5-pyrimidinyl)ethyl_7ethylendiamin,
N-(2-Chloro-5-pyrimidinylmethyl)ethylendiamin,
N-(2-Chloro-5-pyrimidinylmethyl)trimethylendiamin,
N-(2-Isopropyl-5-pyrimidinylmethyl)ethylendiamin,
N-(2-Chlorodifluoromethyl-5-pyrimidinylmethyl)ethylendiamin,
N-(2-Trifluoromethyl-5-pyrimidinylmethyl)ethylendiamin,
N-(2-Bromodifluoromethyl-5-pyrimidinylmethyl)ethylendiamin,
N-(2-Methoxy-5-pyrimidinylmethyl)ethylendiamin,
N-(2-Difluoromethoxy-5-pyrimidinylmethyl)ethylendiamin,
N-(2-Trifluoromethoxy-5-pyrimidinylmethyl)ethylendiamin,
N-/‾2-(2,2,2-Trifluoroethoxy)-5-pyrimidinylmethyl_7-trimethylendiamin,

N-(2-Methylthio-5-pyrimidinylmethyl)ethylendiamin,
N-(2-Ethylthio-5-pyrimidinylmethyl)ethylendiamin,
N-(2-Difluoroethylthio-5-pyrimidinylmethyl)ethylendiamin,
N-(2-Trifluoromethylthio-5-pyrimidinylmethyl)ethylendiamin,
N-/¯2-(2,2,2-Trifluoroethylthio)-5-pyrimidinylmethyl_7-ethylendiamin,
N-(2-Nitro-5-pyrazinylmethyl)ethylendiamin,
N-(2-Cyano-5-pyrazinylmethyl)trimethylendiamin,
N-(2-Chloro-5-pyrazinylmethyl)ethylendiamin,
N-(2-Trifluoromethyl-5-pyrazinylmethyl)ethylendiamin,
N-(3-Fluoro-6-pyridazinylmethyl)ethylendiamin,
N-(3-Methyl-6-pyridazinylmethyl)trimethylendiamin,
N-(4-Pyridazinylmethyl)ethylendiamin,
N-(3-Chloro-6-pyridazinylmethyl)ethylendiamin,
N-(4-Pyridazinylmethyl)trimethylendiamin,
N-(3-Trifluoromethyl-6-pyridazinylmethyl)ethylendiamin,
N-(1,3,5-Triazin-2-ylmethyl)ethylendiamin,
N-(3-Chloro-1,2,4-Triazin-6-ylmethyl)ethylendiamin,
N-(3,5-Dichloro-1,2,4-Triazin-6-ylmethyl)ethylendiamin,
N-(3-Chloro-1,2,4,5-Tetrazin-6-ylmethyl)ethylendiamin,
N-(3-Furylmethyl)ethylendiamin oder -trimethylendiamin,
N-(Furfuryl)ethylendiamin oder -trimethylendiamin,
N-(5-Methylfurfuryl)ethylendiamin oder -trimethylendiamin,
N-(2-Thienylmethyl)ethylendiamin oder -trimethylendiamin,
N-(4-Imidazolylmethyl)ethylendiamin oder -trimethylendiamin,
N-(4-Methyl-5-imidazolylmethyl)ethylendiamin oder -trimethylendiamin,

N-(Tetrahydrofurfuryl)ethylendiamin oder -trimethylendiamin,

N-(5-Methyltetrahydrofurfuryl)ethylendiamin oder -trimethylendiamin,

N-(3-Thienylmethyl)ethylendiamin oder -trimethylendiamin,

N-(2-Pyrrolylmethyl)ethylendiamin oder -trimethylendiamin,

N-(1-Methyl-2-pyrrolylmethyl)ethylendiamin oder -trimethylendiamin,

N-(5-Methyl-2-thienylmethyl)ethylendiamin oder -trimethylendiamin,

N-(5-Bromo-2-thienylmethyl)ethylendiamin oder -trimethylendiamin,

N-(5-Cyanofurfuryl)ethylendiamin oder -trimethylendiamin,

N-(5-Trifluoromethylthio-2-thienylmethyl)ethylendiamin oder -trimethylendiamin,

N-[1-(2-Thienyl)ethyl]ethylendiamin oder -trimethylendiamin,

N-(5-Methyl-3-isoxazolylmethyl)ethylendiamin oder -trimethylendiamin,

N-(5-Isoxazolylmethyl)ethylendiamin oder -trimethylendiamin,

N-(4-Isoxazolylmethyl)ethylendiamin oder -trimethylendiamin,

N-(3-Methyl-5-isoxazolylmethyl)ethylendiamin oder -trimethylendiamin,

N-(3-Trifluoromethyl-5-isoxazolylmethyl)ethylendiamin oder -trimethylendiamin,

N-(3-Chloro-5-isoxazolylmethyl)ethylendiamin oder -trimethylendiamin,

N-(5-Isothiazolylmethyl)ethylendiamin oder -trimethylendiamin,

N-(5-Pyrazolylmethyl)ethylendiamin oder -trimethylendiamin,
N-(4-Pyrazolylmethyl)ethylendiamin oder -trimethylendiamin,
N-(1-Methyl-4-pyrazolylmethyl)ethylendiamin oder -trimethylendiamin,
N-[1-(1-Methyl-4-pyrazolyl)ethyl]ethylendiamin oder -trimethylendiamin,
N-(1-Ethyl-4-pyrazolylmethyl)ethylendiamin oder -trimethylendiamin,
N-(1-Isopropyl-4-pyrazolylmethyl)ethylendiamin oder -trimethylendiamin,
N-(1-Allyl-4-pyrazolylmethyl)ethylendiamin oder -trimethylendiamin,
N-(1-tert-Butyl-4-pyrazolylmethyl)ethylendiamin oder -trimethylendiamin,
N-[1-(2,2,2-Trifluoroethyl)-5-pyrazolylmethyl]-ethylendiamin oder -trimethylendiamin,
N-(3-Methyl-5-pyrazolylmethyl)ethylendiamin oder -trimethylendiamin,
N-(3-Chloro-2-methyl-5-pyrazolylmethyl)ethylendiamin oder -trimethylendiamin,
N-(2,3,5-Trimethyl-4-pyrazolylmethyl)ethylendiamin oder -trimethylendiamin,
N-(5-Oxazolylmethyl)ethylendiamin oder -trimethylendiamin,
N-(4-Methyl-5-oxazolylmethyl)ethylendiamin oder -trimethylendiamin,
N-(4-Thiazolylmethyl)ethylendiamin oder -trimethylendiamin,
N-(5-Thiazolylmethyl)ethylendiamin oder -trimethylendiamin,
N-(2-Methyl-5-thiazolylmethyl)ethylendiamin oder -trimethylendiamin,

N-(2-Chloro-4-thiazolylmethyl)ethylendiamin oder -trimethylendiamin,
N-(2-Chloro-5-thiazolylmethyl)ethylendiamin oder -trimethylendiamin,
N-(2-Trifluoromethyl-5-thiazolylmethyl)ethylendiamin oder -trimethylendiamin,
N-(2-Bromo-5-thiazolylmethyl)ethylendiamin oder -trimethylendiamin,
N-(2,4-Dichloro-5-thiazolylmethyl)ethylendiamin oder -trimethylendiamin,
N-(4-Imidazolylmethyl)ethylendiamin oder -trimethylendiamin,
N-(1-Methyl-2-imidazolylmethyl)ethylendiamin oder -trimethylendiamin,
N-(1,2,4-Triazol-5-ylmethyl)ethylendiamin oder -trimethylendiamin,
N-(1-Methyl-1,2,4-triazol-5-ylmethyl)ethylendiamin oder -trimethylendiamin,
N-(1,2,5-Thiadiazol-4-ylmethyl)ethylendiamin oder -trimethylendiamin,
N-(1,2,3-Thiadiazol-5-ylmethyl)ethylendiamin oder -trimethylendiamin,
N-(3-Methyl-1,2,4-oxadiazol-5-ylmethyl)ethylendiamin oder -trimethylendiamin,
N-(1,3-Dioxolan-2-ylmethyl)ethylendiamin oder -trimethylendiamin,
N-(2,2-Dimethyl-1,3-dioxolan-4-ylmethyl)ethylendiamin oder -trimethylendiamin,
N-(2-Methyl-2-oxazolin-5-ylmethyl)ethylendiamin oder -trimethylendiamin,
N-(2-Trifluoromethyl-2-oxazolin-5-ylmethyl)ethylendiamin oder -trimethylendiamin,
N-(3-Pyrrolylmethyl)ethylendiamin oder -trimethylendiamin,

N-(1-Ethyl-2-pyrrolylmethyl)ethylendiamin oder -trimethylendiamin,
N-(1-Methyl-3-pyrrolylmethyl)ethylendiamin oder -trimethylendiamin,
N-(5-Methyl-3-thienyl)ethylendiamin oder -trimethylendiamin,
N-(5-Methyl-3-pyrrolylmethyl)ethylendiamin oder -trimethylendiamin,
N-(1,5-Dimethyl-3-pyrrolylmethyl)ethylendiamin oder -trimethylendiamin,
N-(2,5-Dimethyl-3-furylmethyl)ethylendiamin oder -trimethylendiamin,
N-(2,5-Dimethyl-3-thienylmethyl)ethylendiamin oder -trimethylendiamin,
N-(5-Fluoro-3-furylmethyl)ethylendiamin oder -trimethylendiamin,
N-(4-Chlorofurfuryl)ethylendiamin oder -trimethylendiamin,
N-(5-Chlorofurfuryl)ethylendiamin oder -trimethylendiamin,
N-(5-Chloro-3-furylmethyl)ethylendiamin oder -trimethylendiamin,
N-(5-Chloro-3-thienylmethyl)ethylendiamin oder -trimethylendiamin,
N-(5-Chloro-1-methyl-3-pyrrolylmethyl)ethylendiamin oder -trimethylendiamin,
N-(5-Bromo-3-furylmethyl)ethylendiamin oder -trimethylendiamin,
N-(5-Nitrofurfuryl)ethylendiamin oder -trimethylendiamin,
N-(4-Nitro-2-thienylmethyl)ethylendiamin oder -trimethylendiamin,
N-(5-Nitro-2-thienylmethyl)ethylendiamin oder -trimethylendiamin,

N-(1-Methyl-5-nitro-3-pyrrolylmethyl)ethylendiamin oder -trimethylendiamin,

N-(5-Cyano-3-furylmethyl)ethylendiamin oder -trimethylendiamin,

N-(5-Cyano-3-thienylmethyl)ethylendiamin oder -trimethylendiamin,

N-(5-Cyano-1-methyl-3-pyrrolylmethyl)ethylendiamin oder -trimethylendiamin,

N-(5-Trifluoromethylfurfuryl)ethylendiamin oder -trimethylendiamin,

N-(5-Difluoromethylfurfuryl)ethylendiamin oder -trimethylendiamin,

N-(5-Trifluoromethyl-3-thienylmethyl)ethylendiamin oder -trimethylendiamin,

N-(1-Methyl-5-trifluoromethyl-3-pyrrolylmethyl)ethylendiamin oder -trimethylendiamin,

N-(5-Methoxy-2-thienylmethyl)ethylendiamin oder -trimethylendiamin,

N-(5-Methylthiofurfuryl)ethylendiamin oder -trimethylendiamin,

N-(2,5-Dimethylthio-3-thienylmethyl)ethylendiamin oder -trimethylendiamin,

N-(5-Trifluoromethylthiofurfuryl)ethylendiamin oder -trimethylendiamin,

N-/¯5-(2,2-Dichlorovinyl)-2-thienylmethyl_7ethylendiamin oder -trimethylendiamin,

N-(5-Ethoxycarbonylfurfuryl)ethylendiamin oder -trimethylendiamin,

N-(5-Formyl-2-thienylmethyl)ethylendiamin oder -trimethylendiamin,

N-(3-Isoxazolylmethyl)ethylendiamin oder -trimethylendiamin,

N-(4-Isoxazolylmethyl)ethylendiamin oder -trimethylendiamin,

N-(3-Ethyl-5-isoxazolylmethyl)ethylendiamin oder -trimethylendiamin,

N-(3-Isopropyl-5-isoxazolylmethyl)ethylendiamin oder -trimethylendiamin,

N-(3-Fluoro-5-isoxazolylmethyl)ethylendiamin oder -trimethylendiamin,

N-(3-Bromo-5-isoxazolylmethyl)ethylendiamin oder -trimethylendiamin,

N-(3-Hydroxy-5-isoxazolylmethyl)ethylendiamin oder -trimethylendiamin,

N-(3-Nitro-5-isoxazolylmethyl)ethylendiamin oder -trimethylendiamin,

N-(3-Cyano-5-isoxazolylmethyl)ethylendiamin oder -trimethylendiamin,

N-(3-Difluoromethyl-5-isoxazolylmethyl)ethylendiamin oder -trimethylendiamin,

N-(3-Chloromethyl-5-isoxazolylmethyl)ethylendiamin oder -trimethylendiamin,

N-(3-Methoxymethyl-5-isoxazolylmethyl)ethylendiamin oder -trimethylendiamin,

N-(3-Isopropoxymethyl-5-isoxazolylmethyl)ethylendiamin oder -trimethylendiamin,

N-(3-Trichloromethyl-5-isoxazolylmethyl)ethylendiamin oder -trimethylendiamin,

N-(3-Methoxy-5-isoxazolylmethyl)ethylendiamin oder -trimethylendiamin,

N-(3-Trifluoromethoxy-5-isoxazolylmethyl)ethylendiamin oder -trimethylendiamin,

N-(2,5-Dimethyl-4-isoxazolylmethyl)ethylendiamin oder -trimethylendiamin,

N-(3-Isothiazolylmethyl)ethylendiamin oder -trimethylendiamin,

N-(4-Isothiazolylmethyl)ethylendiamin oder -trimethylendiamin,

N-(3-Pyrazolylmethyl)ethylendiamin oder -trimethylendiamin,
N-/‾1-(4-Pyrazolyl)ethyl_7ethylendiamin oder -trimethylendiamin,
N-(1-Methyl-3-pyrazolylmethyl)ethylendiamin oder -trimethylendiamin,
N-(1-Methyl-5-pyrazolylmethyl)ethylendiamin oder -trimethylendiamin,
N-(1-Propyl-4-pyrazolylmethyl)ethylendiamin oder -trimethylendiamin,
N-/‾1-(2,2,2-Trifluoroethyl)-3-pyrazolylmethyl_7ethylendiamin oder -trimethylendiamin,
N-(5-Chloro-1-ethyl-3-pyrazolylmethyl)ethylendiamin oder -trimethylendiamin,
N-(5-Chloro-1-isopropyl-3-pyrazolylmethyl)ethylendiamin oder -trimethylendiamin,
N-(3-Chloro-1-methyl-3-pyrazolylmethyl)ethylendiamin oder -trimethylendiamin,
N-(5-Trifluoromethyl-3-pyrazolylmethyl)ethylendiamin oder -trimethylendiamin,
N-(1-Methyl-5-trifluoromethyl-3-pyrazolylmethyl)ethylendiamin oder -trimethylendiamin,
N-(1-Methyl-3-trifluoromethyl-5-pyrazolylmethyl)ethylendiamin oder -trimethylendiamin,
N-(4-Oxazolylmethyl)ethylendiamin oder -trimethylendiamin,
N-(2-Methyl-4-oxazolylmethyl)ethylendiamin oder -trimethylendiamin,
N-(2-Methyl-5-oxazolylmethyl)ethylendiamin oder -trimethylendiamin,
N-(2-Fluoro-5-oxazolylmethyl)ethylendiamin oder -trimethylendiamin,
N-(2-Chloro-5-oxazolylmethyl)ethylendiamin oder -trimethylendiamin,

N-(2-Trifluoromethyl-5-oxazolylmethyl)ethylendiamin oder -trimethylendiamin,
N-(2-Methylthio-5-oxazolylmethyl)ethylendiamin oder -trimethylendiamin,
N-(2-Trifluoromethoxy-5-oxazolylmethyl)ethylendiamin oder -trimethylendiamin,
N-(2,4-Dimethyl-5-oxazolylmethyl)ethylendiamin oder -trimethylendiamin,
N-(5-Ethoxycarbonyl-2-oxazolylmethyl)ethylendiamin oder -trimethylendiamin,
N-/¯1-(5-Thiazolyl)ethyl_7ethylendiamin oder -trimethylendiamin,
N-(2-Methyl-4-thiazolylmethyl)ethylendiamin oder -trimethylendiamin,
N-/¯1-(2-Methyl-5-thiazolyl)ethyl_7ethylendiamin oder -trimethylendiamin,
N-(2-Ethyl-5-thiazolylmethyl)ethylendiamin oder -trimethylendiamin,
N-(2-Isopropyl-5-thiazolylmethyl)ethylendiamin oder -trimethylendiamin,
N-(4-Methyl-5-thiazolylmethyl)ethylendiamin oder -trimethylendiamin,
N-(2-Fluoro-4-thiazolylmethyl)ethylendiamin oder -trimethylendiamin,
N-(2-Fluoro-5-thiazolylmethyl)ethylendiamin oder -trimethylendiamin,
N-/¯1-(2-Chloro-5-thiazolylmethyl)ethyl_7ethylendiamin oder -trimethylendiamin,
N-(2-Nitro-4-thiazolylmethyl)ethylendiamin oder -trimethylendiamin,
N-(2-Nitro-5-thiazolylmethyl)ethylendiamin oder -trimethylendiamin,
N-(2-Cyano-4-thiazolylmethyl)ethylendiamin oder -trimethylendiamin,

N-(2-Cyano-5-thiazolylmethyl)ethylendiamin oder -trimethylendiamin,
N-(2-Methylthio-4-thiazolylmethyl)ethylendiamin oder -trimethylendiamin,
N-(2-Mercapto-5-thiazolylmethyl)ethylendiamin oder -trimethylendiamin,
N-(2-Methylthio-5-thiazolylmethyl)ethylendiamin oder -trimethylendiamin,
N-(2-Difluoromethylthio-5-thiazolylmethyl)ethylendiamin oder -trimethylendiamin,
N-(2-Trifluoromethylthio-5-thiazolylmethyl)ethylendiamin oder -trimethylendiamin,
N-(2-Chlorodifluoromethylthio-5-thiazolylmethyl)ethylendiamin oder -trimethylendiamin,
N-/¯2-(2,2,2-Trifluoroethylthio)-5-thiazolylmethyl_7-ethylendiamin oder -trimethylendiamin,
N-/̿2-/¯2-(2,3,3-Trichloro)propenylthio_7-5-thiazolylmethyl_̳7ethylendiamin oder -trimethylendiamin,
N-(2-Thiocyanato-5-thiazolylmethyl)ethylendiamin oder -trimethylendiamin,
N-(2-Amino-4-thiazolylmethyl)ethylendiamin oder -trimethylendiamin,
N-(2-Acetamino-4-thiazolylmethyl)ethylendiamin oder -trimethylendiamin,
N-(2-Methoxy-4-thiazolylmethyl)ethylendiamin oder -trimethylendiamin,
N-(2-Methoxy-5-thiazolylmethyl)ethylendiamin oder -trimethylendiamin,
N-(2-Trifluoromethoxy-5-thiazolylmethyl)ethylendiamin oder -trimethylendiamin,
N-(2-Difluoromethoxy-5-thiazolylmethyl)ethylendiamin oder -trimethylendiamin,
N-(2-Chloromethyl-5-thiazolylmethyl)ethylendiamin oder -trimethylendiamin,
N-(2-Difluoromethyl-5-thiazolylmethyl)ethylendiamin

oder -trimethylendiamin,
N-(2-Trifluoromethyl-5-thiazolylmethyl)ethylendiamin oder -trimethylendiamin,
N-[2-(1,1,2,2-Tetrafluoroethylthio)-5-thiazolylmethyl]ethylendiamin oder -trimethylendiamin,
N-(2-Cyclopropyl-5-thiazolylmethyl)ethylendiamin oder -trimethylendiamin,
N-(2-Imidazolylmethyl)ethylendiamin oder -trimethylendiamin,
N-(1-Methyl-5-imidazolylmethyl)ethylendiamin oder -trimethylendiamin,
N-(2-Fluoro-4-imidazolylmethyl)ethylendiamin oder -trimethylendiamin,
N-(2-Chloro-4-imidazolylmethyl)ethylendiamin oder -trimethylendiamin,
N-(4-Nitro-2-imidazolylmethyl)ethylendiamin oder -trimethylendiamin,
N-(4-Trifluoromethylthio-2-imidazolylmethyl)ethylendiamin oder -trimethylendiamin,
N-(1,2-Dimethyl-4-imidazolylmethyl)ethylendiamin oder -trimethylendiamin,
N-(1-Methyl-2-trifluoromethylthio-4-imidazolylmethyl)-ethylendiamin oder -trimethylendiamin,
N-(1-Methyl-1,2,3-triazol-4-ylmethyl)ethylendiamin oder -trimethylendiamin,
N-[(1-Methyl-1,2,3-triazol-4-yl)ethyl]ethylendiamin oder -trimethylendiamin,
N-(3-Methyl-1,2,4-triazol-5-ylmethyl)ethylendiamin oder -trimethylendiamin,
N-(3-Trifluoromethyl-1,2,4-triazol-5-ylmethyl)ethylendiamin oder -trimethylendiamin,
N-(1,2,4-Oxadiazol-5-ylmethyl)ethylendiamin oder -trimethylendiamin,
N-(1,3,4-Oxadiazol-2-ylmethyl)ethylendiamin oder -trimethylendiamin,

N-(1,2,3-Oxadiazol-5-ylmethyl)ethylendiamin oder -trimethylendiamin,
N-(3-Trifluoromethyl-1,2,4-oxadiazol-5-ylmethyl)ethylendiamin oder -trimethylendiamin,
N-(2-Methyl-1,3,4-oxadiazol-5-ylmethyl)ethylendiamin oder -trimethylendiamin,
N-(2-Trifluoromethyl-1,3,4-oxadiazol-5-ylmethyl)ethylendiamin oder -trimethylendiamin,
N-[2-(2,2,2-Trifluoroethyl)-1,3,4-oxadiazol-5-ylmethyl]ethylendiamin oder -trimethylendiamin,
N-(1,2,4-Thiadiazol-5-ylmethyl)ethylendiamin oder -trimethylendiamin,
N-(1,2,3-Thiadiazol-4-ylmethyl)ethylendiamin oder -trimethylendiamin,
N-[1-(1,2,3-Thiadiazol-5-yl)ethyl]ethylendiamin oder -trimethylendiamin,
N-(1,3,4-Thiadiazol-2-yl)ethylendiamin oder -trimethylendiamin,
N-[1-(1,3,4-Thiadiazol-2-yl)ethyl]ethylendiamin oder -trimethylendiamin,
N-(1,2,5-Thiadiazol-3-ylmethyl)ethylendiamin oder -trimethylendiamin,
N-(3-Methyl-1,2,4-thiadiazol-5-ylmethyl)ethylendiamin oder -trimethylendiamin,
N-(4-Methyl-1,2,3-thiadiazol-5-ylmethyl)ethylendiamin oder -trimethylendiamin,
N-(2-Methyl-1,3,4-thiadiazol-5-ylmethyl)ethylendiamin oder -trimethylendiamin,
N-(2-Trifluoromethyl-1,3,4-thiadiazol-5-ylmethyl)ethylendiamin oder -trimethylendiamin,
N-(2-Fluoro-1,3,4-thiadiazol-5-ylmethyl)ethylendiamin oder -trimethylendiamin,
N-(2-Chloro-1,3,4-thiadiazol-5-ylmethyl)ethylendiamin oder -trimethylendiamin,

N-(3-Chloro-1,2,5-thiadiazol-4-ylmethyl)ethylendiamin oder -trimethylendiamin,

N-[1-(3-Tetrahydrofuryl)ethyl]ethylendiamin oder -trimethylendiamin,

N-(3-Tetrahydrothienylmethyl)ethylendiamin oder -trimethylendiamin,

N-[1-(3-Tetrahydrothienyl)ethyl]ethylendiamin oder -trimethylendiamin,

N-(1-Methyl-3-pyrrolidinylmethyl)ethylendiamin oder -trimethylendiamin,

N-(1,3-Oxathiolan-2-ylmethyl)ethylendiamin oder -trimethylendiamin,

N-(1,3-Dioxolan-4-ylmethyl)ethylendiamin oder -trimethylendiamin,

N-(1,3-Oxathiolan-4-ylmethyl)ethylendiamin oder -trimethylendiamin,

N-(1,3-Dithiolan-4-ylmethyl)ethylendiamin oder -trimethylendiamin,

N-(Thiazolidin-5-ylmethyl)ethylendiamin oder -trimethylendiamin,

N-(4-Methyl-1,3-dioxolan-2-ylmethyl)ethylendiamin oder -trimethylendiamin,

N-(2-Methyl-1,3-oxathiolan-4-ylmethyl)ethylendiamin oder -trimethylendiamin,

N-(2-Chloromethyl-1,3-dioxolan-4-ylmethyl)ethylendiamin oder -trimethylendiamin,

N-(2-Trifluoromethyl-1,3-dioxolan-4-ylmethyl)ethylendiamin oder -trimethylendiamin,

N-(2-Oxo-1,3-dioxolan-4-ylmethyl)ethylendiamin oder -trimethylendiamin,

N-(3-Formyl-thiazolidin-5-ylmethyl)ethylendiamin oder -trimethylendiamin,

N-(3-Acetyl-thiazolidin-5-ylmethyl)ethylendiamin oder -trimethylendiamin,

N-(3-Thiolen-2-ylmethyl)ethylendiamin oder -trimethylendiamin,

N-(1,1-Dioxo-3-thiolen-3-ylmethyl)ethylendiamin oder -trimethylendiamin,

N-(2-Isoxazolin-5-ylmethyl)ethylendiamin oder -trimethylendiamin,

N-(3-Methyl-2-isoxazolin-5-ylmethyl)ethylendiamin oder -trimethylendiamin,

N-(3-Trifluoromethyl-2-isoxazolin-5-ylmethyl)ethylendiamin oder -trimethylendiamin,

N-/¯3-(2,2,2-Trifluoroethyl)-2-isoxazolin-5-ylmethyl_7-ethylendiamin oder -trimethylendiamin,

N-(2,4-Dimethyl-2-isoxazolin-5-ylmethyl)ethylendiamin oder -trimethylendiamin,

N-(2-Methyl-2-thiazolin-4-ylmethyl)ethylendiamin oder -trimethylendiamin,

N-(2-Oxazolidinon-5-ylmethyl)ethylendiamin oder -trimethylendiamin,

N-(3-Methyl-2-oxo-1,3-oxazolan-5-ylmethyl)ethylendiamin oder -trimethylendiamin,

N-(2-Methylamino-5-thiazolylmethyl)ethylendiamin oder -trimethylendiamin,

N-(2-Trifluoroacetamido-5-thiazolylmethyl)ethylendiamin oder -trimethylendiamin,

N-(3-Methyl-2-thiooxo-thiazolidin-5-ylmethyl)ethylendiamin oder -trimethylendiamin,

N-(3-Chloro-1,2,4-oxadiazol-5-ylmethyl)ethylendiamin oder -trimethylendiamin,

N-(5-Carboxy-2-oxazolylmethyl)ethylendiamin oder -trimethylendiamin,

N-(2-Dimethylamino-5-thiazolylmethyl)ethylendiamin oder -trimethylendiamin,

N-(5-Phenoxyfurfuryl)ethylendiamin oder -trimethylendiamin,

N-(1-Phenyl-4-pyrazolylmethyl)ethylendiamin oder -trimethylendiamin,

N-(1-Benzyl-4-pyrazolylmethyl)ethylendiamin oder -trimethylendiamin,

N-(2-Phenyl-4-thiazolylmethyl)ethylendiamin oder -trimethylendiamin,

N-(1-Benzyl-2-imidazolylmethyl)ethylendiamin oder -trimethylendiamin,

N-(2-Methylsulfinyl-5-thiazolylmethyl)ethylendiamin oder -trimethylendiamin,

N-(2-Methylsulfonyl-5-thiazolylmethyl)ethylendiamin oder -trimethylendiamin,

N-(2-Methylthio-1,3,4-thiadiazol-5-ylmethyl)ethylendiamin oder -trimethylendiamin,

N-(2-Methylsulfonyl-1,3,4-thiadiazol-5-ylmethyl)ethylendiamin oder -trimethylendiamin,

N-(2-Dimethylamino-5-thiazolylmethyl)ethylendiamin oder -trimethylendiamin,

N-(5-Carbamoyl-2-thiazolylmethyl)ethylendiamin oder -trimethylendiamin,

N-(5-Methylaminocarbonyl-2-thiazolylmethyl)ethylendiamin oder -trimethylendiamin,

N-(5-Dimethylaminocarbonyl-2-thiazolylmethyl)ethylendiamin oder -trimethylendiamin,

N-(3-Pyridylmethyl)ethylendiamin oder -trimethylendiamin,

N-[1-(3-Pyridyl)ethyl]ethylendiamin oder -trimethylendiamin,

N-[1-(3-Pyridyl)propyl]ethylendiamin oder -trimethylendiamin,

N-[2-Methyl-1-(3-pyridyl)propyl]ethylendiamin oder -trimethylendiamin,

N-(4-Pyridylmethyl)ethylendiamin oder -trimethylendiamin,

N-(5-Chloro-2-pyridylmethyl)ethylendiamin oder -trimethylendiamin,
N-(2-Fluoro-5-pyridylmethyl)ethylendiamin oder -trimethylendiamin,
N-(2-Chloro-5-pyridylmethyl)ethylendiamin oder -trimethylendiamin,
N-/‾1-(2-Chloro-5-pyridyl)ethyl_7ethylendiamin oder -trimethylendiamin,
N-(2-Nitro-5-pyridylmethyl)ethylendiamin oder -trimethylendiamin,
N-(2-Cyano-5-pyridylmethyl)ethylendiamin oder -trimethylendiamin,
N-(2-Amino-5-pyridylmethyl)ethylendiamin oder -trimethylendiamin,
N-(2-Acetamido-5-pyridylmethyl)ethylendiamin oder -trimethylendiamin,
N-(2-Dimethylamino-5-pyridylmethyl)ethylendiamin oder -trimethylendiamin,
N-(2-Ethoxycarbonyl-5-pyridylmethyl)ethylendiamin oder -trimethylendiamin,
N-(2-Acetyl-5-pyridylmethyl)ethylendiamin oder -trimethylendiamin,
N-(2-Chloro-3-methyl-5-pyridylmethyl)ethylendiamin oder -trimethylendiamin,
N-(2-Difluoromethyl-5-pyridylmethyl)ethylendiamin oder -trimethylendiamin,
N-(5-Trifluoromethyl-2-pyridylmethyl)ethylendiamin oder -trimethylendiamin,
N-(2-Trifluoromethyl-5-pyridylmethyl)ethylendiamin oder -trimethylendiamin,
N-(2-Bromodifluoromethyl-5-pyridylmethyl)ethylendiamin oder -trimethylendiamin,
N-(2-Chlorodifluoromethyl-5-pyridylmethyl)ethylendiamin oder -trimethylendiamin,

N-(Trichloromethyl-5-pyridylmethyl)ethylendiamin oder -trimethylendiamin,

N-/¯2-(2-Chloroethyl)-5-pyridylmethyl_7ethylendiamin oder -trimethylendiamin,

N-/¯2-(2-Fluoroethyl)-5-pyridylmethyl_7ethylendiamin oder -trimethylendiamin,

N-/¯2-(2,2,2-Trifluoroethyl)-5-pyridylmethyl_7ethylendiamin oder -trimethylendiamin,

N-(2-Difluoroethoxy-5-pyridylmethyl)ethylendiamin oder -trimethylendiamin,

N-(2-Trifluoromethoxy-5-pyridylmethyl)ethylendiamin oder -trimethylendiamin,

N-/¯2-(2,2,2-Trifluoroethoxy)-5-pyridylmethyl_7ethylendiamin oder -trimethylendiamin,

N-/¯2-(Trifluoromethylthio)-5-pyridylmethyl_7ethylendiamin oder -trimethylendiamin,

N-(2-Formyl-5-pyridylmethyl)ethylendiamin oder -trimethylendiamin,

N-(2-Chlorodifluoromethylthio-5-pyridylmethyl)ethylendiamin oder -trimethylendiamin,

N-/¯2-(2,2-Dichlorovinyl)-5-pyridylmethyl_7ethylendiamin oder -trimethylendiamin,

N-(5-Trifluoromethyl-2-pyridylmethyl)ethylendiamin oder -trimethylendiamin,

N-(5-Methyl-2-pyridylmethyl)ethylendiamin oder -trimethylendiamin,

N-(6-Methyl-2-pyridylmethyl)ethylendiamin oder -trimethylendiamin,

N-(4-Methyl-2-pyridylmethyl)ethylendiamin oder -trimethylendiamin,

N-(5-Ethyl-2-pyridylmethyl)ethylendiamin oder -trimethylendiamin,

N-(5-Butyl-2-pyridylmethyl)ethylendiamin oder -trimethylendiamin,

N-(4,6-Dimethyl-2-pyridylmethyl)ethylendiamin oder -trimethylendiamin,
N-(3-Chloro-2-pyridylmethyl)ethylendiamin oder -trimethylendiamin,
N-(3,5-Dichloro-2-pyridylmethyl)ethylendiamin oder -trimethylendiamin,
N-(5-Fluoro-2-pyridylmethyl)ethylendiamin oder -trimethylendiamin,
N-(6-Bromo-2-pyridylmethyl)ethylendiamin oder -trimethylendiamin,
N-/¯2-(5-Ethyl-2-pyridyl)ethyl_7ethylendiamin oder -trimethylendiamin,
N-(6-Chloro-4-methyl-2-pyridylmethyl)ethylendiamin oder -trimethylendiamin,
N-(5-Methyl-3-pyridylmethyl)ethylendiamin oder -trimethylendiamin,
N-(2-Methyl-5-pyridylmethyl)ethylendiamin oder -trimethylendiamin,
N-(2-Chloro-3-pyridylmethyl)ethylendiamin oder -trimethylendiamin,
N-(5-Chloro-3-pyridylmethyl)ethylendiamin oder -trimethylendiamin,
N-(5-Bromo-3-pyridylmethyl)ethylendiamin oder -trimethylendiamin,
N-(2-Bromo-5-pyridylmethyl)ethylendiamin oder -trimethylendiamin,
N-(5-Fluoro-3-pyridylmethyl)ethylendiamin oder -trimethylendiamin,
N-(2-Fluoro-5-pyridylmethyl)ethylendiamin oder -trimethylendiamin,
N-/¯1-(2-Fluoro-5-pyridyl)ethyl_7ethylendiamin oder -trimethylendiamin,
N-/¯2-Methyl-1-(2-fluoro-5-pyridyl)propyl_7ethylendiamin oder -trimethylendiamin,

N-(2-Chloro-6-methyl-3-pyridylmethyl)ethylendiamin oder -trimethylendiamin,
N-(2,4-Dichloro-5-pyridylmethyl)ethylendiamin oder -trimethylendiamin,
N-(2,6-Dichloro-3-pyridylmethyl)ethylendiamin oder -trimethylendiamin,
N-(2,4-Dibromo-5-pyridylmethyl)ethylendiamin oder -trimethylendiamin,
N-(2,4-Difluoro-5-pyridylmethyl)ethylendiamin oder -trimethylendiamin,
N-(2-Methoxy-3-pyridylmethyl)ethylendiamin oder -trimethylendiamin,
N-(2-Methoxy-5-pyridylmethyl)ethylendiamin oder -trimethylendiamin,
N-(2-Ethoxy-5-pyridylmethyl)ethylendiamin oder -trimethylendiamin,
N-(2-Isopropoxy-5-pyridylmethyl)ethylendiamin oder -trimethylendiamin,
N-(2-Methylthio-5-pyridylmethyl)ethylendiamin oder -trimethylendiamin,
N-(4-Methyl-2-methylthio-5-pyridylmethyl)ethylendiamin oder -trimethylendiamin,
N-(2-Ethylthio-5-pyridylmethyl)ethylendiamin oder -trimethylendiamin,
N-(2-Methylsulfinyl-5-pyridylmethyl)ethylendiamin oder -trimethylendiamin,
N-(2-Methylsulfonyl-5-pyridylmethyl)ethylendiamin oder -trimethylendiamin,
N-(4-Chloro-2-fluoro-5-pyridylmethyl)ethylendiamin oder -trimethylendiamin,
N-(6-Chloro-2-methyl-3-pyridylmethyl)ethylendiamin oder -trimethylendiamin,
N-(2-Chloro-4-methyl-5-pyridylmethyl)ethylendiamin oder -trimethylendiamin,

N-(2-Allyl-5-pyridylmethyl)ethylendiamin oder -trimethylendiamin,

N-(2-Propargyl-5-pyridylmethyl)ethylendiamin oder -trimethylendiamin,

N-(2,3-Dichloro-5-pyridylmethyl)ethylendiamin oder -trimethylendiamin,

N-/‾2-(1-Propenyl)-5-pyridylmethyl_7ethylendiamin oder -trimethylendiamin,

N-(2-Chloro-4-pyridylmethyl)ethylendiamin oder -trimethylendiamin,

N-(2-Fluoro-4-pyridylmethyl)ethylendiamin oder -trimethylendiamin,

N-(2,6-Dichloro-4-pyridylmethyl)ethylendiamin oder -trimethylendiamin,

N-(2-Methyl-4-pyridylmethyl)ethylendiamin oder -trimethylendiamin,

N-/‾1-(2-Chloro-4-pyridyl)ethyl_7ethylendiamin oder -trimethylendiamin,

N-(2-Chloro-6-methyl-4-pyridylmethyl)ethylendiamin oder -trimethylendiamin,

N-(2,6-Dimethyl-4-pyridylmethyl)ethylendiamin oder -trimethylendiamin,

N-(2-Bromo-4-pyridylmethyl)ethylendiamin oder -trimethylendiamin,

N-(2,6-Dibromo-4-pyridylmethyl)ethylendiamin oder -trimethylendiamin,

N-(3-Chloro-2-fluoro-5-pyridylmethyl)ethylendiamin oder -trimethylendiamin,

N-(3-Bromo-2-fluoro-5-pyridylmethyl)ethylendiamin oder -trimethylendiamin,

N-(2-Chloro-3-fluoro-5-pyridylmethyl)ethylendiamin oder -trimethylendiamin,

N-(3-Chloro-2-methylthio-5-pyridylmethyl)ethylendiamin oder -trimethylendiamin,

2-Amino-1-(4-pyridylmethylamino)propan,
2-Amino-2-methyl-(3-pyridylmethylamino)propan,
N-(4-Pyridylmethyl)-2,2-dimethyltrimethylendiamin,
2-Amino-1-(2-chloro-5-pyridylmethylamino)propan,
N-(2-Chloro-5-pyridylmethyl)-2-methyltrimethylendiamin,
N-(3-Pyridylmethyl)-N'-methylethylendiamin,
N-(2-Chloro-5-pyridylmethyl)-N'-methylethylendiamin oder -trimethylendiamin,
N-(2-Fluoro-5-pyridylmethyl)-N'-isopropylethylendiamin,
N-(2-Chloro-5-pyridylmethyl)-N'-benzylethylendiamin,
N-(2-Chloro-5-pyridylmethyl)-N'-(3-pyridylmethyl)ethylendiamin,
N-(2-Chloro-5-pyridylmethyl)-N'-(1-methyl-4-pyrazolylmethyl)ethylendiamin,
2-Methyl-2-(2-methyl-5-pyridylmethylamino)ethanthiol,
1-Methyl-2-(2-chloro-5-pyridylmethylamino)ethanthiol,
2-(4-Pyridylmethylamino)ethanol,
2-(3-Pyridylmethylamino)ethanol,
3-(2-Methyl-3-pyridylmethyl)propanol,
2-(2-Methyl-5-pyridylmethyl)ethanol,
2-(2-Chloro-5-pyridylmethyl)ethanol,
2-(2-Trifluoromethyl-5-pyridylmethyl)propanol,
N-(1-Methyl-4-pyrazolylmethyl)-2,2-dimethyltrimethylendiamin,
N,N'-Bis(5-methyl-2-furfuryl)ethylendiamin,
N-(3-Methyl-5-isoxazolylmethyl)-N'-(1-methyl-4-pyrazolylmethyl)ethylendiamin oder -trimethylendiamin,
2-(3-Methyl-5-isoxazolylmethylamino)ethanthiol,
3-(1-Isopropyl-4-pyrazolylmethylamino)propanthiol,
2-(1,2,5-Thiadiazol-3-ylmethylamino)ethanthiol,
2-(2-Trifluoromethyl-5-thiazolylmethylamino)ethanthiol,
2-(3-Methyl-5-isoxazolylmethylamino)ethanol,
2-(4-Isothiazolylmethylamino)ethanol,

2-(5-Oxazolylmethylamino)ethanol,
2-(3-Trifluoromethyl-5-isoxazolylmethylamino)ethanol,
2-(5-Pyrimidinylmethylamino)ethanthiol,
2-(3-Trifluoromethyl-6-pyridazinylmethylamino)ethanthiol,
2-(2-Methyl-5-pyrazinylmethylamino)ethanthiol,
2-(3-Pyrazinylmethylamino)ethanol,
2-(3-Chloro-6-pyridazinylmethylamino)ethanol,
2-Amino-1-(2-pyrazinylmethyl)aminopropan,
N-(5-Pyrimidinylmethyl)-N'-(1-methyl-4-pyrazolylmethyl)ethylendiamin und
N-(3-Chloro-6-pyridazinylmethyl)-N'-methylethylendiamin.

Wie bereits oben angegeben wurde, umfaßt die Formel (II) neue Verbindungen.

In dem Fall der unter die Formel (II) fallenden Verbindungen der folgenden Formel (IIa)

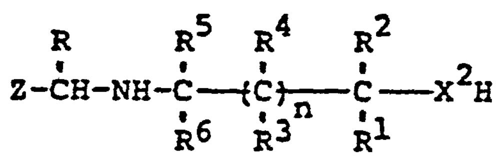

(IIa)

in der n, $R^1$, $R^2$, $R^3$, $R^4$, $R^5$, $R^6$, R und Z die im Vorstehenden angegebenen Bedeutungen haben und $X^2$ ein Sauerstoff-Atom oder $-\dot{N}R^{10}$ bezeichnet, worin $R^{10}$ die im Vorstehenden angegebenen Bedeutungen hat, können die Verbindungen der Formel (IIa) erhalten werden, wenn

(g) die Verbindungen der oben angegebenen Formel (VIII) mit den Verbindungen der Formel (IX)

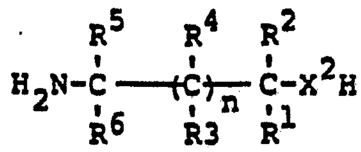

(IX)

in der n, $R^1$, $R^2$, $R^3$, $R^4$, $R^5$, $R^6$ und $X^2$ die im Vorstehenden angegebenen Bedeutungen haben, umgesetzt werden, gegebenenfalls in Anwesenheit inerter Lösungsmittel und in Anwesenheit von Säure-Acceptoren.

Wenn in dem Verfahren (g) beispielsweise Pyrazinylmethylchlorid und Ethylendiamin als Ausgangsstoffe eingesetzt werden, läßt sich der Reaktionsverlauf durch die folgende Gleichung darstellen:

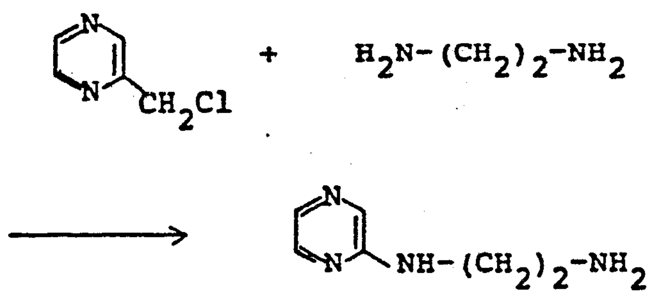

Die Verbindungen der Formel (II) können erhalten werden, wenn

(h) die Verbindungen der Formel (X)

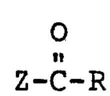

(X) ,

in der Z und R die im Vorstehenden angegebenen Bedeutungen haben, mit den Verbindungen der Formel (XI)

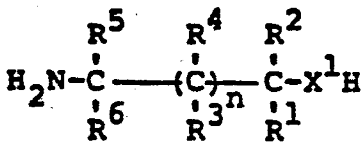 (XI) ,

in der $R^1$, $R^2$, $R^3$, $R^4$, $R^5$, $R^6$ und $X^1$ die im Vorstehenden angegebenen Bedeutungen haben, umgesetzt werden und die entstandenen Produkte reduziert werden, gegebenenfalls in Anwesenheit inerter Lösungsmittel.

Wenn in dem Verfahren (h) beispielsweise 6-Chloronicotinaldehyd und 3-Aminopropanthiol als Ausgangsstoffe eingesetzt werden, läßt sich der Reaktionsverlauf durch die folgende Gleichung darstellen:

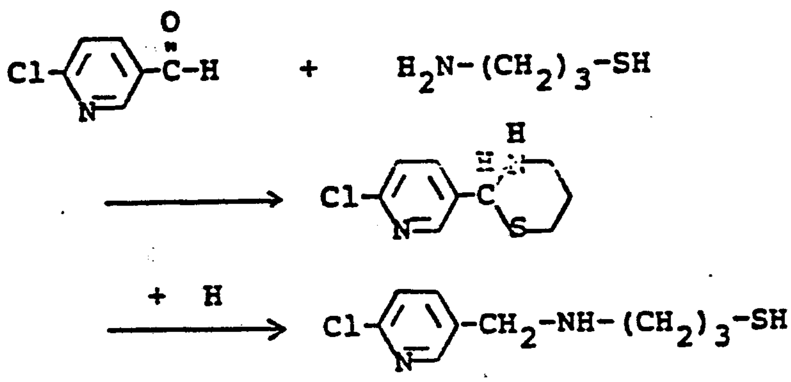

Wenn andererseits in dem Verfahren (h) beispielsweise 5-Pyrimidincarbaldehyd und Ethylendiamin als Ausgangsstoffe eingesetzt werden, läßt sich der Reaktionsverlauf durch die folgende Gleichung darstellen:

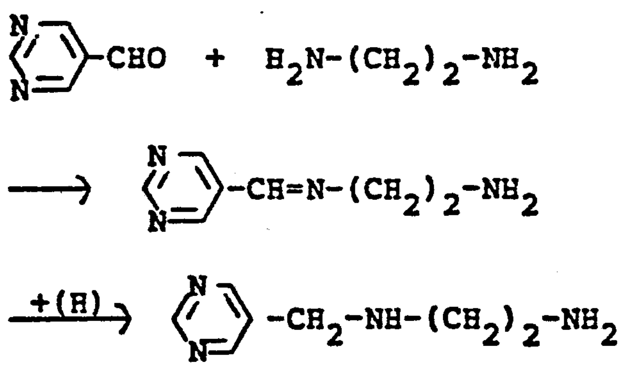

Die Verbindungen der Formel (VIII) in dem Verfahren (g), für die im Folgenden noch Beispiele genannt werden, sind die gleichen wie die Ausgangsstoffe in dem vorstehend erwähnten Verfahren (f).

Die Verbindungen der Formel (IX), die sowohl bekannte als auch neue Verbindungen umfassen, können leicht mittels bekannter Verfahren hergestellt werden.

Als Beispiele für Verbindungen der Formel (IX) seien

Ethylendiamin und

Trimethylendiamin

erwähnt (siehe die DE-OS 2 732 660 und die FR-PS 1 499 785). Weiter zu erwähnen sind auch

2-Aminoethanol und

3-Aminopropanol,

die wohlbekannte Verbindungen in der organischen Chemie sind.

Außerdem zählen auch N-Benzylethylendiamin oder -trimethylendiamin (siehe die JP-OS 78 971/1985 und die DE-OSen 2 514 402 und 2 732 660 sowie die JP-Patentanmeldung 68 551/1985) und N-substituierte Alkylethylendiamine oder -trimethylendiamine, die Ethylendiaminen oder Trimethylendiaminen in der vorstehenden Formel (II) entsprechen, zu Beispielen für Verbindungen der Formel (IX).

In dem Verfahren (g) können die gewünschten Verbindungen der Formel (II) leicht durch Umsetzung der Verbindungen der Formel (VII) mit den Verbindungen der Formel (IX) in inerten Lösungsmitteln erhalten werden, wie das durch das Verfahren (a) veranschaulicht wird, das hiernach noch ausführlich beschrieben wird.

Das Verfahren (g) kann in einfacher Weise durchgeführt werden, indem man mehr als 1 mol, beispielsweise etwa 5 mol, der Verbindungen der Formel (IX) auf 1 mol der Verbindungen der Formel (VIII) bei einer Reaktionstemperatur in dem Bereich von beispielsweise 0°C bis 50°C zur Einwirkung bringt.

Die als Ausgangsstoffe in dem Verfahren (h) eingesetzten Verbindungen der Formel (X) umfassen größtenteils bekannte Verbindungen. Als Beispiele hierfür seien genannt:

6-Chloronicotinaldehyd,
6-Bromonicotinaldehyd,
6-Fluoronicotinaldehyd,
5-Acetyl-2-chloropyridin,
5,6-Dichloronicotinaldehyd,
5-Chloro-6-fluoronicotinaldehyd,
2-Chloro-4-pyridylcarbaldehyd,
3-Chloro-2-pyridincarbaldehyd,
3,5-Dichloro-2-pyridincarbaldehyd,
5-Fluoro-2-pyridincarbaldehyd,
6-Bromo-2-pyridincarbaldehyd,
2-Chloronicotinaldehyd,
5-Chloronicotinaldehyd,
5-Bromonicotinaldehyd,
5-Fluoronicotinaldehyd,
5-Acetyl-2-fluoropyridin,
4,6-Dichloronicotinaldehyd,
4,6-Dibromonicotinaldehyd,
2,6-Difluoro-4-pyridincarbaldehyd,
2-Fluoro-4-pyridincarbaldehyd,
2,6-Dibromo-4-pyridincarbaldehyd,

5-Bromo-6-fluoronicotinaldehyd,
6-Chloro-5-fluoronicotinaldehyd,
2-Chloro-5-propionylpyridin,
Nicotinaldehyd,
4-Pyridincarbaldehyd,
6-Methylnicotinaldehyd,
6-Ethylnicotinaldehyd,
6-Allylnicotinaldehyd,
6-Propargylnicotinaldehyd,
6-Methoxynicotinaldehyd,
6-Methylthionicotinaldehyd,
6-Methylsulfonylnicotinaldehyd,
6-Chloro-4-methylnicotinaldehyd,
3-Acetylpyridin,
6-Nitronicotinaldehyd,
6-Cyanonicotinaldehyd,
6-Methylsulfinylnicotinaldehyd,
6-Phenylnicotinaldehyd,
6-Benzylnicotinaldehyd,
6-Phenoxynicotinaldehyd,
6-(2-Ethoxyethyl)nicotinaldehyd,
6-Trichloromethylnicotinaldehyd,
6-Methoxymethylnicotinaldehyd,
6-Difluoromethoxynicotinaldehyd,
6-Trifluoromethoxynicotinaldehyd,
6-(2,2,2-Trifluoroethoxy)nicotinaldehyd,
6-Chlorodifluoromethylthionicotinaldehyd,
6-Trifluoromethylthionicotinaldehyd,
6-Difluoromethylthionicotinaldehyd,
6-Trifluoromethylsulfonylnicotinaldehyd,
6-Trifluoromethylsulfinylnicotinaldehyd,
6-(2,2-Dichlorovinyl)nicotinaldehyd,
4-Pyrimidincarbaldehyd,

2-Methyl-4-pyrimidincarbaldehyd,
2-Methyl-6-oxo-1H,6H-dihydropyrimidin-4-carbaldehyd,
5-Pyrimidincarbaldehyd,
2-Methyl-5-pyrimidincarbaldehyd,
2-Dimethylamino-5-pyrimidincarbaldehyd,
2,4,6-Trichloro-5-pyrimidincarbaldehyd,
Pyrazylcarbaldehyd,
Acetylpyrazin,
2-Methyl-5-pyrazincarbaldehyd,
3-Pyridazincarbaldehyd,
2-Chloro-4-pyrimidincarbaldehyd,
4-Chloro-6-pyrimidincarbaldehyd,
4-Methyl-6-pyrimidincarbaldehyd,
2-Fluoro-5-pyrimidincarbaldehyd,
5-Acetyl-2-fluoropyrimidin,
2-Chloro-5-pyrimidincarbaldehyd,
2-Isopropyl-5-pyrimidincarbaldehyd,
2-Chlorodifluoromethyl-5-pyrimidincarbaldehyd,
2-Trifluoromethyl-5-pyrimidincarbaldehyd,
2-Bromodifluoromethyl-5-pyrimidincarbaldehyd,
2-Methoxy-5-pyrimidincarbaldehyd,
2-Difluoromethoxy-5-pyrimidincarbaldehyd,
2-Trifluoromethoxy-5-pyrimidincarbaldehyd,
2-(2,2,2-Trifluoroethoxy)-5-pyrimidincarbaldehyd,
2-Methylthio-5-pyrimidincarbaldehyd,
2-Ethylthio-5-pyrimidincarbaldehyd,
2-Difluoroethylthio-5-pyrimidincarbaldehyd,
2-Trifluoromethylthio-5-pyrimidincarbaldehyd,
2-Nitro-5-pyrazincarbaldehyd,
2-Cyano-5-pyrazincarbaldehyd,
2-Chloro-5-pyrazincarbaldehyd,
2-Trifluoromethyl-5-pyrazincarbaldehyd,
3-Fluoro-6-pyridazincarbaldehyd,

3-Methyl-6-pyridazincarbaldehyd,
4-Pyridazincarbaldehyd,
3-Chloro-6-pyridazincarbaldehyd,
3-Trifluoromethyl-6-pyridazincarbaldehyd,
1,3,5-Triazin-2-carbaldehyd,
3-Chloro-1,2,4-triazin-6-carbaldehyd,
3,5-Dichloro-1,2,4-triazin-6-carbaldehyd und
3-Chloro-1,2,4,5-tetrazin-6-carbaldehyd.

Die Verbindungen der Formel (X) können nach verschiedenen herkömmlichen Verfahrensweisen hergestellt werden. Diese werden speziell weiter unter beschrieben.

Beispielsweise können die Pyridincarbaldehyde der Formel (X) dadurch hergestellt werden, daß die entsprechenden Vinylpyridine in einer Ozonolyse-Reaktion umgesetzt werden (siehe J. Org. Chem. 26, 4912-4914), und gemäß der GB-PS 2 002 368 kann 6-Chloronicotinaldehyd aus 2-Chloro-5-pyridylcarbonitril gewonnen werden.

Überdies können die Verbindungen der Formel (X) allgemein ohne Schwierigkeiten gemäß herkömmlicher Verfahren durch Reduktion der entsprechenden Carbonsäuren oder ihrer Ester oder mit Hilfe der Vilsmeyer-Reaktion hergestellt werden.

Beispielsweise können Pyridincarbaldehyde auch durch Reduktion der entsprechenden Pyridincarbonsäuren und ihrer Ester hergestellt werden (siehe Org. React., Vol. 8, 218-257).

Die Verbindungen der Formel (X) können auch direkt durch Ring-Bildung hergestellt werden. Beispielsweise

wird im Hinblick auf 4-Pyrimidincarbaldehyd das entsprechende 2-Methylthio-4-methyl-6-pyrimidincarbaldehyd-acetyl durch Umsetzung von Diethoxyacetylaceton mit S-Methylisothioharnstoff erhalten. Nachfolgende Reduktion und Behandlung mit Salzsäure liefert 4-Methyl-6-pyrimidincarbaldehyd. Die Verwendung von Diethoxyacetylaceton-Derivaten kann zur Synthese ähnlicher Verbindungen wie 4-Pyrimidincarbaldehyd, 2-Methyl-4-pyrimidincarbaldehyd und 2-Trifluoromethyl-4-pyrimidincarbaldehyd (beschrieben in Chem. Ber. 97, S. 3407-3417) führen. Es gibt für die Synthese von 5-Pyrimidincarbaldehyden viele bekannte Verfahren auf dem Gebiet der organischen Chemie.

Beispielsweise läßt sich 5-Pyrimidincarbaldehyd dadurch synthetisieren, daß man eine Formyl-Gruppe durch Vilsmeyer-Reaktion in die 5-Stellung von 4-Hydroxy-6-oxo-dihydropyrimidin einführt, das Produkt zu 4,6-Dichloro-5-formylpyrimidin halogeniert und die erhaltene Verbindung enthalogeniert (Liebigs Ann. Chem. 766, S. 73-83; und Monatsh. Chem. 96, S. 1567-1572). Durch Anwendung dieser Reaktion können 2-alkylsubstituierte und 2-halogenoalkylsubstituierte 5-Pyrimidincarbaldehyde synthetisiert werden. 5-Pyrimidincarbaldehyde mit anderen Substituenten in der 2-Stellung sind in der JP-OS 59 669/1984 beschrieben.

Beispielsweise werden 5-Pyrimidincarbaldehyde mit solchen Substituenten wie Alkyl, Alkoxy, Alkylthio oder Alkylamino in der 2-Stellung werden erhalten durch Reaktion von /¯2-/¯(Dimethylamino)methylen_7propandiyliden_7bis/¯(dimethylaminoperchlorat_7 (beschrieben in Collect. Czech. Chem. Comm. 30, S. 2125) mit geeigneten Amidin-Hydrochloriden.

Was 5-Pyrimidincarbaldehyde mit Halogen in der 2-Stellung betrifft, so kann 2-Chloro-5-pyrimidincarbaldehyd beispielsweise durch Chlorieren von Ethyl-2-oxo-1,2-dihydro-5-pyrimidincarboxylat mit Phosphoroxidchlorid zu Ethyl-2-chloro-5-pyrimidincarboxylat (Chem. Pharm. Bull. 12, S. 804-808; ein ähnliches Beispiel findet sich in J. Org. Chem. 29, S. 1740-1743) und Reduzieren der erhaltenen Verbindung in üblicher Weise erhalten werden. Da das Chlor-Atom in der 2-Stellung Aktivität besitzt, kann es in andere Substituenten umgewandelt werden werden, beispielsweise in 2-Fluor mit Hilfe von Kaliumfluorid.

Was Pyridazincarbaldehyde betrifft, so sind 3- und 4-Pyridazincarbaldehyde auf Seite 213 der Monatsh. Chem., Band 108, und methylsubstituierte Pyridazincarbaldehyde in J. Heterocycl. Chem. 17, S. 1501, beschrieben.

Im übrigen werden die 5-gliedrigen Heterocyclus-carbaldehyde im einzelnen nachstehend beschrieben. Furfural ist eine bekannte Verbindung und kann in einfacher Weise die Einführung eines Halogen-Atoms am Furan-Ring ermöglichen. Beispielsweise lassen sich 5-Chlorofurfural und 4,5-Dichlorofurfural aus Furfural synthetisieren (Zh. Org. Khim. 11, S. 1955-1958). 5-Nitrofurfural ist ebenfalls eine leicht zugängliche bekannte Verbindung. 5-Cyanofurfural ist eine Verbindung, die in Tetrahedron 39, S. 3881 beschrieben ist, und 5-Phenoxyfurfural ist eine Verbindung, die in Chem. Pharm. Bull. 28 (No. 9), S. 2846, beschrieben ist.

Andere Furancarbaldehyde als Furfural, alkylsubstituierte, insbesondere methylsubstituierte Furfurale und

andere Furancarbaldehyde sind ebenfalls bekannte Verbindungen und lassen sich in einfacher Weise erhalten.

Thiophencarbonaldehyd ist eine bekannte Verbindung, und ein Halogen-Atom läßt sich leicht am Thiophen-Ring einführen. Beispielsweise kann 2,3-Dichloro-4-thiophencarbaldehyd aus 3-Thiophencarbaldehyd (Tetrahedron 32, S. 1403-1406) synthetisiert werden. 2,3-Dibromo-5-thiophencarbaldehyd kann aus 2-Thiophencarbaldehyd (J. Org. Chem. 41, S. 2835) synthetisiert werden. Alkylsubstituierte, insbesondere methylsubstituierte Thiophencarbaldehyde sind ebenfalls bekannte Verbindungen. Alkylthiosubstituierte oder halogenoalkylthiosubstituierte Thiophencarbaldehyde lassen sich durch Alkylieren oder Halogenoalkylieren von mercaptosubstituiertem Thiophencarbaldehyd gewinnen. Beispielweise ist 2-Methylthio-5-thiophencarbaldehyd eine bekannte Verbindung, die in Zh. Obshch. Khim., 34, S. 4010-4015, beschrieben ist. Allgemein können alkylthiosubstituierte oder halogenoalkylthiosubstituierte heterocyclische Carbaldehyde mit Hilfe der obigen Verfahren hergestellt werden.

Nitrosubstituierte Thiophencarbaldehyde können leicht durch Nitrierung des Thiophen-Ringes synthetisiert werden. Beispielsweise sind 4-Nitro-2-thiophencarbaldehyd und 2-Nitro-4-thiophencarbaldehyd bekannte Verbindungen, die in Bull. Soc. Chim. France 1963, S. 479-484, beschrieben sind.

Pyrrolcarbaldehyde sind bekannte Verbindungen. 1-Methyl-2-pyrrolcarbaldehyd kann aus 1-Methylpyrrrol durch Vilsmeier-Reaktion oder durch Methylieren von 2-Pyrrolcarbaldehyd hergestellt werden (Beilstein, Band 21, I, Seite 279).

4-Isothiazolcarbaldehyd kann aus 4-Isothiazolylcarbonsäure synthetisiert werden (J. Medicin. Chem. 13, S. 1208-1212) synthetisiert werden, und 5-Isothiazolcarbaldehyd kann aus 5-Isothiazolyl-lithium synthetisiert werden (J. Chem. Soc. 1964, S. 446-451).

5-Pyrazolcarbaldehyd und 3-Methyl-5-pyrazolcarbaldehyd können durch direkte Ring-Synthese hergestellt werden (Chem. Ber. 97, S. 3407-3417). Nach einem ähnlichen Verfahren kann 3-Trifluoromethyl-5-pyrazolcarbaldeyd synthetisiert werden.

Eine Formyl-Gruppe kann in die 4-Position eines N-Alkyl- oder N-Arylpyrazols mit Hilfe der Vilsmeier-Reaktion eingeführt werden. 4-Pyrazolcarbaldehyd kann erhalten werden durch Eliminieren des Benzyl-Restes aus N-Benzyl-4-pyrazolcarbaldehyd (J. Chem. Soc. 1957, S. 3314 und 3315).

4-Methyl-5-imidazolcarbaldehyd und 1-Methyl-5-imidazolcarbaldehyd sind bekannte Verbindungen (J. Pharm. Soc. Japan, 60, S 184-188; J. Amer. Chem. Soc. 71, S. 2444-2448).

Viele substituierte Thiazolcarbaldehyde sind bekannt (JP-OS 206 370/1984; Chem. Abstr. 62, 7764d; Chem. Ber. 101, S. 3872). Beispielsweise läßt sich 2-Chlorothiazol-5-carbaldehyd durch Lithiierung mit Hilfe von Butyllithium und nachfolgende Formylierung synthetisieren. Substituierte 1,3,4-Thiadiazolcarbaldehyde sind ebenfalls bekannte Verbindungen (JP-OS 206 370/1984). 1,2,3-Thiadiazol-5carbaldehyd ist ebenfalls eine bekannte Verbindung (GB-PS 1 113 705).

Die Verbindungen der Formel (XI) in dem Verfahren (h) schließen die Verbindungen der oben bezeichneten Formel (IX) ein.

Daneben werden 2-Aminoethanthiol und 3-Aminopropanthiol beispielhaft genannt (siehe J. Org. Chem. 27, 4712-4713), und auch die auf ihnen aufbauenden Aminoalkanthiole zählen zu diesen Beispielen.

Wenn beispielsweise $X^3$ ein Schwefel-Atom ist, kann das obige Verfahren (h) in der gleichen Weise durchgeführt werden, wie sie in J. Org. Chem. 27, 2452-2457 und 4712-4713, beschrieben ist.

Bei der Durchführung des Verfahrens (c) können in dem ersten Schritt die Thiazolidine oder die Tetrahydrothiazine als Zwischenprodukte dadurch hergestellt werden, daß die Verbindungen der Formel (X) mit den Verbindungen der Formel (XI) in Gegenwart eines inerten Lösungsmittels wie Benzol umgesetzt werden, und in dem nächsten Schritt können die Zwischenprodukte mit Hilfe eines Reduktionsmittels wie Natriumborhydrid, Lithiumaluminiumhydrid, Aluminiumborhydrid, Kaliumborhydrid etc. reduziert werden, wodurch die Verbindungen der Formel (IIb) hergestellt werden.

Bei der Durchführung des Verfahrens (h) in der Praxis können die Thiazolidine oder die Tetrahydrothiazine als Zwischenprodukte nicht nur durch Abdestillieren des flüchtigen Materials nach der Reaktion des ersten Schrittes unter vermindertem Druck, beispielsweise bei 1,33 mbar (1 mmHg) bei 50°C bis 80°C erhalten werden, sondern sie können auch unmittelbar ohne Isolierung der Reduktion unterworfen werden.

In dem Fall, in dem $X^3$ $-\dot{N}-R^{10}$ ist, kann gemäß dem Verfahren (h) die gewünschte Verbindung der Formel (IIb) dadurch erhalten werden, daß die Ausgangsstoffe in einem inerten Lösungsmittel (wie Benzol) unter Rückfluß erhitzt werden und danach die Reaktionsmischung in üblicher Weise unmittelbar ohne Abtrennung der Zwischenstufe der Schiff'schen Base oder des Imins reduziert wird, wie im einzelnen in einem weiter unten angegebenen Arbeitsbeispiel gezeigt wird.

Bei der Durchführung des Verfahrens (h) wird vorzugsweise eine Menge von man mehr als 1 mol, beispielsweise etwa 5 mol, der Verbindungen der Formel (XI) auf 1 mol der Verbindungen der Formel (X) eingesetzt, und die Reaktion wird unter Atmosphärendruck bei einer Temperatur in dem Bereich von gewöhnlich 0°C bis 100°C durchgeführt.

Daneben läßt sich als alternatives Verfahren zur Herstellung von Verbindungen der Formel (II), in denen $X^1$ ein Schwefel-Atom ist, ein Verfahren anführen, bei dem die Verbindungen der Formel (II), in denen $X^1$ ein Sauerstoff-Atom ist, mit einem Halogenierungsmittel wie Thionylchlorid halogeniert werden und danach die erhaltenen Produkte mit Kaliumhydrogensulfid umgesetzt werden.

Die Verbindungen der Formel (III) in dem Verfahren (a) umfassen sowohl bekannte als auch neue Verbindungen.

Als Beispiele für bekannte Verbindungen seien erwähnt (siehe z.B. Chem. Ber. 100, 591-604):

1-Nitro-2,2-bis(methylthio)ethylen,
1-Nitro-2,2-bis(ethylthio)ethylen,
1-Nitro-2,2-bis(benzylthio)ethylen und
2-Nitromethylen-1,3-dithiolan.

Die obigen Verbindungen können in üblicher Weise hergestellt werden, wobei Nitromethan mit Kohlenstoffdisulfid in Gegenwart einer Base umgesetzt wird und das erhaltene Produkt alkyliert wird.

Wenn in dem betreffenden Verfahren andere Nitroalkane an Stelle von Nitromethan eingesetzt werden, können in einfacher Weise ähnliche, der Formel (III) entsprechende Verbindungen hergestellt werden.

Daneben können bei Einsatz acylsubstituierter Nitromethane an Stelle von Nitromethan in dem betreffenden Verfahren andere gewünschte Verbindungen der Formel (III) hergestellt werden.

Wenn beispielsweise Benzoylnitromethan eingesetzt wird, kann 1-Benzoyl-1-nitro-2,2-bis(methylthio)ethylen, eine neue Verbindung, hergestellt werden, und wenn Acetylnitromethan eingesetzt wird, kann 1-Acetyl-1-nitro-2,2-bis(methylthio)ethylen, eine neue Verbindung, hergestellt werden.

Die Verbindungen der Formel (IV) in dem Verfahren (b) sind bekannte Verbindungen (siehe Chem. Abstr. 44, 1011f; JP-OS 137 473/1984). Als Beispiele seien erwähnt:

2,2-Dichloronitroethylen,
1,2,2-Trichloronitroethylen,

1-Fluoro-2,2-dichloronitroethylen,
1-Methyl-2,2-dichloronitroethylen,

Die Verbindungen der Formel (V) in dem Verfahren (c) sind bekannte Verbindungen (siehe J. Org. Chem. 25, 1312; ibid. 28, 1281-1283; Chem.Ber. 75B, 1323-1330; JP-OS 48 978/1985). Als Beispiele seien erwähnt:

2,2,2-Trichloro-1-nitroethan,
1,2,2,2-Tetrachloro-1-nitroethan,
2,2,2-Trifluoro-1-nitroethan.

Die Formel (VI) gibt eine allgemeine Definition der als Ausgangsstoffe in dem Verfahren (e) benötigten Verbindungen, wobei auf die im Vorstehenden angegebenen jeweiligen Bedeutungen von n, $R^1$, $R^2$, $R^3$, $R^4$, $R^5$, $R^6$, X, R und Z Bezug genommen wird.

In der Formel (VI) haben n, $R^1$, $R^2$, $R^3$, $R^4$, $R^5$, $R^6$, X, R und Z vorzugsweise die bereits oben bezeichneten Bedeutungen.

Zu den Verbindungen der Formel (VI) zählen sowohl bekannte als auch neue Verbindungen. Beispielsweise ist 2-Imino-3-(4-pyridylmethyl)thiazolidin in J. Med. Chem. 22, 237-247, offenbart.

Andere, der Formel (VI) entsprechende Verbindungen können in der gleichen Weise hergestellt werden, wie sie in der vorstehenden Literatustelle genannt ist.

Die Verbindungen der Formel (VI) können beispielsweise durch Reaktion der oben bezeichneten Verbindungen mit Cyanhalogeniden hergestellt werden.

Die Reaktion kann in einfacher Weise durch Vermischen der Reaktionspartner unter Rühren in inerten Lösungsmitteln bewerkstelligt werden, und die erhaltenen Produkte lassen sich in Form ihrer Hydrohalogenide erhalten.

Als spezielle Beispiele für die Verbindungen der Formel (VI) (in Form eines Hydrohalogenids) seien die folgenden Verbindungen erwähnt:

Hydrobromide oder Hydrochloride von
1-(2-Chloro-5-pyridylmethyl)-2-iminoimidazolidin,
1-(2-Chloro-5-pyridylmethyl)-2-iminotetrahydropyrimidin,
1-(2-Fluoro-5-pyridylmethyl)-2-iminoimidazolidin,
1-(2-Bromo-5-pyridylmethyl)-2-iminoimidazolidin,
1-(2-Trifluoromethyl-5-pyridylmethyl)-2-iminoimidazolidin,
1-(2-Methyl-5-pyridylmethyl)-2-iminoimidazolidin,
1-(3-Pyridylmethyl)-2-iminoimidazolidin,
1-(3-Pyridylmethyl)-2-iminotetrahydropyrimidin,
1-(2-Trifluoromethoxy-5-pyridylmethyl)-2-iminoimidazolidin und
1-(2-Methoxy-5-pyridylmethyl)-2-iminoimidazolidin.

Die Formel (VII) gibt eine allgemeine Definition der als Ausgangsstoffe in dem Verfahren (f) benötigten Verbindungen, wobei auf die im Vorstehenden angegebenen jeweiligen Bedeutungen von n, $R^1$, $R^2$, $R^3$, $R^4$, $R^5$, $R^6$, X und X Bezug genommen wird.

In der Formel (VII) haben n, $R^1$, $R^2$, $R^3$, $R^4$, $R^5$, $R^6$, X, und Y vorzugsweise die bereits oben bezeichneten Bedeutungen.

Die Verbindungen der Formel (VII) sind meistenteils bekannte Verbindungen.

Als Beispiele seien erwähnt:

2-Nitromethylenimidazolidin,
2-Nitromethylentetrahydropyrimidin,
4,4-Dimethyl-2-nitromethylenimidazolidin,
3-Methyl-2-nitromethylenimidazolidin,
3-Allyl-2-nitromethylenimidazolidin,
3-Propargyl-2-nitromethylenimidazolidin,
3-(3-Chloroallyl)-2-nitromethylenimidazolidin,
3-Acetyl-2-nitromethylenimidazolidin,
3-Chloroacetyl-2-nitromethylenimidazolidin,
3-Benzoyl-2-nitromethylenimidazolidin,
3-p-Tosyl-2-nitromethylenimidazolidin,
2-Nitromethylenthiazolidin,
2-Nitromethylentetrahydro-2H-1,3-thiazin,
2-Nitromethylen-5-methyl-thiazolidin,
2-Nitromethylenoxazolidin,
2-Nitromethylen-4-methyloxazolidin,
2-Nitromethylentetrahydro-2H-1,3-oxazin,
2-Nitromethylenpyrrolidin,
2-Nitromethylenpiperidin,
2-(1-Nitroethyliden)imidazolidin,
2-(1-Nitro-2-fluoroethyliden)imidazolidin,
2-(Phenylnitromethylen)imidazolidin,
2-(1-Nitro-2,2,2-trifluoroethyliden)imidazolidin,
Ethyl-nitro(imidazolidin-2-yliden)acetat,
n-Butyl-nitro(tetrahydropyrimidin-2-yliden)acetat,
o-Tolyl-nitro(imidazolidin-2-yliden)acetat,
p-Chlorophenyl-nitro(imidazolidin-2-yliden)acetat,
p-Nitrophenyl-nitro(imidazolidin-2-yliden)acetat,

2-(Methylthionitromethylen)imidazolidin,
2-(Propylthionitromethylen)imidazolidin,
2-/¯(4-Chlorophenylthio)nitromethylen_7imidazolidin,
2-(Acetylnitromethylen)imidazolidin,
2-(Dinitromethylen)imidazolidin,
2-(Benzoylnitromethylen)imidazolidin,
Ethyl-nitro(1-ethoxycarbonylimidazolidin-2-yliden)-acetat,
Phenyl-nitro(1-phenylthiocarbonylimidazolidin-2-yliden)acetat,
2-(Phenylthionitromethylen)-1-phenylthioimidazolidin,
2-(1-Nitroethyliden)-tetrahydro-2H-1,3-thiazin,
2-(1-Nitro-3-butyliden)thiazolidin,
2-(1-Nitro-3-butyliden)-tetrahydro-2H-1,3-thiazin,
2-(1-Nitro-2-phenylethyliden)thiazolidin,
2-(3-Acetyl-1-nitropropyliden)-tetrahydro-2H-1,3-thiazin,
2-(3-Cyano-1-nitropropyliden)-tetrahydro-2H-1,3-thiazin,
Methyl-4-nitro-4-(thiazolidin-2-yliden)butylat,
2-(2-Ethylthio-1-nitroethyliden)-tetrahydro-2H-1,3-thiazin,
2-(2-Dimethylamino-1-nitroethyliden)thiazolidin,
Ethyl-nitro-(tetrahydro-2H-1,3-thiazin-2-yliden)acetat,
Phenyl-nitro-(tetrahydro-2H-1,3-thiazin-2-yliden)-acetat,
2-Formylnitromethylenthiazolidin,
2-Acetylnitromethylen-tetrahydro-2H-1,3-thiazin,
2-Benzoylnitromethylen-tetrahydro-2H-1,3-thiazin,
2-Phenylthionitromethylen-tetrahydro-2H-1,3-thiazin,
Ethyl-nitro-(oxazolidin-2-yliden)acetat,
Ethyl-nitro-(tetrahydro-2H-1,3-oxazin-2-yliden)acetat,
3-Methyl-2-nitromethylenpyrrolidin,

3-Fluoro-2-nitromethylenpiperidin,
Methyl-nitro-(pyrrolidin-2-yliden)acetat,
3-Methylthio-2-nitromethylenpiperidin,
Ethyl-nitro-(thiazolidin-2-yliden)acetat,
2-Nitroiminoimidazolidin,
4,4-Dimethyl-2-nitroiminoimidazolidin,
2-Nitroiminotetrahydropyrimidin,
3-Methyl-2-nitroiminoimidazolidin,
3-Isopropyl-2-nitroiminoimidazolidin,
3-(2-Ethoxyethyl)-2-nitroiminoimidazolidin,
3-Ethoxycarbonyl-2-nitroiminoimidazolidin,
3-Phenylthio-2-nitroiminoimidazolidin,
3-Formyl-2-nitroiminoimidazolidin,
3-Acetyl-2-nitroiminotetrahydropyrimidin,
3-(2-Bromo-3,3-dimethylbutyryl)-2-nitroiminoimidazolidin,
3-(2-Trifluoromethylbenzoyl)-2-nitroiminoimidazolidin,
3-(2,4-Dichloro-3-methylbenzoyl)-2-nitroiminoimidazolidin,
3-(4-Methoxybenzoyl)-2-nitroiminoimidazolidin,
3-(3-Chloropropylsulfonyl)-2-nitroiminoimidazolidin,
3-(2-Difluoromethoxybenzoyl)-2-nitroiminotetrahydropyrimidin,
3-Phenoxycarbonyl-2-nitroiminoimidazolidin,
3-(2-Formyl)-2-nitroiminotetrahydropyrimidin,
3-(2-Methylthiazol-5-ylcarbonyl)-2-nitroiminoimidazolidin,
3-(Diethoxyphosphono)-2-nitroiminoimidazolidin,
3-(5-Nitro-2-methylbenzolsulfonyl)-2-nitroiminoimidazolidin,
2-Nitroiminothiazolidin,
2-Nitroiminotetrahydro-2H-1,3-thiazin,
2-Nitroiminooxazolidin,

4-Methyl-2-nitroiminooxazolidin,
2-Nitroiminopyrrolidin,
2-Nitroiminopiperidin,
3-Methyl-2-nitroiminopyrrolidin und
2-Nitroiminotetrahydro-2H-1,3-oxazin.

2-Nitromethylen-imidazolidine (oder -tetrahydropyrimidine) der vorstehend bezeichneten Formel (VII) sind bekannte Verbindungen (siehe, beispielsweise, Chem. Ber. 100, 591-604; die BE-PS 821 281 und die US-PS 3 971 774).

Daneben können N-Acyl-Derivate von 2-Nitromethylen-imidazolidinen (oder -tetrahydropyrimidinen) mittels bekannter Verfahren hergestellt werden (siehe die JP-OSen 67 473/1985 und 61 575/1985).

Darüber hinaus können Verbindungen der Formel (VII), in denen neben einer Nitro-Gruppe eine andere Gruppe an die Methylen-Gruppe von 2-Nitromethylen-imidazolidinen (oder -tetrahydropyrimidinen) gebunden ist, mittels bekannter Verfahren hergestellt werden, die in den US-PSen 3 996 372, 4 002 765, 4 042 696, 4 052 411, 4 053 619, 4 053 622 und 4 053 623, der JP-OS 151 727/1977 und der BE-PS 821 282 beschrieben sind.

2-Nitromethylen-thiazolidine (oder -tetrahydro-2H-1,3-thiazine) sind ebenfalls größtenteils bekannte Verbindungen, die leicht hergestellt werden können, beispielsweise durch Reaktion von Aminoalkanthiolen mit Verbindungen der oben angegebenen Formel (III), die durch diejenigen der obigen Formel (IV) oder Formel (V) ersetzt sein können.

Darüber hinaus kann die 2-Position der 2-Nitromethylen-thiazolidine (oder -tetrahydro-2H-1,3-thiazine) mittels verschiedener bekannter Verfahren substituiert werden, wodurch gewünschte Ausgangsstoffe der Formel (VII) erhalten werden (siehe die US-PSen 3 962 234, 4 022 775, 4 024 254, 4 044 128, 4 045 434 und 4 076 813 sowie die JP-OS 151 882/1975).

2-Nitromethylen-oxazolidine (oder -tetrahydro-2H-1,3-oxazine) sind ebenfalls größtenteils bekannte Verbindungen, die leicht hergestellt werden können, beispielsweise durch Reaktion von Aminoalkanolen mit Verbindungen der oben angegebenen Formel (III), die durch diejenigen der obigen Formel (IV) oder Formel (V) ersetzt sein können /‾siehe Adv. Pestic. Sci., Plenary Lect. Symp. Pap. Int. Congr. Pestic. Chem. 4-th, 1978, 206-217 (referiert Chem. Abstr. 91, 103 654), die US-PS 3 907 790 sowie die JP-OSen 151 882/1975 und 151 727/1977_7.

2-Nitromethylen-pyrrolidine (oder -piperidine) sind ebenfalls bekannte Verbindungen, die beispielsweise durch Umsetzung von 2-Methoxypyrrolin-1 mit Nitroalkanen hergestellt werden können (siehe die NL-PSen 7 306 020 und 7 306 145).

2-Nitroimino-Derivate der Formel (VII) sind ebenfalls bekannte Verbindungen.

Beispielsweise sind 2-Nitroiminooxazolidine in J. Amer. Chem. Soc. 73, 2213-2216, beschrieben.

2-Nitroiminoimidazolidine, 2-Nitroiminotetrahydropyrimidine und deren N-Acetyl-Derivate sind in J. Amer. Chem. Soc. 73, 2201-2205, und in der GB-PS 2 055 796 beschrieben.

N-Acyl-Derivate, ausschließlich der N-Acetyl-Derivate, N-Sulfinyl-Derivate, N-Sulfonyl-Derivate und N-Phosphono-Derivate sind neue Verbindungen, die in der gleichen Weise hergestellt werden können, wie in der GB-PS 2 055 796 beschrieben ist.

2-Nitroimino-thiazolidine (oder -tetrahydro-2H-1,3-thiazine) und 2-Nitroimino-pyrrolidine (oder -piperidine), die durch Umsetzung von Nitroguanidin mit den Diaminen, den Aminoalkanolen oder den Aminoalkanthiolen oder durch Reaktion der 2-Imino-Verbindung mit Salpetersäure in Gegenwart von Schwefelsäure hergestellt werden können, werden ebenfalls in der oben genannten GB-PS beschrieben.

Die Formel (VIII) gibt eine allgemeine Definition der als Ausgangsstoffe in dem Verfahren (f) benötigten Verbindungen, wobei auf die im Vorstehenden angegebenen jeweiligen Z und R Bezug genommen wird.

In der Formel (VIII) haben Z und R vorzugsweise die bereits oben bezeichneten Bedeutungen.

Die gemäß der vorliegenden Erfindungen einsetzbaren Verbindungen der Formel (VIII) umfassen bekannte Verbindungen, die beispielsweise bereits in J. Org. Chem. 34, 3547; J. Medicin. Chem. 14, 211-213 und 557-558 (1971); der US-PS 4 332 944 und in J. Heterocycl. Chem. 1979, 16, 333-337, offenbart sind.

Als Beispiele seien erwähnt:

3-Picolylchlorid,
1-(3-Pyridyl)ethylchlorid,
1-(3-Pyridyl)propylchlorid,
2-Methyl-1-(3-pyridyl)propylchlorid,
4-Picolylchlorid,
5-Chloro-2-pyridylmethylchlorid,
2-Fluoro-5-pyridylmethylchlorid,
2-Chloro-5-pyridylmethylchlorid,
1-(2-Chloro-5-pyridyl)ethylchlorid,
2-Nitro-5-pyridylmethylchlorid,
2-Cyano-5-pyridylmethylchlorid,
2-Amino-5-pyridylmethylchlorid,
2-Acetamido-5-pyridylmethylchlorid,
2-Dimethylamino-5-pyridylmethylchlorid,
2-Ethoxycarbonyl-5-pyridylmethylchlorid,
2-Acetyl-5-pyridylmethylchlorid,
2-Chloro-3-methyl-5-pyridylmethylchlorid,
2-Difluoromethyl-5-pyridylmethylchlorid,
5-Trifluoromethyl-2-pyridylmethylchlorid,
2-Bromodifluoromethyl-5-pyridylmethylchlorid,
2-Chlorodifluoromethyl-5-pyridylmethylchlorid,
2-Trichloromethyl-5-pyridylmethylchlorid,
2-(2-Chloroethyl)-5-pyridylmethylchlorid,
2-(2-Fluoroethyl)-5-pyridylmethylchlorid,
2-(2,2,2-Trifluoroethyl)-5-pyridylmethylchlorid,
2-Difluoroethoxy-5-pyridylmethylchlorid,
2-Trifluoroethoxy-5-pyridylmethylchlorid,
2-(2,2,2-Trifluoroethoxy)-5-pyridylmethylchlorid,
2-(Trifluoromethylthio)-5-pyridylmethylchlorid,
2-Formyl-5-pyridylmethylchlorid,
2-Chlorodifluoromethylthio-5-pyridylmethylchlorid,

2-(2,2-Dichlorovinyl)-5-pyridylmethylchlorid,
5-Trifluoromethyl-2-pyridylmethylchlorid,
5-Methyl-2-pyridylmethylchlorid,
6-Methyl-2-pyridylmethylchlorid,
4-Methyl-2-pyridylmethylchlorid,
5-Ethyl-2-pyridylmethylchlorid,
5-Butyl-2-pyridylmethylchlorid,
4,6-Dimethyl-2-pyridylmethylchlorid,
3-Chloro-2-pyridylmethylchlorid,
3,5-Dichloro-2-pyridylmethylchlorid,
5-Fluoro-2-pyridylmethylchlorid,
6-Bromo-2-pyridylmethylchlorid,
6-Chloro-4-methyl-2-pyridylmethylchlorid,
5-Methyl-3-pyridylmethylchlorid,
2-Methyl-5-pyridylmethylchlorid,
2-Chloro-3-pyridylmethylchlorid,
5-Chloro-3-pyridylmethylchlorid,
5-Bromo-3-pyridylmethylchlorid,
2-Bromo-5-pyridylmethylchlorid,
5-Fluoro-3-pyridylmethylchlorid,
2-Fluoro-5-pyridylmethylchlorid,
1-(2-Fluoro-5-pyridyl)ethylchlorid,
2-Methyl-1-(2-fluoro-5-pyridyl)propylchlorid,
2-Chloro-6-methyl-3-pyridylmethylchlorid,
2,4-Dichloro-5-pyridylmethylchlorid,
2,6-Dichloro-5-pyridylmethylchlorid,
2,4-Dibromo-5-pyridylmethylchlorid,
2,4-Difluoro-5-pyridylmethylchlorid,
2-Methoxy-3-pyridylmethylchlorid,
2-Methoxy-5-pyridylmethylchlorid,
2-Ethoxy-5-pyridylmethylchlorid,
2-Isopropoxy-5-pyridylmethylchlorid,
2-Methylthio-3-pyridylmethylchlorid,

2-Methylthio-5-pyridylmethylchlorid,
4-Methyl-2-methylthio-5-pyridylmethylchlorid,
2-Ethylthio-5-pyridylmethylchlorid,
2-Methylsulfinyl-5-pyridylmethylchlorid,
2-Methylsulfonyl-5-pyridylmethylchlorid,
4-Chloro-2-fluoro-5-pyridylmethylchlorid,
6-Chloro-2-methyl-3-pyridylmethylchlorid,
2-Chloro-4-methyl-5-pyridylmethylchlorid,
2-Allyl-5-pyridylmethylchlorid,
2-Propargyl-5-pyridylmethylchlorid,
2,3-Dichloro-5-pyridylmethylchlorid,
2-(1-Propenyl)-5-pyridylmethylchlorid,
2-Chloro-4-pyridylmethylchlorid,
2-Fluoro-4-pyridylmethylchlorid,
2,6-Dichloro-4-pyridylmethylchlorid,
2,6-Difluoro-4-pyridylmethylchlorid,
2-Methyl-4-pyridylmethylchlorid,
1-(2-Chloro-4-pyridyl)ethylchlorid,
2-Chloro-6-methyl-4-pyridylmethylchlorid,
2,6-Dimethyl-4-pyridylmethylchlorid,
2-Bromo-4-pyridylmethylchlorid,
2,6-Dibromo-4-pyridylmethylchlorid,
3-Chloro-2-fluoro-5-pyridylmethylchlorid,
3-Bromo-2-fluoro-5-pyridylmethylchlorid,
2-Chloro-3-fluoro-5-pyridylmethylchlorid,
3-Chloro-2-methylthio-5-pyridylmethylchlorid,
2-Ethyl-5-pyridylmethylchlorid,
2-Phenyl-5-pyridylmethylchlorid,
2-Benzyl-5-pyridylmethylchlorid,
2-Phenoxy-5-pyridylmethylchlorid,
2-(2-Ethoxyethyl)-5-pyridylmethylchlorid,
2-Methoxymethyl-5-pyridylmethylchlorid,
2-Difluoromethoxy-5-pyridylmethylchlorid,

2-(2,2,2-Trifluoroethoxy)-5-pyridylmethylchlorid,
2-Trifluoromethylsulfonyl-5-pyridylmethylchlorid,
2-Trifluoromethylsulfinyl-5-pyridylmethylchlorid,
3-Furylmethylchlorid,
Furfurylchlorid,
5-Methylfurfurylchlorid,
2-Thienylmethylchlorid,
4-Imidazolylmethylchlorid,
4-Methyl-5-imidazolylmethylchlorid,
Tetrahydrofurfurylchlorid,
5-Methyltetrahydrofurfurylchlorid,
3-Thienylmethylchlorid,
2-Pyrrolylmethylchlorid,
1-Methyl-2-pyrrolylmethylchlorid,
5-Methyl-2-thienylmethylchlorid,
5-Bromo-2-thienylmethylchlorid,
5-Cyanofurfurylchlorid,
5-Trifluoromethylthio-2-thienylmethylchlorid,
1-(2-Thienyl)ethylchlorid,
5-Methyl-3-isoxazolylmethylchlorid,
5-Isoxazolylmethylchlorid,
4-Isoxazolylmethylchlorid,
3-Methyl-5-isoxazolylmethylchlorid,
3-Trifluoromethyl-5-isoxazolylmethylchlorid,
3-Chloro-5-isoxazolylmethylchlorid,
5-Isothiazolylmethylchlorid,
5-Pyrazolylmethylchlorid,
4-Pyrazolylmethylchlorid,
1-Methyl-4-pyrazolylmethylchlorid,
1-(1-Methyl-4-pyrazolyl)ethylchlorid,
1-Ethyl-4-pyrazolylmethylchlorid,
1-Isopropyl-4-pyrazolylmethylchlorid,
1-Allyl-4-pyrazolylmethylchlorid,

1-tert-Butyl-4-pyrazolylmethylchlorid,
1-(2,2,2-Trifluoroethyl)-5-pyrazolylmethylchlorid,
3-Methyl-5-pyrazolylmethylchlorid,
3-Chloro-2-methyl-5-pyrazolylmethylchlorid,
2,3,5-Trimethyl-4-pyrazolylmethylchlorid,
5-Oxazolylmethylchlorid,
4-Methyl-5-oxazolylmethylchlorid,
4-Thiazoylmethylchlorid,
5-Thiazoylmethylchlorid,
2-Methyl-5-thiazoylmethylchlorid,
2-Chloro-4-thiazoylmethylchlorid,
2-Chloro-5-thiazoylmethylchlorid,
2-Trifluoromethyl-5-thiazolylmethylchlorid,
2-Bromo-5-thiazolylmethylchlorid,
2,4-Dichloro-5-thiazolylmethylchlorid,
4-Imidazolylmethylchlorid,
1-Methyl-2-imidazolylmethylchlorid,
1,2,4-Triazol-5-ylmethylchlorid,
1-Methyl-1,2,4-triazol-5-ylmethylchlorid,
1,2,5-Thiadiazol-4-ylmethylchlorid,
1,2,3-Thiadiazol-5-ylmethylchlorid,
3-Methyl-1,2,4-oxadiazol-5-ylmethylchlorid,
1,3-Dioxolan-2-ylmethylchlorid,
2,2-Dimethyl-1,3-dioxolan-4-ylmethylchlorid,
2-Methyl-2-oxazolin-5-ylmethylchlorid,
2-Trifluoromethyl-2-oxazolin-5-ylmethylchlorid,
3-Pyrrolylmethylchlorid,
1-Ethyl-2-pyrrolylmethylchlorid,
1-Methyl-3-pyrrolylmethylchlorid,
5-Methyl-3-thienylchlorid,
5-Methyl-3-pyrrolylmethylchlorid,
1,5-Dimethyl-3-pyrrolylmethylchlorid,
2,5-Dimethyl-3-furylmethylchlorid,

2,5-Dimethyl-3-thienylmethylchlorid,
5-Fluoro-3-furylmethylchlorid,
4-Chlorofurfurylchlorid,
5-Chlorofurfurylchlorid,
5-Chloro-3-furylmethylchlorid,
5-Chloro-3-thienylmethylchlorid,
5-Chloro-1-methyl-3-pyrrolylmethylchlorid,
5-Bromo-3-furylmethylchlorid,
5-Nitrofurfurylchlorid,
4-Nitro-2-thienylmethylchlorid,
5-Nitro-2-thienylmethylchlorid,
1-Methyl-5-nitro-3-pyrrolylmethylchlorid,
5-Cyano-3-furylmethylchlorid,
5-Cyano-3-thienylmethylchlorid,
5-Cyano-1-methyl-3-pyrrolylmethylchlorid,
5-Trifluoromethylfurfurylchlorid,
5-Difluoromethylfurfurylchlorid,
5-Trifluoromethyl-3-thienylmethylchlorid,
1-Methyl-5-trifluoromethyl-3-pyrrolylmethylchlorid,
5-Methoxy-2-thienylmethylchlorid,
5-Methylfurfurylchlorid,
2,5-Dimethylthio-3-thienylmethylchlorid,
5-Trifluoromethylthiofurfurylchlorid,
5-(2,2-Dichlorovinyl)-2-thienylmethylchlorid,
5-Ethoxycarbonylfurfurylchlorid,
5-Formyl-2-thienylmethylchlorid,
3-Isoxazolylmethylchlorid,
4-Isoxazolylmethylchlorid,
3-Ethyl-5-isoxazolylmethylchlorid,
3-Isopropyl-5-isoxazolylmethylchlorid,
3-Fluoro-5-isoxazolylmethylchlorid,
3-Bromo-5-isoxazolylmethylchlorid,
3-Hydroxy-5-isoxazolylmethylchlorid,

3-Nitro-5-isoxazolylmethylchlorid,
3-Cyano-5-isoxazolylmethylchlorid,
3-Difluoromethyl-5-isoxazolylmethylchlorid,
3-Chlorodifluoromethyl-5-isoxazolylmethylchlorid,
3-Methoxymethyl-5-isoxazolylmethylchlorid,
3-Isopropoxymethyl-5-isoxazolylmethylchlorid,
3-Trichloromethyl-5-isoxazolylmethylchlorid,
3-Methoxy-5-isoxazolylmethylchlorid,
3-Trifluoromethoxy-5-isoxazolylmethylchlorid,
2,5-Dimethyl-4-isoxazolylmethylchlorid,
3-Isothiazolylmethylchlorid,
4-Isothiazolylmethylchlorid,
3-Pyrazolylmethylchlorid,
1-(4-Pyrazolyl)ethylchlorid,
1-Methyl-3-pyrazolylmethylchlorid,
1-Methyl-5-pyrazolylmethylchlorid,
1-Propyl-4-pyrazolylmethylchlorid,
1-(2,2,2-Trifluoroethyl)-3-pyrazolylmethylchlorid,
5-Chloro-1-ethyl-3-pyrazolylmethylchlorid,
5-Chloro-1-isopropyl-3-pyrazolylmethylchlorid,
3-Chloro-1-methyl-5-pyrazolylmethylchlorid,
5-Trifluoromethyl-3-pyrazolylmethylchlorid,
1-Methyl-5-trifluoromethyl-3-pyrazolylmethylchlorid,
1-Methyl-3-trifluoromethyl-5-pyrazolylmethylchlorid,
4-Oxazolylmethylchlorid,
2-Methyl-4-oxazolylmethylchlorid,
2-Methyl-5-oxazolylmethylchlorid,
2-Fluoro-5-oxazolylmethylchlorid,
2-Chloro-5-oxazolylmethylchlorid,
2-Trifluoromethyl-5-oxazolylmethylchlorid,
2-Methylthio-5-oxazolylmethylchlorid,
2-Trifluoromethoxy-5-oxazolylmethylchlorid,
2,4-Dimethyl-5-oxazolylmethylchlorid,

5-Ethoxycarbonyl-2-oxazolylmethylchlorid,
1-(5-Thiazolyl)ethylchlorid,
2-Methyl-4-thiazolylmethylchlorid,
1-(2-Methyl-5-thiazolyl)ethylchlorid,
2-Ethyl-5-thiazolylmethylchlorid,
2-Isopropyl-5-thiazolylmethylchlorid,
4-Methyl-5-thiazolylmethylchlorid,
2-Fluoro-4-thiazolylmethylchlorid,
2-Fluoro-5-thiazolylmethylchlorid,
1-(2-Chloro-5-thiazolyl)ethylchlorid,
2-Nitro-4-thiazolylmethylchlorid,
2-Nitro-5-thiazolylmethylchlorid,
2-Cyano-4-thiazolylmethylchlorid,
2-Cyano-5-thiazolylmethylchlorid,
2-Methylthio-4-thiazolylmethylchlorid,
2-Mercapto-5-thiazolylmethylchlorid,
2-Methylthio-5-thiazolylmethylchlorid,
2-Difluoromethylthio-5-thiazolylmethylchlorid,
2-Trifluoromethylthio-5-thiazolylmethylchlorid,
2-Chlorodifluoromethylthio-5-thiazolylmethylchlorid,
2-(2,2,2-Trifluoroethylthio)-5-thiazolylmethylchlorid,
2-/¯2-(2,3,3-Trichloro)propenylthio_7-thiazolylmethylchlorid,
2-Thiocyanato-5-thiazolylmethylchlorid,
2-Amino-4-thiazolylmethylchlorid,
2-Acetamino-4-thiazolylmethylchlorid,
2-Methoxy-4-thiazolylmethylchlorid,
2-Methoxy-5-thiazolylmethylchlorid,
2-Trifluoromethoxy-5-thiazolylmethylchlorid,
2-Difluoromethoxy-5-thiazolylmethylchlorid,
2-Chloromethyl-5-thiazolylmethylchlorid,
2-Difluoromethyl-5-thiazolylmethylchlorid,
2-Trifluoromethyl-5-thiazolylmethylchlorid,

2-(1,1,2,2-Tetrafluoroethyl)-5-thiazolylmethylchlorid,
2-Cyclopropyl-5-thiazolylmethylchlorid,
2-Imidazolylmethylchlorid,
1-Methyl-5-imidazolmethylchlorid,
2-Fluoro-4-imidazolmethylchlorid,
2-Chloro-4-imidazolmethylchlorid,
4-Nitro-2-imidazolmethylchlorid,
2-Trifluoromethylthio-4-imidazolmethylchlorid,
1,2-Dimethyl-4-imidazolmethylchlorid,
1-Methyl-2-trifluoromethyl-4-imidazolmethylchlorid,
1-Methyl-1,2,3-triazol-4-ylmethylchlorid,
1-(1-Methyl-1,2,3-triazol-4-yl)ethylchlorid,
3-Methyl-1,2,4-triazol-5-ylmethylchlorid,
3-Trifluoromethyl-1,2,4-triazol-5-ylmethylchlorid,
1,2,4-Oxadiazol-5-ylmethylchlorid,
1,3,4-Oxadiazol-2-ylmethylchlorid,
1,2,3-Oxadiazol-5-ylmethylchlorid,
3-Trifluoromethyl-1,2,4-oxadiazol-5-ylmethylchlorid,
2-Methyl-1,3,4-oxadiazol-5-ylmethylchlorid,
2-Trifluoromethyl-1,3,4-oxadiazol-5-ylmethylchlorid,
2-(2,2,2-Trifluoroethyl)-1,3,4-oxadiazol-5-ylmethylchlorid,
1,2,4-Thiadiazol-5-ylmethylchlorid,
1,2,3-Thiadiazol-4-ylmethylchlorid,
1-(1,2,3-Thiadiazol-5-yl)ethylchlorid,
1,3,4-Thiadiazol-2-ylmethylchlorid,
1-(1,3,4-Thiadiazol-2-yl)ethylchlorid,
1,2,5-Thiadiazol-3-ylmethylchlorid,
3-Methyl-1,2,4-thiadiazol-5-yl)ethylchlorid,
4-Methyl-1,2,3-thiadiazol-5-yl)ethylchlorid,
2-Methyl-1,3,4-thiadiazol-5-yl)ethylchlorid,
2-Trifluoromethyl-1,3,4-thiadiazol-5-yl)ethylchlorid,
2-Fluoro-1,3,4-thiadiazol-5-yl)ethylchlorid,

2-Chloro-1,3,4-thiadiazol-5-yl)ethylchlorid,
3-Chloro-1,2,5-thiadiazol-4-yl)ethylchlorid,
1-(3-Tetrahydrofuryl)ethylchlorid,
3-Tetrahydrothienylmethylchlorid,
1-(3-Tetrahydrothienyl)ethylchlorid,
1-Methyl-3-pyrrolidinylmethylchlorid,
1,3-Oxathiolan-2-ylmethylchlorid,
1,3-Dioxolan-4-ylmethylchlorid,
1,3-Oxathiolan-4-ylmethylchlorid,
1,3-Dithiolan-4-ylmethylchlorid,
Thiazolidin-5-ylmethylchlorid,
4-Methyl-1,3-dioxolan-2-ylmethylchlorid,
2-Methyl-1,3-oxathiolan-4-ylmethylchlorid,
2-Chloromethyl-1,3-dioxolan-4-ylmethylchlorid,
2-Trifluoromethyl-1,3-dioxolan-4-ylmethylchlorid,
2-Oxo-1,3-dioxolan-4-ylmethylchlorid,
3-Formyl-thiazolidin-5-ylmethylchlorid,
3-Acetyl-thiazolidin-5-ylmethylchlorid,
3-Thiolen-5-ylmethylchlorid,
2H,5H-1,1-Dioxo-3-thiolen-3-ylmethylchlorid,
2-Isoxazolin-5-ylmethylchlorid,
3-Methyl-2-isoxazolin-5-ylmethylchlorid,
3-Trifluoromethyl-2-isoxazolin-5-ylmethylchlorid,
3-(2,2,2-Trifluoroethyl)-2-isoxazolin-5-ylmethylchlorid,
2,4-Dimethyl-2-oxazolin-4-ylmethylchlorid,
2-Methyl-1,3-thiazolin-4-ylmethylchlorid,
2-Oxazolidon-5-ylmethylchlorid,
3-Methyl-2-thiazolidinon-5-ylmethylchlorid,
2-Methylamino-5-thiazolylmethylchlorid,
2-Trifluoroacetamido-5-thiazolylmethylchlorid,
3-Methyl-2-thiooxo-thiazolidin-5-ylmethylchlorid,
3-Chloro-1,2,4-oxadiazol-5-ylmethylchlorid,

5-Carboxy-2-oxazolylmethylchlorid,
2-Dimethylamino-5-thiazolylmethylchlorid,
5-Phenoxyfurfurylchlorid,
1-Phenyl-4-pyrazolylmethylchlorid,
1-Benzyl-4-pyrazolylmethylchlorid,
2-Phenyl-4-thiazolylmethylchlorid,
1-Benzyl-2-imidazolylmethylchlorid,
2-Methylsulfinyl-5-thiazolylmethylchlorid,
2-Methylsulfonyl-5-thiazolylmethylchlorid,
2-Carbamoyl-5-thiazolylmethylchlorid,
2-Methylaminocarbonyl-5-thiazolylmethylchlorid,
2-Dimethylaminocarbonyl-5-thiazolylmethylchlorid,
2-Methylthio-1,3,4-thiadiazol-2-ylmethylchlorid,
2-Methylsulfonyl-1,3,4-thiadiazol-2-ylmethylchlorid,
4-Pyrimidinylmethylchlorid,
2-Methyl-6-oxo-1H,6H-dihydropyrimidin-4-ylmethylchlorid,
5-Pyrimidinylmethylchlorid,
2-Methyl-5-pyrimidinylmethylchlorid,
2-Dimethylamino-5-pyrimidinylmethylchlorid,
2,4,6-Trichloro-5-pyrimidinylmethylchlorid,
Pyrazinylmethylchlorid,
1-(Pyrazinylethyl)chlorid,
2-Methyl-5-pyrazinylmethylchlorid,
3-Pyridazinylmethylchlorid,
2-Chloro-4-pyrimidinylmethylchlorid,
4-Chloro-6-pyrimidinylmethylchlorid,
4-Methyl-6-pyrimidinylmethylchlorid,
2-Fluoro-5-pyrimidinylmethylchlorid,
1-(2-Fluoro-5-pyrimidinyl)ethylchlorid,
2-Chloro-5-pyrimidinylmethylchlorid,
2-Isopropyl-5-pyrimidinylmethylchlorid oder -bromid,
2-Chlorodifluoromethyl-5-pyrimidinylmethylchlorid,

2-Trifluoromethyl-5-pyrimidinylmethylchlorid,
2-Bromodifluoromethyl-5-pyrimidinylmethylchlorid,
2-Methoxy-5-pyrimidinylmethylchlorid,
2-Difluoromethoxy-5-pyrimidinylmethylchlorid,
2-Trifluoromethoxy-5-pyrimidinylmethylchlorid,
2-(2,2,2-Trifluoroethoxy)-5-pyrimidinylmethylchlorid,
2-Methylthio-5-pyrimidinylmethylchlorid,
2-Ethylthio-5-pyrimidinylmethylchlorid,
2-Difluoroethylthio-5-pyrimidinylmethylchlorid,
2-Trifluoromethylthio-5-pyrimidinylmethylchlorid,
2-Nitro-5-pyrazinylmethylchlorid,
2-Cyano-5-pyrazinylmethylchlorid,
2-Chloro-5-pyrazinylmethylchlorid,
2-Trifluoromethyl-5-pyrazinylmethylchlorid,
3-Fluoro-6-pyridazinylmethylchlorid,
3-Methyl-6-pyridazinylmethylchlorid,
4-Pyridazinylmethylchlorid,
3-Chloro-6-pyridazinylmethylchlorid,
3-Trifluoromethyl-6-pyridazinylmethylchlorid,
1,3,5-Triazin-2-ylmethylchlorid,
3-Chloro-1,2,3-triazin-6-ylmethylchlorid,
3,5-Dichloro-1,2,3-triazin-6-ylmethylchlorid und
3-Chloro-1,2,4,5-tetrazin-6-ylmethylchlorid.

An Stelle der im Vorstehenden aufgeführten Chloride können beispielhaft auch die Bromide oder die p-Toluolsulfonate genannt werden. Sie werden im Folgenden speziell beschrieben.

Die obigen Halogenide, beispielsweise die Chloride, können ohne Schwierigkeiten durch Chlorierung der entsprechenden Alkohole mit Thionylchlorid hergestellt werden.

Beispielsweise kann 2-Chloro-5-pyridylmethylchlorid durch Chlorierung von 2-Chloro-5-pyridylmethylalkohol mit Thionylchlorid erhalten werden (siehe J. Org. Chem. 34, 3547).

Die Bromide können ebenfalls durch Bromierung einer Methyl-Gruppe in der Seitenkette mit N-Bromosuccinimid hergestellt werden.

Einige der trifluoromethylsubstituierten oder trifluoromethoxysubstituierten Pyridylalkohole sind in J. Med. Chem. 13, S. 1124-1130, beschrieben. Unter Anwendung dieser Arbeitstechniken der Synthese wird 2-Methyl-5-trifluoromethyl-pyridin, das durch Reaktion von 6-Methylnicotinsäure mit Flußsäure und Schwefeltetrafluorid erhalten wird, in das N-Oxid überführt, die Umlagerungs-Reaktion des N-Oxids vermag 5-Trifluormethyl-2-pyridylmethylalkohol zu liefern.

Diese Reaktion läßt sich auch auf die Synthese von 5-Methyl-2-trifluoromethylpyridin aus 5-Methylpicolinsäure anwenden. 2-Trifluoromethyl-5-pyridylmethylbromid (oder -chlorid), die gewünschte Ausgangssubstanz, kann durch Mono-Halogenierung des oben erwähnten 5-Methyl-2-trifluoromethylpyridins mit N-Bromosuccinimid oder N-Chlorosuccinimid synthetisiert werden.

2-Trifluoromethoxy-5-pyridylmethylbromid (oder -chlorid) kann in ähnlicher Weise durch Reaktion von 5-Methyl-2-trifluoromethoxypyridin, gewonnen aus 2-Hydroxy-5-methylpyridin, mit N-Bromosuccinimid oder N-Chlorosuccinimid erhalten werden.

Da das Halogen in der o-Stellung des Pyridin-Rings aktiv ist, kann ein 2-Halogenoalkoxy-5-pyridylmethylalkohol beispielsweise durch die Reaktion von 6-Chloronicotinsäure mit einem Überschuß and Natriumalkoxid erhalten werden. synthetisiert werden. Die Reduktion dieser Verbindung vermag den Ausgangsstoff, den 2-Halogenoalkoxy-5-pyridylmethylalkohol, zu liefern.

Halogenomethylsubstituierte Furane und Thiophene sind bekannte Verbindungen. Beispielsweise ist 2-Ethoxycarbonyl-5-chloromethylfuran eine bekannte Verbindung, die in Liebigs Annalen der Chemie 580, S. 176, beschrieben ist. 2-Bromomethyl-5-trifluoromethylfuran wird erhalten durch Halogenierung der Seitenkette von 2-Methyl-5-trifluoromethylfuran mit einem Halogenierungsmittel wie N-Bromosuccinimid (NBS) (US-PS 3 442 913).

Viele bromsubstituierte heterocyclische Verbindung lassen sich durch Bromierung der entsprechenden methylsubstituierten heterocyclischen Verbindungen mit N-Bromosuccinimid gewinnen.

Halogenomethylsubstituierte Isoxazole können erhalten werden durch Halogenierung von Methylisoxazolen mit NBS, oder Hydroxymethylisoxazol läßt sich mit Hilfe von Thionylchlorid leicht in Chloromethylisoxazol überführen. Beispielsweise ist 5-Bromomethylisoxazol eine in der DE-OS 2 716 687 beschriebene Verbindung, und 4-Bromomethylisoxazol ist eine in Chem. Abstr. 65, 2242h, beschriebene Verbindung.

Chloromethylsubstituierte heterocyclische Verbindungen können vermittels einer Chloromethylierungs-Reaktion

synthetisiert werden. 4-Chloromethylisoxazol und 4-Chloromethyl-3,5-dimethylisoxazol, die in Zh. Obshch. Khim. 34, S. 4010-4015, beschrieben sind, sind hierfür gute Beispiele. Weiterhin können halogenomethylsubstituierte Isoxazole auch durch direkte Ring-Synthese hergestellt werden. 3-Bromo-5-bromomethylisoxazol (Rend. Ist. Lombaro Sci. Pt. I. Classe Sci. Mat. e Nat. 94, S. 729-740) und 5-Bromomethyl-3-methylisoxazol (JP-OS 59 156/1977) sind hierfür gute Beispiele.

5-Chloro-3-trifluoromethylisoxazol kann durch Synthetisieren von 3-Trifluoromethyl-5-hydroxymethylisoxazol und Chlorieren desselben mit Thionylchlorid gemäß der Beschreibung in Bull. Chem. Soc. Japan 57, S. 2184-2187, erhalten werden. Die Verwendung anderer Halogenoalkyle an Stelle von Trifluoromethyl in der obigen Reaktion kann zur Synthese der entsprechenden Halogenoalkyl-isoxazole führen. Wie im Vorstehenden angegeben wurde, lassen sich Halogenoalkyl-heterocyclische Verbindung in einfacher Weise allgemein durch Behandeln der entsprechenden Alkohole mit Halogenierungsmitteln erhalten werden, für die Thionylchlorid ein typisches Beispiel ist.

5-Chloromethyl-3-hydroxyisoxazol wird nach der Beschreibung in Tetrahedron Letters 1965, No. 25, S. 2077-2079, synthetisiert. Chlorierung desselben mit Phosphonylchlorid etc. vermag 3-Chloro-5-chloromethylisoxazol zu liefern.

Halogensubstituierte halogenomethylsubstituierte Isothiazole lassen sich beispielsweise dadurch erhalten,

daß halogensubstituierte methylsubstituierte Isothiazole mit Halogenierungsmitteln wie NBS in halogensubstituierte bromomethylsubstituierte Isothiazole überführt werden. 5-Bromo-3-bromomethylisothiazol (beschrieben in J. Chem. Soc. 1965, S. 7274-7276) ist ein gutes Beispiel.

4-Chloromethylpyrazol kann leicht durch Chlorieren von 4-Hydroxymethylpyrazol mit Thionylchlorid erhalten werden (J. Amer. Chem. Soc. 71, S. 3994-4000).

Was die Halogeno-halogenomethylpyrazole betrifft, so wird beispielsweise 3-Ethoxycarbonyl-5-hydroxy-1-methylpyrazol gemäß den Angaben in Chem. Pharm. Bull. 31, No. 4, S. 1228-1234, synthetisiert. Die nachfolgende Chlorierung mit Phosphonylchlorid ergibt 5-Chloro-1-methyl-3-pyrazolylcarbonylchlorid. Die Reduktion des Chlorids mit Natriumborhydrid liefert 5-Chloro-3-hydroxymethyl-1-methylpyrazol. Die Chlorierung dieses Produkts in üblicher Weise kann zu 5-Chloro-3-chloromethyl-1-methylpyrazol führen.

Was die halogenomethylsubstituierten Oxazole betrifft, so kann durch Chlorierung vpn Hydroxymethyloxazol mit Thionylchlorid etc. Chloromethyloxazol erhalten werden. Die betreffenden Verbindungen können auch durch direkte Ring-Synthese hergestellt werden.

Beispielsweise ist 5-Bromomethyl-2-methyloxazol eine bekannte Verbindung (J. Amer. Chem. Soc. 104, S. 4461-4465), und 2-Bromoethyl-5-ethoxycarbonyloxazol ist ebenfalls eine bekannte Verbindung (JP-OS 108 771/1984).

4-Halogenomethylthiazole lassen sich direkt synthetisieren, beispielsweise durch Reaktion von Dihalogenoacetonen mit Thioacylamiden wie Thioacetamid (J. Amer. Chem. Soc. 56, S. 470-471 und ibid. 73, S. 2936).

5-Halogenomethylthiazole lassen sich erhalten durch Reaktion eines Thioacylamids mit α-Chloro-α-formylethylacetat, Reduktion des erhaltenen 5-Ethoxycarbonylthiazols mit Lithiumaluminiumhydrid in üblicher Weise und Halogenierung des erhaltenen 5-Hydroxymethylthiazols. 5-Chloromethyl-2-methylthiazol, das in Zh. Obshch. Khim. 32, S. 570-575, und in J. Amer. Chem. Soc. 104, S. 4461-4465, beschrieben ist, ist ein gutes Beispiel.

Die Reaktion von Thioharnstoff an Stelle des Thioacylamids vermag 2-Amino-4-chloromethyl- oder 2-Amino-5-chloromethylthiazol zu liefern, und über eine Diazotierung kann weiterhin ein Halogen-Atom etc. eingeführt werden. Dieses Halogen ist aktiv und kann mit Hilfe eines Natriumalkoxids in eine 2-Alkoxy-Gruppe umgewandelt werden (JP-OS 5972/1979 und J. Chem. Soc. Perkin I, 1982, S. 159-164).

2-Halogeno-4- oder -5-bromomethylthiazol kann durch Bromierung der 2-Halogeno-4- oder -5-methylthiazole mit NBS synthetisiert werden.

Der Einsatz von Ammoniumdithiocarbamat (Org. Synthesis, Coll. Vol. III, S. 763) an Stelle des oben genannten Thioacetamids vermag 4- oder 5-Halogenomethyl-2-mercaptothiazole zu liefern. Deren Alkylierung oder Halogenoalkylierung kann zu den 2-Alkylthio-4- oder -5-halogenomethylthiazolen oder 2-substituierten 4- oder

5-Halogenomethylthiazolen führen (J. Amer. Chem. Soc. 75, S. 102-103).

Ein Halogenomethylimidazol, beispielsweise Chloromethylimidazol, kann erhalten werden durch Hydroxymethylierung eines N-Alkylimidazols mit Formaldehyd, gegebenenfalls Dealkylierung unter Bildung eines Hydroxymethylimidazols und Chlorierung mit Thionylchlorid etc. in üblicher Weise (beschrieben in J. Amer. Chem. Soc. 71, S. 383-386). 4-Hydroxymethylimidazol als ein Beispiel für die Hydroxymethylimidazole kann direkt aus Fructose, Formaldehyd und Ammoniak synthetisiert werden (beschrieben in Org. Synthesis, Coll. Vol. III, S. 460).

Hydroxymethyltriazol kann beispielsweise gemäß der in J. Amer. Chem. Soc. 77, S. 1538-1540, beschriebenen Arbeitsweise synthetisiert werden, und durch Chlorierung desselben kann Chloromethyltriazol erhalten werden.

Halogenomethyloxazole und Halogenomethylthiazole lassen sich jeweils synthetisieren durch Bromierung methylsubstituierter Oxazole bzw. methylsubstituierter Thiazole mit NBS. 3-Bromomethyl-1,2,5-thiazol, das in der JP-OS 24 963/1974 beschrieben ist, und 3-Bromomethyl-4-chloro-1,2,5-thiazol sind gute Beispiele hierfür. Die Halogenomethyloxadiazole und Halogenomethylthiadiazole können durch direkte Ring-Synthese aufgebaut werden. Neben anderen sind 5-Chloro-3-chloromethyl-1,2,4-thiadiazol, das in J. Org. Chem. 27, S. 2589-2592, beschrieben ist, 3-Chloro-5-chloromethyl-1,2,4-oxadiazol, das in der DE-OS 2 054 342 beschrieben ist, und

5-Chloromethyl-3-methyl-1,2,4-oxadiazol, das in Bull. Soc. Chim. Belges 73, S. 793-798, beschrieben ist, gute Beispiele hierfür. Das in der FR-PS 1 373 290 beschriebene, in der 2-Stellung substituierte 5-Chloromethyl-1,3,4-oxadiazol oder -thiadiazol ist ein anderes gutes Beispiel.

Chloromethylsubstituierte heterocyclische Verbindungen können synthetisiert werden durch Reduktion von Carbonsäuren oder Ester-Derivaten derselben mit Lithiumaluminiumhydrid zu ihrer Umwandlung in Alkohol-Derivate und anschließende Chlorierung der Alkohol-Derivate mit Thionylchlorid etc.. Als Beispiele hierfür beschreibt die JP-OS 89 633/1984 5-Chloromethyl-1,2,3-thiadiazol, 4-Chloromethyl-1-methyl-1,2,3,5-Tetrazol und 4-Chloromethyl-1-methyl-1,2,3-triazol.

Halogenoalkylsubstituierte gesättigte oder teilweise ungesättigte heterocyclische Verbindungen können in chloromethylsubstituierte Produkte beispielsweise durch Chlorierung ihrer Alkohol-Derivate in üblicher Weise umgewandelt werden.

Bromomethyldioxolan und Bromomethyloxothiolan können dadurch synthetisiert werden, daß Dimethylbromoacetal mit Ethylenglycol umgesetzt wird oder daß Dimethylbromoacetal mit 2-Mercaptoethanol umgesetzt wird oder daß ein Alkohol oder ein Keton mit Epibromohydrin umgesetzt wird. Beispielsweise ist 2-Bromomethyl-1,3-dioxolan in Beilstein, Band 19, II, S. 8, beschrieben.

Was chloromethylsubstituierte Oxazolin-Verbindungen betrifft, so kann 5-Chloromethyl-3-methyl-2-isoxazolin

gemäß den Angaben in Pak. J. Sci. Res. 30, S. 91-94, hergestellt werden. 5-Chloromethyl-3-trifluoromethyl-2-isoxazolin kann durch Chlorieren von 3-Trifluoromethyl-5-hydroxymethyl-2-isoxazolin, das in Bull. Chem. Soc. Japan 57, S. 2184-2187, beschrieben ist, in üblicher Weise hergestellt werden. 5-Chloromethyl-2-methyloxazolin ist eine Verbindung, die in Tetrahedron 34, S. 3537-3544, beschrieben ist.

4-Chloromethyl-2-methyl-2-thiazolin kann synthetisiert werden durch Chlorierung von 4-Hydroxymethyl-2-methyl-2-thiazolin, das in Heterocycles 4, S. 1687-1692, beschrieben ist. 5-Chloromethyl-3-methyl-2-oxazolidin-2-on ist eine Verbindung, die in der DE-OS 1 932 219 beschrieben ist. 3-Bromomethyl-1,1-dioxo-3-thiolen ist eine in der US-PS 4 561 764 beschriebene Verbindung und kann durch Bromierung mit NBS hergestellt werden.

5-Pyrimidinylmethylalkohol wird aus 5-Pyrimidincarbaldehyd erhalten, und 2-Chloro-5-pyrimidinylmethylalkohol wird aus 2-Chloro-5-pyrimidincarbaldehyd erhalten. 3-Pyridazinylmethylalkohol kann aus Furfurylacetat synthetisiert werden (Acta Chem. Scand. 1, S. 619). Was Pyrazinylalkylhalogenide betrifft, so können Methylpyrazin oder Dimethylpyrazin, das leicht zugänglich ist, mit Hilfe von N-Chlorosuccinimid in Chloromethylpyrazin überführt werden (Synthesis 1984, S. 676-679). Diese Reaktion läßt sich auch anwenden auf Methylpyrazine mit anderen Substituenten und auf halogensubstituierte Methylpyridazine wie 3-Chloro-6-methylpyridazin (beschrieben in J. Chem. Soc. 1947, S. 242), und mittels dieser Reaktion kann 3-Chloro-6-pyridazinylmethylchlorid synthetisiert werden. Weiterhin

läßt sich 2-Chloro-5-pyrimidinylmethylbromid gewinnen aus 2-Chloro-5-methylpyrimidin (Reacts. Sposobnost. Org. Soedin. 5, S. 824-837) und N-Bromsuccinimid.

Wie in J. Heterocycl. Chem. 19, S. 407, und in Chem. Pharm. Bull. 28, S. 3057 und 3063, beschrieben ist, kann Ethyl-2-chloropyrazin-5-carboxylat synthetisiert und zu dem entsprechenden Methanol (DE-OS 2 910 824) reduzieren.

Was Triazinylalkylhalogenide betrifft, so kann beispielsweise 1,3,5-Triazin-2-ylmethylchlorid durch Reaktion von 2-Methyl-1,3,5-triazin mit N-Chlorosuccinimid erhalten werden (J. Org. Chem. 29, S. 1527-1537). 3,5-Dichloro-6-methyl-1,2,4-triazin (beschrieben in J. Med. Chem. 10, S. 883-887) und 3-Chloro-6-methyl-1,2,4,5-tetrazin (J. Org. Chem. 46, S. 5102-5109) können in ähnlicher Weise durch Umsetzung mit N-Chlorosuccinimid chloriert werden.

In den Verbindungen der Formel (I), in denen X eine -NH- -Gruppe oder Y eine =CH- -Gruppe ist und die mittels der vorstehenden Verfahren (a), (b), (c), (d) (e) oder (f) erhalten werden können, kann jedes Wasserstoff-Atom der -NH- -Gruppe und der =CH- -Gruppe durch andere Gruppen substituiert sein oder an andere Gruppen addiert sein.

Als spezielle Beispiele können einige Verbindungen mit aktiven olefinischen Bindungen, etwa Methylvinylketon, Ethylacrylat und Acrylnitril, an das Wasserstoff-Atom der =CH- -Gruppe durch Michael-Reaktion angelagert werden (siehe die JP-OS 151 882/1975).

Darüber hinaus kann durch eine Mannich-Reaktion oder ähnliche Reaktionen spezifisch eine Dialkylaminomethyl-Gruppe am α-Kohlenstoff-Atom der Nitromethylen-Gruppe eingeführt werden, und ebenso können auch aktive Aldehyde wie Formaldehyd und Chloral addiert werden (siehe die JP-OS 151 882/1975).

Im übrigen kann jedes Wasserstoff-Atom der obigen -NH -Gruppe und =CH -Gruppe auch durch Halogenierungsmittel wie N-Chlorosuccinimid, N-Bromosuccinimid, Perchlorylfluorid und Halogen an sich halogeniert werden (siehe die US-PSen 3 933 809 und 3 962 233 und die JP-OS 54 532/1974).

In den obigen Fällen kann die Formel (I) die folgende Form

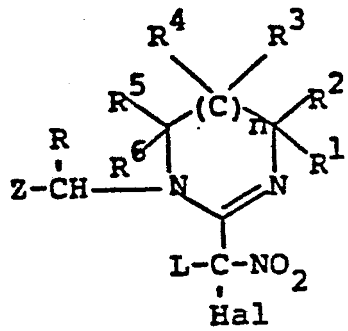

haben, worin n, $R^1$, $R^2$, $R^3$, $R^4$, $R^5$, $R^6$, R, Z und Hal die oben angegebenen Bedeutungen haben und L ein Halogen-Atom, eine Phenylthio-Gruppe oder eine Alkoxycarbonyl-Gruppe bezeichnet.

Glyoxalsäure und Dimethylformaldehyddimethylacetal können auch mit dem α-Kohlenstoff-Atom der Nitromethylen-Gruppe umgesetzt werden, und die Formel (I) kann die folgenden Formen annehmen

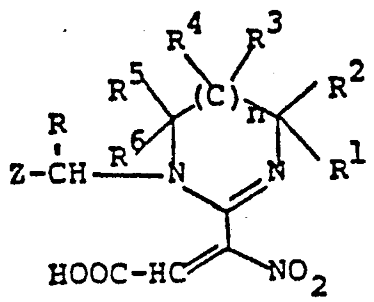 , 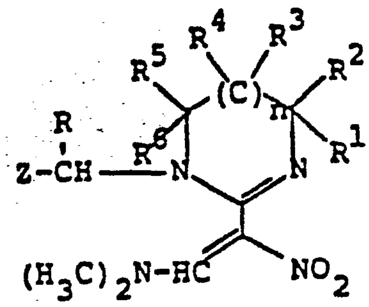 ,

worin n, $R^1$, $R^2$, $R^3$, $R^4$, $R^5$, $R^6$, R und Z die oben angegebenen Bedeutungen haben.

Das Stickstoff-Atom der obigen -NH -Gruppe und das α-Kohlenstoff-Atom der Nitromethylen-Gruppe können unabhängig acyliert, sulfinyliert oder sulfonyliert werden (siehe die NL-PS 7 306 145, die US-PSen 3 985 736, 3 996 372, 4 020 061, 4 022 775, 4 052 411, 4 053 662 und 4 076 813).

Acylisocyanate und Sulfonylisocyanate können mit dem α-Kohlenstoff-Atom der Nitromethylen-Gruppe zur Reaktion gebracht werden (siehe die US-PSen 4 013 766, 4 025 634, 4 029 791 und 4 034 091).

Das Stickstoff-Atom der obigen -NH -Gruppe kann auch alkyliert werden (siehe die BE-PS 821 282), und mit Hilfe dieser Reaktion kann die 3-Stellung der Imidazolidine oder Tetrahydropyrimidine alkyliert werden, um die entsprechenden Verbindungen der Formel (I) zu erhalten.

Bei der Durchführung des Verfahrens (a) können geeignete Verdünnungsmittel sämtliche inerten organischen Lösungsmittel sein.

Beispiele für solche Verdünnungsmittel umfassen Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Nitrile wie Acetonitril, Propionitril und Acrylnitril; Alkohole wie Methanol, Ethanol, Isopropanol, Butanol und Ethylenglycol; Säureamide wie Dimethylformamid und Dimethylacetamid; Sulfone und Sulfoxide wie Dimethylsulfoxid und Sulfolan; und Basen wie Pyridin.

Das Verfahren (a) kann in einem weiten Temperaturbereich durchgeführt werden. Im allgemeinen kann es bei einer Temperatur zwischen etwa -20°C und dem Siedepunkt der Mischung, vorzugsweise zwischen etwa 50°C und etwa 120°C, durchgeführt werden. Die Reaktion wird zweckmäßigerweise unter normalem Atmosphärendruck durchgeführt, jedoch ist es auch möglich, unter erhöhtem oder vermindertem Druck zu arbeiten.

In dem Verfahren (a) können die gewünschten neuen Verbindungen der Formel (I) beispielsweise dadurch erhalten werden, daß die Verbindungen der Formel (II) mit 1 bis etwa 1,2 mol, vorzugsweise 1 bis etwa 1,1 mol, der Verbindung der Formel (III) auf 1 mol der Verbindung der Formel (II) in einem inerten Lösungsmittel wie einem Alkohol (z.B. Methanol oder Ethanol) unter Rückfluß erhitzt werden, bis die Mercaptan-Entwicklung beendet ist.

Bei der Durchführung der Verfahren (b) und (c) können geeignete Verdünnungsmittel die oben beispielhaft für das Verfahren (a) genannten inerten Lösungsmittel sein. Als Basen seien beispielsweise Hydroxide, Carbonate, Hydrogencarbonate und Alkoholate von Alkalimetallen und auch tertiäre Amine wie Triethylamin, Diethylamin und Pyridin genannt.

Die Verfahren (b) und (c) können in einem weiten Temperaturbereich durchgeführt werden, im allgemeinen bei einer Temperatur zwischen etwa -20°C und dem Siedepunkt der Mischung, vorzugsweise zwischen etwa 0°C und etwa 50°C.

Die Reaktion wird vorzugsweise unter normalem Atmosphärendruck durchgeführt, jedoch ist es auch möglich, unter erhöhtem oder vermindertem Druck zu arbeiten.

In den vorstehenden Verfahren (b) und (c) können beispielsweise etwa 1 bis etwa 5 mol, vorzugsweise etwa 2 bis etwa 4 mol einer Base und etwa 0,9 bis etwa 4 mol, vorzugsweise etwa 1 bis etwa 3 mol, der Verbindungen der Formeln (IV) und (V) auf 1 mol der Verbindung der Formel (II) eingesetzt werden.

Bei der Durchführung des Verfahrens (d) können geeignete Verdünnungsmittel die oben beispielhaft für das Verfahren (a) genannten inerten Lösungsmittel sein.

Gemäß dem Verfahren (d) können die gewünschten Verbindungen der Formel (I) in einfacher Weise erhalten werden, beispielsweise durch Umsetzung von 1 mol der Verbindungen der Formel (II) mit 1 bis etwa 1,2 mol,

vorzugsweise 1 bis etwa 1,1 mol, Nitroguanidin unter Erhitzen in einem Wasser-Lösungsmittel.

Das Verfahren (d) kann beispielsweise bei einer Temperatur von etwa 0°C bis etwa 100°C, vorzugsweise etwa 30°C bis etwa 80°C, durchgeführt werden. Die Reaktion wird vorzugsweise unter normalem Atmosphärendruck durchgeführt, jedoch kann sie unter erhöhtem oder vermindertem Druck durchgeführt werden.

Bei der praktischen Durchführung des Verfahrens (e) werden die Verbindungen der Formel (VI) gewöhnlich vor der Reaktion in einer Säure wie konz. Schwefelsäure gelöst.

Bei der Verfahrensdurchführung werden die Verbindungen der Formel (VI) und rauchende Salpetersäure (mit einer Reinheit von wenigstens 98 %) zur Gewinnung der gewünschten Verbindungen der Formel (I) bei tiefen Temperaturen, vorzugsweise bei 0°C oder darunter, umgesetzt (unter Anwendung der Arbeitsweise der GB-Patentanmeldung 2 055 796).

Die in dem vorstehenden Verfahren eingesetzte Verbindung der Formel (VI) liegt, wie oben angegeben ist, als Folge ihrer Synthese im allgemeinen in der Form eines Hydrohalogenid-Salzes vor, und gewöhnlich wird sie vor ihrer Verwendung bei dem obigen Verfahren (e) in üblicher Weise neutralisiert.

Bei der praktischen Durchführung des Verfahrens (f) können geeignete Verdünnungsmittel die oben beispielhaft für das Verfahren (a) genannten inerten organischen Lösungsmittel sein. Als Basen seien beispislweise

Hydride wie Natriumhydrid und Kaliumhydrid und auch Hydroxide und Carbonate von Alkalimetallen genannt.

Das Verfahren (f) kann in einem weiten Temperaturbereich durchgeführt werden, im allgemeinen bei einer Temperatur zwischen etwa 0°C und etwa 100°C, vorzugsweise zwischen etwa 10°C und etwa 80°C.

Die Reaktion (f) wird vorzugsweise unter normalem Atmosphärendruck durchgeführt, jedoch ist es auch möglich, unter erhöhtem oder vermindertem Druck zu arbeiten.

In dem Verfahren (f) können die gewünschten Verbindungen der Formel (I) beispielsweise erhalten werden durch Umsetzung der Verbindungen der Formel (VII), in Anwesenheit von 1,1 bis etwa 1,2 mol Natriumhydrid als Base pro 1 mol der Verbindung (VII), mit 1 bis etwa 1,2 mol, vorzugsweise 1 bis etwa 1,1 mol, der Verbindungen der Formel (VIII) pro 1 mol der Verbindung (VII) in einem inerten Lösungsmittel wie Dimethylformamid. In dem Verfahren (f) wird für die Reaktion bevorzugt, die Verbindung der Formel (VII) vor der Reaktion mit Hilfe von Natriumhydrid in ihr Natrium-Salz zu überführen. Im Hinblick auf die Eigenschaften des Natriumhydrids wird eine solche Reaktion zweckmäßigerweise unter einer Atmosphäre von Stickstoff-Gas durchgeführt.

Die Verbindungen der Formel (I) gemäß der vorliegenden Erfindung umfassen Tautomere, wie dies durch nachstehenden Formel dargestellt ist:

Für den Fall, in dem X NH ist und Y CH ist:

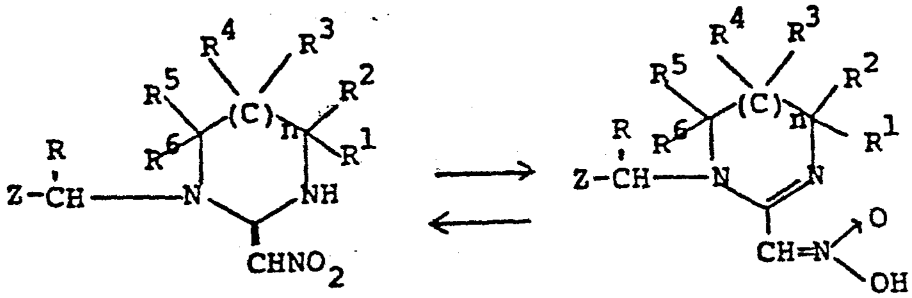

Für den Fall, in dem X NH ist und Y N ist:

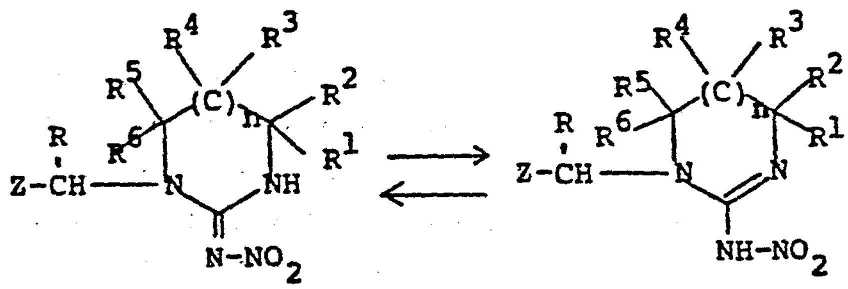

Darüber hinaus können diejenigen Verbindungen der Formel (I) in den Fällen, in denen Y C-$R^9$ ist, die entsprechenden E- und Z-Iosmeren umfassen.

Die Verbindungen der Formel (I) können auch in Form eines Salzes vorliegen. Beispiele für solche Salze sind Salze anorganischer Säuren, Sulfonat-Salze, Salze organischer Säuren und Metall-Salze.

Die erfindungsgemäßen aktiven Verbindungen zeigen potente insektizide Eigenschaften. Sie können aus diesem Grunde als Insektizide eingesetzt werden. Die aktiven Verbindungen können zur Bekämpfung und Ausrottung eines weiten Bereichs von Schädlingen verwendet

werden, darunter saugende Insekten, beißende Insekten und andere Pflanzenparasiten, Schädlinge in Getreidevorräten und gelagerten Körnerfrüchten und gesundheitsgefährdende Schädlinge.

Beispiele für die zu bekämpfenden Schädlinge sind im Folgenden angegeben.

<u>Insekten der Ordnung Coleoptera</u>

Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus;

<u>Insekten der Ordnung Lepidoptera</u>

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,

Galleria mellonella,
Plutella maculipennis und
Phyllocnistis citrella;

<u>Insekten der Ordnung Hemiptera</u>

Nephotettix cincticeps,
Nilaparvata lugens,
Pseudococcus comstocki,
Unaspis yanonensis,
Myzus persicae,
Aphis pomi,
Aphis gossypii
Rhopalosiphum pseudobrassicae,
Stephanitis nashi,
Nazara spp.,
Cimex lectularius,
Trialeurodes vaporariorum und
Psylla spp.;

<u>Insekten der Ordnung Orthoptera</u>

Blatella germanica,
Periplaneta americana,
Gryllotalpa africana und
Locusta migratoria migratoriodes;

<u>Insekten der Ordnung Isoptera</u>

Deucotermes speratus und
Coptotermes formosanus;

<u>Insekten der Ordnung Diptera</u>

Musca domestica,
Aedes aegypti,
Hylemia platura

0192060

Culex pipiens,
Anopheles sinensis und
Culex tritaeniorhynchus.

Auf dem Gebiet der Veterinärmedizin sind die neuen Verbindungen gemäß der vorliegenden Erfindung gegenüber verschiedenen schädlichen Tierparasiten (inneren und äußeren Parasiten) wie Insekten und Würmern wirksam. Beispiele für solche Tierparasiten sind nachstehend angegeben.

Insekten

Gastrophilus spp.,
Stomoxys spp.,
Trichodectes spp.,
Rhodnius spp. und
Ctenocephalides canis.

Substanzen mit pestizider Aktivität gegen Schädlinge, darunter sämtliche der oben beispielhaft angegebenen Species, können in der vorliegenden Anmeldung gelegentlich einfach als "Insektizide" bezeichnet sein.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz,

Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele

Beispiel 1

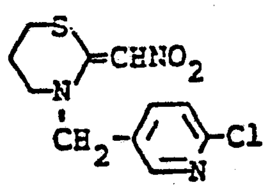

(Verbindung Nr. 1)

Eine Mischung aus N-(2-Chloro-5-pyridylmethyl)-3-aminopropanthiol (4,3 g), 1-Nitro-2,2-bis(methylthio)ethylen (3,3 g) und Ethanol (40 ml) wurde 10 h im Stickstoff-Strom unter Rückfluß erhitzt. Nach der Reaktion wurden etwa 2/3 des Ethanols unter vermindertem Druck abdestilliert. Nach und nach wurde zu der Reaktionsmischung Ether zugesetzt, um Kristalle auszufällen. Die Kristalle wurden durch Filtration gesammelt und mit einer Mischung aus Ethanol und Ether gewaschen, wonach das gewünschte 3-(2-Chloro-5-pyridylmethyl)-2-nitromethylentetrahydro-2H-1,3-thiazin (1,3 g) in Form gelber Kristalle erhalten wurde. Schmp. 164-166°C.

Beispiel 2

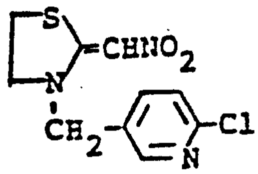

(Verbindung Nr. 2)

2-Nitromethylenthiazolidin (2,9 g) wurde in trockenem Acetonitril (30 ml) gelöst, und 60-proz. Natriumhydrid (0,9 g) wurde bei Raumtemperatur in einem Stickstoff-Strom hinzugefügt. Anschließend wurde die Mischung bei Raumtemperatur gerührt, bis die Wasserstoff-Antwicklung aufhörte. Dann wurde eine Lösung von 2-Chloro-5-pyridylmethylchlorid (3,2 g) in trockenem Acetonitril (5 ml) hinzugegeben, und die Mischung wurde 1 d bei Raumtemperatur gerührt. Danach wurde das Acetonitril unter vermindertem Druck abdestilliert, und zu dem Rückstand wurde Dichloromethan hinzugegeben. Die Mischung wurde dann mit Wasser gewaschen. Dichloromethan wurde aus der Dichloromethan-Schicht abdestilliert. Das zurückbleibende Öl wurde durch Chromatographie an einer Silicagel-Säule gereinigt, wonach das gewünschte 3-(2-Chloro-5-pyridylmethyl)-2-nitromethylenthiazolidin (1,6 g) erhalten wurde. Schmp. 177-179°C.

Beispiel 3

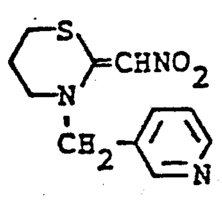

(Verbindung Nr. 3)

Eine Mischung aus N-(3-Pyridylmethyl)-3-aminopropanthiol (1,8 g), 1-Nitro-2,2-bis(methylthio)ethylen (1,7 g) und Ethanol (40 ml) wurde 5 h im Stickstoff-Strom unter Rückfluß erhitzt. Nach der Reaktion wurden etwa 2/3 des Ethanol-Volumens unter vermindertem Druck abdestilliert. Nach und nach wurde zu der Reaktionsmischung Ether zugesetzt, um Kristalle auszufällen. Die Kristalle wurden durch Filtration gesammelt und mit einer Mischung aus Ethanol und Ether gewaschen, wonach das gewünschte 3-(3-Pyridylmethyl)-2-nitromethylen-tetrahydro-2H-1,3-thiazin (1,2 g) erhalten wurde. Schmp. 143-146°C.

Beispiel 4

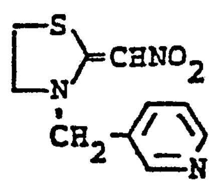

(Verbindung Nr. 4)

2-Nitromethylenthiazolidin (2,9 g) wurde in trockenem Acetonitril (30 ml) suspendiert, und 60-proz. Natriumhydrid (0,9 g) wurde in einem Stickstoff-Strom hinzugefügt. Anschließend wurde die Mischung bei Raumtemperatur gerührt, bis die Wasserstoff-Entwicklung aufhörte. Dann wurde eine Lösung von 3-Picolylchlorid (3,2 g) in trockenem Acetonitril (5 ml) hinzugegeben, und die Mischung wurde 3 h bei Raumtemperatur gerührt. Danach wurde das Acetonitril unter vermindertem Druck abdestilliert. Zu dem Rückstand wurde Dichloromethan hinzugegeben. Die Mischung wurde dann mit Wasser gewaschen. Dichloromethan wurde dann aus der Dichloromethan-Schicht abdestilliert. Das zurückbleibende Öl wurde durch Säulen-Chromatographie gereinigt, wonach das gewünschte 3-(3-Pyridylmethyl)-2-nitromethylenthiazolidin (0,5 g) erhalten wurde. Schmp. 96-100°C.

Beispiel 5

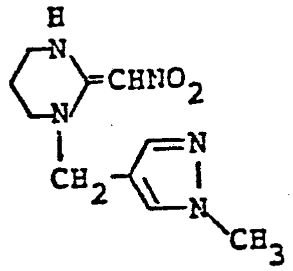

(Verbindung Nr. 5)

Eine Mischung aus 16,8 g N-(1-Methyl-4-pyrazolylmethyl)trimethylendiamin, 16,5 g 1-Nitro-2,2-bis-(methylthio)ethylen und 200 ml Ethanol wurde unter Rückfluß erhitzt, bis die Entwicklung von Methylmercaptan aufhörte. Die Mischung wurde abgekühlt, und die ausgeschiedenen Kristalle wurden durch Filtration gesammelt. Waschen mit Methanol lieferte 16,6 g des gewünschten 1-(1-Methyl-4-pyrazolylmethyl)-2-(nitromethylen)tetrahydropyrimidins in Form blaßgelber Kristalle. Schmp. 186-190°C.

Beispiel 6

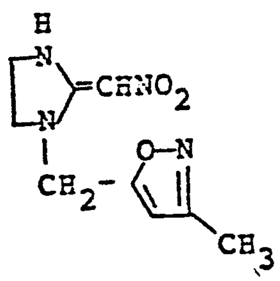

(Verbindung Nr. 6)

Nit 189

Eine Mischung aus 15,5 g N-(3-Methyl-5-isoxazolyl-methyl)ethylendiamin, 16,5 g 1-Nitro-2,2-bis(methyl-thio)ethylen und 200 ml Ethanol wurde unter Rückfluß erhitzt, bis die Entwicklung von Methylmercaptan aufhörte. Hierfür war eine Zeitspanne von etwa 3 h erforderlich. Die Mischung wurde dann auf Raumtemperatur abgekühlt, worauf das gewünschte Produkt sich in Form von Kristallen abschied. Die Kristalle wurden durch Filtration gesammelt. Waschen mit Ethanol lieferte 12,5 g des gewünschten 1-(3-Methyl-5-isoxazolylmethyl)-2-(nitromethylen)imidazolidins in Form blaßgelber Kristalle. Schmp. 168-170°C.

Beispiel 7

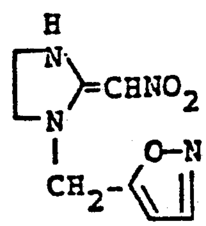

(Verbindung Nr. 7)

2-Nitromethylenimidazolidin (12,9 g) wurde in 60 ml trockenem Dimethylformamid gelöst, und 4,4 g 60-proz. Natriumhydrid (0,9 g) wurde nach und nach bei Raumtemperatur in einem Stickstoff-Strom hinzugefügt. Anschließend wurde zur Bildung des Natrium-Salzes der Imidazolidin-Verbindung die Mischung 3 h bei Raumtemperatur bis 30°C gerührt. Dann wurden 11,8 g 5-Isoxazolylmethylchlorid bei Raumtemperatur hinzugegeben, und die Mischung wurde 24 h bei Raumtemperatur gerührt. Die Reaktionsmischung wurde vorsichtig in 150 ml Eiswasser gegossen und zweimal mit Dichloromethan extrahiert. Dichloromethan wurde von den Dichloromethan-Schichten abdestilliert, wonach 12 g des gewünschten 1-(5-Isoxazolylmethyl)-2-(nitromethylen)imidazolidins in Form brauner Kristalle erhalten wurden. Schmp. 156-158°C.

Beispiel 8

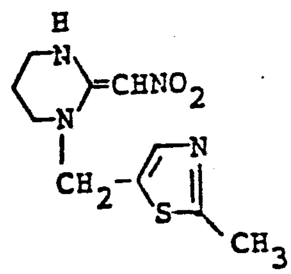

(Verbindung Nr. 8)

N-(2-Methyl-5-thiazolylmethyl)trimethylendiamin (18,5 g) wurde in 100 ml Acetonitril gelöst, und 1-Nitro-2,2-bis(methylthio)ethylen (16,5 g) wurde hinzugefügt. Die Mischung wurde unter Rühren 6 h zum Rückfluß erhitzt. Nach der Reaktion wurde die Reaktionsmischung auf Raumtemperatur abgekühlt. Die entstandenen Kristalle wurden durch Filtration gesammelt und mit Methanol gewaschen, wonach 10,2 g des gewünschten 1-(2-Methyl-5-thiazolylmethyl)-2-(nitromethylen)tetrahydropyrimidins erhalten wurden. Schmp. 204-207°C.

Beispiel 9

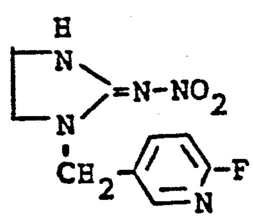

(Verbindung Nr. 9)

Eine Mischung aus 2-Fluoro-5-pyridylmethylbromid (9,5 g), 2-(Nitroimino)imidazolidin (6,5 g), Kaliumcarbonat (7,6 g) und Acetonitril (100 ml) wurde 2 h unter Rückfluß erhitzt. Nach der Reaktion wurde die Reaktionsmischung auf Raumtemperatur abgekühlt, und kaltes Wasser (100 ml) wurde zugegeben. Die erhaltenen Kristalle wurden durch Filtration gesammelt und mit Ether gewaschen, wonach schwach gefärbtes 1-(2-Fluoro-5-pyridylmethyl)-2-(nitroimino)imidazolidin (6,0 g) als die gewünschte Verbindung erhalten wurde. Schmp. 121-124°C.

Beispiel 10

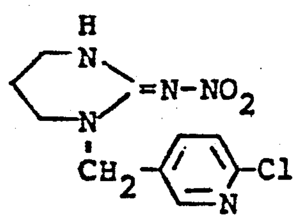

(Verbindung Nr. 10)

Eine aus N-(2-Chloro-5-pyridylmethyl)trimethylendiamin (10 g), Nitroguanidin (5,7 g) und Wasser (80 ml) bestehende Lösung wurde 3 h auf 80°C erhitzt. Die Reaktionsmischung wurde auf Raumtemperatur gekühlt und zweimal mit je 50 ml Dichloromethan extrahiert. Dichloromethan wurde von den Extrakten abdestilliert, und der teerartige Rückstand wurde durch Säulenchromatographie an Silicagel gereinigt, wodurch fast farbloses 1-(2-Chloro-5-pyridylmethyl)-2-nitroimino)tetrahydropyrimidin (6,1 g) erhalten wurde. Schmp. 113-117°C.

 

Beispiel 11-i

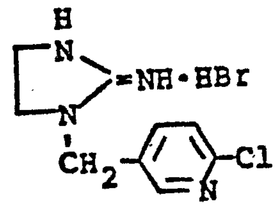

Eine Lösung von N-(2-Chloro-5-pyridylmethyl)ethylenediamin (18,6 g) in Toluol (200 ml) wurde bei Raumtemperatur gerührt, und Cyanbromid (10,6 g) wurde portionsweise hinzugefügt. Danach wurde die Mischung weiter gerührt. Da das gewünschte 1-(2-Chloro-5-pyridylmethyl)-2-iminoimidazolidin in Form des Hydrobromids ausfiel, wurde die Reaktionsmischung filtriert, und der Rückstand wurde mit Ether gewaschen. Schmp. 202-205°C.

Beispiel 11-ii

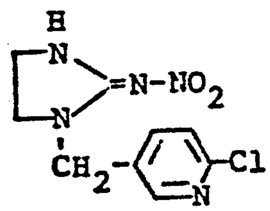

(Verbindung Nr. 11)

Das in Beispiel 11-i synthetisierte Hydrobromid (5,8 g) wurde bei 0°C zu 98-proz. Schwefelsäure (30 ml) hinzugefügt. Dann wurden bei 0°C unter Rühren 2 ml rauchende Salpetersäure tropfenweise dazugegeben. Nach der Zugabe wurde die Mischung 2 h bei 0°C gerührt. Die Reaktionsmischung wurde in Eiswasser (100 g) gegossen und mit Dichloromethan extrahiert. Dichloromethan wurde aus dem Extrakt unter vermindertem Druck abdestilliert, wonach blaßgelbe Kristalle erhalten wurden. Die Kristalle wurden mit Ether gewaschen, wonach 1-(2-Chloro-5-pyridylmethyl)-2-iminoimidazolidin (1,5 g) erhalten wurde. Schmp. 136-139°C.

Nit 189

0192060

Beispiel 12

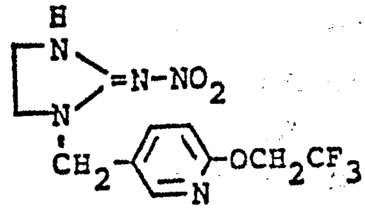

(Verbindung Nr. 12)

Natriumhydrid (0,48 g) wurde zu einer Lösung von 2,2,2-Trifluoroethanol (2,4 g) in Toluol (30 ml) hinzugefügt, und die Mischung wurde gerührt, bis die Wasserstoff-Entwicklung aufhörte. Als Produkt wurde dabei das 2,2,2-Trifluoroethanol-Natrium-Salz hergestellt. Zu dem Produkt wurden 1-(2-Chloro-5-pyridylmethyl)-2-(nitroimino)imidazolidin (5,1 g), das nach der Arbeitsweise des Beispiels 3 hergestellt worden war, und eine katalytische Menge 4-Dimethylaminopyridin hinzugefügt.Die Mischung wurde 10 h unter Rühren auf 80°C erhitzt. Nach dem Kühlen der Reaktionsmischung wurden die ausgeschiedenen Kristalle durch Filtration gesammelt, mit Wasser und mit Ether gewaschen und danach durch Silicagel-Chromatographie gereinigt, wodurch 1-/¯2-(2,2,2-Trifluoroethoxy)-5-pyridylmethyl_7-2-(nitroimino)imidazolidin (1,5 g) erhalten wurde. Schmp. 109-112°C.

Beispiel 13

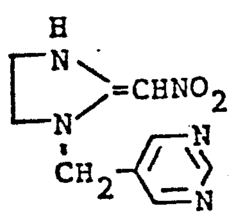

(Verbindung Nr. 13)

Eine Mischung aus N-(1-Pyrimidinylmethyl)ethylendiamin (15,2 g), 1-Nitro-2,2-bis(methylthio)ethylen (14,9 g) und Ethanol (100 g) wurde unter Rühren zum Rückfluß erhitzt, bis die Entwicklung von Methylmercaptan aufhörte (etwa 3 h). Die Mischung wurde auf Raumtemperatur abgekühlt, und die entstandenen Kristalle wurden durch Filtration gesammelt. Die Kristalle wurden mit Ethanol gewaschen und getrocknet, wonach 1-(5-Pyridinylmethyl)-2-(nitromethylen)imidazolidin (12,7 g) in Form blaßgelber Kristalle erhalten wurde. Dieses Produkt zersetzt sich bei 236°C.

Beispiel 14

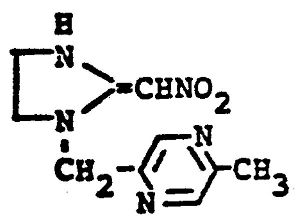

(Verbindung Nr. 14)

2-Nitromethylenimidazolidin (12,9 g) wurde in trockenem Dimethylformamid (100 ml) gelöst, und bei Raumtemperatur wurde 60-proz. Natriumhydrid in Öl (4,4 g) hinzugefügt. Die Mischung wurde bei Raumtemperatur gerührt, bis die Wasserstoff-Entwicklung aufhörte. Dann wurde 2-Methyl-5-pyrazinylmethylchlorid (14,3 g) bei Raumtemperatur hinzugegeben, und die Mischung wurde 8 h bei 40°C gerührt. Nach dem Kühlen auf Raumtemperatur wurde die Reaktionsmischung 200 ml Wasser gegeben und mit Dichloromethan extrahiert. Nach dem Abdestillieren des Dichloromethans aus der organischen Schicht unter ver-

0192060

mindertem Druck wurde 1-(2-Methyl-5-pyrazinylmethyl)-2-(nitromethylen)imidazolidin (5,4 g) in Form gelber Kristalle mit einem Schmp. 163-166°C erhalten.

Beispiel 15

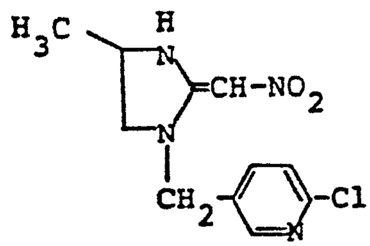

(Verbindung Nr. 15)

Eine Mischung aus 2 g 2-Amino-1-(2-chloro-5-pyridylmethylamino)propan, 1,6 g 1-Nitro-2,2-bis(methylthio)-ethylen und 20 ml Methanol wurde 5 h unter Rühren zum Rückfluß erhitzt. Nach dem Stehen bei Raumtemperatur schied sich ein kristallines Produkt ab. Das Produkt wurde unter Saugen abfiltriert, mit Methanol gewaschen und dann im Vakuum getrocknet. 1,9 g 1-(2-Chloro-5-pyridylmethyl)-4-methyl-2-(nitromethylen)imidazolidin wurden erhalten in Form hellgelber Kristalle. Schmp. 170-174°C.

Beispiel 16

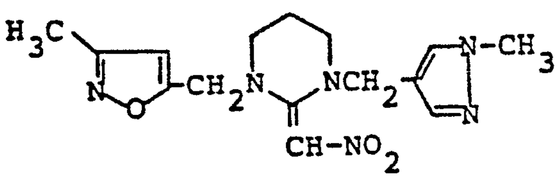

(Verbindung Nr. 16)

Eine Mischung aus 2,6 g N-(3-Methyl-5-isoxazolylmethyl)-N'-(1-methyl-4-pyrazolyl)trimethylendiamin, 1,6 g 1-Nitro-2,2-bis(methylthio)ethylen und 10 ml Ethanol wurde 15 h unter Rühren zum Rückfluß erhitzt. Nach der Reaktion wurde das Ethanol durch Vakuumdestillation entfernt. Der teerartige Rückstand wurde durch Chromatographie an einer Silicagel-Säule gereinigt. Das Produkt, 0,7 g 1-(3-Methyl-5-isoxazolylmethyl)-3-(1-methyl-4-pyrazolyl)-2-nitromethylen)tetrahydropyrimidin wurde in Form eines viskosen Öls erhalten. $n_D^{20}$ 1,5670.

Beispiel 17

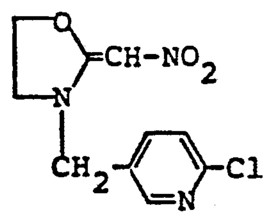

(Verbindung Nr. 17)

6,6 g fein gepulvertes 1-Nitro-2,2-bis(methylthio)ethylen wurden mit 7,5 g 2-(2-Chloro-5-pyridylmethylamino)-ethanol gut vermischt. Die Mischung wurde 30 min oder so lange, bis die Bildung von Methylmercaptan aufhörte, auf 110°C bis 120°C erhitzt. Das erhaltene Öl wurde auf Raumtemperatur abgekühlt und durch Chromatographie an einer Silicagel-Säule gereinigt. Das Produkt, 3-(2-Chloro-5-pyridylmethyl)-2-(nitromethylen)oxazolidin, wog 1,7 g. Schmp. 123-124°C.

Beispiel 18

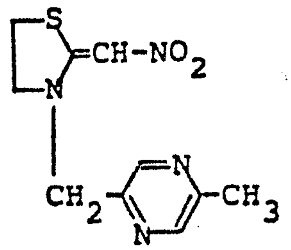

(Verbindung Nr. 18)

2,6 g Kaliumhydroxid wurden in 20 ml wasserfreiem Ethanol gelöst. Zu der Lösung wurden 3,7 g 2-(2-Methyl-5-pyrazinylmethylamino)ethanthiol unter einer Stickstoff-Atmosphäre hinzugefügt. Die Lösung wurde dann auf 0°C gekühlt, und 3,0 g 2,2-Dichloro-1-nitroethylen wurden tropfenweise bei 0°C bis 10°C dazuzugegeben. Nach der Zugabe wurde die Mischung 1 h bei 10°C gerührt. Das Ethanol wurde durch Vakuumdestillation entfernt, dem Rückstand wurde Chloroform zugesetzt, und die Chloroform-Schicht wurde mit einer 1-proz. Natriumhydroxid-Lösung und mit Wasser gewaschen. Die Chloroform-Schicht wurde in üblicher Weise behandelt, wonach hellgelbe Kristalle von 3-(2-Methyl-5-pyrazinylmethyl)-2-(nitromethylen)thiazolidin erhalten wurden, die 2,0 g wogen. Schmp. 147-150°C.

Beispiel 19

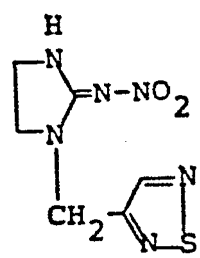

(Verbindung Nr. 19)

Eine Mischung aus 4,7 g N-(1,2,5-Thiadiazol-3-ylmethyl)ethylendiamin, 3,4 g Nitroguanidin und 20 ml Wasser wurde 1 h bei 50°C bis 60°C und dann 20 min bei 70°C gerührt. Die erhaltene Lösung wurde langsam auf 5°C gekühlt, wobei das Produkt ausfiel. Das kristalline Produkt wurde durch Filtration gesammelt, mit Wasser und Methanol gewaschen und getrocknet. Die Ausbeute an 1-(1,2,5-Thiadiazol-3-ylmethyl)-2-(nitroimino)imidazolidin betrug 2,9 g. Schmp. 162-165°C.

Beispiel 20

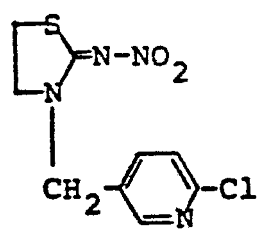

(Verbindung Nr. 20)

Eine Mischung aus 2,9 g 2-Nitroiminothiazolidin, 2,9 g wasserfreiem Kaliumcarbonat, 3,2 g 2-Chloro-5-pyridylmethylchlorid und 50 ml Acetonitril wurde 5 h unter kräftigem Rühren zum Rückfluß erhitzt. Nach der Reaktion wurde der größte Teil des Acetonitrils durch Destillation entfernt, und Wasser wurde dem Rückstand zugesetzt. Nachdem sich ein festes Produkt abgeschieden hatte, wurde es durch Filtration gesammelt. Das rohe Material wurde aus Ethanol umkristallisiert, wonach das gewünschte 3-(2-Chloro-5-pyridylmethyl)-2-(nitroimino)-thiazolidin erhalten wurde. Ausbeute 3,8 g. Schmp. 137-138°C.

Beispiel 21

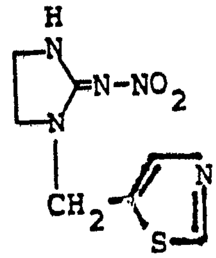

(Verbindung Nr. 21)

Zu einer Mischung aus 1,3 g fein gepulvertem 2-Nitroiminoimidazolidin und 30 ml trockenem Acetonitril wurden portionsweise 0,4 g Natriumhydrid (60 % in Öl) bei Raumtemperatur hinzugefügt, und die Mischung wurde gerührt, bis die Entwicklung von Wasserstoff-Gas aufhörte. Eine Lösung von 1,7 g 2-Chloro-5-thiazolylmethylchlorid in 10 ml Acetonitril wurde dann tropfenweise bei Raumtemperatur hinzugegeben. Nach der Zugabe wurde die Mischung 3 h bei Raumtemperatur gerührt und dann in Eiswasser gegossen. Die organische Schicht wurde mit Dichloromethan extrahiert, und der Dichloromethan-Extrakt wurde mit 1-proz. Natriumhydroxid-Lösung und mit Wasser gewaschen. Das Produkt erstarrte nach dem Abdampfen des Dichloromethans und wurde mit Wasser gewaschen und getrocknet. Das gewünschte 1-(2-Chloro-5-thiazolylmethyl)-2-(nitroimino)imidazolidin wog 1,4 g. Schmp. 147-150°C.

Beispiel 22

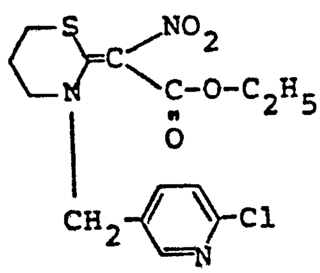

(Verbindung Nr. 22)

Nach der in Beispiel 7 beschriebenen Arbeitsweise wurde eine gummiartige Substanz aus 2,3 g Ethyl-nitro(tetrahydro-2H-1,3-thiazin-2-yliden)acetat und 1,6 g 2-Chloro-5-pyridylmethylchlorid erhalten. Die Substanz wurde mit Ethanol verrieben. Das unlösliche Material wurde mit Hexan gewaschen und dann durch Chromatographie an einer Silicagel-Säule gereinigt. Das gewünschte Ethyl-/¯3-(2-Chloro-5-pyridylmethyl)tetrahydro-2H-1,3-thiazin-2-yliden_7acetat wog 0,2 g. Schmp. 180-184°C.

Beispiel 23

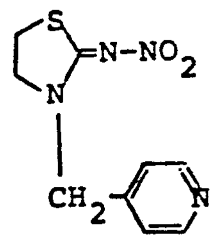

(Verbindung Nr. 23)

5,8 g 2-Imino-3-(4-pyridylmethyl)thiazolidin wurden bei -5°C bis 0°C zu 20 ml konz. Schwefelsäure hinzugefügt. 6 ml rauchende Salpetersäure wurden dann bei der gleichen Temperatur tropfenweise zu der Lösung dazugegeben. Nach 30-minütigem Rühren bei 0°C bis 5°C wurde die Mischung auf zerkleinertes Eis gegossen. Zweimalige Extraktion mit Dichloromethan und nachfolgende Behandlung in üblicher Weise ergab 1,4 g des gewünschten 2-Nitroimino-3-(4-pyridylmethyl)thiazolidins. Schmp. 151-152°C.

Nit 189

Beispiel 24

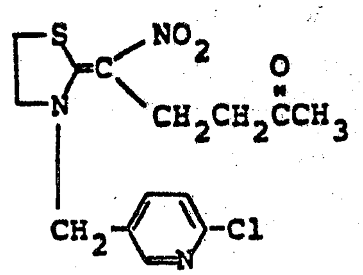

(Verbindung Nr. 24)

Eine Mischung aus 2,7 g 3-(2-Chloro-5-pyridylmethyl)-2-(nitromethylen)thiazolidin, 3,5 g Methylvinylketon und 30 ml Chloroform wurde 2 d bei 40°C unter einer Stickstoff-Atmosphäre gerührt. Danach wurden 3,5 g Methylvinylketon zu der Reaktionsmischung hinzugefügt, und diese wurde weitere 2 d bei der gleichen Temperatur gerührt. Nach dem Entfernen des flüchtigen Materials im Vakuum wurde der Rückstand durch Chromatographie an einer Silicagel-Säule gereinigt. Das gewünschte 3-(2-Chloro-5-pyridylmethyl)-2-(1-nitro-4-oxopentyliden)-thiazolidin wog 0,1 g. Schmp. 75-80°C.

Beispiel 25

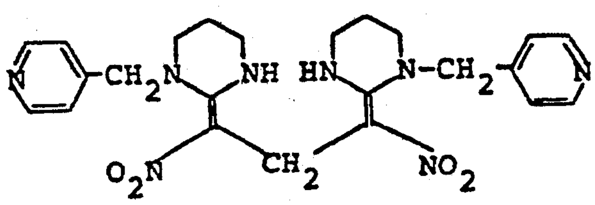

(Verbindung Nr. 25)

Eine Mischung aus 2,3 g 1-(4-Pyridylmethyl)-2-(nitromethylen)tetrahydropyrimidin, 0,5 g Paraformaldehyd und

30 ml Dioxan wurde 2 h bei 70°C bis 80°C kräftig gerührt. Nach der Reaktion wurde das Dioxan unter vermindertem Druck auf etwa 1/3 seines Volumens abgedampft. Das kristallisierte Produkt wurde filtriert und getrocknet. Die Ausbeute des gewünschten Bis-/‾α-nitro-1-(4-pyridylmethyl)tetrahydropyrimidin-2-ylidenmethyl_7methan betrug 1,8 g. Schmp. 222-223°C

Beispiel 26

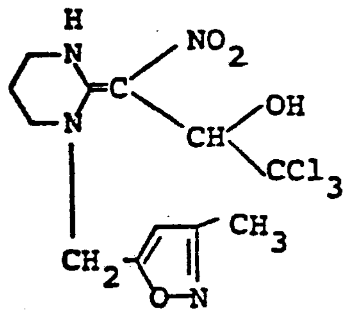

(Verbindung Nr. 26)

Zu einer Lösung von 2,4 g 1-(3-Methyl-5-isoxazolylmethyl)-2-(nitromethylen)tetrahydropyrimidin und 30 ml trockenem Dichloromethan wurden 1,6 g Trichloroacetaldehyd bei Raumtemperatur hinzugefügt. Nach dreistündigem Rühren bei 25°C bis 30°C wurde das kristallisierte Produkt abfiltriert, mit Ether gewaschen und getrocknet. Das gewünschte 1-(3-Methyl-5-isoxazolylmethyl)-2-(1-nitro-2-hydroxy-3,3,3-trichloropropyliden)tetrahydropyrimidin wog 3,1 g. Schmp. 128-130°C (Zers.).

Beispiel 27

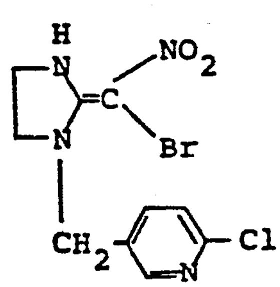

(Verbindung Nr. 27)

2,5 g 1-(2-Chloro-5-pyridylmethyl)-2-(nitromethylen)-imidazolidin wurden in 40 ml trockenem Dichloromethan gelöst, und 10 ml Wasser wurden zugegeben. Eine Lösung von 1,6 g Brom in 10 ml Dichloromethan wurde unter gutem Rühren bei 0°C bis 5°C innerhalb von 10 min hinzugefügt. Nach 10-minütigem Rühren bei 0°C bis 5°C wurde das kristallisierte Produkt filtriert, mit kaltem Wasser und einer kleinen Menge Dichloromethan gewaschen und getrocknet. Die Ausbeute an 1-(2-Chloro-5-pyridylmethyl)-2-(bromonitromethylen)imidazolidin betrug 2,5 g. Schmp. 110-115°C (Zers.).

Beispiel 28

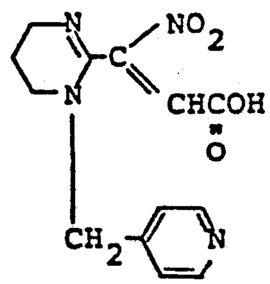

(Verbindung Nr. 28)

Zu einer Mischung aus 1,2 g fein gepulvertem 1-(4-Pyridylmethyl)-2-(nitromethylen)tetrahydropyrimidin in 20 ml Methanol wurden 2 Tropfen konz. Schwefelsäure und dann 1,6 g 25-proz. wäßrige Glyoxylsäure hinzugefügt. Die Mischung wurde 3 h bei Raumtemperatur gerührt. Nach dem Einstellen auf pH 7 wurde das abgeschiedene Produkt filtriert, mit Wasser, Methanol und Ether gewaschen und getrocknet. Die gewünschte 3-/¯1-(4-Pyridylmethyl)-1H,4H,5H,6H-tetrahydropyrimidin-4-yl_7-3-nitroacrylsäure wog 1,1 g. Schmp. 150-155°C (Zers.).

0192060

Beispiel 29

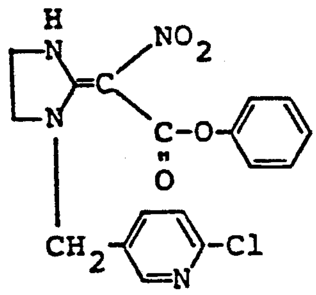

(Verbindung Nr. 29)

3,1 g Phenylchloroformiat wurden unterhalb von 0°C zu einer Lösung von 1,6 g Methylimidazol in 50 ml Dichloromethan hinzugefügt. Nach 30-minütigem Rühren bei der gleichen Temperatur wurden 2,5 g 1-(2-Chloro-5-pyridylmethyl)-2-(nitromethylen)imidazolidin zu der Mischung gegeben, und die erhaltene Mischung wurde 24 h bei Raumtemperatur gerührt. Die Mischung wurde dann jeweils mit Wasser, 1-proz. Salzsäure und 1-proz. Natriumhydroxid-Lösung gewaschen. Das Entfernen des Dichloromethans durch Destillation liefert glasiges Phenyl-nitro/¯1-(2-chloro-5-pyridylmethyl)-3-phenoxycarbonylimidazolidin-2-yliden_7acetat (3,2 g). Dieses wurde in 20 ml Dimethylformamid gelöst. 1,7 g Natriumcarbonat wurden der Lösung zugesetzt, und die Mischung wurde 3 d bei Raumtemperatur gerührt. Danach wurde Wasser zugegeben, und die organische Schicht wurde mit Dichloromethan extrahiert. Der Extrakt wurde mit 1-proz. Natriumhydroxid-Lösung und mit Wasser gewaschen. Nach dem Abdampfen des Dichloromethans wurde der Rückstand durch Chromatographie an einer Silicagel-Säure gereinigt. Das gewünschte Produkt, Phenyl-nitro-/¯1-(2-chloro-5-pyridylmethyl)imidazolidin-2-yliden_7-acetat, wog 0,2 g. Schmp. 224-228°C (Zers.).

Beispiel 30

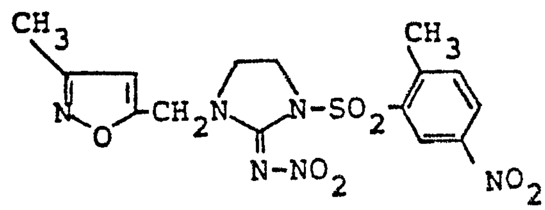

(Verbindung Nr. 30)

0,2 g Natriumhydrid (60 % in Öl) wurden zu einer Lösung von 1,1 g 1-(3-Methylisoxazol-5-ylmethyl)-2-nitroiminoimidazolidin in 15 ml trockenem Dimethylformamid hinzugefügt, und die Mischung wurde gerührt, bis die Entwicklung von Wasserstoff-Gas beendet war. 1,2 g 2-Methyl-5-nitrobenzolsulfonylchlorid wurde dann zu der Mischung dazugegeben. Nach 1-tägigem Rühren bei Raumtemperatur wurde die Mischung in Eiswasser gegossen, und die organische Schicht wurde mit Dichloromethan extrahiert. Das Dichloromethan wurde durch Destillation entfernt, wonach ein kristallines Produkt erhalten wurde. Dieses wurde mit Ether gewaschen. Das gewünschte 1-(2-Methyl-5-nitrobenzolsulfonyl)-3-(3-methylisoxazol-5-ylmethyl)-2-nitroiminoimidazolidin wog 1,1 g. Schmp. 154-156°C.

Beispiel 31

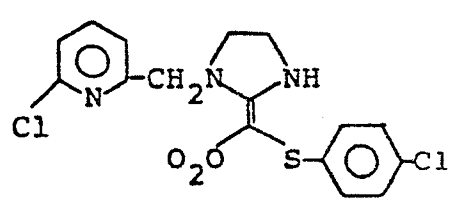

(Verbindung Nr. 31)

Nit 189

0,2 g Natriumhydrid (60 % in Öl) wurden zu einer Lösung von 1,3 g 1-(2-Chloro-5-pyridylmethyl)-2-nitromethylenimidazolidin in 15 ml trockenem Dimethylformamid hinzugefügt, und die Mischung wurde bei Raumtemperatur gerührt, bis die Entwicklung von Wasserstoff-Gas beendet war. 0,9 g 4-Chlorobenzolsulfenylchlorid wurde dann bei 0°C unter Rühren tropfenweise zu der Lösung dazugegeben. Nach 1-stündigem Rühren bei Raumtemperatur wurde die Reaktionsmischung in Eiswasser gegossen. Die abgeschiedenen Kristalle wurden filtriert und Ethylacetat umkristallisiert, wonach 1-(2-Chloro-5-pyridylmethyl)-2-/¯(4-chlorophenylthio)nitromethylen_7imidazolidin erhalten wurde. Die Ausbeute betrug 1,3 g. Schmp. 159-161°C.

Beispiel 32

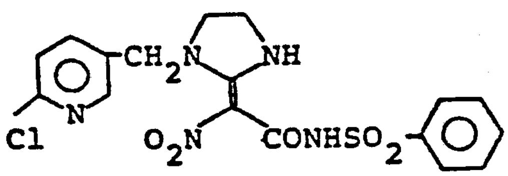

(Verbindung Nr. 32)

Eine Lösung von 0,9 g Benzolsulfonylisocyanat in 10 ml trockenem Dichloromethan wurde tropfenweise bei Raumtemperatur zu einer Lösung von 1,3 g 1-(2-Chloro-5-pyridylmethyl)-2-nitromethylenimidazolidin in 25 ml trockenem Dichloromethan hinzugefügt. Die Lösung wurde 2 h bei der gleichen Temperatur gerührt, und danach wurde etwa die Hälfte des Dichloromethan-Volumens unter vermindertem Druck abgedampft. Das Produkt, das auskristallisierte, wurde filtriert und mit Ether gewaschen. Das gewünschte N-Benzolsulfonyl-2-/¯1-(2-chloro-5-pyridylmethyl)imidazolidin-2-yliden_7-2-nitroacetamid wog 1 g. Schmp. 95-100°C.

Beispiel 33

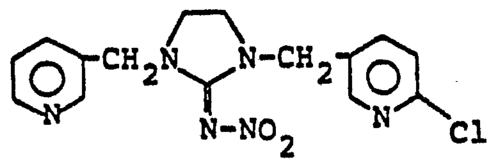

(Verbindung Nr. 33)

Eine Mischung aus 2,2 g 2-Nitroimino-1-(3-pyridylmethyl)imidazolidin, 1,6 g 2-Chloro-5-chloromethylpyridin und 1,4 g wasserfreiem Kaliumcarbonat in 30 ml Acetonitril wurde 16 h unter Rühren zum Rückfluß erhitzt. Dann wurde das Acetonitril unter vermindertem Druck entfernt. Dem Rückstand wurde Dichloromethan zugesetzt, und die Mischung wurde mit Wasser und mit 1-proz. Natriumhydroxid-Lösung gewaschen. Nach dem Entfernen des Dichloromethans unter vermindertem Druck wurde der Rückstand durch Chromatographie an einer Silicagel-Säule gereinigt, wonach 1-(2-Chloro-5-pyridylmethyl)-2-nitroimino-3-(3-pyridylmethyl)imidazolidin erhalten wurde. Die Ausbeute betrug 2,1 g. Schmp. 143-144°C.

Beispiel 34

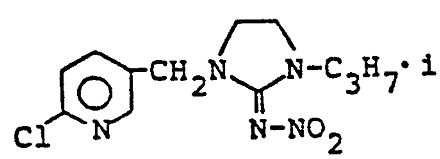

(Verbindung Nr. 34)

0,4 g Natriumhydrid (60 % in Öl) wurde portionsweise zu einer Lösung von 2,6 g 1-(2-Chloro-5-pyridylmethyl)-2-nitroiminoimidazolidin in 20 ml trockenem Dimethylformamid hinzugefügt. Die Mischung wurde bei Raumtemperatur gerührt, bis die Entwicklung von Wasserstoff beendet war. Eine Lösung von 1,5 g Isopropylbromid in 5 ml trockenem Dimethylformamid wurde tropfenweise bei Raumtemperatur zu der Mischung hinzugegeben, und nach der Zugabe wurde die Mischung 3 h bei Raumtemperatur gerührt. Die Reaktionsmischung wurde dann in Eiswasser gegossen, und die ausgefallenen Kristalle wurden filtriert. Sie wurden aus Ethanol umkristallisiert, wonach 1-(2-Chloro-5-pyridylmethyl)-3-isopropyl-2-nitroiminoimidazolidin erhalten wurden. Die Ausbeute betrug 1,5 g. Schmp. 138-142°C.

Beispiel 35

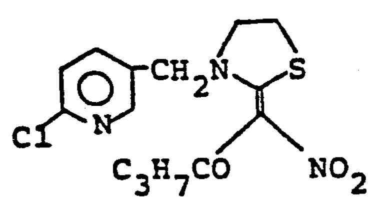

(Verbindung Nr. 35)

Eine Mischung aus 2,7 g 3-(2-Chloro-5-pyridylmethyl)-2-nitromethylenthiazolidin und 8 ml Buttersäureanhydrid wurde 8 h bei 60°C unter einer Stickstoff-Atmosphäre gerührt. Die flüchtigen Stoffe wurden dann durch Destillation bei 1,33 mbar (1 mmHg) entfernt, wobei die Bad-Temperatur unter 60°C gehalten wurde. Der Rückstand wurde in Dichloromethan gelöst und mit 1-proz. Natriumhydroxid-Lösung gewaschen. Die Dichloromethan-Schicht

wurde danach durch Chromatographie an einer Silicagel-Säule gereinigt, wonach viskoses öliges 1-/‾3-(2-Chloro-5-pyridylmethyl)thiazolidin-2-yliden_7-1-nitro-2-pentanon erhalten wurde. Die Ausbeute betrug 0,15 g. $n_D^{20}$ 1,6342.

Nach den gleichen Verfahren, wie sie in den vorstehenden Beispielen beschrieben sind, wurden die in den nachfolgenden Tabellen aufgeführten Verbindungen der Formel (I) erhalten.

In den Tabellen bedeutet die Kennzeichnung " - " für $R^3$ und $R^4$, daß n 0 ist, und in diesem Fall bezeichnet die entsprechende Ring-Struktur einen 5-gliedrigen heterocyclischen Ring.

Tabelle 1 umfaßt den Fall, in dem X ein Schwefel-Atom ist und die Verbindungen der Formel (I) durch die folgende Formel dargestellt werden:

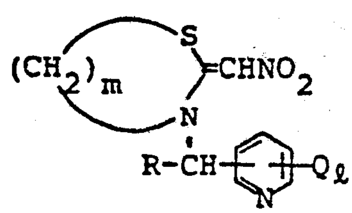

0192060

Tabelle 1

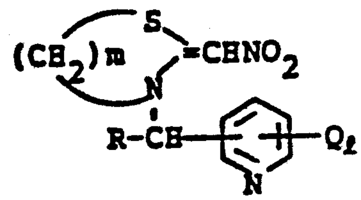

| Verbindung Nr. | m | R | Bindungs-Position des Pyridin-Rings | $Q_\ell$ |
|---|---|---|---|---|
| 36 | 2 | H | 5- | 2-F |
| 37 | 3 | H | 5- | 2-F |
| 38 | 2 | $-CH_3$ | 5- | 2-Cl |
| 39 | 2 | H | 5- | 2-Br |
| 40 | 2 | H | 5- | $2,3-Cl_2$ |
| 41 | 2 | H | 5- | $2,3,4,6-F_4$ |
| 42 | 2 | H | 4- | 2-Cl |
| 43 | 3 | H | 5- | 2-Br |
| 44 | 2 | H | 5- | 2-F, 3-Cl |
| 45 | 2 | H | 2- | 3-Cl |
| 46 | 3 | H | 2- | 5-Cl |
| 47 | 2 | H | 2- | $3,5-Cl_2$ |
| 48 | 3 | H | 2- | 5-F |
| 49 | 2 | H | 2- | 6-Br |
| 50 | 3 | H | 3- | 2-Cl |
| 51 | 3 | H | 3- | 5-Cl |
| 52 | 2 | H | 3- | 5-Br |
| 53 | 2 | H | 3- | 5-F |

| | | | | |
|---|---|---|---|---|
| 54 | 2 | $-CH_3$ | 5- | 2-F |
| 55 | 3 | H | 5- | $2,4-Cl_2$ |
| 56 | 2 | H | 5- | $2,4-Br_2$ |
| 57 | 2 | H | 4- | $2,6-F_2$ |
| 58 | 3 | H | 4- | 2-F |
| 59 | 2 | H | 4- | $2,6-Br_2$ |
| 60 | 2 | H | 5- | 2-F, 3-Br |
| 61 | 2 | H | 5- | 2-Cl, 3-F |
| 62 | 2 | $-C_2H_5$ | 5- | $2-Cl_3$ |
| 63 | 2 | H | 4- | - |
| 64 | 3 | H | 4- | - |
| 65 | 2 | H | 5- | $2-CH_3$ |
| 66 | 3 | H | 5- | $2-CH_3$ |
| 67 | 2 | H | 5- | $2-C_2H_5$ |
| 68 | 2 | H | 5- | $2-CH_2CH=CH_2$ |
| 69 | 2 | H | 5- | $2-CH_2C \equiv CH$ |
| 70 | 3 | H | 5- | $2-OCH_3$ |
| 71 | 2 | H | 5- | $2-SCH_3$ |
| 72 | 2 | H | 5- | $2-\overset{O}{\underset{O}{\overset{\|}{\underset{\|}{S}}}}-CH_3$ |
| 73 | 2 | H | 5- | $2-Cl, 3-CH_3$ |
| 74 | 2 | $-CH_3$ | 3- | - |
| 75 | 2 | H | 5- | $2-CF_3$ |
| 76 | 3 | H | 5- | $2-CF_3$ |
| 77 | 2 | H | 5- | $2-NO_2$ |
| 78 | 3 | H | 5- | $2-NO_2$ |
| 79 | 2 | H | 5- | 2-CN |

Nit 189

Tabelle 1 - Fortsetzung

| | | | | |
|---|---|---|---|---|
| 80 | 3 | H | 5- | 2-CN |
| 81 | 2 | H | 5- | $2-\overset{O}{\overset{\|}{S}}-CH_3$ |
| 82 | 2 | H | 5- | 2-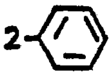 |
| 83 | 2 | H | 5- | $2-CH_2-$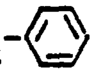 |
| 84 | 3 | H | 5- | 2-O-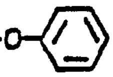 |
| 85 | 2 | H | 5- | $2-CCl_3$ |
| 86 | 3 | H | 5- | $2-C_2H_4OC_2H_5$ |
| 87 | 2 | H | 5- | $2-CH_2OCH_3$ |
| 88 | 2 | H | 5- | $2-OCHF_2$ |
| 89 | 2 | H | 5- | $2-OCF_3$ |
| 90 | 2 | H | 5- | $2-OCH_2CF_3$ |
| 91 | 3 | H | 5- | $2-SCClF_2$ |
| 92 | 2 | H | 5- | $2-SCF_3$ |
| 93 | 2 | H | 5- | $2-CHF_2$ |
| 94 | 2 | H | 5- | $2-\underset{\underset{O}{\|}}{\overset{\overset{O}{\|}}{S}}-CF_3$ |
| 95 | 2 | H | 5- | $2-\overset{\overset{O}{\|}}{S}-CF_3$ |
| 96 | 2 | H | 5- | $2-CH=CCl_2$ |

Neben Tabelle 1 umfaßt Tabelle 2 den Fall, in dem X ein Schwefel-Atom ist.

Nit 189

Tabelle 2

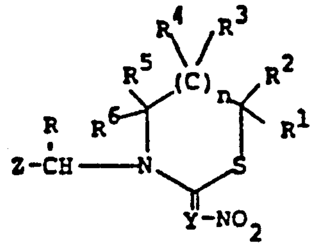

| Verb. Nr. | Z | R | $R^1$ | $R^2$ | $R^3$ | $R^4$ | $R^5$ | $R^6$ | n | Y | |
|---|---|---|---|---|---|---|---|---|---|---|---|
| 97 | 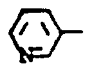 | H | H | H | – | – | H | H | 0 | N | |
| 98 | 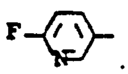 | H | H | H | – | – | H | H | 0 | N | |
| 99 | 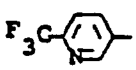 | H | H | H | – | – | H | H | 0 | N | |
| 100 | 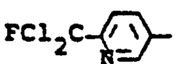 | H | H | H | H | H | H | H | 1 | N | |
| 101 | 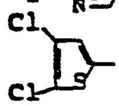 | H | H | H | – | – | H | H | 0 | N | |
| 102 | 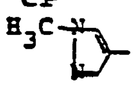 | H | H | H | – | – | H | H | 0 | N | |
| 103 | 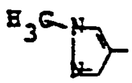 | H | H | H | H | H | H | H | 1 | N | |
| 104 | 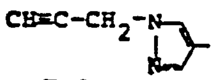 | H | H | H | – | – | H | H | 0 | N | |
| 105 | 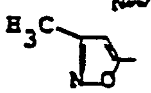 | H | H | H | – | – | H | H | 0 | N | Schmp. 143-145°C |
| 106 | 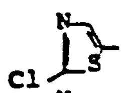 | H | H | H | – | – | H | H | 0 | N | |
| 107 | 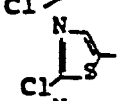 | H | H | H | H | H | H | H | 1 | N | |
| 108 | 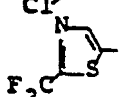 | H | H | H | – | – | H | H | 0 | N | |
| 109 | 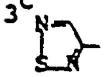 | H | H | H | – | – | H | H | 0 | N | |
| 110 | 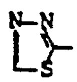 | H | H | H | H | H | H | H | 1 | N | |
| 111 | 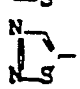 | H | H | H | – | – | H | H | 0 | N | |
| 112 | 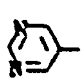 | H | H | H | – | – | H | H | 0 | CH | |

Nit 189

| Verb. Nr. | Z | R | R¹ | R² | R³ | R⁴ | R⁵ | R⁶ | n | Y | |
|---|---|---|---|---|---|---|---|---|---|---|---|
| 113 | 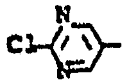 | H | H | H | – | – | H | H | 0 | CH | |
| 114 | 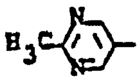 | H | H | H | H | H | H | H | 1 0 | CH | |
| 115 | 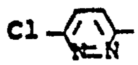 | H | H | H | – | – | H | H | 0 | CH | |
| 116 | 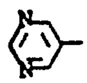 | H | H | H | – | – | H | H | 0 | N | |
| 117 | 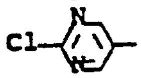 | H | H | H | – | – | H | H | 0 | N | |
| 118 | 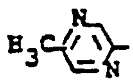 | H | H | H | – | – | H | H | 0 | N | |
| 119 | 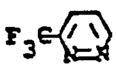 | H | H | H | – | – | H | H | 0 | N | |
| 120 | 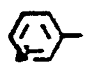 | H | H | H | H | H | H | H | 1 | $C-CH_3$ | |
| 121 | 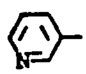 | H | H | H | – | – | H | H | 0 | $C-CH_2F$ | |
| 122 | 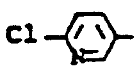 | H | H | H | – | – | H | H | 0 | $C-CF_3$ | |
| 123 | 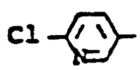 | H | H | H | – | – | H | H | 0 | $C-CH_2OCH_3$ | |
| 124 | 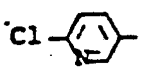 | H | H | H | – | – | H | H | 0 | $C-CH_2SC_2H_5$ | |
| 125 | 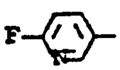 | H | H | H | H | H | H | H | 1 | $C-CH_2N(CH_3)_2$ | |
| 126 | 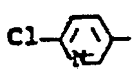 | H | H | H | H | H | H | H | 1 | $C-CH_2CH=CH_2$ | |
| 127 | 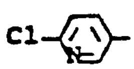 | H | H | H | – | – | H | H | 0 | $C-(CH_2)_2CN$ | |
| 128 | 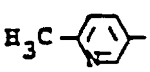 | H | H | H | – | – | H | H | 0 | $C-(CH_2)_2COOCH_3$ | |
| 129 | 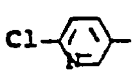 | H | H | H | – | – | H | H | 0 | $C-CH(OH)CCl_3$ | |
| 130 | 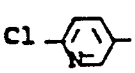 | H | H | H | H | H | H | H | 1 | 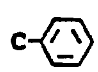 | |
| 131 | 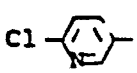 | H | H | H | – | – | H | H | 0 | $C-COCH_3$ | $n_D^{25}$ 1,6358 |
| 132 | 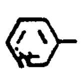 | H | H | H | H | H | H | H | 1 | $C-COCH_2OCH_3$ | |

Nit 189

| Verb. Nr. | Z | R | $R^1$ | $R^2$ | $R^3$ | $R^4$ | $R^5$ | $R^6$ | n | Y | |
|---|---|---|---|---|---|---|---|---|---|---|---|
| 133 | 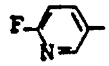 | H | H | H | – | – | H | H | 0 | $C-COCCl_3$ | |
| 134 | 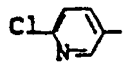 | H | H | H | – | – | H | H | 0 | C-CO-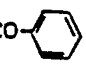 | |
| 135 | 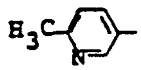 | H | H | H | H | H | H | H | 1 | C-CO-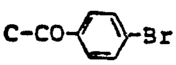 | |
| 136 | 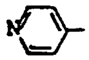 | H | H | H | H | H | H | H | 1 | $C-COOCH_3$ | |
| 137 | 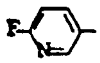 | H | H | H | H | H | H | H | 1 | $C-COOC_2H_5$ | |
| 138 | 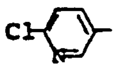 | H | H | H | – | – | H | H | 0 | $C-COOC_2H_5$ | |
| 139 | 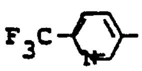 | H | H | H | – | – | H | H | 0 | C-COO-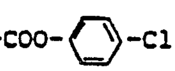 | |
| 140 | 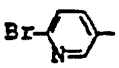 | H | H | H | – | – | H | H | 0 | $C-S-(CH_2)_3CH_3$ | |
| 141 | 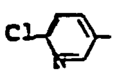 | H | H | H | – | – | H | H | 0 | C-S-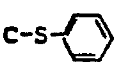 | |
| 142 | 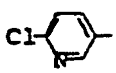 | H | H | H | – | – | H | H | 0 | C-CONHCO-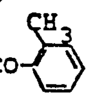 | |
| 143 | 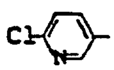 | H | H | H | – | – | H | H | 0 | $C-CONHSO_2$-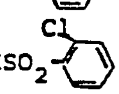 | |
| 144 | 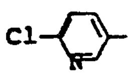 | H | H | $CH_3$ | – | – | H | H | 0 | N | |
| 145 | 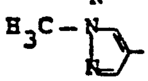 | H | H | H | – | – | H | H | 0 | C-COO-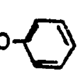 | |
| 146 | 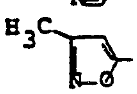 | H | H | H | H | H | H | H | 1 | $C-COOC_2H_5$ | $n_D^{24}$ 1,5978 |
| 147 | 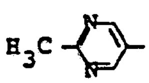 | H | H | H | – | – | H | H | 0 | $C-COCH_3$ | |
| 148 | 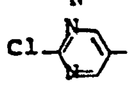 | H | H | H | – | – | H | H | 0 | $C-COOCH_3$ | |
| 149 | 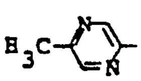 | H | H | H | – | – | H | H | 0 | $C-COOCH_2CF_3$ | |
| 150 | 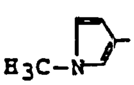 | H | H | H | – | – | H | H | 0 | CH | |
| 151 | 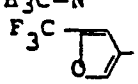 | H | H | H | H | H | H | H | 1 | CH | |
| 152 | 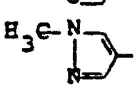 | H | H | H | H | H | H | H | 1 | CH | |

0192060

| Verb. Nr. | Z | R | $R^1$ | $R^2$ | $R^3$ | $R^4$ | $R^5$ | $R^6$ | n | Y | |
|---|---|---|---|---|---|---|---|---|---|---|---|
| 153 | 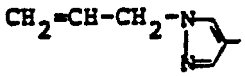 | H | H | H | – | – | H | H | 0 | CH | |
| 154 | 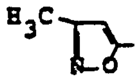 | H | H | H | H | H | H | H | 1 | CH | |
| 155 | 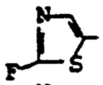 | H | H | H | H | H | H | H | 1 | CH | |
| 156 | 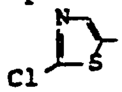 | H | H | H | H | H | H | H | 1 | CH | |
| 157 | 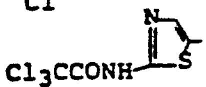 | H | H | H | – | – | H | H | 0 | CH | |
| 158 | 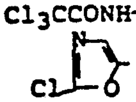 | H | H | H | – | – | H | H | 0 | CH | |
| 159 | 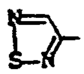 | H | H | H | – | – | H | H | 0 | CH | |
| 160 | 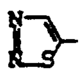 | H | H | H | H | H | H | H | 1 | CH | |

Tabelle 3 umfaßt den Fall, in dem X $CH-R^8$ ist.

Nit 189

Tabelle 3

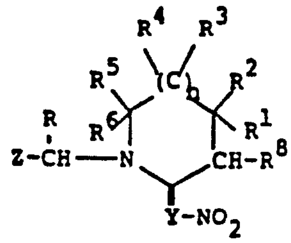

| Verb. Nr. | Z | R | $R^1$ | $R^2$ | $R^3$ | $R^4$ | $R^5$ | $R^6$ | n | $R^8$ | Y | |
|---|---|---|---|---|---|---|---|---|---|---|---|---|
| 161 | 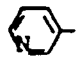 | $CH_3$ | H | H | H | – | – | H | 0 | H | CH | |
| 162 | 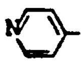 | H | H | H | H | H | H | H | 1 | H | CH | |
| 163 | 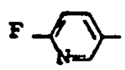 | H | H | H | – | – | H | H | 0 | H | CH | |
| 164 | 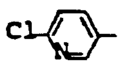 | H | H | H | – | – | H | H | 0 | H | CH | |
| 165 | 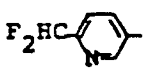 | H | H | H | – | – | H | H | 0 | H | CH | |
| 166 | 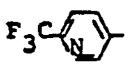 | H | H | H | – | – | H | H | 0 | H | CH | |
| 167 | 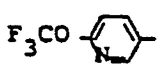 | H | H | H | – | – | H | H | 0 | H | CH | |
| 168 | 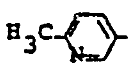 | H | H | H | – | – | H | H | 0 | H | CH | |
| 169 | 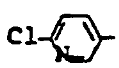 | H | H | H | – | – | H | H | 0 | H | N | |
| 170 | 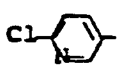 | H | H | H | H | H | H | H | 1 | H | N | $n_D^{20}$ 1,5995 |
| 171 | 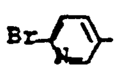 | H | H | H | – | – | H | H | 0 | H | N | |
| 172 | 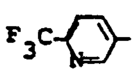 | H | H | H | – | – | H | H | 0 | H | N | |
| 173 | 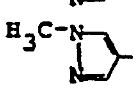 | $CH_3$ | H | H | – | – | H | H | 0 | H | CH | |
| 174 | 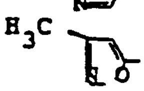 | H | H | H | – | – | H | H | 0 | H | CH | |
| 175 | 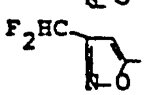 | H | H | H | H | H | H | H | 1 | H | CH | |
| 176 | 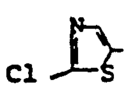 | H | H | H | – | – | H | H | 0 | H | CH | |
| 177 | 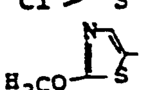 | H | H | H | – | – | H | H | 0 | H | CH | |
| 178 | 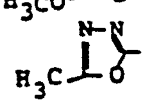 | H | H | H | H | H | H | H | 1 | H | CH | |

Nit 189

| Verb. Nr. | Z | R | $R^1$ | $R^2$ | $R^3$ | $R^4$ | $R^5$ | $R^6$ | n | $R^8$ | Y | |
|---|---|---|---|---|---|---|---|---|---|---|---|---|
| 179 | 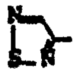 | H | H | H | – | – | H | H | 0 | H | CH | |
| 180 | 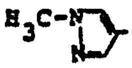 | H | H | H | – | – | H | H | 0 | H | N | |
| 181 | 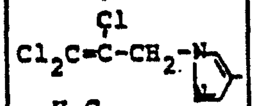 | H | H | H | H | H | H | H | 1 | H | N | |
| 182 | 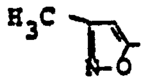 | H | H | H | – | – | H | H | 0 | H | N | |
| 183 | 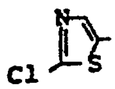 | H | H | H | – | – | H | H | 0 | 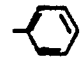 | N | |
| 184 | 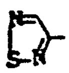 | H | H | H | – | – | H | H | 0 | H | N | |
| 185 | 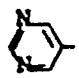 | H | H | H | H | H | H | H | 0 | H | CH | |
| 186 | 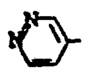 | H | H | H | – | – | H | H | 0 | H | CH | |
| 187 | 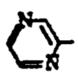 | $CH_3$ | H | H | – | – | H | H | 0 | 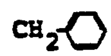 | CH | |
| 188 | 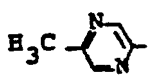 | H | H | H | – | – | H | H | 0 | H | CH | |
| 189 | 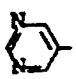 | H | H | H | – | – | H | H | 0 | H | N | |
| 190 | 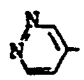 | H | H | H | – | – | H | H | 0 | H | N | |
| 191 | 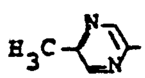 | H | H | H | – | – | H | H | 0 | H | N | |
| 192 | 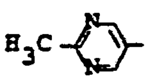 | H | H | H | – | – | H | H | 0 | H | N | |
| 193 | 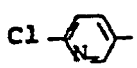 | H | H | H | H | H | H | H | 1 | $CH_3$ | CH | |
| 194 | 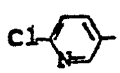 | H | H | H | – | – | H | H | 0 | H | $C-COOC_2H_5$ | |
| 195 | 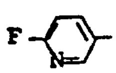 | H | H | H | – | – | H | H | 0 | H | 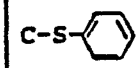 | |
| 196 | 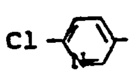 | H | H | H | – | – | H | H | 0 | H | C-Br | |
| 197 | 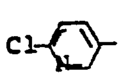 | H | H | H | H | H | H | H | 1 | H | 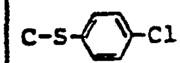 | |
| 198 | 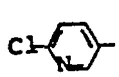 | H | H | H | – | – | H | H | 0 | $CH_3$ | N | |

Tabelle 4 umfaßt den Fall, in dem X ein Sauerstoff-Atom ist.

Nit 189

Tabelle 4

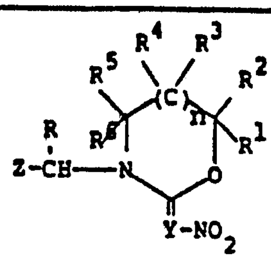

| Verb. Nr. | Z | R | $R^1$ | $R^2$ | $R^3$ | $R^4$ | $R^5$ | $R^6$ | n | Y | |
|---|---|---|---|---|---|---|---|---|---|---|---|
| 199 | 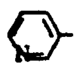 | H | H | H | – | – | H | H | 0 | CH | Schmp. 137-140°C |
| 200 | 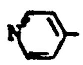 | H | H | H | H | H | H | H | 1 | CH | |
| 201 | 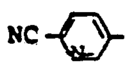 | H | H | H | – | – | H | H | 0 | CH | |
| 202 | 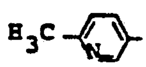 | H | H | H | – | – | H | H | 0 | CH | |
| 203 | 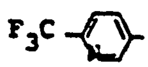 | H | H | H | – | – | H | H | 0 | CH | |
| 204 | 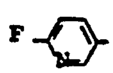 | H | H | H | – | – | H | H | 0 | N | |
| 205 | 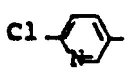 | H | H | H | – | – | H | H | 0 | N | |
| 206 | 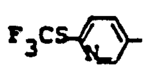 | H | H | H | – | – | H | H | 0 | N | |
| 207 | 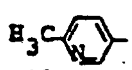 | H | H | H | – | – | H | H | 0 | N | |
| 208 | 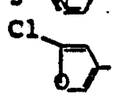 | H | H | H | – | – | H | H | 0 | CH | |
| 209 | 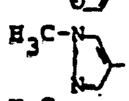 | H | H | H | – | – | H | H | 0 | CH | |
| 210 | 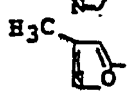 | H | H | H | – | – | H | H | 0 | CH | |
| 211 | 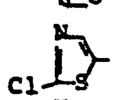 | H | H | H | – | – | H | H | 0 | CH | |
| 212 | 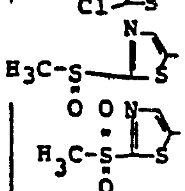 | H | H | H | H | H | H | H | 1 | CH | |
| 213 | | H | H | H | H | H | H | H | 1 | CH | |
| 214 | 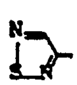 | H | H | H | – | – | H | H | 0 | CH | |

Nit 189

| Verb. Nr. | Z | R | $R^1$ | $R^2$ | $R^3$ | $R^4$ | $R^5$ | $R^6$ | n | Y | |
|---|---|---|---|---|---|---|---|---|---|---|---|
| 215 | 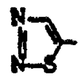 | H | H | H | H | H | H | H | 1 | CH | |
| 216 | 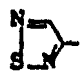 | H | H | H | – | – | H | H | 0 | N | |
| 217 | 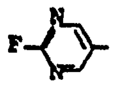 | H | H | H | – | – | H | H | 0 | CH | |
| 218 | 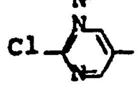 | H | H | H | – | – | H | H | 0 | CH | |
| 219 | 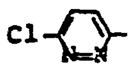 | H | H | H | – | – | H | H | 0 | CH | |
| 220 | 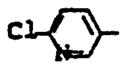 | H | H | H | – | – | H | H | 0 | $C-CH_3$ | |
| 221 | 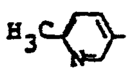 | H | H | H | – | – | $CH_3$ | H | 0 | CH | |
| 222 | 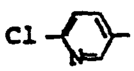 | H | H | H | – | – | H | H | 0 | C-COO- 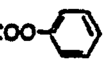 | |
| 223 | 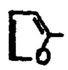 | H | H | H | – | – | H | H | 0 | C-CO- 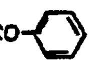 | |
| 224 | 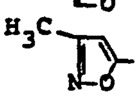 | H | H | H | – | – | H | H | 0 | $C-CO-(CH_2)_2CH_3$ | |

Tabelle 5 umfaßt den Fall, in dem die Verbindungen der Formel (I) durch die folgende Formel dargestellt werden:

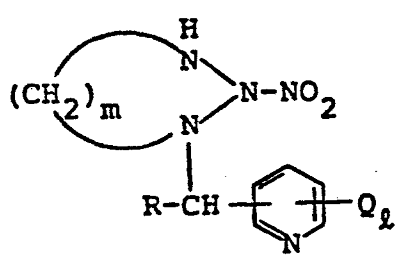

0192060

Tabelle 5

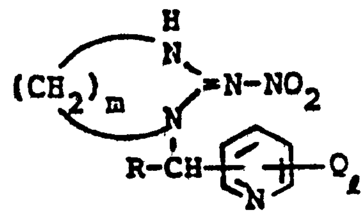

| Verbindung Nr. | m | R | Bindungs-Position am Pyridin-Ring | $Q_\ell$ | |
|---|---|---|---|---|---|
| 225 | 2 | H | 3- | - | Schmp. 90-94°C (Zers.) |
| 226 | 3 | H | 3- | - | |
| 227 | 2 | $-CH_3$ | 3- | - | |
| 228 | 2 | $-C_2H_5$ | 3- | - | |
| 229 | 2 | $-CH(CH_3)_2$ | 3- | - | |
| 230 | 2 | H | 4- | - | Schmp. 154-157°C |
| 231 | 3 | H | 4- | - | Schmp. 163-165°C |
| 232 | 2 | H | 2- | 5-Cl | |
| 233 | 3 | H | 5- | 2-F | |
| 234 | 2 | $-CH_3$ | 5- | 2-Cl | |
| 235 | 2 | H | 5- | 2-Br | |
| 236 | 2 | H | 5- | $2-CH_3$ | Schmp. 157-160°C |
| 237 | 3 | H | 5- | $2-CH_3$ | Schmp. 155,5-158,5°C |
| 238 | 2 | H | 5- | $2-C_2H_5$ | |
| 239 | 2 | H | 5- | $2-OCH_3$ | |
| 240 | 2 | H | 5- | $2-OC_2H_5$ | |
| 241 | 2 | H | 5- | $2-SCH_3$ | |

Nit 189

Tabelle 5 - Fortsetzung



| | | | | | |
|---|---|---|---|---|---|
| 242 | 2 | H | 5- | $2\text{-}NO_2$ | |
| 243 | 2 | H | 5- | 2-CN | |
| 244 | 2 | H | 5- | $2\text{-}NH_2$ | |
| 245 | 3 | H | 5- | $2\text{-}NH\overset{O}{\overset{\|}{C}}CH_3$ | |
| 246 | 2 | H | 5- | $2\text{-}N(CH_3)_2$ | |
| 247 | 2 | H | 5- | $2\text{-}\overset{O}{\overset{\|}{C}}\text{-}OC_2H_5$ | |
| 248 | 2 | H | 5- | $2\text{-}\underset{O}{\underset{\|}{C}}\text{-}CH_3$ | |
| 249 | 2 | H | 5- | $2\text{-}\overset{O}{\overset{\|}{S}}\text{-}CH_3$ | |
| 250 | 2 | H | 5- | $2\text{-}\underset{O}{\underset{\|}{\overset{O}{\overset{\|}{S}}}}\text{-}CH_3$ | |
| 251 | 2 | H | 5- | $2\text{-}CHF_2$ | |
| 252 | 2 | H | 5- | $2\text{-}CF_3$ | Schmp. 134-146°C |
| 253 | 3 | H | 5- | $2\text{-}CF_3$ | |
| 254 | 2 | H | 5- | $2\text{-}CCl_3$ | |
| 255 | 2 | H | 5- | $2\text{-}CF_2Cl$ | |
| 256 | 2 | H | 5- | $2\text{-}CH_2CH_2F$ | |
| 257 | 2 | H | 5- | $2\text{-}CH_2CH_2Cl$ | |
| 258 | 2 | H | 5- | $2\text{-}CH_2CF_3$ | |
| 259 | 3 | H | 5- | $2\text{-}OCHF_2$ | |
| 260 | 2 | H | 5- | $2\text{-}OCF_3$ | |

Tabelle 5 - Fortsetzung



| | | | | | |
|---|---|---|---|---|---|
| 261 | 3 | H | 5- | $2-OCH_2CF_3$ | |
| 262 | 2 | H | 5- | $2-SCF_3$ | |
| 263 | 2 | H | 5- | $2-SCF_2Cl$ | |
| 264 | 2 | H | 5- | 3-Br | Schmp. 198-201°C |
| 265 | 2 | H | 2- | $5-CF_3$ | |
| 266 | 2 | H | 5- | $2-CH_2C{\equiv}CH$ | |
| 267 | 2 | H | 5- | $2-CH_2CH{=}CH_2$ | |
| 268 | 2 | H | 5- | $2-CH{=}C(Cl)_2$ | |
| 269 | 2 | H | 5- | $2,3-Cl_2$ | |
| 270 | 2 | H | 5- | $2-Cl, 3-CH_3$ | |
| 271 | 2 | H | 4- | $2,3,5,6-F_4$ | |
| 272 | 2 | H | 5- | 2-CHO | |
| 273 | 2 | H | 5- | $2-CF_2Br$ | |

Tabelle 6 umfaßt den Fall, in dem Z eine gegebenenfalls substituierte Pyridyl-Gruppe ist und X $N-R^7$ ist; die Verbindungen der Formel (I) werden durch die folgende Formel dargestellt:

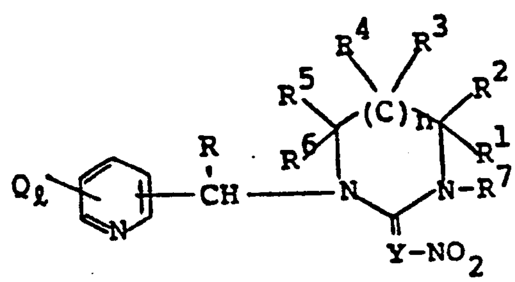

In Tabelle 6 bedeutet die Kennzeichnung " - " für $Q_\ell$ die Abwesenheit eines Substituenten und die Kennzeichnung " - " für $R^2$ und $R^5$ gemeinsam, daß $R^2$ zusammen mit $R^5$ eine Einfachbindung bildet.

Nit 189

Tabelle 6

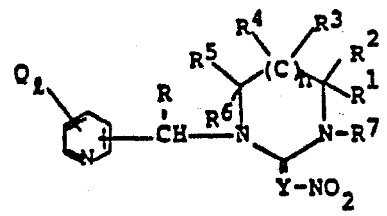

| Verb. Nr. | $Q_l$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 274 | – | 4-Position | H | H | $CH_3$ | – | – | H | H | 0 | H | CH | Schmp. 184-187°C |
| 275 | – | 3-Position | $CH_3$ | H | $CH_3$ | – | – | H | H | 0 | H | CH | |
| 276 | – | 3-Position | H | $CH_3$ | $CH_3$ | – | – | H | H | 0 | H | CH | Schmp. 177-180°C |
| 277 | – | 4-Position | H | H | H | $CH_3$ | $CH_3$ | H | H | 1 | H | CH | Schmp. 190-191°C |
| 278 | – | 4-Position | H | H | H | OH | H | H | H | 1 | H | CH | Schmp. 209-212°C |
| 279 | 2-F | 5-Position | H | H | $CH_3$ | – | – | H | H | 0 | H | CH | |
| 280 | 2-Cl | 5-Position | H | H | H | H | $CH_3$ | H | H | 1 | H | CH | |
| 281 | 2-Cl | 5-Position | H | H | $C_2H_5$ | – | – | H | H | 0 | H | CH | |
| 282 | 2-Cl | 5-Position | H | H | $CH_3$ | – | – | $CH_3$ | H | 0 | H | CH | |
| 283 | 2-Br | 5-Position | H | H | $CH_3$ | – | – | H | H | 0 | H | CH | |
| 284 | 2-$CF_3$ | 5-Position | H | H | $CH_3$ | – | – | H | H | 0 | H | CH | |
| 285 | 2-$CH_3$ | 5-Position | H | H | $CH_3$ | – | – | H | H | 0 | H | CH | |
| 286 | 2-$CF_3$ | 5-Position | H | H | $CH_3$ | – | – | H | H | 0 | H | CH | |
| 287 | 2-$NO_2$, 3-$OCH_3$ | 5-Position | H | H | $CH_3$ | – | – | H | H | 0 | H | CH | |
| 288 | – | 3-Position | H | H | H | – | – | H | H | 0 | $CH_3$ | CH | Schmp. 107-110°C |

| Verb. Nr. | $Q_l$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 289 | 2-F | 5-Position | H | H | H | – | – | H | H | 0 | $CH(CH_3)_2$ | CH | |
| 290 | 2-Cl | 5-Position | H | H | H | – | – | H | H | 0 | $CH_3$ | CH | Schmp. 128-130°C |
| 291 | 2-Cl | 5-Position | H | H | H | H | H | H | H | 1 | $CH_3$ | CH | |
| 292 | 2-Cl | 5-Position | H | H | H | – | – | H | H | 0 | OH | CH | |
| 293 | 2-Cl | 5-Position | H | H | H | – | – | H | H | 0 | $OC_2H_5$ | CH | |
| 294 | 2-Cl | 5-Position | H | H | H | – | – | H | H | 0 | 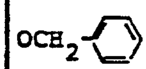 | CH | |
| 295 | 2-Cl | 5-Position | H | H | H | – | – | H | H | 0 | $CH_2OC_2H_5$ | CH | |
| 296 | 2-Cl | 5-Position | H | H | H | – | – | H | H | 0 | $CH_2CH_2OC_4H_9$ | CH | |
| 297 | 2-Cl | 5-Position | H | H | H | – | – | H | H | 0 | $CH_2CH_2SC_2H_5$ | CH | |
| 298 | 2-Cl | 5-Position | H | H | H | – | – | H | H | 0 | $CH_2CH=CH_2$ | CH | |
| 299 | 2-Cl | 5-Position | H | H | H | – | – | H | H | 0 | $CH_2CH_2CN$ | CH | |
| 300 | – | 3-Position | H | H | H | – | – | H | H | 0 | 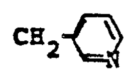 | CH | Schmp. 175-178°C |
| 301 | – | 4-Position | H | H | H | – | – | H | H | 0 | 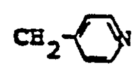 | CH | Schmp. 176-180°C |
| 302 | 2-F | 5-Position | H | H | H | H | H | H | H | 1 | 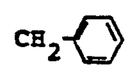 | CH | |
| 303 | 2-Cl | 5-Position | H | H | H | – | – | H | H | 0 | 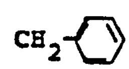 | CH | Schmp. 152-153°C |
| 304 | 2-Cl | 5-Position | H | H | H | – | – | H | H | 0 | 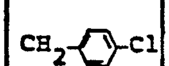 | CH | Schmp. 158-160°C |
| 305 | 2-Cl | 5-Position | H | H | H | – | – | H | H | 0 | 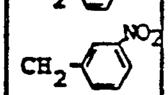 | CH | |
| 306 | 2-$CH_3$ | 5-Position | H | H | H | – | – | H | H | 0 | 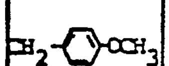 | CH | |
| 307 | 2-Cl | 5-Position | H | H | H | – | – | H | H | 0 | 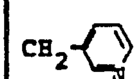 | CH | $n_D^{20}$ 1,6495 |
| 308 | 2-Cl | 5-Position | H | H | H | – | – | H | H | 0 | 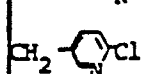 | CH | Schmp. 154-156°C |

Nit 189

| Verb. Nr. | $Q_1$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 309 | 2-F | 5-Position | H | H | H | – | – | H | H | 0 | 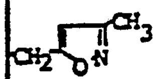 | CH | |
| 310 | 2-Cl | 5-Position | H | H | H | – | – | H | H | 0 | 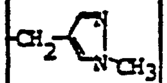 | CH | $n_D^{20}$ 1,6480 |
| 311 | 2-Br | 5-Position | H | H | H | – | – | H | H | 0 | 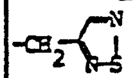 | CH | |
| 312 | 2-Cl | 5-Position | H | H | H | – | – | H | H | 0 | 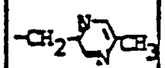 | CH | |
| 313 | 2-Cl | 5-Position | H | H | H | – | – | H | H | 0 | 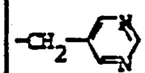 | CH | |
| 314 | – | 3-Position | H | H | H | – | – | H | H | 0 | H | C-Cl | |
| 315 | 2-Cl | 5-Position | H | H | H | – | – | H | H | 0 | H | C-F | |
| 316 | 2-Cl | 5-Position | H | H | $CH_3$ | – | – | H | H | 0 | H | C-Br | |
| 317 | – | 4-Position | H | H | H | H | H | H | H | 1 | H | $C-CH_3$ | |
| 318 | 2-Cl | 5-Position | H | H | H | – | – | H | H | 0 | H | $C-CH_3$ | |
| 319 | 2-Cl | 5-Position | H | H | H | – | – | H | H | 0 | H | $C-CH_2F$ | |
| 320 | 2-Cl | 5-Position | H | H | H | – | – | H | H | 0 | H | $C-CF_3$ | |
| 321 | – | 3-Position | H | H | H | – | – | H | H | 0 | H | $C-CH_2OH$ | |
| 322 | 2-F | 5-Position | H | H | H | H | H | H | H | 1 | H | OH<br>$C-CHCCl_3$ | |
| 323 | 2-Cl | 5-Position | H | H | H | – | – | H | H | 0 | H | OH<br>$C-CHCCl_3$ | Schmp. 135-140°C |
| 324 | 2-Cl | 5-Position | H | H | H | – | – | H | H | 0 | H | 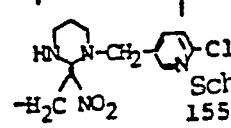 | Schmp. 155-158°C |
| 325 | – | 3-Position | H | H | H | H | H | H | H | 1 | H | C-OH | |

Nit 189

| Verb. Nr. | $Q_l$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 326 | 2-Cl | 5-Position | H | H | H | – | – | H | H | 0 | H | $C-OCH_3$ | |
| 327 | 2-Cl | 5-Position | H | H | H | – | – | H | H | 0 | H | 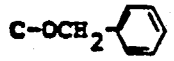 | |
| 328 | – | 4-Position | H | H | H | – | – | H | H | 0 | H | $C-S-C_3H_7$ | |
| 329 | $2-CH_3$ | 5-Position | H | H | H | – | – | H | H | 0 | H | 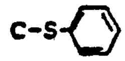 | |
| 330 | – | 3-Position | H | H | H | – | – | H | H | 0 | H | 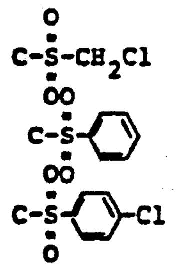 | |
| 331 | 2-Cl | 5-Position | H | H | H | – | – | H | H | 0 | H | | |
| 332 | $2-CF_3$ | 5-Position | H | H | H | – | – | H | H | 0 | H | | |
| 333 | 2-Cl | 5-Position | H | H | H | – | – | H | H | 0 | H | $C-COCH_3$ | |
| 334 | 2-F | 5-Position | H | H | H | – | – | H | H | 0 | H | $C-COCCl_3$ | |
| 335 | $2-NO_2$ | 5-Position | H | H | H | – | – | H | H | 0 | H | $C-COCH=CH_2$ | |
| 336 | 2-Cl | 5-Position | H | H | H | – | – | H | H | 0 | H | $C-COC_3H_7$ | Schmp. 102-105°C |
| 337 | 2-Cl | 5-Position | H | H | H | – | – | H | H | 0 | H | 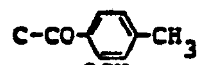 | Schmp. 213-215°C |
| 338 | – | 3-Position | H | H | H | H | H | H | H | 1 | H | 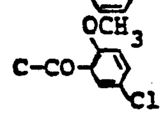 | |
| 339 | – | 4-Position | $CH_3$ | H | H | – | – | H | H | 0 | H | $C-COOCH_3$ | |
| 340 | 2-Cl | 5-Position | H | H | H | – | – | H | H | 0 | H | $C-COOC_2H_5$ | Schmp. 169-171°C |
| 341 | 2-CN | 5-Position | H | H | H | – | – | H | H | 0 | H | $C-COOC_4H_9$ | |
| 342 | 2-F | 5-Position | H | P | H | – | – | H | H | 0 | H | 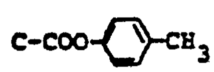 | |
| 343 | 2-Cl | 5-Position | H | H | H | – | – | H | H | 0 | H | 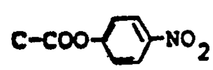 | |
| 344 | 2-Cl | 5-Position | H | H | H | – | – | H | H | 0 | H | $C-COS-C_4H_9$ | |

| Verb. Nr. | Q | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 345 | 2-Cl | 5-Position | H | H | H | – | – | H | H | 0 | H | C-CONHCO-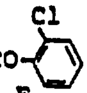 | Schmp. 89-94°C (Zers.) |
| 346 | $2-CF_3$ | 5-Position | H | H | H | – | – | H | H | 0 | H | C-CONHCO-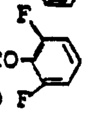 | |
| 347 | $2,3-Cl_2$ | 5-Position | H | H | H | – | – | H | H | 0 | H | 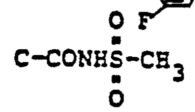 | |
| 348 | $2-CHF_2$ | 5-Position | H | H | H | – | – | H | H | 0 | H | 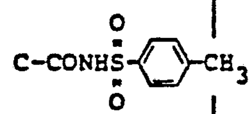 | |
| 349 | – | 3-Position | H | H | H | – | – | H | H | 0 | $CH_3$ | $C-COCH_3$ | |
| 350 | 2-Cl | 5-Position | H | H | H | – | – | H | H | 0 | $CH_3$ | $C-COC_3H_7$ | Schmp. 100-105°C |
| 351 | 2-Cl | 5-Position | H | H | H | – | – | H | H | 0 | $COCH_3$ | $C-COCH_3$ | |
| 352 | $2-CH_3$ | 5-Position | H | H | H | – | – | H | H | 0 | Cl | C-Cl | |
| 353 | – | 3-Position | H | H | H | H | H | H | H | 1 | $COOC_2H_5$ | $C-COOC_2H_5$ | |
| 354 | 2-Cl | 5-Position | H | H | H | – | – | H | H | 0 | COO-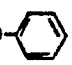 | C-COO-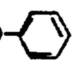 | Glas |
| 355 | $2-CF_3$ | 5-Position | H | H | H | – | – | H | H | 0 | COO-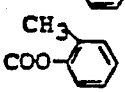 | C-COO-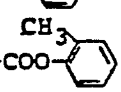 | |
| 356 | – | 4-Position | H | H | H | – | – | H | H | 0 | COS-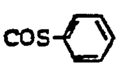 | COS-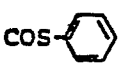 | |
| 357 | 2-Cl | 5-Position | H | H | H | – | – | H | H | 0 | S-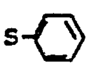 | C-S-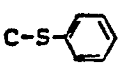 | |
| 358 | – | 3-Position | H | H | H | $CH_3$ | H | H | H | 1 | H | N | |
| 359 | 2-Cl | 5-Position | H | H | $CH_3$ | – | – | H | H | 0 | H | N | |
| 360 | 2-Cl | 5-Position | H | $CH_3$ | $CH_3$ | – | – | H | H | 0 | H | N | |
| 361 | 2-Cl | 5-Position | H | H | H | – | – | H | H | 0 | $CH_3$ | N | |
| 362 | 2-SCN | 5-Position | H | H | H | – | – | H | H | 0 | $CH(CH_3)_2$ | N | |

Nit 189

| Verb. Nr. | $Q_1$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 363 | 2-F | 5- Position | H | H | H | – | – | H | H | 0 | $CH_2CH_2Cl$ | N | |
| 364 | 2-Cl | 5- Position | H | H | H | – | – | H | H | 0 | $CH_2CH{=}CH_2$ | N | $n_D^{30}$ 1,5854 |
| 365 | 2-Cl | 5- Position | H | H | H | – | – | H | H | 0 | $CH_2CCl{=}CCl_2$ | N | |
| 366 | 2-Cl | 5- Position | H | H | H | – | – | H | H | 0 | $CH_2CH_2CN$ | N | |
| 367 | 2-F | 5- Position | H | H | H | – | – | H | H | 0 | $CH_2C{\equiv}CH$ | N | |
| 368 | $2-CF_3$ | 5- Position | H | H | H | – | – | H | H | 0 | $CH_2CH_2N(C_2H_5)_2$ | N | |
| 369 | 2-Cl | 5- Position | H | H | H | H | H | H | H | 1 | $CH_2OCH_3$ | N | |
| 370 | – | 3- Position | H | H | H | – | – | H | H | 0 | $CH_2CH_2SC_2H_5$ | N | |
| 371 | $2-CH_2F$ | 5- Position | H | H | H | – | – | H | H | 0 | $CH_2SC_4H_9$ | N | |
| 372 | 2-Cl | 5- Position | H | H | H | – | – | H | H | 0 | $CH_2CN$ | N | |
| 373 | 2-Cl | 5- Position | H | H | H | – | – | H | H | 0 | $CH_2Si(CH_3)_3$ | N | |
| 374 | $5-CF_3$ | 2- Position | H | H | H | – | – | H | H | 0 | $CH_2Si(CH_3)_3$ | N | |
| 375 | 2-Cl | 5- Position | H | H | H | H | H | H | H | 1 | 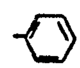 | N | |
| 376 | $2-NO_2$ | 5- Position | H | H | H | – | – | H | H | 0 | $CH_2$- 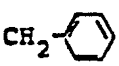 | N | |
| 377 | – | 3- Position | H | H | H | – | – | H | H | 0 | $CH_2$ 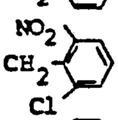 | N | |
| 378 | 2-Cl | 5- Position | H | H | H | – | – | H | H | 0 | $CH_2$- 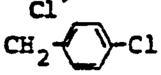 | N | |
| 379 | $5-OCF_3$ | 2- Position | H | H | H | – | – | H | H | 0 | $CH_2$ 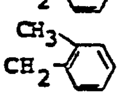 | N | |
| 380 | 2-Cl | 5- Position | H | H | H | – | – | H | H | 0 | $CH_2$- 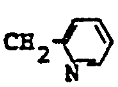 | N | Schmp. 126-128°C |
| 381 | 2-Cl | 5- Position | H | H | H | – | – | H | H | 0 | $CH_2$- 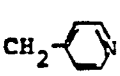 | N | Schmp. 146-148°C |
| 382 | 2-Cl | 5- Position | H | H | H | – | – | H | H | 0 | $CH_2$- 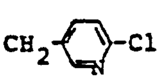 | N | Schmp. 128-131°C |

Nit 189

| Verb. Nr. | $Q_1$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 383 | — | 3-Position | H | H | H | — | — | H | H | 0 | 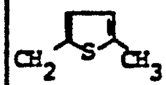 | N | |
| 384 | 2-$CH_3$ | 5-Position | H | H | H | H | H | H | H | 1 | 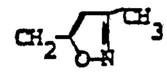 | N | |
| 385 | 2-Cl | 5-Position | H | H | H | H | H | H | H | 1 | 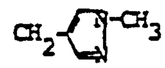 | N | |
| 386 | 2-Cl | 5-Position | H | H | H | H | H | H | H | 1 | 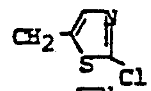 | N | |
| 387 | 2-F | 5-Position | H | H | H | — | — | H | H | 0 | 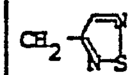 | N | |
| 388 | 2-Cl | 5-Position | H | H | H | — | — | H | H | 0 | 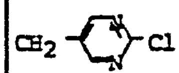 | N | |
| 389 | 2-Cl | 5-Position | H | H | H | — | — | H | H | 0 | 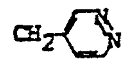 | N | |
| 390 | 2-$CH_3$ | 5-Position | H | H | H | — | — | H | H | 0 | 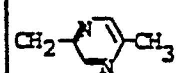 | N | |
| 391 | 2-F | 5-Position | H | H | H | — | — | H | H | 0 | $CH_2COCH_3$ | N | |
| 392 | 2-Cl | 5-Position | H | H | H | — | — | H | H | 0 | 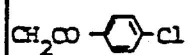 | N | |
| 393 | 2-Cl | 5-Position | H | H | H | — | — | H | H | 0 | 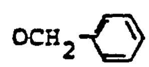 | N | |
| 394 | — | 3-Position | $CH_3$ | H | H | — | — | H | H | 0 | $COCH_3$ | N | |
| 395 | 2-Cl | 5-Position | H | H | H | — | — | H | H | 0 | CHO | N | |
| 396 | 2-Cl | 5-Position | H | H | H | — | — | H | H | 0 | $COCH_3$ | N | Schmp. 144,5-146°C |
| 397 | 2-Cl | 5-Position | H | H | H | H | H | H | H | 1 | $COCH_3$ | N | |
| 398 | 2-F | 5-Position | H | H | H | — | — | H | H | 0 | $COCCl_3$ | N | |
| 399 | 2-Cl, 4-F | 5-Position | H | H | H | — | — | H | H | 0 | $COC_3H_7$ | N | |
| 400 | 2-$C_2H_5$ | 5-Position | H | H | H | — | — | H | H | 0 | $COCH_3$ | N | |
| 401 | 2-Cl | 5-Position | H | H | H | — | — | H | H | 0 | $COC(CH_3)_3$ | N | |
| 402 | 2-$CH_3$ | 5-Position | H | H | H | — | — | H | H | 0 | Br<br>$COCH-C(CH_3)_3$ | N | |

Nit 189

| Verb. Nr. | $Q_1$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 403 | 2,3-$F_2$ | 5-Position | H | H | H | – | – | H | H | 0 | $COCH_2OCH_3$ | N | |
| 404 | 2-Br | 5-Position | H | H | H | – | – | H | H | 0 | $COCH=CH_2$ | N | |
| 405 | 2-Cl | 5-Position | H | H | H | – | – | H | H | 0 | 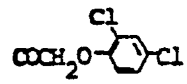 | N | |
| 406 | – | 3-Position | H | H | H | – | – | H | H | 0 | 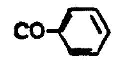 | N | |
| 407 | – | 4-Position | H | H | H | H | H | H | H | 1 | 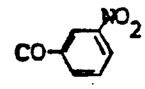 | N | |
| 408 | 2-Cl | 5-Position | H | H | H | – | – | H | H | 0 | 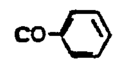 | N | Schmp. 146-150°C |
| 409 | 2-F | 5-Position | H | H | H | – | – | H | H | 0 | 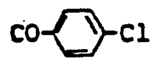 | N | Schmp. 149-152°C |
| 410 | 2-Cl | 5-Position | H | H | H | – | – | H | H | 0 | 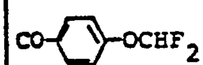 | N | |
| 411 | 2-Cl | 5-Position | H | H | H | – | – | H | H | 0 | 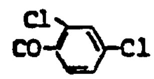 | N | |
| 412 | – | 3-Position | H | H | H | – | – | H | H | 0 | 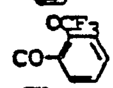 | N | |
| 413 | 2-CH=$CCl_2$ | 5-Position | H | H | H | – | – | H | H | 0 | 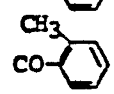 | N | |
| 414 | 2-$CF_3$ | 5-Position | H | H | H | – | – | H | H | 0 | 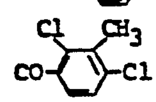 | N | |
| 415 | 2-$CH_3$ | 5-Position | H | H | H | – | – | H | H | 0 | 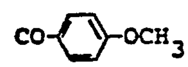 | N | |
| 416 | 2-Cl | 5-Position | H | H | H | – | – | H | H | 0 | 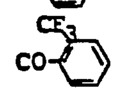 | N | |
| 417 | 2-Cl | 5-Position | H | H | H | – | – | H | H | 0 | 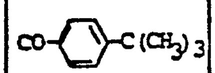 | N | |
| 418 | 2-Cl | 5-Position | H | H | H | – | – | H | H | 0 | 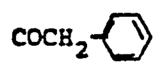 | N | |
| 419 | – | 3-Position | H | H | H | H | H | H | H | 1 | 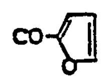 | N | |
| 420 | 2-F | 5-Position | H | H | H | – | – | H | H | 0 | 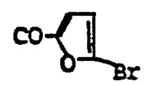 | N | |
| 421 | 2-$CH_3$ | 5-Position | H | H | H | – | – | H | H | 0 | 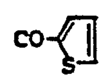 | N | |
| 422 | 2-Cl | 5-Position | H | H | H | – | – | H | H | 0 | 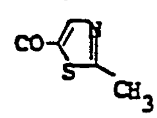 | N | |

Nit 189

| Verb. Nr. | $Q_1$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 423 | 2,6-$Cl_2$ | 4-Position | H | H | H | – | – | H | H | 0 | CO-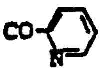 | N | |
| 424 | 2-Cl | 5-Position | H | H | H | – | – | H | H | 0 | CO-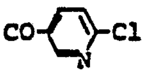-Cl | N | Schmp. 118-122°C |
| 425 | 2-Cl | 5-Position | H | H | H | – | – | H | H | 0 | CO-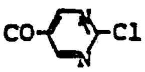-Cl | N | |
| 426 | – | 3-Position | H | H | H | H | $CH_3$ | H | H | 1 | $COOCH_3$ | N | |
| 427 | 2-F | 5-Position | H | H | H | – | – | H | H | 0 | $COOC_2H_5$ | N | |
| 428 | 2-Cl | 5-Position | H | H | H | – | – | H | H | 0 | $COOC_2H_5$ | N | Schmp. 125-126°C |
| 429 | 2-$CH_3$ | 5-Position | H | H | H | – | – | H | H | 0 | $COOC_4H_9$ | N | |
| 430 | 2-Cl | 5-Position | H | H | H | – | – | H | H | 0 | $COOCH_2CF_3$ | N | |
| 431 | 2-Cl | 5-Position | H | H | H | – | – | H | H | 0 | COO-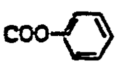 | N | Schmp. 135-140°C |
| 432 | 2-Cl | 5-Position | $CH_3$ | H | H | – | – | H | H | 0 | COO-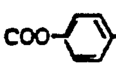-$CH_3$ | N | |
| 433 | 2-Cl | 5-Position | H | H | H | – | – | H | H | 0 | COO-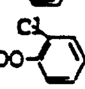-Cl | N | |
| 434 | 2-$CHF_2$ | 5-Position | H | H | H | – | – | H | H | 0 | $COOCH_2$-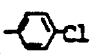-Cl | N | |
| 435 | 2-Cl | 5-Position | H | H | H | – | – | H | H | 0 | $COSC_2H_5$ | N | |
| 436 | 2-Cl | 5-Position | H | H | H | – | – | H | H | 0 | COS-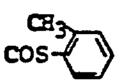 | N | |
| 437 | 2,3-$F_2$ | 5-Position | H | H | H | – | – | H | H | 0 | O<br>S-$CH_3$<br>O | N | |
| 438 | 2-Cl | 5-Position | H | H | H | – | – | H | H | 0 | O<br>S-$CH_3$<br>O | N | Schmp. 191-192°C |
| 439 | 2-Cl | 5-Position | H | H | H | – | – | H | H | 0 | O<br>S-$CH_2Cl$<br>O | N | |
| 440 | 2-Cl | 5-Position | H | H | H | – | – | H | H | 0 | O<br>S-$CH_2CH_2Cl$<br>O | N | |

Nit 189

| Verb. Nr. | $Q_{\ell}$ | Bindungs-Position am Pyridin-Ring | R | $R^1$ | $R^2$ | $R^3$ | $R^4$ | $R^5$ | $R^6$ | n | $R^7$ | Y | |
|---|---|---|---|---|---|---|---|---|---|---|---|---|---|
| 441 | 2-$COOCH_3$ | 6-Position | H | H | H | – | – | H | H | 0 | 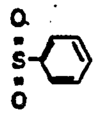 | N | |
| 442 | 2-Cl | 5-Position | H | H | H | – | – | H | H | 0 | 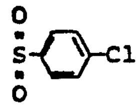 | N | |
| 443 | 2-Cl | 5-Position | H | H | H | – | – | H | H | 0 | 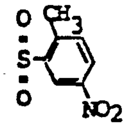 | N | |
| 444 | 2-Cl | 5-Position | H | H | H | – | – | H | H | 0 | $CON(CH_3)_2$ | N | Schmp. 186-188°C |
| 445 | 2-Cl | 5-Position | H | H | H | – | – | H | H | 0 | 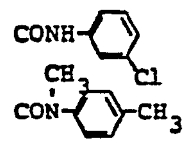 | N | Schmp. 120-123°C |
| 446 | 2-F | 5-Position | H | H | H | – | – | H | H | 0 | | N | |
| 447 | 2-Cl, 6-F | 5-Position | H | H | H | – | – | H | H | 0 | 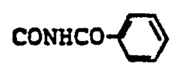 | N | |
| 448 | 2-CN | 5-Position | H | H | H | – | – | H | H | 0 | 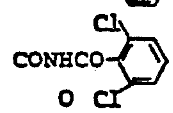 | N | |
| 449 | – | 4-Position | H | H | H | H | H | H | H | 1 | $CONHSO_2-CH_3$ | N | |
| 450 | 2-Cl | 5-Position | H | H | H | – | – | H | H | 0 | 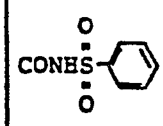 | N | |
| 451 | 2-Cl | 5-Position | H | H | H | – | – | H | H | 0 | 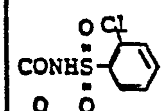 | N | |
| 452 | – | 3-Position | H | H | H | – | – | H | H | 0 | $P(O)(OCH_3)_2$ | N | $n_D^{24}$ 1,5760 |
| 453 | 2-Cl | 5-Position | H | H | H | – | – | H | H | 0 | $P(O)(OC_2H_5)(SC_3H_7)$ | N | $n_D^{20}$ 1,5615 |
| 454 | 2-Cl | 5-Position | H | H | H | – | – | H | H | 0 | $P(S)(OC_2H_5)_2$ | N | |
| 455 | 2-Cl | 5-Position | H | H | H | – | – | H | H | 0 | 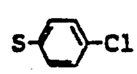 | N | |
| 456 | 2-Cl | 5-Position | H | H | – | H | H | – | H | 1 | H | CH | |

Neben Tabelle 5 umfassen
Tabelle 7 den Fall, in dem die Verbindungen der Formel (I) durch die folgende Formel dargestellt werden:

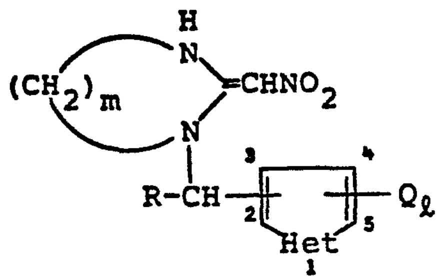

,

Tabelle 8 den Fall, in dem die Verbindungen der Formel (I) durch die folgende Formel dargestellt werden:

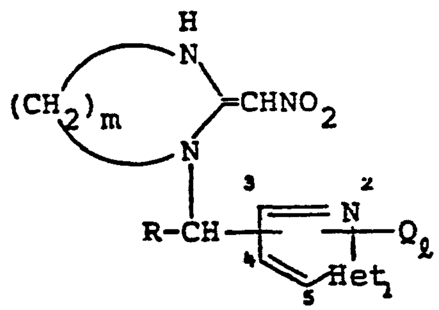

,

Tabelle 9 den Fall, in dem die Verbindungen der Formel (I) durch die folgende Formel dargestellt werden:

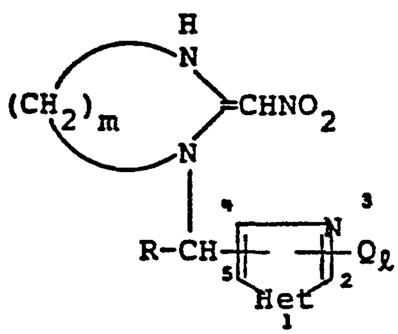

,

und Tabelle 10 den Fall, in dem die Verbindungen der Formel (I) durch die folgende Formel dargestellt werden:

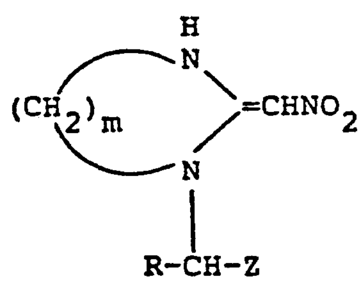

.

Tabelle 7

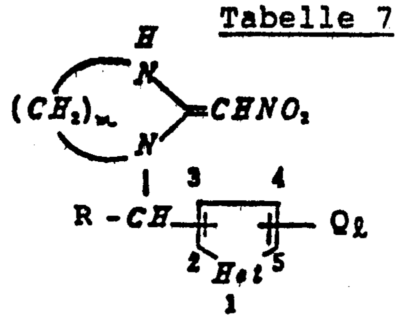

| Verbindung Nr. | Het | Bindungs-Position des Heterocyclus | $Q_\ell$ | R | m | |
|---|---|---|---|---|---|---|
| 457 | O | 2 | - | H | 2 | |
| 458 | O | 2 | - | H | 3 | Schmp. 194~196℃ |
| 459 | O | 3 | - | H | 3 | |
| 460 | S | 2 | - | H | 2 | |
| 461 | S | 2 | - | H | 3 | Schmp. 189~190℃ |
| 462 | S | 3 | - | H | 3 | Schmp. 196~198℃ |
| 463 | S | 3 | - | $-CH_3$ | 2 | |
| 464 | N | 2 | 1 - H | H | 2 | Schmp. 201~205℃ |
| 465 | N | 2 | 1 - H | H | 3 | Schmp. 183~185℃ |
| 466 | N | 3 | 1 - H | H | 3 | |
| 467 | N | 2 | $1-CH_3$ | H | 2 | |
| 468 | N | 2 | $1-CH_3$ | H | 3 | Schmp. 207~213℃ |
| 469 | N | 2 | $1-C_2H_5$ | H | 2 | |
| 470 | N | 3 | $1-CH_3$ | H | 3 | |
| 471 | O | 2 | $5-CH_3$ | H | 2 | Schmp. 151~152℃ |
| 472 | O | 2 | $5-CH_3$ | H | 3 | |
| 473 | O | 3 | $5-CH_3$ | H | 2 | |
| 474 | S | 2 | $5-CH_3$ | H | 2 | |
| 475 | S | 3 | $5-CH_3$ | H | 3 | |

| | | | | | | |
|---|---|---|---|---|---|---|
| 476 | N | 3 | 1-H, 5-$CH_3$ | H | 3 | |
| 477 | N | 3 | 1-H, 5-$CH_3$ | H | 2 | |
| 478 | O | 3 | 2, 5-$(CH_3)_2$ | H | 3 | |
| 479 | S | 3 | 2, 5-$(CH_3)_2$ | H | 3 | |
| 480 | O | 3 | 5-F | H | 2 | |
| 481 | O | 2 | 4-Cl | H | 2 | |
| 482 | O | 2 | 5-Cl | H | 2 | Schmp. 130~131℃ |
| 483 | O | 3 | 5-Cl | H | 3 | |
| 484 | S | 3 | 5-Cl | H | 2 | |
| 485 | N | 3 | 1-$CH_3$, 5-Cl | H | 3 | |
| 486 | O | 3 | 5-Br | H | 2 | |
| 487 | S | 2 | 5-Br | H | 3 | Schmp. 184~186℃ |
| 488 | O | 2 | 4, 5-$Cl_2$ | H | 2 | |
| 489 | S | 3 | 4, 5-$Cl_2$ | H | 2 | |
| 490 | S | 2 | 4, 5-$Br_2$ | H | 2 | |
| 491 | O | 2 | 5-$NO_2$ | H | 2 | |
| 492 | S | 2 | 4-$NO_2$ | H | 3 | |
| 493 | S | 2 | 5-$NO_2$ | H | 2 | |
| 494 | N | 3 | 1-$CH_3$, 5-$NO_2$ | H | 3 | |
| 495 | O | 2 | 5-CN | H | 2 | Schmp. 212~215℃ |
| 496 | O | 3 | 5-CN | H | 3 | |
| 497 | S | 3 | 5-CN | H | 2 | |
| 498 | N | 3 | 1-$CH_3$, 5-CN | H | 2 | |
| 499 | O | 2 | 5-$CF_3$ | H | 2 | |
| 500 | O | 2 | 5-$CHF_2$ | H | 3 | |
| 501 | S | 3 | 5-$CF_3$ | H | 3 | |

Nit 189

| | | | | | | |
|---|---|---|---|---|---|---|
| 502 | N | 3 | 1-$CH_3$, 5-$CF_3$ | H | 2 | |
| 503 | S | 2 | 5-$OCH_3$ | H | 2 | |
| 504 | O | 2 | 5-$SCH_3$ | H | 3 | |
| 505 | S | 3 | 2,5-$(SCH_3)_2$ | H | 2 | |
| 506 | O | 2 | 5-$SCF_3$ | H | 2 | Schmp. 143~144℃ |
| 507 | O | 2 | 5-$SCF_3$ | H | 3 | Schmp. 120~124℃ |
| 508 | S | 2 | 5-$CH{=}CCl_2$ | H | 2 | |
| 509 | O | 2 | 5-O- 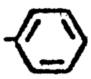 | H | 2 | Schmp. 127~129℃ |
| 510 | O | 2 | 5-$COOC_2H_5$ | H | 2 | |
| 511 | S | 2 | 5-CHO | H | 2 | |
| 512 | S | 2 | 4-$CH_3$ | -$CH_3$ | 2 | Schmp. 170~171,5℃ |

Tabelle 8

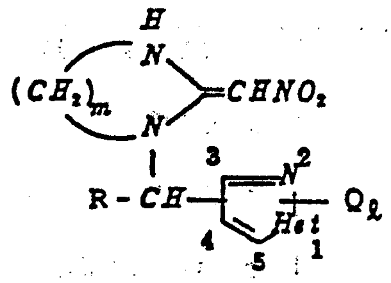

| Verbindung Nr. | Het | Bindungs-Position des Heterocyclus | $Q_\ell$ | R | m | |
|---|---|---|---|---|---|---|
| 513 | O | 3 | - | H | 3 | |
| 514 | O | 4 | - | H | 2 | |
| 515 | O | 4 | - | H | 3 | |
| 516 | O | 4 | - | $-CH_3$ | 2 | |
| 517 | O | 5 | - | H | 3 | |
| 518 | O | 3 | $5-CH_3$ | H | 3 | Schmp. 186~188°C |
| 519 | O | 5 | $3-CH_3$ | H | 3 | Schmp. 200~201°C |
| 520 | O | 5 | $3-C_2H_5$ | H | 2 | |
| 521 | O | 5 | $3-C_3H_7-iso$ | H | 2 | |
| 522 | O | 5 | $3-F$ | H | 3 | |
| 523 | O | 5 | $3-Cl$ | H | 2 | |
| 524 | O | 5 | $3-Br$ | H | 2 | |
| 525 | O | 5 | $3-OH$ | H | 2 | |
| 526 | O | 5 | $3-NO_2$ | H | 3 | |
| 527 | O | 5 | $3-CN$ | H | 3 | |
| 528 | O | 5 | $3-CF_3$ | H | 2 | |
| 529 | O | 5 | $3-CF_3$ | H | 3 | Schmp. 177~180°C |
| 530 | O | 5 | $3-CHF_2$ | H | 2 | |

Nit 189

0192060

| | | | | | | |
|---|---|---|---|---|---|---|
| 531 | O | 5 | 3-$CF_2Cl$ | H | 2 | |
| 532 | O | 5 | 3-$CH_2Cl$ | H | 2 | |
| 533 | O | 5 | 3-$CH_2OCH_3$ | H | 2 | |
| 534 | O | 5 | 3-$CH_2OCH(CH_3)_2$ | H | 2 | |
| 535 | O | 5 | 3-$CCl_3$ | H | 2 | |
| 536 | O | 5 | 3-$OCH_3$ | H | 3 | |
| 537 | O | 5 | 3-$OCF_3$ | H | 2 | |
| 538 | O | 4 | 2,5-$(CH_3)_2$ | H | 2 | |
| 539 | S | 3 | - | H | 3 | |
| 540 | S | 4 | - | H | 2 | |
| 541 | S | 5 | - | H | 2 | |
| 542 | S | 3 | 5-$CH_3$ | H | 3 | |
| 543 | S | 5 | 3-$CH_3$ | H | 2 | |
| 544 | S | 5 | 3-F | H | 2 | |
| 545 | S | 3 | 5-Cl | H | 2 | |
| 546 | S | 5 | 3-Cl | H | 2 | |
| 547 | S | 3 | 5-Br | H | 2 | |
| 548 | S | 5 | 3-$NO_2$ | H | 3 | |
| 549 | S | 5 | 3-$SCF_3$ | H | 2 | |
| 550 | N | 5 | 1-H | H | 2 | Schmp. 190～193℃ |
| 551 | N | 5 | 1-H | H | 3 | |
| 552 | N | 4 | 1-H | H | 2 | Schmp. 196～198℃ |
| 553 | N | 4 | 1-H | -$CH_3$ | 2 | |
| 554 | N | 4 | 1-H | H | 3 | Schmp. 222～225℃ |
| 555 | N | 3 | 1-$CH_3$ | H | 2 | Schmp. 212～215℃ |
| 556 | N | 4 | 1-$CH_3$ | H | 2 | Schmp. 179～180℃ |
| 557 | N | 4 | 1-$CH_3$ | -$CH_3$ | 3 | |

| | | | | | | |
|---|---|---|---|---|---|---|
| 558 | N | 5 | 1-$CH_3$ | H | 2 | |
| 559 | N | 4 | 1-$C_2H_5$ | H | 2 | |
| 560 | N | 4 | 1-$C_2H_5$ | H | 3 | Schmp. 145~148℃ |
| 561 | N | 4 | 1-$C_3H_7$ | H | 3 | Schmp. 99~101℃ |
| 562 | N | 4 | 1-$C_3H_7$-iso | H | 2 | |
| 563 | N | 4 | 1-$C_3H_7$-iso | H | 3 | Schmp. 136~137℃ |
| 564 | N | 4 | 1-$CH_2CH{=}CH_2$ | H | 3 | Schmp. 109~112℃ |
| 565 | N | 4 | 1-$CH_2C{\equiv}CH$ | H | 2 | |
| 566 | N | 4 | 1-$C_4H_9$-tert | H | 2 | Schmp. 126~128℃ |
| 567 | N | 4 | 1-$C_4H_9$-tert | H | 3 | Schmp. 153~156℃ |
| 568 | N | 4 | 1- 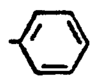 | H | 2 | Schmp. 151~153℃ |
| 569 | N | 4 | 1- 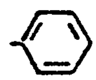 | H | 3 | Schmp. 177~180℃ |
| 570 | N | 4 | 1-$CH_2$- 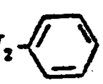 | H | 2 | Schmp. 111~113℃ |
| 571 | N | 4 | 1-$CH_2$- 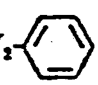 | H | 3 | Schmp. 159~163℃ |
| 572 | N | 4 | 1-$CF_3$ | H | 2 | |
| 573 | N | 3 | 1-$CH_2CF_3$ | H | 2 | |
| 574 | N | 4 | 1-$CH_2CF_3$ | H | 2 | |
| 575 | N | 5 | 1-H, 3-$CH_3$ | H | 2 | Schmp. 183~185℃ |
| 576 | N | 5 | 1-H, 3-$CH_3$ | H | 3 | |
| 577 | N | 3 | 1-H, 5-Cl | H | 2 | |
| 578 | N | 3 | 1-$CH_3$, 5-F | H | 2 | |
| 579 | N | 3 | 1-$CH_3$, 5-Cl | H | 2 | Schmp. 195~198℃ |
| 580 | N | 3 | 1-$CH_3$, 5-Cl | H | 3 | Schmp. 222~224℃ |
| 581 | N | 3 | 1-$C_2H_5$, 5-Cl | H | 2 | |

| | | | | | | |
|---|---|---|---|---|---|---|
| 582 | N | 3 | 1-$C_3H_7$-iso,5-Cl | H | 2 | |
| 583 | N | 5 | 1-$CH_3$ ,5-Cl | H | 2 | |
| 584 | N | 3 | 1-H,5-$CF_3$ | H | 2 | |
| 585 | N | 3 | 1-$CH_3$ ,5-$CF_3$ | H | 2 | |
| 586 | N | 5 | 1-$CH_3$ ,5-$CF_3$ | H | 2 | |
| 587 | N | 4 | 1,3,5-$(CH_3)_3$ | H | 3 | Schmp. 192~194℃ |
| 588 | N | 5 | 1-$CH_2CF_3$ | H | 2 | Schmp. 165~168℃ |

Nit 189

Tabelle 9

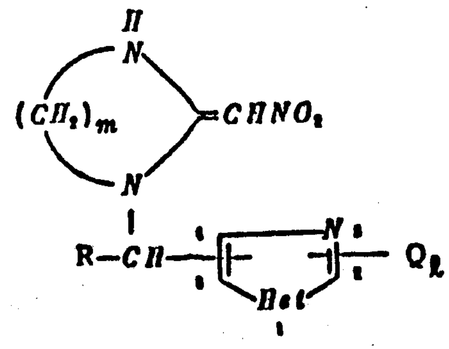

| Verbindung Nr. | Het | Bindungs-Positicn des Heterocyclus | Qℓ | R | m | |
|---|---|---|---|---|---|---|
| 589 | O | 4 | — | H | 2 | |
| 590 | O | 5 | — | H | 2 | |
| 591 | O | 5 | — | H | 3 | |
| 592 | O | 4 | 2-$CH_3$ | H | 2 | |
| 593 | O | 5 | 2-$CH_3$ | H | 2 | |
| 594 | O | 5 | 2-$CH_3$ | H | 3 | |
| 595 | O | 5 | 4-$CH_3$ | H | 2 | Schmp. 200~202°C |
| 596 | O | 5 | 4-$CH_3$ | H | 3 | Schmp. 221~224°C |
| 597 | O | 5 | 2-F | H | 2 | |

Nit 189

| | | | | | | |
|---|---|---|---|---|---|---|
| 598 | O | 5 | 2—Cl | H | 2 | |
| 599 | O | 5 | 2—Cl | H | 3 | |
| 600 | O | 5 | 2—$CF_3$ | H | 2 | |
| 601 | O | 5 | 2—$SCF_3$ | H | 2 | |
| 602 | O | 5 | 2—$OCF_3$ | H | 2 | |
| 603 | O | 5 | 2, 4—$(CH_3)_2$ | H | 2 | |
| 604 | O | 2 | 5—$COOC_2H_5$ | H | 3 | |
| 605 | S | 4 | — | H | 2 | Schmp. 213~216℃ |
| 606 | S | 4 | — | H | 3 | Schmp. 181~183℃ |
| 607 | S | 5 | — | H | 2 | Schmp. 169~174℃ |
| 608 | S | 5 | — | —$CH_3$ | 2 | |
| 609 | S | 5 | — | —$C_3H_7$—iso | 2 | |
| 610 | S | 5 | — | H | 3 | |
| 611 | S | 4 | 2—$CH_3$ | H | 2 | Schmp. 170~172℃ |
| 612 | S | 4 | 2—$CH_3$ | H | 3 | Schmp. 203~206℃ |
| 613 | S | 5 | 2—$CH_3$ | H | 2 | Schmp. 162~166℃ |

0192060

| | | | | | | |
|---|---|---|---|---|---|---|
| 614 | S | 5 | 2—$CH_3$ | —$CH_3$ | 2 | |
| 615 | S | 5 | 2—$CH_3$ | —$CH_3$ | 3 | |
| 616 | S | 5 | 2—$C_2H_5$ | H | 2 | |
| 617 | S | 5 | 2—$C_3H_7$—iso | H | 3 | |
| 618 | S | 5 | 4—$CH_3$ | H | 2 | |
| 619 | S | 4 | 2—F | H | 2 | |
| 620 | S | 4 | 2—Cl | H | 2 | Schmp. 162～165℃ |
| 621 | S | 4 | 2—Cl | H | 3 | Schmp. 190～194℃ |
| 622 | S | 5 | 2—F | H | 3 | |
| 623 | S | 5 | 2—Cl | H | 2 | Schmp. 191～192℃ |
| 624 | S | 5 | 2—Cl | H | 3 | Schmp. 203～205℃ |
| 625 | S | 5 | 2—Cl | —$CH_3$ | 3 | |
| 626 | S | 5 | 2—Cl | —$C_2H_5$ | 3 | |
| 627 | S | 5 | 2, 4—$Cl_2$ | H | 2 | Schmp. 179～181℃ |
| 628 | S | 4 | 2—$NO_2$ | H | 2 | |
| 629 | S | 5 | 2—$NO_2$ | H | 3 | |

| | | | | | | |
|---|---|---|---|---|---|---|
| 630 | S | 4 | $2-CN$ | H | 2 | |
| 631 | S | 5 | $2-CN$ | H | 2 | |
| 632 | S | 4 | $2-SCH_3$ | H | 3 | |
| 633 | S | 5 | $2-SH$ | H | 2 | |
| 634 | S | 5 | $2-SCH_3$ | H | 3 | |
| 635 | S | 5 | $2-SCHF_2$ | H | 2 | |
| 636 | S | 5 | $2-SCF_3$ | H | 2 | |
| 637 | S | 5 | $2-SCF_2Cl$ | H | 2 | |
| 638 | S | 5 | $2-SCH_2CF_3$ | H | 2 | |
| 639 | S | 5 | $2-SCH_2C(Cl)=CCl_2$ | H | 2 | |
| 640 | S | 5 | $2-SCN$ | H | 2 | |
| 641 | S | 4 | $2-NH_2$ | H | 2 | Schmp. 165~167℃ |
| 642 | S | 4 | $2-NHC(=O)CH_3$ | H | 2 | |
| 643 | S | 4 | $2-OCH_3$ | H | 3 | |

Nit 189

| | | | | | | |
|---|---|---|---|---|---|---|
| 644 | S | 5 | 2—$OCH_3$ | H | 3 | |
| 645 | S | 5 | 2—$OCF_3$ | H | 2 | |
| 646 | S | 5 | 2—$OCHF_2$ | H | 3 | |
| 647 | S | 5 | 2—$CH_2Cl$ | H | 2 | |
| 648 | S | 5 | 2—$CHF_2$ | H | 2 | |
| 649 | S | 5 | 2—$CF_3$ | H | 2 | |
| 650 | S | 5 | 2—$CF_3$ | H | 3 | |
| 651 | S | 5 | 2—$CF_2CHF_2$ | H | 2 | |
| 652 | S | 5 | 2— 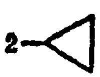 | H | 3 | |
| 653 | S | 4 | 2— 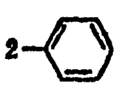 | H | 2 | Schmp. 169~172℃ |
| 654 | S | 4 | 2— 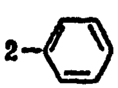 | H | 3 | Schmp. 158~159℃ |
| 655 | N | 2 | 1—H | H | 2 | Schmp. 239~240℃ |
| 656 | N | 4 | 1—H | H | 2 | |
| 657 | N | 4 | 1—H | H | 3 | Schmp. 169~173℃ |

Nit 189

| | | | | | | |
|---|---|---|---|---|---|---|
| 658 | N | 2 | $1-CH_3$ | H | 2 | Schmp. 248~252℃ |
| 659 | N | 5 | $1-CH_3$ | H | 2 | |
| 660 | N | 2 | $1-CH_2-$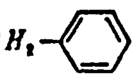 | H | 2 | Schmp. 142~145℃ |
| 661 | N | 5 | $1-H, 4-CH_3$ | H | 2 | |
| 662 | N | 4 | $1-H, 2-F$ | H | 3 | |
| 663 | N | 4 | $1-H, 2-Cl$ | H | 2 | |
| 664 | N | 2 | $1-H, 4-NO_2$ | H | 2 | |
| 665 | N | 4 | $1-H, 2-SCF_3$ | H | 2 | |
| 666 | N | 4 | $1, 2-(CH_3)_2$ | H | 2 | |
| 667 | N | 4 | $1-CH_3, 2-CF_3$ | H | 2 | |
| 668 | O | 2 | $5-COOH$ | H | 3 | |
| 669 | S | 5 | $2-NHCH_3$ | H | 2 | |
| 670 | S | 5 | $2-N(CH_3)_2$ | H | 3 | Schmp. mp. 188~191℃ |
| 671 | S | 5 | $2-NH\overset{O}{\overset{\|}{C}}CF_3$ | H | 2 | |
| 672 | S | 5 | $2-CH_3$ | H | 4 | |

0192060

| | | | | | | |
|---|---|---|---|---|---|---|
| 673 | S | 2 | 5—$CONH_2$ | H | 2 | |
| 674 | S | 2 | 5—$CONHCH_3$ | H | 2 | |
| 675 | S | 2 | 5—$CON(CH_3)_2$ | H | 3 | |
| 676 | S | 5 | 2—$SOCH_3$ | H | 2 | |
| 677 | S | 5 | 2—$SO_2CH_3$ | H | 2 | |
| 678 | S | 5 | 2—$N(CH_3)_2$ | H | 2 | Schmp. 149～150℃ |
| 679 | S | 5 | 2—$SCH_3$ | H | 2 | Schmp. 150～153℃ |
| 680 | S | 5 | 2—Br | H | 3 | |

Nit 189

Tabelle 10

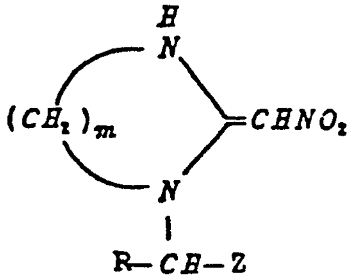

| Verbindung Nr. | Z | R | m | |
|---|---|---|---|---|
| 681 | 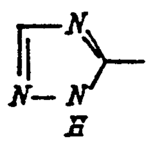 | H | 2 | Schmp. 206~210℃ |
| 682 | 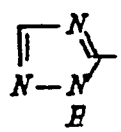 | H | 3 | |
| 683 | 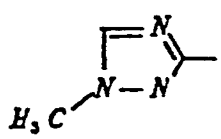 | H | 2 | Schmp. 265~267℃ |
| 684 | 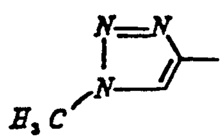 | H | 2 | |
| 685 | 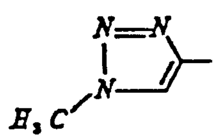 | H | 3 | |
| 686 | 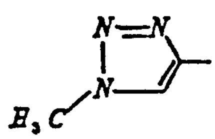 | $-CH_3$ | 2 | |

Nit 189

| | | | | |
|---|---|---|---|---|
| 687 | 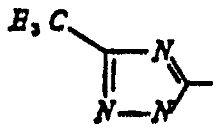 | H | 2 | |
| 688 | 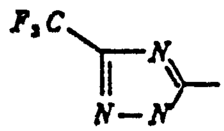 | H | 2 | |
| 689 | 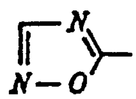 | H | 2 | |
| 690 | 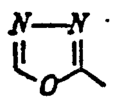 | H | 3 | |
| 691 | 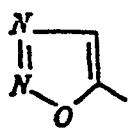 | H | 2 | |
| 692 | 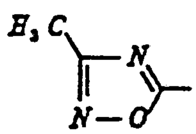 | H | 2 | Schmp. 221~225℃ |
| 693 | 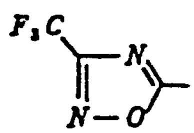 | H | 2 | |
| 694 | 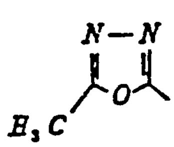 | H | 2 | Schmp. 178~180℃ |
| 695 | 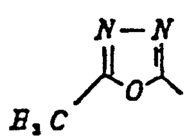 | H | 3 | |

| | | | | |
|---|---|---|---|---|
| 696 | 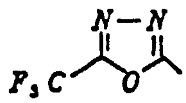 | H | 2 | |
| 697 | 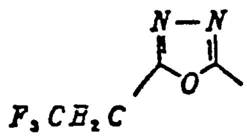 | H | 2 | |
| 698 | 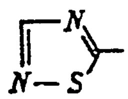 | H | 2 | |
| 699 | 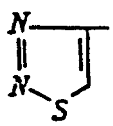 | H | 2 | |
| 700 | 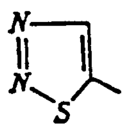 | H | 2 | |
| 701 | 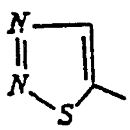 | H | 3 | |
| 702 | 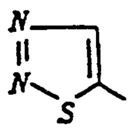 | $-CH_3$ | 2 | |
| 703 | 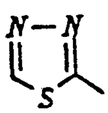 | H | 2 | |
| 704 | 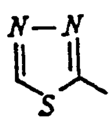 | $-CH_3$ | 3 | |
| 705 | 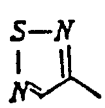 | H | 2 | Schmp. 188~190℃ |

Nit 189

| | | | | |
|---|---|---|---|---|
| 706 | 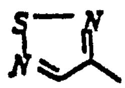 | H | 3 | Schmp. 207~210℃ |
| 707 | 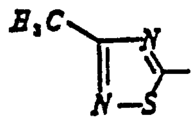 | H | 2 | |
| 708 | 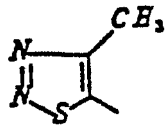 | H | 2 | |
| 709 | 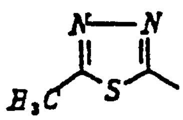 | H | 2 | |
| 710 | 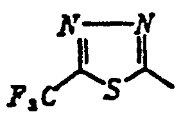 | H | 3 | |
| 711 | 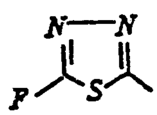 | H | 2 | |
| 712 | 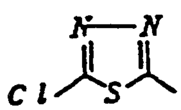 | H | 2 | |
| 713 | 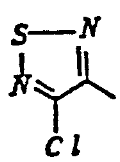 | H | 2 | |
| 714 | 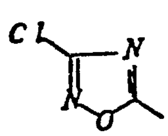 | H | 2 | |

| | | | | |
|---|---|---|---|---|
| 715 | 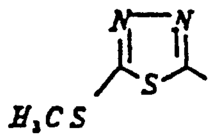 | H | 2 | |
| 716 | 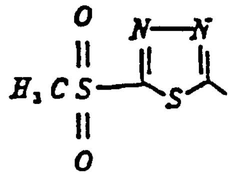 | H | 2 | |
| 717 | 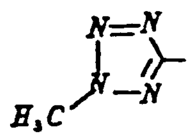 | H | 2 | |

| | | | | |
|---|---|---|---|---|
| 718 | 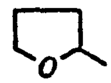 | H | 2 | Schmp. 163~166℃ |
| 719 | 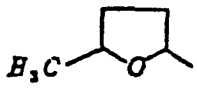 | H | 2 | |
| 720 | 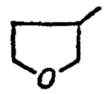 | H | 3 | |
| 721 | 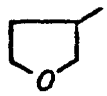 | $-CH_3$ | 3 | |
| 722 | 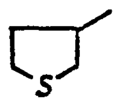 | H | 2 | |
| 723 | 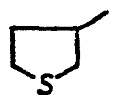 | $-CH_3$ | 2 | |
| 724 | 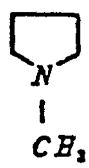 | H | 2 | |
| 725 | 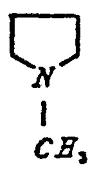 | H | 3 | |

| | | | | |
|---|---|---|---|---|
| 726 | 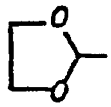 | H | 3 | Schmp. 144~148℃ |
| 727 | 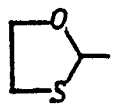 | H | 2 | |
| 728 | 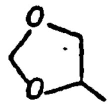 | H | 3 | |
| 729 | 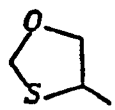 | H | 2 | |
| 730 | 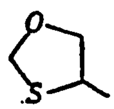 | H | 3 | |
| 731 | 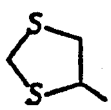 | H | 2 | |
| 732 | 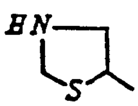 | H | 2 | |
| 733 | 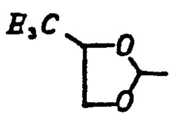 | H | 2 | |
| 734 | 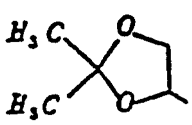 | H | 2 | Schmp. 142~143℃ |
| 735 | 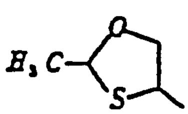 | H | 2 | |
| 736 | 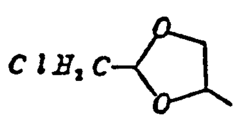 | H | 3 | |
| 737 | 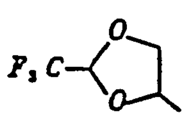 | H | 2 | |

| | | | | |
|---|---|---|---|---|
| 738 | 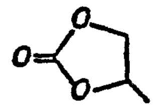 | H | 2 | |
| 739 | 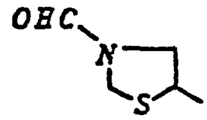 | H | 2 | |
| 740 | 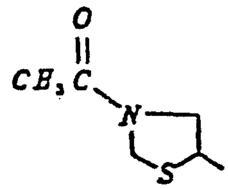 | H | 3 | |
| 741 | 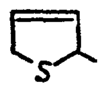 | H | 2 | |
| 742 | 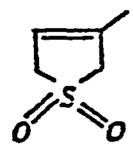 | H | 2 | |
| 743 | 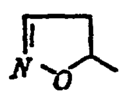 | H | 2 | |
| 744 | 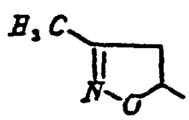 | H | 2 | Schmp. 212~215℃ |
| 745 | 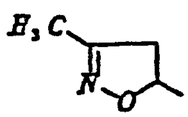 | H | 3 | |
| 746 | 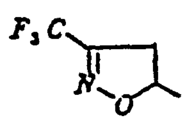 | H | 2 | |
| 747 | 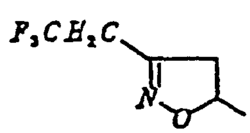 | H | 2 | |
| 748 | 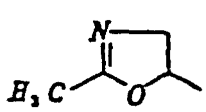 | H | 3 | |

Nit 189

| | | | | |
|---|---|---|---|---|
| 749 | 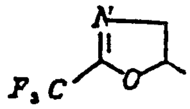 | H | 2 | |
| 750 | 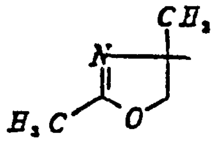 | H | 2 | |
| 751 | 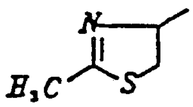 | H | 2 | |
| 752 | 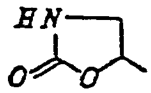 | H | 2 | |
| 753 | 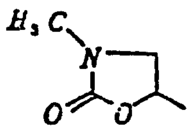 | H | 2 | |
| 754 | 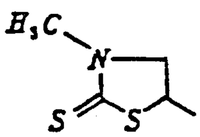 | H | 2 | |

| | | | | |
|---|---|---|---|---|
| 755 | 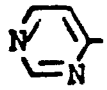 | H | 2 | Schmp. 185-187°C |
| 756 | 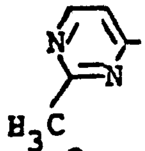 | H | 2 | Schmp. 216-217°C |
| 757 | 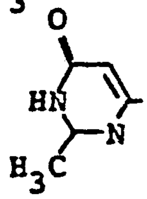 | H | 3 | |
| 758 | 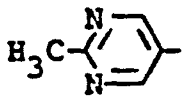 | H | 2 | Schmp. 188-189°C |
| 759 | 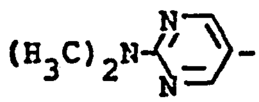 | H | 2 | Schmp. 200-203°C |
| 760 | 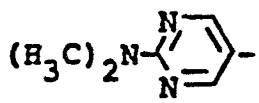 | H | 3 | Schmp. 219-222°C |
| 761 | 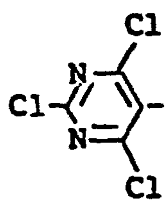 | H | 2 | |
| 762 | 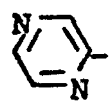 | H | 2 | Schmp. 259-260°C |
| 763 | 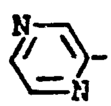 | $-CH_3$ | 2 | Schmp. 173-174°C |

| | | | | |
|---|---|---|---|---|
| 764 | 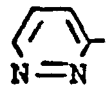 | H | 2 | Schmp. 216-218°C |
| 765 | 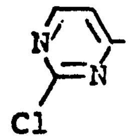 | H | 2 | |
| 766 | 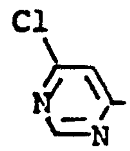 | H | 2 | |
| 767 | 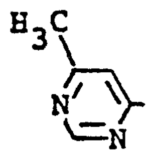 | H | 3 | |
| 768 | 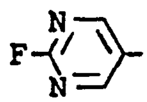 | H | 2 | |
| 769 | 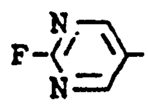 | H | 3 | |
| 770 | 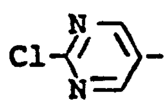 | H | 2 | |
| 771 | 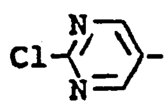 | H | 3 | |
| 772 | 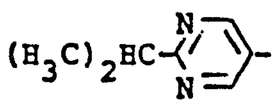 | H | 2 | |
| 773 | 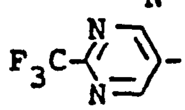 | H | 2 | |
| 774 | 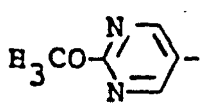 | H | 2 | |

Nit 189

| | | | | |
|---|---|---|---|---|
| 775 | 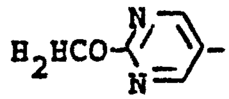 | H | 2 | |
| 776 | 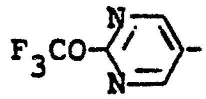 | H | 2 | |
| 777 | 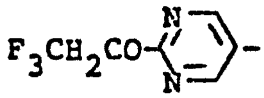 | H | 3 | |
| 778 | 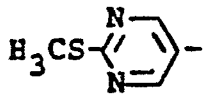 | H | 2 | |
| 779 | 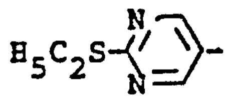 | H | 2 | |
| 780 | 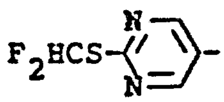 | H | 2 | |
| 781 | 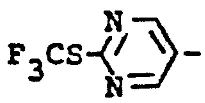 | H | 2 | |
| 782 | 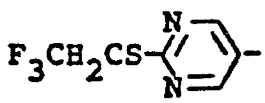 | H | 2 | |
| 783 | 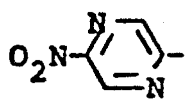 | H | 2 | |
| 784 | 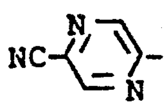 | H | 3 | |
| 785 | 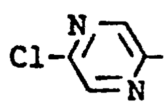 | H | 2 | |
| 786 | 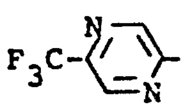 | H | 2 | |

| | | | | |
|---|---|---|---|---|
| 787 | 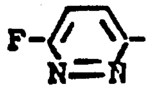 | H | 2 | |
| 788 | 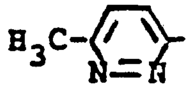 | H | 3 | |
| 789 | 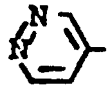 | H | 2 | |
| 790 | 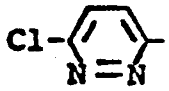 | H | 2 | Schmp. 172-175°C |
| 791 | 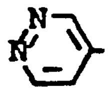 | H | 3 | |
| 792 | 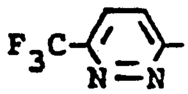 | H | 2 | |
| 793 | 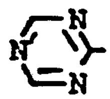 | H | 2 | |
| 794 | 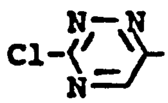 | H | 2 | |
| 795 | 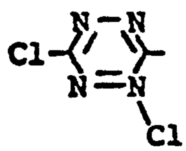 | H | 2 | |
| 796 | 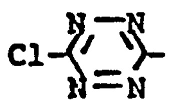 | H | 2 | |
| 797 | 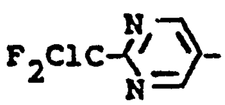 | H | 2 | |
| 798 | 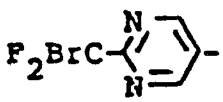 | H | 2 | |

Neben den Tabellen 5 bis 10 umfaßt Tabelle 11 den Fall, in dem X $N-R^7$ ist.

Nit 189

Tabelle 11

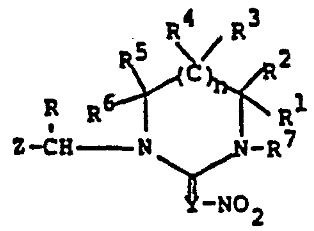

| Verb. Nr. | Z | R | $R^1$ | $R^2$ | $R^3$ | $R^4$ | $R^5$ | $R^6$ | n | $R^7$ | Y | |
|---|---|---|---|---|---|---|---|---|---|---|---|---|
| 799 | 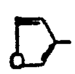 | H | H | H | – | – | H | H | 0 | H | N | |
| 800 | 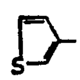 | $CH_3$ | H | H | – | – | H | H | 0 | H | N | |
| 801 | 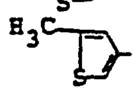 | H | H | H | H | H | H | H | 1 | H | N | |
| 802 | 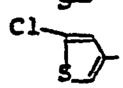 | H | H | H | H | H | H | H | 1 | H | N | |
| 803 | 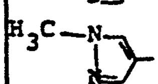 | H | H | H | H | H | H | H | 1 | H | N | |
| 804 | 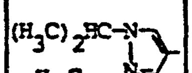 | H | H | H | H | H | H | H | 1 | H | N | |
| 805 | 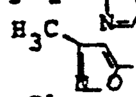 | H | H | H | – | – | H | H | 0 | H | N | mp. 149-150°C |
| 806 | 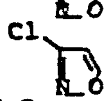 | H | H | H | – | – | H | H | 0 | H | N | |
| 807 | 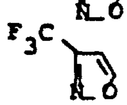 | H | H | H | – | – | H | H | 0 | H | N | |
| 808 | 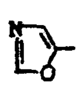 | H | H | H | H | H | H | H | 1 | H | N | |
| 809 | 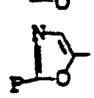 | H | H | H | – | – | H | H | 0 | H | N | |
| 810 | 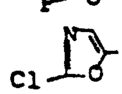 | H | H | H | – | – | H | H | 0 | H | N | |
| 811 | 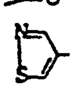 | H | H | H | H | H | H | H | 1 | H | N | |
| 812 | 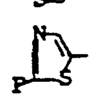 | H | H | H | – | – | H | H | 0 | H | N | |
| 813 | 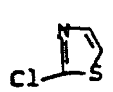 | H | H | H | H | H | H | H | 1 | H | N | Schmp. 123-128°C |
| 814 | 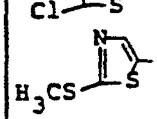 | H | H | H | H | H | H | H | 1 | H | N | |

Nit 189

| Verb. Nr. | Z | R | $R^1$ | $R^2$ | $R^3$ | $R^4$ | $R^5$ | $R^6$ | n | $R^7$ | Y | |
|---|---|---|---|---|---|---|---|---|---|---|---|---|
| 815 | 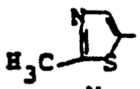 | H | H | H | H | H | H | H | 1 | H | N | |
| 816 | 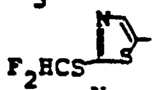 | H | H | H | H | H | H | H | 1 | H | N | |
| 817 | 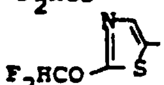 | H | H | H | H | H | H | H | 1 | H | N | |
| 818 | 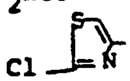 | H | H | H | – | – | H | H | 0 | H | N | Schmp. 141-145°C |
| 819 | 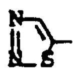 | H | H | H | H | H | H | H | 1 | H | N | |
| 820 | 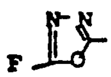 | H | H | H | H | H | H | H | 1 | H | N | |
| 821 | 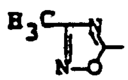 | H | H | H | H | H | H | H | 1 | H | N | |
| 822 | 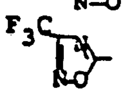 | H | H | H | H | H | H | H | 1 | H | N | |
| 823 | 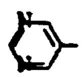 | H | H | H | – | – | H | H | 0 | H | N | |
| 824 | 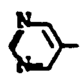 | $CH_3$ | H | H | – | – | H | H | 0 | H | N | |
| 825 | 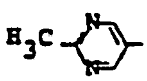 | H | H | H | – | – | H | H | 0 | H | N | |
| 826 | 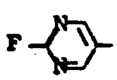 | H | H | H | – | – | H | H | 0 | H | N | |
| 827 | 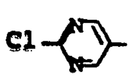 | H | H | H | – | – | H | H | 0 | H | N | Schmp. 181-183°C |
| 828 | 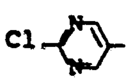 | H | H | H | H | H | H | H | 1 | H | N | |
| 829 | 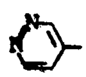 | H | H | H | – | – | H | H | 0 | H | N | |
| 830 | 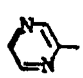 | H | H | H | – | – | H | H | 0 | H | N | |
| 831 | 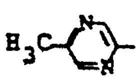 | H | H | H | – | – | H | H | 0 | H | N | Schmp. 142-144°C |
| 832 | 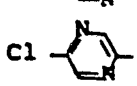 | H | H | H | – | – | H | H | 0 | H | N | |
| 833 | 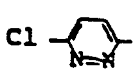 | H | H | H | – | – | H | H | 0 | H | N | Schmp. 216-219°C |
| 834 | 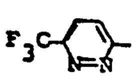 | H | H | H | – | – | H | H | 0 | H | N | |

| Verb. Nr. | Z | R | $R^1$ | $R^2$ | $R^3$ | $R^4$ | $R^5$ | $R^6$ | n | $R^7$ | Y | |
|---|---|---|---|---|---|---|---|---|---|---|---|---|
| 835 | 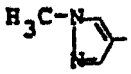 | H | H | H | $CH_3$ | $CH_3$ | H | H | 1 | H | CH | Schmp. 210-213°C |
| 836 | 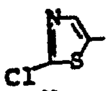 | H | H | H | H | H | H | H | 1 | $CH_3$ | CH | |
| 837 | 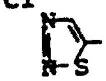 | H | H | H | H | H | H | H | 1 | $C_2H_5$ | CH | |
| 838 | 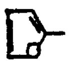 | H | H | H | – | – | H | H | 0 | 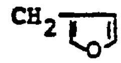 | CH | |
| 839 | 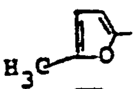 | H | H | H | – | – | H | H | 0 | 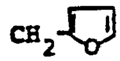 | CH | |
| 840 | 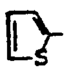 | H | H | H | – | – | H | H | 0 | 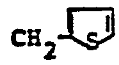 | CH | |
| 841 | 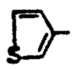 | H | H | H | – | – | H | H | 0 | 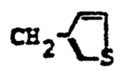 | CH | Schmp. 154-156°C |
| 842 | 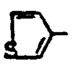 | H | H | H | H | H | H | H | 1 | 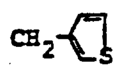 | CH | Glas |
| 843 | 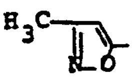 | H | H | H | – | – | H | H | 0 | 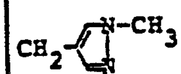 | CH | $n_D^{20}$ 1,5980 |
| 844 | 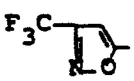 | H | H | H | H | H | H | H | 1 | 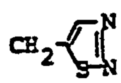 | CH | |
| 845 | 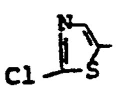 | H | H | H | H | H | H | H | 1 | 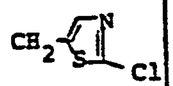 | CH | |
| 846 | 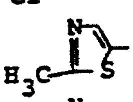 | H | H | H | – | – | H | H | 0 | 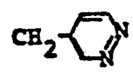 | CH | |
| 847 | 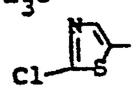 | H | H | H | – | – | H | H | 0 | 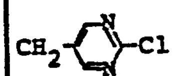 | CH | |
| 848 | 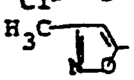 | H | H | H | – | – | H | H | 0 | H | $C-COOC_2H_5$ | Schmp. 153-155°C |
| 849 | 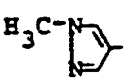 | H | H | H | H | H | H | H | 1 | H | 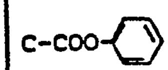 | |
| 850 | 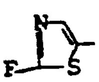 | H | H | H | H | H | H | H | 1 | H | 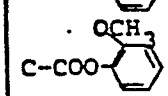 | |
| 851 | 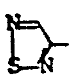 | H | H | H | H | H | H | H | 1 | H | $C-CO(CH_2)_3CH_3$ | |
| 852 | 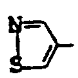 | H | H | H | – | – | H | H | 0 | H | 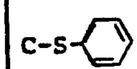 | |
| 853 | 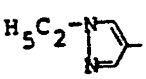 | H | H | H | – | – | H | H | 0 | $CH_3$ | N | |

Nit 189

0192060

| Verb. Nr. | Z | R | $R^1$ | $R^2$ | $R^3$ | $R^4$ | $R^5$ | $R^6$ | n | $R^7$ | Y | |
|---|---|---|---|---|---|---|---|---|---|---|---|---|
| 854 | 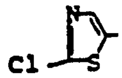 | H | H | H | – | – | H | H | 0 | $C(CH_3)_3$ | N | |
| 855 | 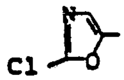 | H | H | H | H | H | H | H | 1 | $CH_2SCH_3$ | N | |
| 856 | 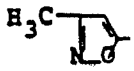 | H | H | H | H | H | H | H | 1 | $CH_2CH_2OC_2H_5$ | N | |
| 857 | 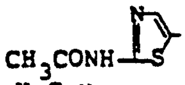 | H | H | H | – | – | H | H | 0 | $CH_2COCH_3$ | N | |
| 858 | 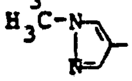 | H | H | H | – | – | H | H | 0 | $COCH_3$ | N | |
| 859 | 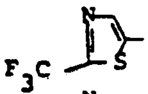 | H | H | H | H | H | H | H | 1 | CHO | N | |
| 860 | 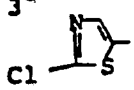 | H | H | H | H | H | H | H | 1 | $COCH_3$ | N | |
| 861 | 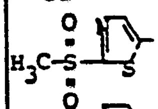 | H | H | H | – | – | H | H | 0 | $CON(C_2H_5)_2$ | N | |
| 862 | 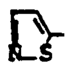 | H | H | H | – | – | H | H | 0 | 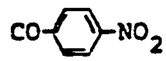 | N | |
| 863 | 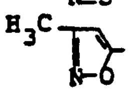 | H | H | H | – | – | H | H | 0 | $COCH_3$ | N | Schmp. 134-136°C |
| 864 | 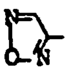 | H | H | H | – | – | H | H | 0 | 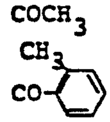 | N | |
| 865 | 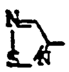 | H | H | H | – | – | H | H | 0 | 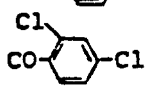 | N | |
| 866 | 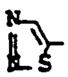 | H | H | H | – | – | H | H | 0 | 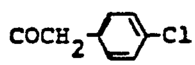 | N | |
| 867 | 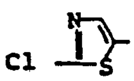 | H | H | H | – | – | H | H | 0 | $COOC_2H_5$ | N | |
| 868 | 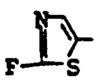 | H | H | H | H | H | H | H | 1 | 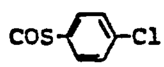 | N | |
| 869 | 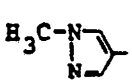 | H | H | H | – | – | H | H | 0 | 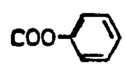 | N | |
| 870 | 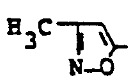 | H | H | H | – | – | H | H | 0 | 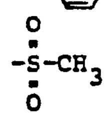 | N | Schmp. 156-158°C |
| 871 | 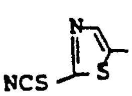 | H | H | H | H | H | H | h | 1 | 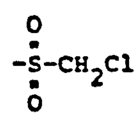 | N | |

Nit 189

0192060

| Verb. Nr. | Z | R | $R^1$ | $R^2$ | $R^3$ | $R^4$ | $R^5$ | $R^6$ | n | $R^7$ | Y | |
|---|---|---|---|---|---|---|---|---|---|---|---|---|
| 872 | 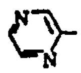 | H | $CH_3$ | H | – | – | H | H | 0 | H | CH | |
| 873 | 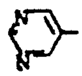 | H | H | H | – | – | H | H | 0 | H | 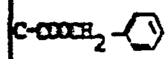 | |
| 874 | 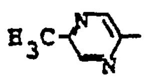 | H | H | H | – | – | H | H | 0 | H | 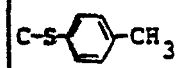 | |
| 875 | 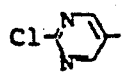 | H | H | H | – | – | H | H | 0 | $CH_3$ | N | |
| 876 | 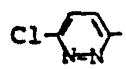 | H | H | H | – | – | H | H | 0 | $COCH_2OCH_3$ | N | |
| 877 | 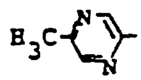 | H | H | H | – | – | H | H | 0 | Br<br>$COCH-C(CH_3)_3$ | N | |
| 878 | 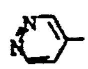 | H | H | H | – | – | H | H | 0 | 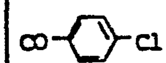 | N | |
| 879 | 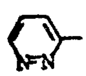 | H | H | H | – | – | H | H | 0 | 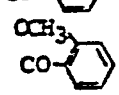 | N | |
| 880 | 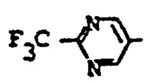 | H | H | H | – | – | H | H | 0 | 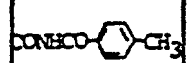 | N | |
| 881 | 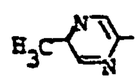 | H | H | H | – | – | H | H | 0 | 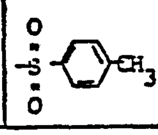 | N | |

Tabelle 12 umfaßt den Fall, in dem die Verbindungen der Formel (I) durch die folgende Formel dargestellt werden:

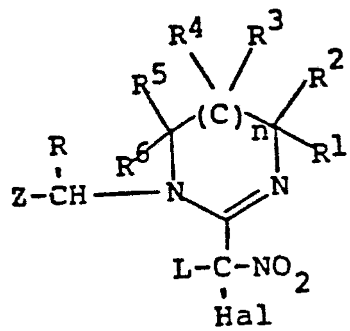

Nit 189

Tabelle 12

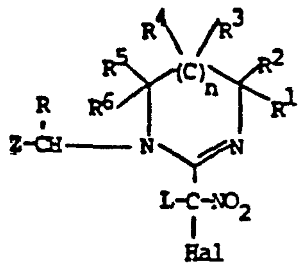

| Verb. Nr. | Z | R | $R^1$ | $R^2$ | $R^3$ | $R^4$ | $R^5$ | $R^6$ | n | L | Hal | |
|---|---|---|---|---|---|---|---|---|---|---|---|---|
| 882 | 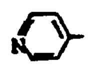 | H | H | H | – | – | H | H | 0 | Cl | Cl | |
| 883 | 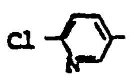 | H | H | H | – | – | H | H | 0 | Cl | Cl | |
| 884 | 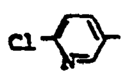 | H | H | H | – | – | H | H | 0 | Br | Br | Schmp. 140-143°C (Zers.) |
| 885 | 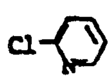 | H | H | H | – | – | H | H | 0 | Cl | Br | |

Tabelle 13 umfaßt den Fall, in dem die Verbindungen der Formel (I) durch die folgende Formel dargestellt werden:

Nit 189

Tabelle 13

| Verb. Nr. | Verbindungen | |
|---|---|---|
| 886 | 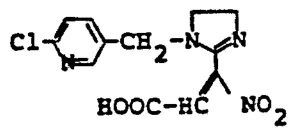 | mp. 170-175°C (Zers.) |
| 887 | 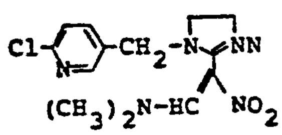 | |

Typische Beispiele für die Synthese der Zwischenprodukt-Verbindungen der Formel (II) sind im Folgenden angegeben.

Beispiel 36

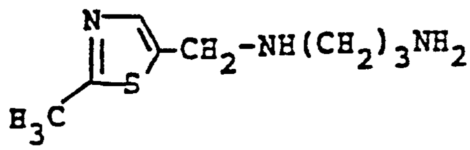

Trimethylendiamin (37 g) wurde in 120 ml Acetonitril gelöst, und eine Lösung von 14,8 g 5-Chloromethyl-2-methylthiazol in 30 ml Acetonitril wurde tropfenweise bei 10°C bis 15°C dazugegeben. Nach der Zuagbe wurde die Mischung eine Weile bei 30°C bis 40°C gerührt, und danach wurden 8 g einer 50-proz. wäßrigen Natriumhydroxid-Lösung hinzugefügt. Anschließend wurden die flüchtigen Materialien bei einer Bad-Temperatur von weniger als 50°C unter 6,67 mbar (5 mmHg) entfernt. Die anorganischen Stoffe wurden aus dem Rückstand durch Filtration entfernt, wonach 16,7 g N-(2-Methyl-5-thiazolylmethyl)trimethylendiamin (Reinheit etwa 90 %) als farbloses Öl erhalten wurden. $n_D^{22}$: 1,5126.

Beispiel 37

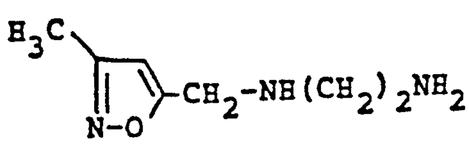

Ethylendiamin (30 g) wurde in 120 g Acetonitril gelöst, und eine Lösung von 17,6 g 5-Bromomethyl-3-methylisoxazol in 30 ml Acetonitril wurde tropfenweise bei

5°C bis 10°C hinzugefügt. Nach der Zugabe wurde die Mischung 1 h gerührt, wobei darauf geachtet wurde, daß die Temperatur des Reaktionssystems nicht über 20°C anstieg. Danach wurde die Hauptmenge der flüchtigen Stoffe im Vakuum (weniger als 2,67 mbar (2 mmHg)) entfernt, wobei die Bad-Temperatur bei 20°C gehalten wurde. Dem Rückstand wurde Eiswasser zugesetzt, und die Mischung wurde mit Dichloromethan extrahiert. Die Dichloromethan-Schicht wurde entwässert, und das Dichloromethan wurde unter vermindertem Druck abdestilliert, wonach 10,0 g N-(3-Methyl-5-isoxazolylmethyl)-ethylendiamin (Reinheit etwa 95 %) als farbloses Öl erhalten wurden.

NMR-Spektrum (δ in $CDCl_3$): NH, $NH_2$: 1,47 (ppm); $-CH_2CH_2-$: 2,23; $-CH_3$: 2,7; $-CH_2$: 5,8; Hetero-H: 5,9.

Beispiel 38

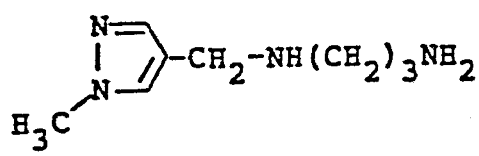

Bei Raumtemperatur wurden 11 g 1-Methyl-4-pyrazolcarbaldehyd langsam zu 37 g Ethylendiamin in 150 ml trockenem Acetonitril hinzugefügt. Molekularsieb 4A (ein Produkt der Wako Pure Chemicals, Co.) wurde als Entwässerungsmittel der Lösung zugesetzt. Die Mischung wurde 2 h bei Raumtemperatur gerührt und dann filtriert. Das Acetonitril wurde aus dem Filtrat unter vermindertem Druck abdestilliert. Dem Rückstand wurden 100 ml Ethanol zugesetzt, und anschließend wurden 4 g Natriumborhydrid nach und nach bei Raumtemperatur hinzugefügt. Die Mischung wurde 2 h bei Raumtemperatur gerührt, und

das Ethanol wurde unter vermindertem Druck abdestilliert. Zu dem Rückstand wurde Wasser gegeben, und die Mischung wurde mit Dichloromethan extrahiert. Das Lösungsmittel wurde aus der Dichloromethan-Schicht abdestilliert, und der Rückstand wurde im Vakuum destilliert, wonach 10 g N-(1-Methyl-4-pyrazolylmethyl)trimethylendiamin als farbloses Öl erhalten wurden. Sdp. 120-125°C/1,07 mbar (0,8 mmHg).

Beispiel 39

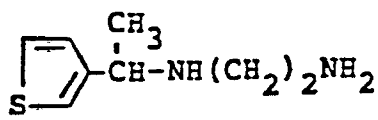

Eine Lösung aus 12,6 g 3-Acetylthiophen, 30 g Ethylendiamin und 150 ml Benzol wurde unter Rühren zum Rückfluß erhitzt, wobei das Wasser als Azeotrop entfernt wurde. Als keine Wasser-Bildung mehr zu beobachtet war, wurde das Benzol unter vermindertem Druck abdestilliert. Ethanol (100 ml) wurde dem Rückstand zugesetzt, und anschließend wurden 4 g Natriumborhydrid nach und nach hinzugefügt. Die Mischung wurde dann 2 h bei 40°C gerührt. Das Ethanol wurde unter vermindertem Druck abdestilliert. Eine kleine Menge anorganisches Material wurde aus dem Rückstand entfernt. Die anschließende Vakuumdestillation lieferte 8,2 g N-/¯1-(3-Thienyl)-ethyl_7ethylendiamin. Sdp. 102-105°C/4,67 mbar (3,5 mmHg).

Neue Verbindungen der Formel (II), die nach den gleichen Verfahren erhalten wurden, wie sie in den Beispielen 36 bis 39 beschrieben wurden, sind im Folgenden aufgeführt.

0192060

Tabelle 14

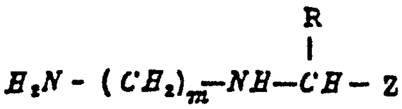

| Z | R | m | |
|---|---|---|---|
| 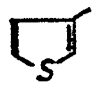 | H | 3 | |
| 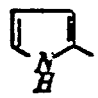 | H | 2 | $n_D^{20}$ 1,5523 |
| 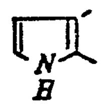 | H | 3 | $n_D^{20}$ 1,5495 |
| 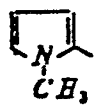 | H | 3 | |
| 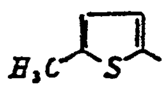 | H | 2 | Sdp. 108~110℃ / 5,3 mbar (4 mmHg) |
| 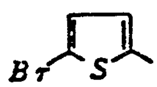 | H | 3 | Sdp. 135~140℃ / 0,67 mbar (0,5 mmHg) |
| 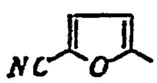 | H | 2 | |
| 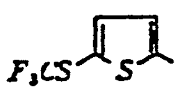 | H | 2 | $n_D^{20}$ 1,4775 |
| 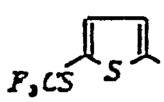 | H | 3 | $n_D^{21}$ 1,4752 |
| 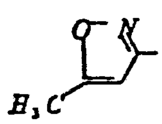 | H | 3 | |
| 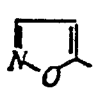 | H | 3 | |

Nit 189

| | | | |
|---|---|---|---|
| 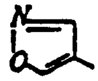 | H | 3 | |
| 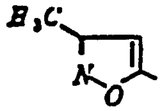 | H | 3 | |
| 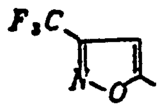 | H | 3 | $n_D^{20}$ 1,4683 |
| 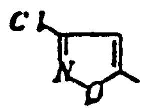 | H | 2 | |
| 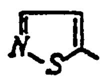 | H | 3 | |
| 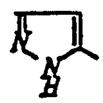 | H | 2 | $n_D^{20}$ 1,5335 |
| 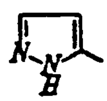 | H | 3 | $n_D^{20}$ 1,5230 |
| 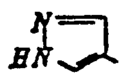 | H | 2 | |
| 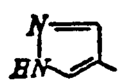 | H | 3 | |
| 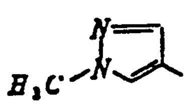 | H | 2 | Sdp. 125~127℃ / 1,33 mbar (1 mmHg) |
| 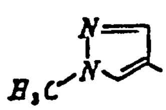 | $-CH_3$ | 2 | Sdp. 133~135℃ / 1,6 mbar (1,2 mmHg) |

| | | | |
|---|---|---|---|
| 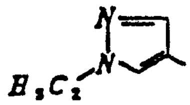 | H | 3 | $n_D^{20}$ 1,5251 |
| 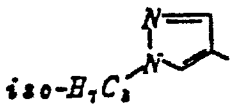 | H | 3 | |
| 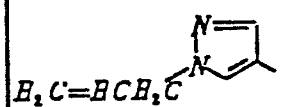 | H | 3 | $n_D^{20}$ 1,5085 |
| 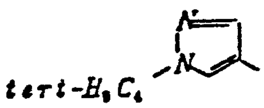 | H | 2 | $n_D^{20}$ 1,5045 |
| 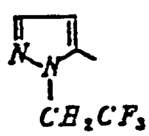 | H | 2 | $n_D^{20}$ 1,4651 |
| 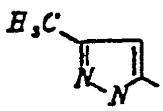 | H | 2 | |
| 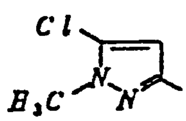 | H | 2 | $n_D^{20}$ 1,4940 |
| 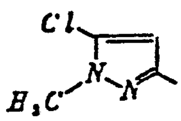 | H | 3 | $n_D^{20}$ 1,4904 |
| 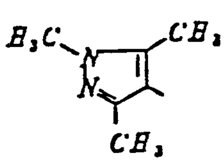 | H | 3 | |
| 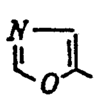 | H | 2 | $n_D^{23}$ 1,5003 |

| | | | |
|---|---|---|---|
| 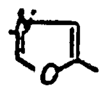 | H | 3 | |
| 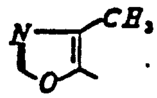 | H | 2 | $n_D^{27}$ 1,5160 |
| 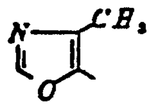 | H | 3 | $n_D^{27}$ 1,5130 |
| 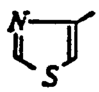 | H | 2 | $n_D^{20}$ 1,5722 |
| 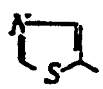 | H | 2 | |
| 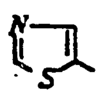 | H | 3 | |
| 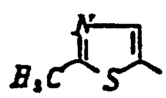 | H | 2 | $n_D^{24}$ 1,5205 |
| 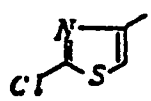 | H | 2 | $n_D^{24}$ 1,5691 |
| 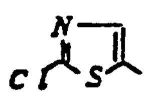 | H | 3 | |
| 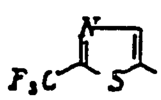 | H | 2 | |
| 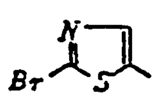 | H | 2 | |
| 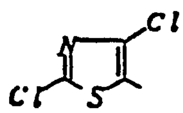 | H | 2 | $n_D^{20}$ 1,5788 |

0192060

| | | | |
|---|---|---|---|
| 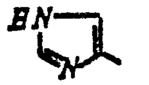 | H | 3 | |
| 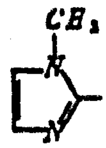 | H | 2 | |
| 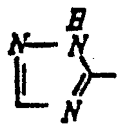 | H | 2 | |
| 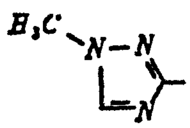 | H | 2 | |
| 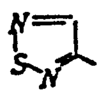 | H | 2 | $n_D^{20}$ 1,5457 |
| 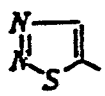 | H | 2 | |
| 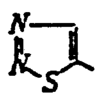 | H | 3 | |
| 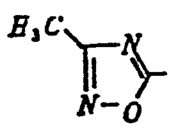 | H | 2 | |
| 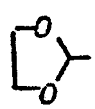 | H | 3 | Sdp. 90 °C/ 3,33 mbar (2,5 mmHg) |
| 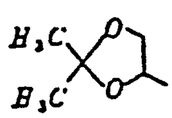 | H | 2 | $n_D^{17}$ 1,4715 |
| 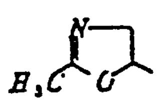 | H | 2 | |
| 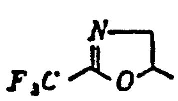 | H | 2 | |

0192060

Beispiel 40

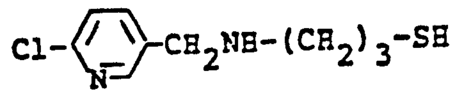

Eine Lösung aus 6-Chloronicotinaldehyd (14,2 g), 2-Aminoethanthiol (7,7 g) und Benzol (80 ml) wurde 5 h unter Rühren erhitzt, wobei das Wasser als Azeotrop entfernt wurde. Nach der Reaktion wurde das Benzol unter vermindertem Druck abdestilliert, und weitere flüchtige Stoffe wurden bei 1,33 mbar (1 mmHg) und 70°C entfernt, wonach 2-(2-Chloro-5-pyridyl)thiazolidin (18 g) als Rückstand erhalten wurde. 10 g 2-(2-Chloro-5-pyridyl)thiazolidin wurden in 100 ml Ethanol gelöst, und Natriumborhydrid wurde hinzugefügt. Unter Rühren wurde die Mischung allmählich erwärmt und anschließend 1 h unter Rückfluß erhitzt. Das Ethanol wurde unter vermindertem Druck abdestilliert, und der Rückstand wurde mit Chloroform versetzt. Unlösliche Stoffe wurden durch Filtration abgetrennt, und die Chloroform-Schicht wurde mit Wasser gewaschen und entwässert. Das Chloroform wurde unter vermindertem Druck abdestilliert, wonach das gewünschte N-(2-Chloro-5-pyridylmethyl-2-aminoethanthiol (8,3 g) erhalten wurde. $n_D^{24}$: 1,5917.

Beispiel 41

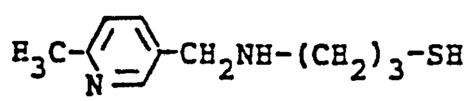

Eine Lösung aus 6-Methylnicotinaldehyd (12,1 g), 3-Aminopropanthiol (9,1 g) und Benzol (120 ml) wurde

Nit 189

5 h unter Rühren erhitzt, wobei das Wasser als Azeotrop entfernt wurde. Nach der Reaktion wurde das Benzol unter vermindertem Druck abdestilliert, und flüchtige Stoffe wurden bei 1,33 mbar (1 mmHg) und 70°C entfernt. Als Rückstand wurde 3-Pyridyltetrahydrothiazin (16,5 g) erhalten. Dann wurde 3-Pyridyltetrahydrothiazin (10 g) in 100 ml Ethanol gelöst, und Natriumborhydrid wurde hinzugefügt. Die Mischung wurde unter Rühren allmählich erwärmt und anschließend 1 h unter Rückfluß erhitzt. Das Ethanol wurde unter vermindertem Druck abdestilliert. Der Rückstand wurde mit Chloroform versetzt, und unlösliche Stoffe wurden durch Filtration abgetrennt. Die Chloroform-Schicht wurde mit Wasser gewaschen und entwässert. Nach dem Abdestillieren des Chloroforms unter vermindertem Druck wurde das gewünschte N-(3-Pyridyl)-3-aminopropanthiol (6,2 g) erhalten. $n_D^{24}$: 1,5733.

Verbindungen der Formel (II) mit den nachstehenden Formeln wurden nach der gleichen Arbeitsweise wie in der Beispielen 40 und 41 hergestellt:

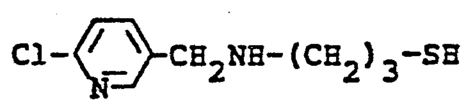

N-(2-Chloro-5-pyridylmethyl)-3-aminopropanthiol ($n_D^{22}$: 1,5890).

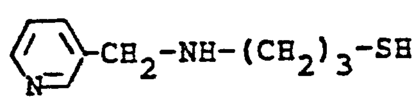

$n_D^{20}$: 1,5748 (Nr. II-4)

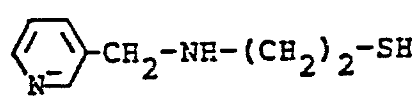

$n_D^{23}$: 1,5817 (Nr. II-5)

Beispiel 42

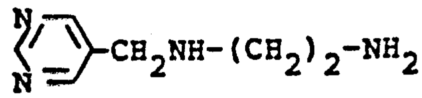

Ethylendiamin (60 g) wurde in Benzol (200 ml) gelöst, und 5-Formylpyrimidin (21,6 g) wurde bei Raumtemperatur dazugegeben. Anschließend wurde die Mischung erwärmt und 3 h unter Rückfluß erhitzt, wobei das Wasser als Azeotrop entfernt wurde. Nach der Reaktion wurden das Benzol und das überschüssige Ethylendiamin unter vermindertem Druck abdestilliert. Der Rückstand wurde in Ethanol (200 ml) gelöst. Natriumborhydrid (8,4 g) wurde portionsweise zu dieser Lösung bei Raumtemperatur hinzugefügt, und anschließend wurde die Mischung 5 h bei Raumtemperatur gerührt. Das Ethanol wurde unter vermindertem Druck abdestilliert. Dichloromethan (100 ml) wurde dem Rückstand zugesetzt, und der in Dichloromethan lösliche Anteil wurde abgetrennt. Aus der Dichloromethan-Schicht wurde das Dichloromethan unter vermindertem Druck abdestilliert, wonach N-(5-Pyrimidinylmethyl)ethylendiamin (25,8 g) als farbloses Öl erhalten wurde. $n_D^{25}$ = 1,5532.

Beispiel 43

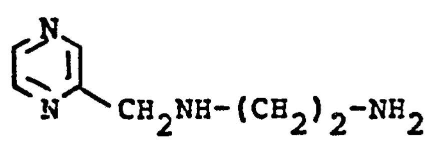

Ethylendiamin (30 g) wurde in Acetonitril (200 ml) gelöst, und Pyrazinylmethylchlorid (12,9 g) wurde tropfenweise bei 5°C zu 10°C zu dieser Lösung hinzugegeben.

Nach der Zugabe wurde die Mischung 1 h bei Raumtemperatur gerührt. Dann wurde eine 50-proz. wäßrige Lösung von Natriumhydroxid (8 g) zugesetzt, und anschließend wurden flüchtige Stoffe bei einer Bad-Temperatur von 60°C unter 6,67 mbar (5 mmHg) entfernt. Das anorganische Salz wurde dann durch Filtration entfernt. Auf diese Weise wurde N-(Pyrazinylmethyl)ethylendiamin (14,1 g) als farbloses Öl erhalten. $n_D^{20}$ = 1,5359.

Beispiel 44

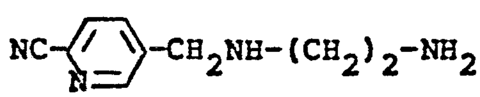

Eine Lösung von 2-Cyano-5-pyridylmethylchlorid (4,6 g) in Acetonitril (20 ml) wurde tropfenweise bei 0°C bis 5°C zu einer Lösung von Ethylendiamin (9 g) in Acetonitril (50 ml) hinzugefügt. Nach der Zugabe wurde die Mischung 3 h bei Raumtemperatur gerührt. Acetonitril und das überschüssige Ethylendiamin wurden unter vermindertem Druck aus der Reaktionsmischung abdestilliert. Dem Rückstand wurde Dichloromethan zugesetzt, und der in Dichloromethan lösliche Anteil wurde abgetrennt. Das Dichloromethan wurde unter vermindertem Druck abdestilliert. Die flüchtigen Stoffe wurden bei 50°C und 1,33 mbar (1 mmHg) entfernt, wonach N-(2-Cyano-5-pyridylmethyl)ethylendiamin (4,5 g) als farbloses Öl erhalten wurde. $n_D^{20}$ = 1,5718.

Beispiel 45

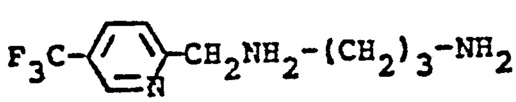

Nit 189

5-Trifluoromethylpicolinaldehyd (3,5 g) wurde tropfenweise bei Raumtemperatur zu einer Lösung von Trimethylendiamin (7,4 g) in Benzol (70 ml) hinzugefügt. Nach der Zugabe wurde die Mischung allmählich unter Rühren erwärmt und danach 2 h zum Rückfluß erhitzt, wobei Wasser als Azeotrop abgetrennt wurde. Das Benzol wurde unter vermindertem Druck abdestilliert, und dann wurde der Rückstand in Ethanol (100 ml) gelöst. Unter Rühren wurde bei 10°C bis 15°C Natriumborhydrid (0,9 g) nach und nach hinzugefügt. Die Mischung wurde dann 2 h bei Raumtemperatur gerührt. Ethanol wurde bei weniger als 30°C abdestilliert. Dem Rückstand wurde Dichloromethan zugesetzt, und der in Dichloromethan lösliche Anteil wurde abgetrennt. Das Dichloromethan wurde unter vermindertem Druck abdestilliert, und die flüchtigen Stoffe wurden bei 1,33 mbar (1 mmHg) und weniger als 60°C entfernt, wonach N-(5-Trifluoromethyl-2-pyridylmethyl)trimethylendiamin (3,5 g) als farbloses Öl erhalten wurde. $n_D^{20}$ = 1,4651.

Beispiel 46

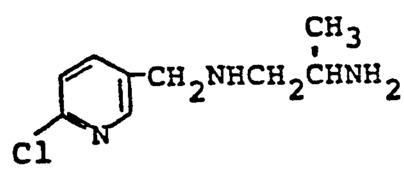

Eine Lösung von 8,1 g 2-Chloro-5-chloromethylpyridin in 30 ml Acetonitril wurde tropfenweise im Laufe von 2 h bei 0°C unter kräftigem Rühren zu einer Mischung von 14,8 g 1,2-Diaminopropan, 5 g einer 40-proz. Natriumhydroxid-Lösung und 100 ml Acetonitril hinzugefügt. Nachdem eine kurze Zeit bei Raumtemperatur gerührt worden war, wurden das Wasser, das Acetonitril und das

überschüssige 1,2-Diaminopropan unter vermindertem Druck entfernt. Das anorganische Salz wurde unter Saugen aus dem Rückstand abfiltriert. Das Filtrat war das gewünschte Produkt, 2-Amino-1-(2-chloro-5-pyridylmethylamino)propan (9,3 g). $n_D^{17}$ = 1,5450.

In der gleichen Weise wie in Beispiel 46 wurden die folgenden Verbindungen hergestellt:

N-Methyl-N'-3-pyridylmethylethylendiamin, Sdp. 140°C/ 3,33 mbar (2,5 mmHg);
2-Amino-2-methyl-1-(3-pyridylmethylamino)propan, Sdp. 115°C/ 2,0 mbar (1,5 mmHg);
2-Aminomethyl-2-methyl-1-(1-methyl-4-pyrazolylmethyl)-propan, $n_D^{25}$ = 1,5109.

Beispiel 47

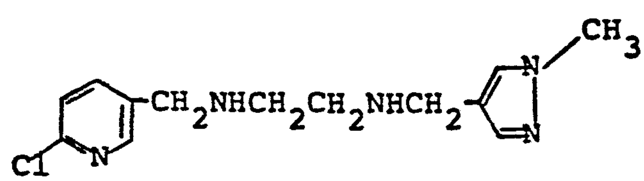

Eine Lösung von 18,6 g N-(2-Chloro-5-pyridylmethyl)-ethylendiamin und 11 g 1-Methylpyrazol-4-carbaldehyd in 150 ml Benzol wurde auf einem erwärmten Wasserbad gerührt. Nach kurzer Zeit schied sich aus der Lösung Wasser ab, was bedeutete, daß die Schiff'sche Base erhalten worden war. Benzol und Wasser wurden dann unter vermindertem Druck entfernt, und 100 ml Ethanol wurden dem Rückstand zugesetzt. Zu der Lösung wurden 3,8 g Natriumborhydrid portionsweise bei Raumtemperatur hinzugefügt, und die Mischung wurde 1 d gerührt. Nach dem Entfernen des Ethanols unter vermindertem Druck

wurde der Rückstand in Dichloromethan gelöst und mit Wasser gewaschen. Die Behandlung der Dichloromethan-Lösung in der üblichen Weise ergab das gewünschte Zwischenprodukt, N-(2-Chloro-5-pyridylmethyl)-N'-(1-methyl-4-pyrazolylmethyl)ethylendiamin als viskoses Öl. Die Ausbeute betrug 23,5 g. $n_D^{20}$ = 1,5655.

In der gleichen Weise wie in Beispiel 47 wurden beispielsweise die folgenden Verbindungen hergestellt:

N-(3-Pyridylmethyl)-N'-(2-chloro-5-pyridylmethyl)ethylendiamin, $n_D^{20}$ = 1,5846.
N-(3-Methyl-5-isoxazolylmethyl)-N'-(1-methyl-4-pyrazolylmethyl)trimethylendiamin, $n_D^{20}$ = 1,5224.

Beispiel 48

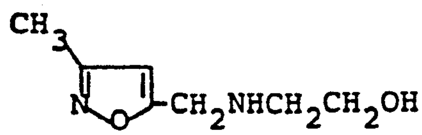

Eine Lösung von 7 g 5-Bromomethyl-3-methylisoxazol in 20 ml Acetonitril wurde tropfenweise unterhalb von 10°C zu einer Lösung von 12,2 g 2-Aminoethanol in 100 ml Acetonitril hinzugefügt. Nachdem eine Weile bei Raumtemperatur gerührt worden war, wurden das Acetonitril und das überschüssige 2-Aminoethanol unter vermindertem Druck entfernt. Chloroform wurde dem Rückstand zugesetzt, und die Mischung wurde mit einer kleinen Menge Wasser gewaschen. Die Behandlung der Chloroform-Lösung in der üblichen Weise lieferte das gewünschte 2-(3-Methyl-5-isoxazolylmethylamino)ethanol. Die Ausbeute betrug 4,4 g. $n_D^{20}$ = 1,5130.

0192060

In der gleichen Weise wie in Beispiel 48 wurden beispielsweise die folgenden Verbindungen hergestellt:

N-(2-Chloro-5-pyridylmethylamino)propanol, $n_D^{27} = 1,5391$.
N-(4-Pyridylmethyl)ethanolamin, Sdp. 148-150°C/ 4 mbar (3 mmHg).

Beispiel 49

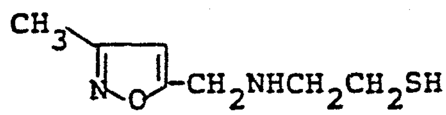

Zu einer Lösung von 15,6 g 2-(3-Methyl-5-isoxazolylmethylamino)ethanol in 100 ml Chloroform wurden eine katalytische Menge Pyridin und sodann 15 g Thionylchlorid bei Raumtemperatur hinzugegeben. Nach der Zugabe wurde die Mischung 30 min zum Rückfluß erhitzt, und das Abdampfen des flüchtigen Materials im Vakuum ergab die rohe chlorierte Verbindung in Form ihres Hydrochlorids. Schmp. 136-139°C.

Eine ethanolische Kaliumhydrogensulfid-Lösung wurde hergestellt durch Sättigen einer Lösung von 13,4 g Kaliumhydroxid in 120 ml Ethanol mit Hydrogensulfid-Gas. Zu der erhaltenen Lösung wurde das obige Chlorid-Hydrochlorid bei 25°C bis 30°C unter Rühren portionsweise hinzugefügt. Die Reaktionsmischung erwärmte sich langsam und wurde dann 2 h bei 60°C gerührt. Nach dem Kühlen auf Raumtemperatur wurde das anorganische Salz rasch unter Saugen abfiltriert. Das Lösungsmittel wurde aus dem Filtrat unter vermindertem Druck entfernt, wonach 2-(3-Methyl-5-isoxazolylmethylamino)ethanthiol (12,9 g) als Öl erhalten wurde. $n_D^{25} = 1,5490$.

0192060

In der gleichen Weise wie in Beispiel 49 wurde beispielsweise die folgende Verbindung hergestellt:

2-(2-Methyl-5-pyrazinylamino)ethylmercaptan, $n_D^{28}$ = 1,5581.

Beispiel 50

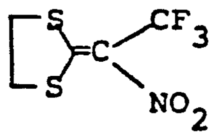

2,72 g Ethylentrithiocarbonat und 2,52 g Dimethylsulfat wurden miteinander vermischt und 1 h auf 100°C erhitzt. 10 ml Essigsäure und 2,02 g Triethylamin wurdem dem entstandenen 2-Methylmercapto-1,3-dithiolaniummethylsulfat hinzugefügt. Danach wurden 2,6 g 2,2,2-Trifluoronitroethan unter Kühlung in einem Eisbad zu der Mischung hinzugefügt. Die gesamte Mischung wurde langsam auf 100°C aufgeheizt und 4 h auf dieser Temperatur gehalten. Nach dem Stehenlassen über Nacht bei Raumtemperatur wurden 100 ml Wasser zu der Reaktionsmischung hinzugefügt. Die ausgefallenen Kristalle wurden abfiltriert und aus Ethanol umkristallisiert, wonach 1-Nitro-2,2-ethylendimercapto-1-trifluoromethylethylen erhalten wurde. Die Ausbeute betrug 4,35 g. Schmp. 130-133°C.

Beispiel 51

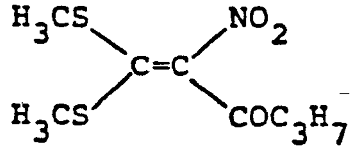

Nit 189

Zu einer Lösung von 13,1 g 1-Nitro-2-pentanon in 200 ml Dimethylsulfoxid in 100 ml Chloroform wurden bei 10°C bis 20°C tropfenweise 44 g einer 20-proz. Natriumhydroxid-Lösung hinzugefügt. Danach wurden 12 g Kohlenstoffdisulfid tropfenweise bei 10°C zu der Lösung hinzugegeben, und die Mischung wurde 2 h bei 0°C bis 10°C erhitzt. In die Mischung wurden unter Eiskühlung 57 g Methyliodid eingetropft, und danach wurde die Reaktionsmischung über Nacht bei Raumtemperatur stehen gelassen. Danach wurde sie in Eiswasser gegossen, und die organische Schicht wurde mit Dichloromethan extrahiert. Der Extrakt wurde einige Male mit Wasser gewaschen, und nach dem Entfernen des Lösungsmittels wurde der Rückstand durch Chromatographie an einer Silicagel-Säule gereinigt, wonach das gewünschte 1-Butyroyl-1-nitro-2,2-bis(methylthio)ethylen erhalten wurde. Die Ausbeute betrug 3,0 g. $n_D^{22}$ = 1,5880.

In der gleichen Weise wie in Beispiel 51 wurde beispielsweise die folgende Verbindung hergestellt:

1-Benzolsulfonyl-1-nitro-2,2-bis(methylthio)ethylen, $n_D^{20}$ = 1,5868.

Beispiel 52

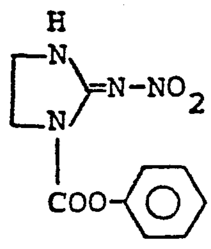

2,5 g Natriumhydrid (60 % in Öl) wurden in kleinen Anteilen zu einer Lösung von 3,9 g 2-Nitroiminoimidazolidin in trockenem Dimethylformamid hinzugefügt. Nach

der Zugabe wurde die Mischung gerührt, bis die Entwicklung von Wasserstoff-Gas beendet war. Die Mischung wurde auf -5°C gekühlt, und 4,7 g Phenylchloroformiat wurden tropfenweise unterhalb von 0°C hinzugefügt. Nach einstündigem Rühren bei Raumtemperatur wurde die Reaktionsmischung in Eiswasser gegossen, auf pH 7 eingestellt und mit Dichloromethan extrahiert. Die nach dem Entfernen des Dichloromethans zurückbleibenden weißen Kristalle wurden mit Ether gewaschen, wonach 1-(Phenoxycarbonyl)-2-nitroiminoimidazolidin im Gewicht von 5,1 g erhalten wurden. Schmp. 171-175°C.

In der gleichen Weise wie in Beispiel 52 wurden beispielsweise die folgenden Verbindungen hergestellt:

1-(2-Methyl-5-nitrobenzolsulfonyl)-2-(nitroimino)imidazolidin, Schmp. 193-197°C;
1-(2,4-Dichlorobenzoyl)-2-(nitroimino)imidazolidin, Schmp. 184-186°C.

Beispiel 53

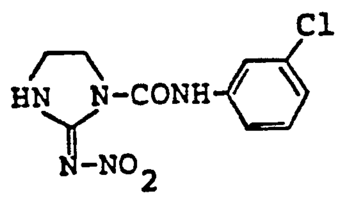

Eine Lösung von 3,9 g 2-Nitroimidazolidin und 4,6 g 3-Chlorophenylisocyanat in 80 m trockenem Acetonitril wurde 3 h zum Rückfluß erhitzt. Nach dem Kühlen auf Raumtemperatur wurden die ausgefallenen Kristalle filtriert und mit Ether gewaschen, wonach das gewünschte 1-(3-Chlorophenylcarbamoyl-2-nitroiminoimidazolidin erhalten wurde. Die Ausbeute betrug. 4,7 g. Schm. 214-216°C.

Nit 189

Verwendungsbeispiele

Vergleichs-Verbindung A-1:

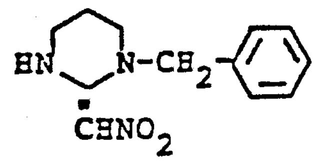

(die in der DE-OS 2 514 402 beschriebene Verbindung);

Vergleichs-Verbindung A-2:

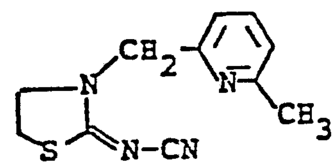

(die in der JP-OS 196 877/1984 beschriebene Verbindung);

Vergleichs-Verbindung A-3:

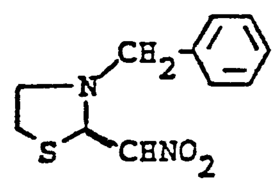

(die in dem gleichen Patent-Dokument wie oben beschriebene Verbindung);

Vergleichs-Verbindung A-4:

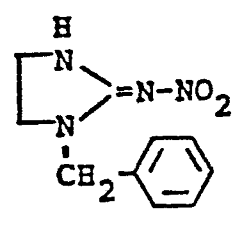

(die in Can. J. Chem. 38, S. 1787-1796, beschriebene Verbindung).

Beispiel 54

Test an gegen Organophosphor-Mittel resistenten Nephotettix cincticeps:

Herstellung eines Test-Präparats:

| | | | |
|---|---|---|---|
| Lösungsmittel: | Xylol | | 3 Gew.-Teile |
| Emulgator: | Polyoxyethylen-alkylphenylether | | 1 Gew.-Teil |

Zur Herstellung eines geeigneten Test-Präparats wurde 1 Gew.-Teil der aktiven Verbindung mit der oben bezeichneten Menge Lösungsmittel, das die oben angegebene Menge Emulgator enthielt, vermischt. Die Mischung wurde mit Wasser auf eine vorher festgesetzte Konzentration verdünnt.

Test-Verfahren:

Auf Reispflanzen von etwa 10 cm Höhe, die jeweils in Töpfe von 12 cm Durchmesser gepflanzt waren, wurden pro Topf 10 ml der mit Wasser verdünnten, eine vorbestimmte Wirkstoff-Konzentration aufweisenden Lösungen, die wie oben angegeben hergestellt wurden, gesprüht. Die aufgesprühte Chemikalie wurde trocknen gelassen, und über jeden Topf wurde ein Drahtkorb von 7 cm Durchmesser und 14 cm Höhe gestülpt; 30 ausgewachsene weibliche Exemplare von Nephotettix cincticeps, die gegen Organophosphor-Insektizide resistent waren, wurden unter jedem Drahtkorb ausgesetzt. Die Töpfe wurden jeweils in einem Raum mit konstanter Temperatur aufbewahrt, und 2 Tage später wurde die Zahl der toten Insekten bestimmt und das Vernichtungsverhältnis berechnet.

Als Ergebnis wurde gefunden, daß beispielsweise die Verbindungen Nr. 1, 2, 3, 4, 5, 6, 8, 9, 10, 11, 13, 14, 15, 16, 17, 19, 20, 22, 26, 27, 31, 32, 33, 34, 35, 36, 39, 65, 66, 75, 76, 105, 164, 290, 304, 323, 336, 340, 396, 409, 431, 444, 453, 518, 556, 612, 623, 624, 705, 706, 755, 762, 813, 827, 831, 884 ein Vernichtungsverhältnis von 100 % bei einer Wirkstoff-Konzentration von 8 ppm zeigten.

Im Gegensatz dazu zeigten im Vergleich
die Verbindung A-1 ein Vernichtungsverhältnis von 65 % bei einer Wirkstoff-Konzentration von 40 ppm,
die Verbindung A-2 ein Vernichtungsverhältnis von 40 % bei einer Wirkstoff-Konzentration von 200 ppm und ein Vernichtungsverhältnis von 0 % bei einer Wirkstoff-Konzentration von 40 ppm,
die Verbindung A-3 ein Vernichtungsverhältnis von 0 % bei einer Wirkstoff-Konzentration von 200 ppm und
die Verbindung A-4 ein Vernichtungsverhältnis von 30 % bei einer Wirkstoff-Konzentration von 200 ppm,

Beispiel 55

Test an Laternenträgern

Eine mit Wasser verdünnten, eine vorbestimmte Wirkstoff-Konzentration aufweisende Lösungen, die wie in Beispiel 54 hergestellt worden war, wurde in einer Menge von 10 ml pro Topf auf Reispflanzen von etwa 10 cm Höhe gesprüht, die in Töpfen von 12 cm Durchmesser gezogen worden waren. Die aufgesprühte Chemikalie wurde trocknen gelassen, und über jeden Topf wurde ein Drahtkorb von 7 cm Durchmesser und 14 cm Höhe gestülpt. 30 ausgewachsene weibliche Exemplare von Nilaparvata lugens Stal eines Stammes, der Resistenz gegen

Organophosphor-Chemikalien zeigte, wurden unter jedem Drahtkorb ausgesetzt. Die Töpfe wurden jeweils in einem Raum mit konstanter Temperatur aufbewahrt, und 2 Tage später wurde die Zahl der toten Insekten bestimmt. Das Vernichtungsverhältnis wurde berechnet.

In der gleichen Weise wurde das Vernichtungsverhältnis berechnet für Sogatella furcifera Horvath und organophosphorresistente Laodelphax stratellus Fallen.

Als Ergebnis wurde gefunden, daß beispielsweise die Verbindungen Nr. 1, 2, 3, 4, 5, 6, 7, 8, 9, 10, 11, 12, 13, 14, 15, 16, 17, 18, 19, 20, 22, 29, 35, 37, 39, 43, 65, 70, 75, 79, 146, 164, 230, 303, 308, 324, 331, 350, 396, 409, 424, 453, 518, 529, 550, 556, 579, 612, 623, 624, 649, 701, 705, 706, 755, 756, 758, 848 ein Vernichtungsverhältnis von 100 % bei einer Wirkstoff-Konzentration von 40 ppm gegen jeden der Laternenträger zeigten.

Im Gegensatz dazu zeigten im Vergleich
die Verbindung A-1 bei einer Wirkstoff-Konzentration
von 40 ppm ein Vernichtungsverhältnis
von 50 % gegen N. Lugens und
von 40 % gegen S. furcifera und L. Striatellus,
die Verbindung A-2 bei einer Wirkstoff-Konzentration
von 200 ppm ein Vernichtungsverhältnis
von 30 % gegen N. Lugens,
von 20 % gegen L. Striatella und
von 50 % gegen S. furcifera
und bei einer Wirkstoff-Konzentration von 40 ppm ein Vernichtungsverhältnis von 0 % gegen jeden der Laternenträger,

die Verbindung A-3 bei einer Wirkstoff-Konzentration von 200 ppm ein Vernichtungsverhältnis von 0 % gegen jeden der Laternenträger und
die Verbindung A-4 bei einer Wirkstoff-Konzentration von 200 ppm ein Vernichtungsverhältnis
von 100 % gegen N. Lugens und
von 0 % gegen S. furcifera und L. Striatellus.

Beispiel 56

Test an gegen Organophosphor-Mittel resistenten Myzodes persicae (grünen Pfirsichblattläusen):

Test-Verfahren:
Gezüchtete grüne Pfirsichblattläuse, die gegen Organophosphor-Chemikalien und Carbamat-Chemikalien Resistenz zeigten, wurden auf Setzlingen von Eierfrüchten (schwarzen länglichen Auberginen) von etwa 20 cm Höhe ausgesetzt, die in unglasierten Töpfen mit einem Durchmesser von 15 cm gezogen worden waren (etwa 200 Blattläuse pro Setzling). Einen Tag nach dem Aussetzen wurde eine wie in Beispiel 54 hergestellte wäßrige Verdünnung jeder der aktiven Verbindungen mit einer vorher festgelegten Konzentration in genügender Menge mit Hilfe einer Spritzpistole auf die Pflanzen aufgesprüht. Nach dem Sprühen wurden die Töpfe in einem Gewächshaus bei 28°C stehen gelassen. 24 Stunden nach dem Sprühen wurde das Vernichtungsverhältnis berechnet. Für jede Verbindung wurde der Test als Doppelbestimmung durchgeführt.

Als Ergebnis wurde gefunden, daß beispielsweise die Verbindungen Nr. 1, 2, 3, 4, 5, 6, 8, 9, 10, 11, 12, 13, 14, 15, 16, 17, 18, 19, 20, 21, 22, 24, 25, 26, 27, 28, 29, 30, 31, 32, 33, 35, 71, 74, 75, 98, 105, 164,

230, 231, 373, 396, 409, 518, 529, 550, 556, 579, 612, 623, 624, 649, 705, 706, 755, 756, 758, 763, 831 ein Vernichtungsverhältnis von 100 % bei einer Wirkstoff-Konzentration von 200 ppm zeigten.

Im Gegensatz dazu zeigten im Vergleich
die Verbindung A-1 ein Vernichtungsverhältnis von 80 % bei einer Wirkstoff-Konzentration von 1 000 ppm und von 30 % bei einer Wirkstoff-Konzentration von 200 ppm
die Verbindung A-3 ein Vernichtungsverhältnis von 60 % bei einer Wirkstoff-Konzentration von 1 000 ppm und von 0 % bei einer Wirkstoff-Konzentration von 200 ppm und
die Verbindung A-4 ein Vernichtungsverhältnis von 60 % bei einer Wirkstoff-Konzentration von 1 000 ppm und von 0 % bei einer Wirkstoff-Konzentration von 200 ppm.

Die Beispiele 54, 55 und 56 sind typische Beispiele für insektizide Verwendungen, und die Verbindungen der vorliegenden Erfindungen, die hierfür beispielhaft gewählt wurden, sind ebenfalls typische Beispiele. Die vorliegende Erfindung ist jedoch nicht allein auf diese Beispiele beschränkt.

P a t e n t a n s p r ü c h e

1. Neue heterocyclische Verbindungen der Formel (I)

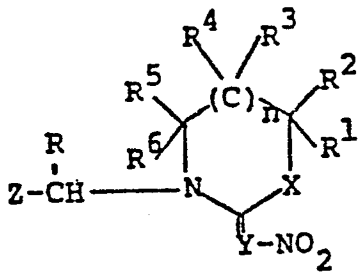

(I)

,

in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

X ein Schwefel-Atom, ein Sauerstoff-Atom oder eine der folgenden Gruppen $-\overset{|}{N}-R^7$ oder $-\overset{|}{C}H-R^8$ bezeichnet, worin

$R^7$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch

wenigstens einen Substituenten substituiert sein kann, der ausgewählt ist aus der aus Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Alkenylcarbonyl-Gruppe, eine Alkylcarbonyl-Gruppe, die durch wenigstens einen Substituenten substituiert sein kann, der ausgewählt ist aus der aus Alkoxy-Gruppen, einer Phenoxy-Gruppe, Alkylthio-Gruppen und Halogen-Atomen bestehenden Klasse, eine Benzoyl-Gruppe, die durch wenigstens einen Substituenten substituiert sein kann, der ausgewählt ist aus der aus Halogen-Atomen, Alkyl-Gruppen, Halogenomethyl-Gruppen, Alkoxy-Gruppen, Halogenoalkoxy-Gruppen und einer Nitro-Gruppe bestehenden Klasse, eine Benzylcarbonyl-Gruppe, die durch eine Alkyl-Gruppe und/oder ein Halogen-Atom substituiert sein kann, eine Alkoxycarbonyl-Gruppe, die durch ein Halogen-Atom substituiert sein kann, eine

Alkylthiocarbonyl-Gruppe, eine Phenoxycarbonyl-Gruppe, die durch wenigstens einen Substituenten substituiert sein kann, der ausgewählt ist aus der aus einer Methyl-Gruppe, einer Methoxy-Gruppe, Halogenomethyl-Gruppen, Halogenomethoxy-Gruppen, Halogen-Atomen und einer Nitro-Gruppe bestehenden Klasse, eine Phenylthiocarbonyl-Gruppe, die durch ein Halogen-Atom und/oder eine Alkyl-Gruppe substituiert sein kann, eine Benzyloxycarbonyl-Gruppe, eine Monoalkyl- oder Dialkylaminocarbonyl-Gruppe, eine Phenylaminocarbonyl-Gruppe, die durch wenigstens einen Substituenten substituiert sein kann, der ausgewählt ist aus der aus Alkyl-Gruppen, Halogenoalkyl-Gruppen und Halogen-Atomen bestehenden Klasse, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert sein kann, eine Phenylthio-Gruppe, die durch eine Alkyl-Gruppe und/oder ein Halogen-Atom substituiert sein kann, eine Alkylsulfonyl-Gruppe, die durch ein Halogen-Atom substituiert sein kann, eine Phenylsulfonyl-Gruppe, die durch wenigstens einen Substituenten substituiert sein kann, der ausgewählt ist aus der aus Alkyl-Gruppen, Halogen-Atomen und einer Nitro-Gruppe bestehenden Klasse, eine Alkylcarbonylmethyl-Gruppe, eine Phenacyl-Gruppe, die durch ein Halogen-Atom und/oder eine Alkyl-Gruppe substituiert sein

kann, eine Organophosphono-Gruppe, eine Organothiophosphono-Gruppe, die folgenden Gruppen $-CH_2-W$ oder $-CO-W$,
worin W eine 5- bis 6-gliedrige heterocyclische Gruppe bezeichnet, die wenigstens ein aus der aus Sauerstoff-Atomen, Schwefel-Atomen und Stickstoff-Atomen bestehenden Klasse ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen bestehenden Klasse, darstellt,

$R^8$ ein Wasserstoff-Atom, eine Alkyl-Gruppe, eine Aryl-Gruppe oder eine Benzyl-Gruppe darstellt,

Y ein Stickstoff-Atom oder die nachstehende Gruppe $=\overset{|}{C}-R^9$ bezeichnet, worin

$R^9$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus Halogen-Atomen, einer Hydroxy-Gruppe, Alkoxy-Gruppen, Alkylthio-Gruppen, einer Cyano-Gruppe, Mono- oder Dialkylamino-Gruppen, Alkylcarbonyl-Gruppen, Alkoxycarbonyl-Gruppen und Phenoxycarbonyl-Gruppen bestehenden Klasse, eine Alkenyl-Gruppe, die durch ein Halogen-Atom substituiert sein kann, eine Alkinyl-Gruppe, eine Phenyl-Gruppe, die durch eine Alkyl-Gruppe und/oder ein Halogen-Atom substituiert sein kann, eine Alkylcarbonyl-Gruppe, die durch ein Halogen-Atom substituiert sein kann, eine Alkenylcarbonyl-Gruppe, eine

Benzoyl-Gruppe, die durch wenigstens einen Substituenten substituiert sein kann, der ausgewählt ist aus der aus Halogen-Atomen, Alkyl-Gruppen und Alkoxy-Gruppen bestehenden Klasse, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus Halogen-Atomen, Alkyl-Gruppen, Alkoxy-Gruppen und einer Nitro-Gruppe bestehenden Klasse, eine Phenylthiocarbonyl-Gruppe, die durch eine Alkyl-Gruppe und/oder ein Halogen-Atom substituiert sein kann, eine Benzyloxycarbonyl-Gruppe, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert sein kann, eine Alkylsulfonylaminocarbonyl-Gruppe, eine Alkylthio-Gruppe, eine Alkylsulfonyl-Gruppe, die durch ein Halogen-Atom substituiert sein kann, eine Phenylthio-Gruppe, die durch eine Alkyl-Gruppe und/oder ein Halogen-Atom substituiert sein kann, eine Phenylsulfonyl-Gruppe, die durch eine Alkyl-Gruppe und/oder ein Halogen-Atom substituiert sein kann, und außerdem

$R^9$ eine Bis-Form der Formel (I) über eine Methylen-Gruppe zu bilden vermag,

R ein Wasserstoff-Atom oder eine Alkyl-Gruppe darstellt und

Z eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen, einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer Thioxo-Gruppe, Halogenoalkenylthio-Gruppen, Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die

gegebenenfalls durch einen der gleichen Substituenten substituiert sind, wie sie für die Aryl-Gruppen angegeben ist, bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf.

2. Verbindungen nach Anspruch 1, dadurch gekennzeichnet, daß

n 0 oder 1 bezeichnet,

$R^1$, $R^2$, $R^5$ und $R^6$ unabhängig voneinander ein Wasserstoff-Atom oder eine Alkyl-Gruppe mit 1 bis 4 Kohlenstoff-Atomen bezeichnen,

$R^3$ und $R^4$ unabhängig voneinander ein Wasserstoff-Atom, eine Hydroxy-Gruppe oder eine Alkyl-Gruppe mit 1 bis 4 Kohlenstoff-Atomen bezeichnen,

X ein Schwefel-Atom, ein Sauerstoff-Atom oder eine der folgenden Gruppen $-N-R^7$ oder $-CH-R^8$ bezeichnet,

$R^7$ ein Wasserstoff-Atom, ein Fluor-Atom, ein Chlor-Atom, ein Brom-Atom, eine Hydroxy-Gruppe, eine Alkoxy-Gruppe mit 1 bis 4 Kohlenstoff-Atomen, eine Benzyloxy-Gruppe, eine Alkyl-Gruppe mit 1 bis 4 Kohlenstoff-Atomen, die durch wenigstens einen Substituenten substituiert sein kann, der ausgewählt ist aus der aus Alkoxy-Gruppen mit 1 bis 4 Kohlenstoff-Atomen, Alkylthio-Gruppen mit 1 bis 4 Kohlenstoff-Atomen, einer Cyano-Gruppe, einem Fluor-Atom, einem Chlor-Atom, einem Brom-Atom, einer Dimethylamino-Gruppe und Trimethylsilyl bestehenden Klasse, eine Alkenyl-Gruppe mit 2 bis 3

Kohlenstoff-Atomen, die durch ein Chlor-Atom substituiert sein kann, eine Alkinyl-Gruppe mit 2 bis 3 Kohlenstoff-Atomen, eine Benzyl-Gruppe, die durch wenigstens einen Substituenten substituiert sein kann, der ausgewählt ist aus der aus einer Methyl-Gruppe, einer Methoxy-Gruppe, einem Fluor-Atom, einem Chlor-Atom, einem Brom-Atom und einer Nitro-Gruppe bestehenden Klasse, eine Formyl-Gruppe, eine Alkenylcarbonyl-Gruppe mit einem Alkenyl-Teil mit 2 bis 3 Kohlenstoff-Atomen, eine Alkyl-Gruppe mit 1 bis 5 Kohlenstoff-Atomen, die durch wenigstens einen Substituenten substituiert sein kann, der ausgewählt ist aus der aus einer Methoxy-Gruppe, einer Phenoxy-Gruppe, einem Fluor-Atom, einem Chlor-Atom und einem Brom-Atom bestehenden Klasse, eine Benzoyl-Gruppe, die durch wenigstens einen Substituenten substituiert sein kann, der ausgewählt ist aus der aus einem Fluor-Atom, einem Chlor-Atom, einem Brom-Atom, einer Methyl-Gruppe, einer Trifluoromethyl-Gruppe, einer Methoxy-Gruppe, einer Difluoromethoxy-Gruppe, einer Trifluoromethoxy-Gruppe und einer Nitro-Gruppe bestehenden Klasse, eine Benzylcarbonyl-Gruppe, die durch wenigstens einen Substituenten substituiert sein kann, der ausgewählt ist aus der aus einem Fluor-Atom, einem Chlor-Atom und einem Brom-Atom bestehenden Klasse, eine Alkoxycarbonyl-Gruppe mit einem Alkyl mit 1 bis 4 Kohlenstoff-Atomen, die durch ein Fluor-Atom und/oder ein Chlor-Atom substituiert sein kann, eine Alkylthiocarbonyl-Gruppe mit einem Alkyl mit 1 bis 4 Kohlenstoff-Atomen, eine Phenoxycarbonyl-Gruppe, die durch wenigstens einen Substituenten substituiert sein kann, der ausgewählt ist aus der aus

einer Methyl-Gruppe, einem Fluor-Atom, einem Chlor-Atom und einem Brom-Atom bestehenden Klasse, eine Phenylthiocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Benzyloxycarbonyl-Gruppe, eine Dimethylaminocarbonyl-Gruppe, eine Phenylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Benzoylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Phenylsulfonylaminocarbonyl-Gruppe, die durch wenigstens einen Substituenten substituiert sein kann, der ausgewählt ist aus der aus einem Fluor-Atom, einem Chlor-Atom und einem Brom-Atom bestehenden Klasse, eine Phenylthio-Gruppe, eine Alkylsulfonyl-Gruppe, die durch ein Fluor-Atom und/oder ein Chlor-Atom substituiert sein kann, eine Phenylsulfonyl-Gruppe, die durch wenigstens einen Substituenten substituiert sein kann, der ausgewählt ist aus der aus einer Methyl-Gruppe, einem Fluor-Atom, einem Chlor-Atom, einem Brom-Atom und einer Nitro-Gruppe bestehenden Klasse, eine Methylcarbonylmethyl-Gruppe, eine Phenacyl-Gruppe, die durch ein Fluor-Atom und/oder ein Chlor-Atom substituiert sein kann, eine Organophosphono-Gruppe, eine Organothiophosphono-Gruppe, die folgenden Gruppen $-CH_2-W$ oder $-CO-W$,

W eine 5- bis 6-gliedrige heterocyclische Gruppe bezeichnet, die ein oder zwei aus der aus Sauerstoff-Atomen, Schwefel-Atomen und Stickstoff-Atomen bestehenden Klasse ausgewählte Hetero-Atome enthält und die durch wenigstens einen Substituenten substituiert sein kann, der ausgewählt ist aus der aus einem Fluor-Atom, einem Chlor-Atom, einem Brom-Atom und Alkyl-Gruppen mit 1 bis 4 Kohlenstoff-Atomen bestehenden Klasse,

$R^8$ ein Wasserstoff-Atom, eine Alkyl-Gruppe mit 1 bis 4 Kohlenstoff-Atomen, eine Phenyl-Gruppe oder eine Benzyl-Gruppe darstellt,

Y ein Stickstoff-Atom oder die nachstehende Gruppe $=\dot{C}-R^9$ bezeichnet,

$R^9$ ein Wasserstoff-Atom, ein Fluor-Atom, ein Chlor-Atom, ein Brom-Atom, eine Hydroxy-Gruppe, eine Alkoxy-Gruppe mit 1 bis 4 Kohlenstoff-Atomen, eine Benzyloxy-Gruppe, eine Alkyl-Gruppe mit 1 bis 4 Kohlenstoff-Atomen, die durch wenigstens einen Substituenten substituiert sein kann, der ausgewählt ist aus der aus einem Fluor-Atom, einem Chlor-Atom, einer Hydroxy-Gruppe, einer Alkoxy-Gruppe mit 1 bis 2 Kohlenstoff-Atomen, Alkylthio-Gruppen mit 1 bis 2 Kohlenstoff-Atomen, einer Cyano-Gruppe, einer Dimethylamino-Gruppe, Alkylcarbonyl-Gruppen mit einem Alkyl mit 1 bis 2 Kohlenstoff-Atomen und Alkoxycarbonyl-Gruppen mit einem Alkyl mit 1 bis 2 Kohlenstoff-Atomen bestehenden Klasse, eine Alkenyl-Gruppe mit 2 bis 3 Kohlenstoff-Atomen, eine Phenyl-Gruppe, eine Alkylcarbonyl-Gruppe mit einem Alkyl mit 1 bis 4 Kohlenstoff-Atomen, die durch wenigstens einen Substituenten substituiert sein kann, der ausgewählt ist aus der aus einer Methoxy-Gruppe, einem

Chlor-Atom und einem Fluor-Atom bestehenden Klasse, eine Alkenylcarbonyl-Gruppe mit einem Alkenyl mit 2 bis 3 Kohlenstoff-Atomen, eine Benzoyl-Gruppe, die durch wenigstens einen Substituenten substituiert sein kann, der ausgewählt ist aus der aus einem Fluor-Atom, einem Chlor-Atom, einem Brom-Atom, einer Methoxy-Gruppe und einer Methyl-Gruppe bestehenden Klasse, eine Alkoxycarbonyl-Gruppe, die durch ein Fluor-Atom und/oder ein Chlor-Atom substituiert sein kann, eine Alkylthiocarbonyl-Gruppe mit einem Alkyl mit 1 bis 4 Kohlenstoff-Atomen, eine Phenoxycarbonyl-Gruppe, die durch wenigstens einen Substituenten substituiert sein kann, der ausgewählt ist aus der aus einem Fluor-Atom, einem Chlor-Atom, einem Brom-Atom, einer Methyl-Gruppe, einer Methoxy-Gruppe und einer Nitro-Gruppe bestehenden Klasse, eine Phenylthiocarbonyl-Gruppe, eine Benzyloxycarbonyl-Gruppe, eine Benzoylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Phenylsulfonylaminocarbonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, eine Alkylsulfonylaminocarbonyl-Gruppe mit einem Alkyl mit 1 bis 4 Kohlenstoff-Atomen, eine Alkylthio-Gruppe mit 1 bis 4 Kohlenstoff-Atomen, eine Alkylsulfonyl-Gruppe, die durch ein Fluor-Atom und/oder ein Chlor-Atom substituiert sein kann,

eine Phenylthio-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, oder eine Phenylsulfonyl-Gruppe, die durch wenigstens einen Substituenten substituiert sein kann, der ausgewählt ist aus der aus einer Methyl-Gruppe, einem Fluor-Atom, einem Chlor-Atom und einem Brom-Atom bestehenden Klasse, außerdem

$R^9$ eine Bis-Form der Formel (I) über eine Methylen-Gruppe zu bilden vermag,

R ein Wasserstoff-Atom oder eine Methyl-Gruppe darstellt und

Z eine 5- bis 6-gliedrige heterocyclische Gruppe bezeichnet, die ein bis drei Hetero-Atome ausgewählt aus der aus einem Sauerstoff-Atom, einem Schwefel-Atom und einem Stickstoff-Atom bestehenden Klasse enthält, von denen wenigstens eines ein Stickstoff-Atom ist, und die durch wenigstens einen Substituenten substituiert sein kann, der ausgewählt ist aus der aus einem Fluor-Atom, einem Chlor-Atom, einem Brom-Atom, Alkyl-Gruppen mit 1 bis 4 Kohlenstoff-Atomen, die durch ein Fluor-Atom und/oder ein Chlor-Atom substituiert sein können, einer Nitro-Gruppe, einer Cyano-Gruppe, Alkylsulfinyl-Gruppen mit 1 bis 4 Kohlenstoff-Atomen, Alkylsulfonyl-Gruppen mit 1 bis 4 Kohlenstoff-Atomen, Alkoxy-Gruppen mit 1 bis 4 Kohlenstoff-Atomen, die durch ein Fluor-Atom und/oder ein Chlor-Atom substituiert ein können, Alkylthio-Gruppen mit 1 bis 4 Kohlenstoff-Atomen, die durch ein Fluor-Atom und/oder ein Chlor-Atom substituiert ein können, Alkenyl-Gruppen mit 2 bis 3

Kohlenstoff-Atomen, die durch ein Chlor-Atom substituiert sein können, eine Acetamid-Gruppe, die durch ein Fluor-Atom und/oder ein Chlor-Atom substituiert ein kann, Alkoxycarbonyl-Gruppen mit einem Alkyl mit 1 bis 4 Kohlenstoff-Atomen, einer Thiocyanato-Gruppe, Alkinyl-Gruppen mit 2 bis 4 Kohlenstoff-Atomen, einer Amino-Gruppe, einer Methylamino-Gruppe, einer Dimethylamino-Gruppe, einer Acetyl-Gruppe, einer Formyl-Gruppe, einer Carboxy-Gruppe, einer Hydroxy-Gruppe, einer Mercapto-Gruppe, Cycloalkyl-Gruppen mit 3 bis 7 Kohlenstoff-Atomen, einer Oxo-Gruppe, einer Thioxo-Gruppe, Alkenylthio-Gruppen, die durch ein Fluor-Atom, ein Chlor-Atom und/oder ein Brom-Atom substituiert sind, Alkoxyalkyl-Gruppen mit insgesamt 2 bis 4 Kohlenstoff-Atomen, Alkylaminocarbonyl-Gruppen mit einem Alkyl mit 1 bis 2 Kohlenstoff-Atomen, Dialkylaminocarbonyl-Gruppen mit einem Alkyl mit 1 bis 2 Kohlenstoff-Atomen, einer Phenyl-Gruppe, einer Phenoxy-Gruppe und einer Benzyl-Gruppe bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf.

3. Verbindungen nach Anspruch 1, dadurch gekennzeichnet, daß

n 0 oder 1 bezeichnet,

$R^1$, $R^2$, $R^5$ und $R^6$ unabhängig voneinander ein Wasserstoff-Atom oder eine Methyl-Gruppe bezeichnen,

$R^3$ und $R^4$ unabhängig voneinander ein Wasserstoff-Atom oder eine Methyl-Gruppe bezeichnen,

X ein Schwefel-Atom, ein Sauerstoff-Atom oder eine der folgenden Gruppen $-\overset{|}{N}-R^7$ oder $-\overset{|}{C}H-R^8$ bezeichnet,

$R^7$ ein Wasserstoff-Atom, eine Alkyl-Gruppe, die durch wenigstens einen Substituenten substituiert sein kann, der ausgewählt ist aus der aus einer Methoxy-Gruppe, einer Ethoxy-Gruppe, einer Methylthio-Gruppe, einer Ethylthio-Gruppe, einer Cyano-Gruppe, einem Fluor-Atom, einem Chlor-Atom und einer Trimethylsilyl-Gruppe bestehenden Klasse,
eine Allyl-Gruppe, die durch ein Chlor-Atom substituiert sein kann,
eine Propargyl-Gruppe,
eine Benzyl-Gruppe, die durch eine Methyl-Gruppe und/oder ein Chlor-Atom substituiert sein kann,
eine Formyl-Gruppe, eine Vinylcarbonyl-Gruppe,
eine Alkylcarbonyl-Gruppe mit einem Alkyl mit 1 bis 3 Kohlenstoff-Atomen, die durch wenigstens einen Substituenten substituiert sein kann, der ausgewählt ist aus der aus einer Methoxy-Gruppe, einer Phenoxy-Gruppe und einem Chlor-Atom bestehenden Klasse,
eine Benzoyl-Gruppe, die durch wenigstens einen Substituenten substituiert sein kann, der ausgewählt ist aus der aus einem Chlor-Atom, einem Brom-Atom, einer Methyl-Gruppe, einer Trifluoromethyl-Gruppe, einer Methoxy-Gruppe und einer Nitro-Gruppe bestehenden Klasse,
eine Benzylcarbonyl-Gruppe, die durch ein Chlor-Atom substituiert sein kann,
eine Alkoxycarbonyl-Gruppe mit einem Alkyl mit 1 bis 2 Kohlenstoff-Atomen, die durch ein Fluor-Atom und/oder ein Chlor-Atom substituiert sein kann,

eine Alkylthiocarbonyl-Gruppe mit einem Alkyl mit 1 bis 2 Kohlenstoff-Atomen,
eine Phenoxycarbonyl-Gruppe, die durch eine Methyl-Gruppe und/oder ein Chlor-Atom substituiert sein kann,
eine Phenylthiocarbonyl-Gruppe, die durch ein Chlor-Atom substituiert sein kann,
eine Benzyloxycarbonyl-Gruppe, eine Dimethylaminocarbonyl-Gruppe, eine Phenylaminocarbonyl-Gruppe,
eine Benzoylaminocarbonyl-Gruppe,
eine Phenylsulfonylaminocarbonyl-Gruppe, die durch eine Methyl-Gruppe und/oder ein Chlor-Atom substituiert sein kann,
eine Phenylthio-Gruppe,
eine Methylsulfonyl-Gruppe, die durch ein Chlor-Atom substituiert sein kann,
eine Phenylsulfonyl-Gruppe, die durch eine Methyl-Gruppe substituiert sein kann,
eine Methylcarbonylmethyl-Gruppe,
eine Phenacyl-Gruppe, die durch ein Chlor-Atom substituiert sein kann,
eine O,O-Diethylthionophosphono-Gruppe,
eine O-Ethyl-S-n-propylthiolophosphono-Gruppe,
die folgenden Gruppen $-CH_2-W$ oder $-CO-W$,

W eine 5- bis 6-gliedrige heterocyclische Gruppe bezeichnet, die ein oder zwei aus der aus Sauerstoff-Atomen, Schwefel-Atomen und Stickstoff-Atomen bestehenden Klasse ausgewählte Hetero-Atome enthält und die durch ein Fluor-Atom, ein Chlor-Atom, ein Brom-Atom und eine Methyl-Gruppe substituiert sein kann,

$R^8$ ein Wasserstoff-Atom, eine Methyl-Gruppe, eine Phenyl-Gruppe oder eine Benzyl-Gruppe darstellt,

Y ein Stickstoff-Atom oder die nachstehende Gruppe $=\overset{|}{C}-R^9$ bezeichnet,

$R^9$ ein Wasserstoff-Atom, ein Chlor-Atom, ein Brom-Atom, eine Hydroxy-Gruppe, eine Methoxy-Gruppe, eine Benzyloxy-Gruppe, eine Alkyl-Gruppe, die durch wenigstens einen Substituenten substituiert sein kann, der ausgewählt ist aus der aus einem Fluor-Atom, einem Chlor-Atom, einer Hydroxy-Gruppe, einer Methoxy-Gruppe, einer Cyano-Gruppe, einer Dimethylamino-Gruppe, einer Acetyl-Gruppe und einer Methoxycarbonyl-Gruppe bestehenden Klasse,

eine Allyl-Gruppe, eine Phenyl-Gruppe,

eine Acetyl-Gruppe, die durch ein Chlor-Atom substituiert sein kann, eine Vinylcarbonyl-Gruppe, eine Allylcarbonyl-Gruppe, eine Benzoyl-Gruppe, eine Alkoxycarbonyl-Gruppe mit einem Alkyl mit 1 bis 2 Kohlenstoff-Atomen, die durch ein Fluor-Atom substituiert sein kann,

eine n-Butylthiocarbonyl-Gruppe, eine Phenoxycarbonyl-Gruppe, die durch ein Chlor-Atom und/oder eine Methyl-Gruppe substituiert sein kann, eine Phenylthiocarbonyl-Gruppe, eine Benzyloxycarbonyl-Gruppe, eine Benzoylaminocarbonyl-Gruppe, die durch ein Chlor-Atom substituiert sein kann, eine Phenylsulfonylaminocarbonyl-Gruppe, die durch eine Methyl-Gruppe substituiert sein kann, eine Methylsulfonylaminocarbonyl-Gruppe

eine Propylthio-Gruppe, eine Methylsulfonyl-Gruppe, die durch ein Fluor-Atom und/oder ein Chlor-Atom substituiert sein kann, eine Phenylthio-Gruppe, die durch ein Chlor-Atom substituiert sein kann, oder eine Phenylsulfonyl-Gruppe, zusätzlich

$R^9$ eine Bis-Form der Formel (I) über eine Methylen-Gruppe zu bilden vermag,

R ein Wasserstoff-Atom oder eine Methyl-Gruppe darstellt und

Z eine 5- bis 6-gliedrige heterocyclische Gruppe bezeichnet, die ein bis drei Hetero-Atome ausgewählt aus der aus einem Sauerstoff-Atom, einem Schwefel-Atom und einem Stickstoff-Atom bestehenden Klasse enthält, von denen wenigstens eines ein Stickstoff-Atom ist, und die durch wenigstens einen Substituenten substituiert sein kann, der ausgewählt ist aus der aus einem Fluor-Atom, einem Chlor-Atom, einem Brom-Atom, einer Methyl-Gruppe, Fluoroalkyl-Gruppen mit 1 bis 2 Kohlenstoff-Atomen, einer Methoxy-Gruppe, einer Methylthio-Gruppe, einer Methylsulfinyl-Gruppe, einer Methylsulfonyl-Gruppe, einer Nitro-Gruppe, einer Cyano-Gruppe, einer Trifluoromethoxy-Gruppe, einer Trifluoromethylthio-Gruppe, einer Allyl-Gruppe, einer Acetamid-Gruppe, einer Methoxycarbonyl-Gruppe, einer Acetyl-Gruppe, einer Formyl-Gruppe und einer Carboxy-Gruppe bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf.

4. Verbindungen nach Ansprüchen 1 bis 3, ausgewählt aus 3-(2-Chloro-5-pyridylmethyl)-2-(nitromethylen)-tetrahydro-2H-1,3-thiazin der folgenden Formel

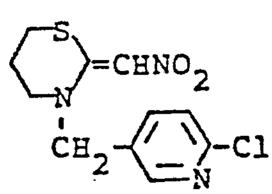

,

3-(2-Chloro-5-pyridylmethyl)-2-(nitromethylen)-thiazolidin der folgenden Formel

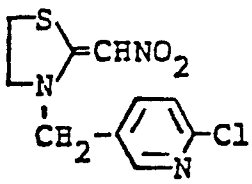

,

3-(2-Fluoro-5-pyridylmethyl)-2-(nitromethylen)-tetrahydro-2H-1,3-thiazin der folgenden Formel

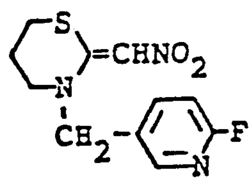

,

3-(2-Fluoro-5-pyridylmethyl)-2-(nitromethylen)-thiazolidin der folgenden Formel

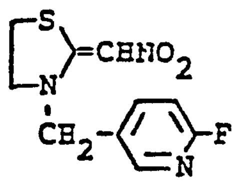

,

3-(2-Bromo-5-pyridylmethyl)-2-(nitromethylen)-tetrahydro-2H-1,3-thiazin der folgenden Formel

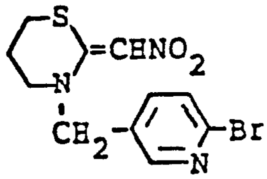

,

3-(2-Bromo-5-pyridylmethyl)-2-(nitromethylen)-thiazolidin der folgenden Formel

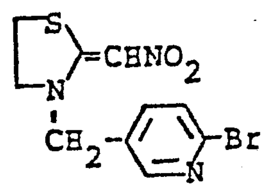

,

3-/¯1-(2-Chloro-5-pyridyl)ethyl_7-2-(nitromethylen)-thiazolidin der folgenden Formel

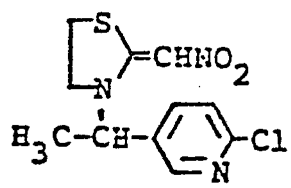

,

3-(2-Methyl-5-pyridylmethyl)-2-(nitromethylen)-thiazolidin der folgenden Formel

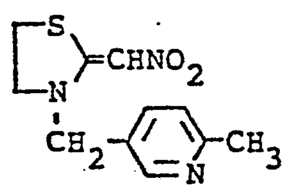

,

3-(2-Methyl-5-pyridylmethyl)-2-(nitromethylen)-tetrahydro-2H-1,3-thiazin der folgenden Formel

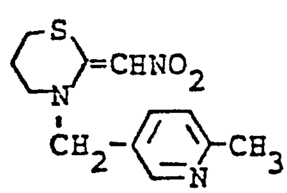

,

3-(2-Trifluoromethyl-5-pyridylmethyl)-2-(nitromethylen)thiazolidin der folgenden Formel

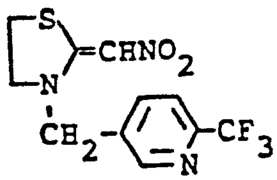

,

3-(2-Ethyl-5-pyridylmethyl)-2-(nitromethylen)-thiazolidin der folgenden Formel

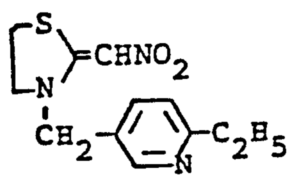

,

3-(3-Pyridylmethyl)-2-(nitromethylen)-tetrahydro-2H-1,3-thiazin der folgenden Formel

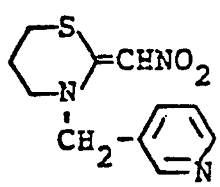

,

1-(5-Pyrazolylmethyl)-2-(nitromethylen)imidazolidin der folgenden Formel

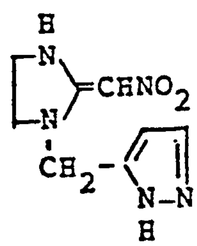

,

Nit 189

1-(5-Chloro-1-methyl-3-pyrazolylmethyl)-2-(nitromethylen)imidazolidin der folgenden Formel

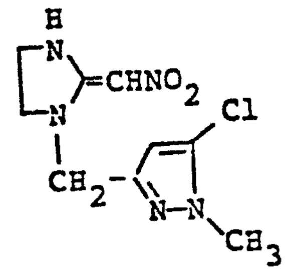

,

1-(1-Methyl-4-pyrazolylmethyl)-2-(nitromethylen)tetrahydropyrimidin der folgenden Formel

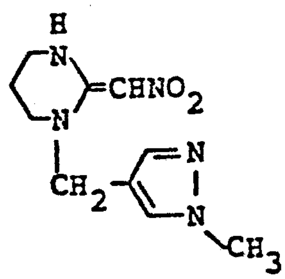

,

1-(1-Methyl-4-pyrazolylmethyl)-2-(nitromethylen)imidazolidin der folgenden Formel

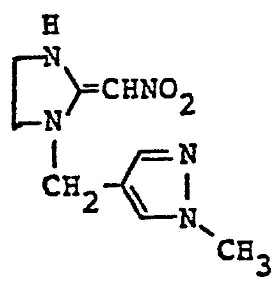

,

1-(3-Trifluoromethyl-5-isoxazolylmethyl)-2-(nitromethylen)tetrahydropyrimidin der folgenden Formel

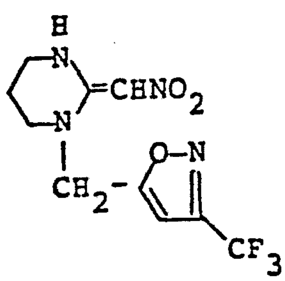

,

Nit 189

1-(3-Methyl-5-isoxazolylmethyl)-2-(nitromethylen)imid-azolidin der folgenden Formel

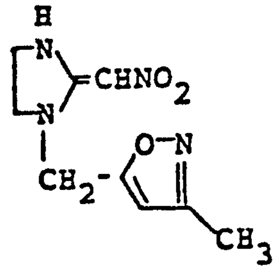

,

1-(3-Methyl-5-isoxazolylmethyl)-2-(nitromethylen)tetra-hydropyrimidin der folgenden Formel

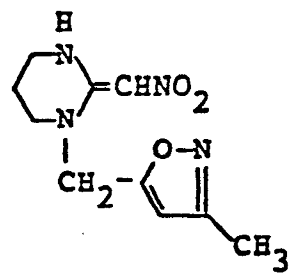

,

1-(5-Isoxazolylmethyl)-2-(nitromethylen)imidazolidin der folgenden Formel

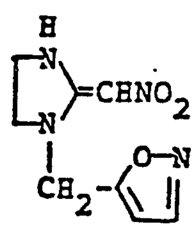

,

1-(2-Methyl-5-thiazolylmethyl)-2-(nitromethylen)imid-azolidin der folgenden Formel

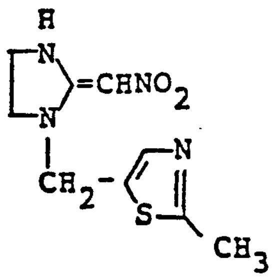

,

1-(2-Chloro-5-thiazolylmethyl)-2-(nitromethylen)tetrahydropyrimidin der folgenden Formel

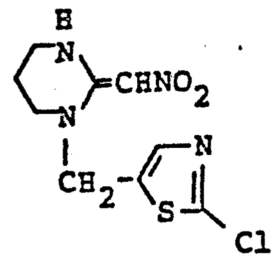

,

1-(2-Trifluoromethyl-5-thiazolylmethyl)-2-(nitromethylen)imidazolidin der folgenden Formel

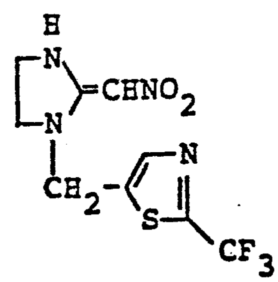

,

1-(1,2,5-Thiadiazol-3-ylmethyl)-2-(nitromethylen)tetrahydropyrimidin der folgenden Formel

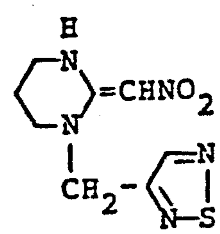

,

1-(1,2,3-Thiadiazol-5-ylmethyl)-2-(nitromethylen)tetrahydropyrimidin der folgenden Formel

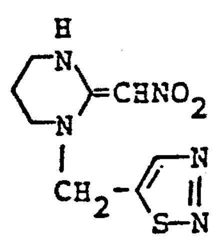

,

Nit 189

1-(2-Methyl-5-thiazolylmethyl)-2-(nitromethylen)tetrahydropyrimidin der folgenden Formel

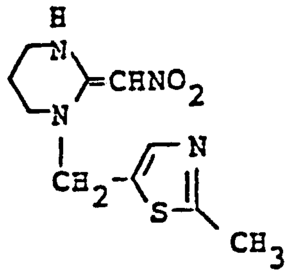

,

1-(2-Chloro-5-thiazolylmethyl)-2-(nitromethylen)imidazolidin der folgenden Formel

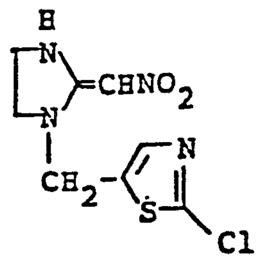

,

1-(5-Thiazolylmethyl)-2-(nitromethylen)tetrahydropyrimidin der folgenden Formel

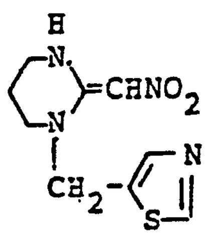

,

1-(5-Pyrimidinylmethyl)-2-(nitromethylen)imidazolidin der folgenden Formel

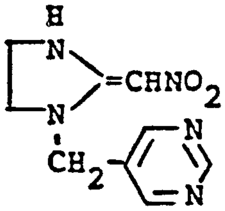

,

1-(2-Methyl-5-pyrimidinylmethyl)-2-(nitromethylen)imidazolidin der folgenden Formel

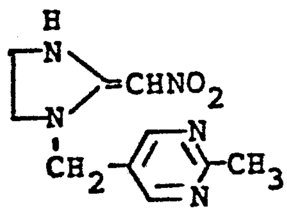,

1-(2-Pyrazinylmethyl)-2-(nitromethylen)imidazolidin der folgenden Formel

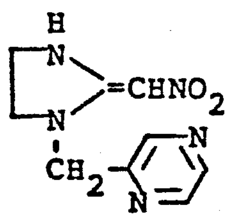,

1-(5-Methyl-2-pyrazinylmethyl)-2-(nitromethylen)imidazolidin der folgenden Formel

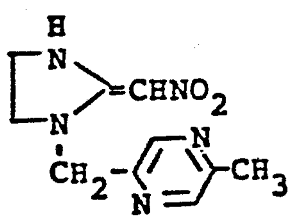,

1-(2-Chloro-5-pyrimidinylmethyl)-2-(nitromethylen)-tetrahydropyrimidin der folgenden Formel

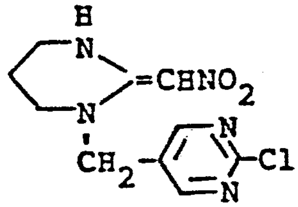,

Nit 189

1-(2-Chloro-5-pyrimidinylmethyl)-2-(nitromethylen)imidazolidin der folgenden Formel

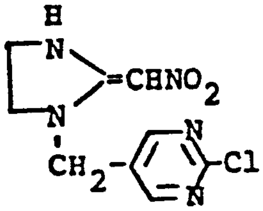

,

1-(2-Chloro-5-pyridylmethyl)-2-(nitroimino)tetrahydropyrimidin der folgenden Formel

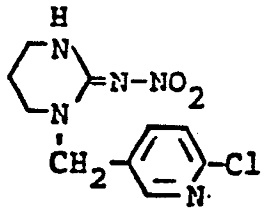

,

1-(2-Chloro-5-pyridylmethyl)-2-(nitroimino)imidazolidin der folgenden Formel

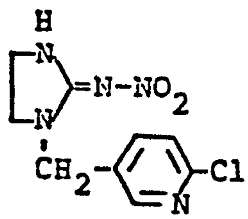

,

1-(2-Chloro-5-pyridylmethyl)-2-(nitromethylen)pyrrolidin der folgenden Formel

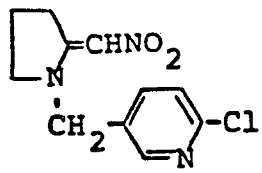

,

1-(2-Chloro-5-thiazolylmethyl)-2-(nitroimino)tetrahydropyrimidin der folgenden Formel

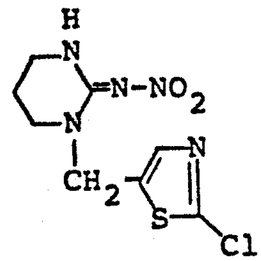

,

1-(2-Chloro-5-pyrimidinylmethyl)-2-(nitroimino)imidazolidin der folgenden Formel

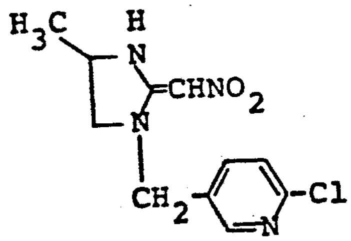

,

1-(2-Chloro-5-pyridylmethyl)-3-(3-pyridylmethyl)-2-(nitromethylen)imidazolidin der folgenden Formel

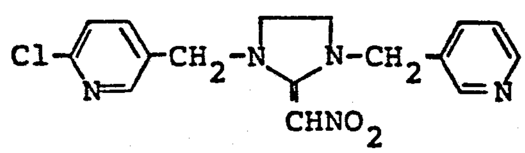

,

1-(2-Chloro-5-pyridylmethyl)-2-(bromonitromethylen)-imidazolidin der folgenden Formel

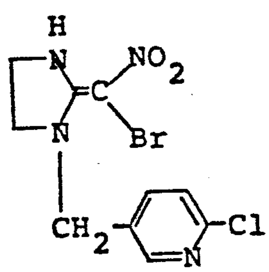

,

Nit 189

1-(2-Chloro-5-pyridylmethyl)-2-(1-nitro-2-oxopentyliden)imidazolidin der folgenden Formel

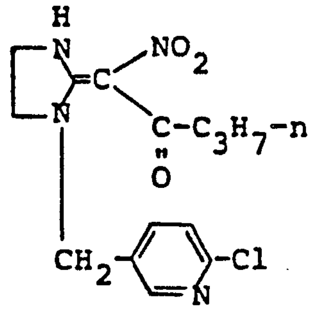

,

Ethyl-nitro/¯3-(2-chloro-5-pyridylmethyl)thiazolidin-2-yliden_7acetat der folgenden Formel

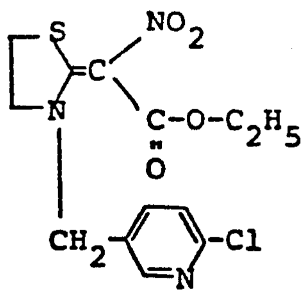

,

1-Acetyl-3-(2-chloro-5-pyridylmethyl)-2-(nitroimino)imidazolidin der folgenden Formel

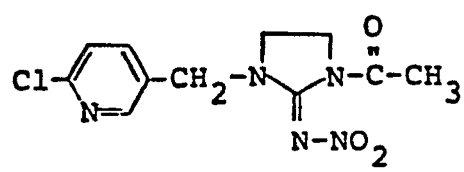

,

N-Phenylsulfonyl-nitro-/¯1-(2-chloro-5-pyridylmethyl)-imidazolidin-2-yliden_7acetamid der folgenden Formel

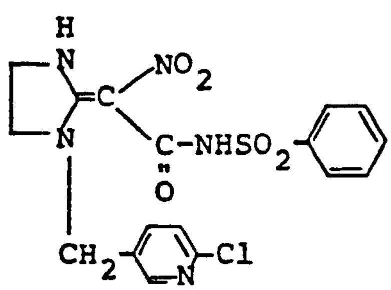

und

Nit 189

3-(2-Chloro-5-pyridylmethyl)-2-(nitroimino)-thiazolidin der folgenden Formel

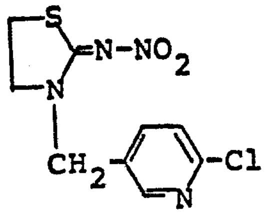

.

5. Verfahren zur Herstellung neuer heterocyclischer Verbindungen der Formel (Ia)

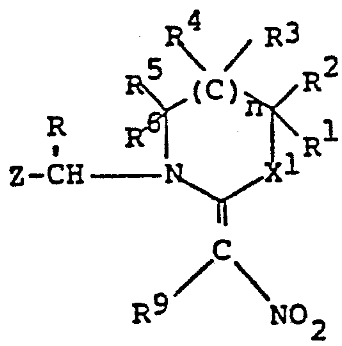

(Ia)

,

in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

$X^1$ ein Schwefel-Atom, ein Sauerstoff-Atom oder die folgende Gruppen $-\overset{|}{N}-R^{10}$ bezeichnet, worin

$R^{10}$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus

Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Phenylthio-Gruppe, die durch eine Alkyl-Gruppe und/oder ein Halogen-Atom substituiert sein kann, eine Alkylcarbonylmethyl-Gruppe, eine Phenacyl-Gruppe, die durch ein Halogen-Atom und/oder eine Alkyl-Gruppe substituiert sein kann, die folgende Gruppe $-CH_2-W$,
worin W eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen bestehenden Klasse, darstellt,

$R^9$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch

wenigstens einen Substituenten substituiert sein kann, der ausgewählt ist aus der aus Halogen-Atomen, einer Hydroxy-Gruppe, Alkoxy-Gruppen, Alkylthio-Gruppen, einer Cyano-Gruppe, Mono- oder Dialkylamino-Gruppen, Alkylcarbonyl-Gruppen, Alkoxycarbonyl-Gruppen und Phenoxycarbonyl-Gruppen bestehenden Klasse, eine Alkenyl-Gruppe, die durch ein Halogen-Atom substituiert sein kann, eine Alkinyl-Gruppe, eine Phenyl-Gruppe, die durch eine Alkyl-Gruppe und/oder ein Halogen-Atom substituiert sein kann, eine Alkylcarbonyl-Gruppe, die durch ein Halogen-Atom substituiert sein kann, eine Alkenylcarbonyl-Gruppe, eine Benzoyl-Gruppe, die durch wenigstens einen Substituenten substituiert sein kann, der ausgewählt ist aus der aus Halogen-Atomen, Alkyl-Gruppen und Alkoxy-Gruppen bestehenden Klasse, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus Halogen-Atomen, Alkyl-Gruppen, Alkoxy-Gruppen und einer Nitro-Gruppe bestehenden Klasse, eine Phenylthiocarbonyl-Gruppe, die durch eine Alkyl-Gruppe und/oder ein Halogen-Atom substituiert sein kann, eine Benzyloxycarbonyl-Gruppe, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-

Gruppe und/oder ein Halogen-Atom substituiert sein kann, eine Alkylsulfonylaminocarbonyl-Gruppe, eine Alkylthio-Gruppe, eine Alkylsulfonyl-Gruppe, die durch ein Halogen-Atom substituiert sein kann, eine Phenylthio-Gruppe, die durch eine Alkyl-Gruppe und/oder ein Halogen-Atom substituiert sein kann, eine Phenylsulfonyl-Gruppe, die durch eine Alkyl-Gruppe und/oder ein Halogen-Atom substituiert sein kann, und außerdem

$R^9$ eine Bis-Form der Formel (I) über eine Methylen-Gruppe zu bilden vermag,

R ein Wasserstoff-Atom oder eine Alkyl-Gruppe darstellt und

Z eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen, einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer

Thioxo-Gruppe, Halogenoalkenylthio-Gruppen, Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die gegebenenfalls durch einen der gleichen Substituenten substituiert sind, wie sie für die Aryl-Gruppen angegeben ist, bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

$R^9$ ein Wasserstoff-Atom bezeichnet, dann

Z nicht für die Pyridyl-Gruppe stehen darf,

dadurch gekennzeichnet, daß

(a) die Verbindungen der Formel (II)

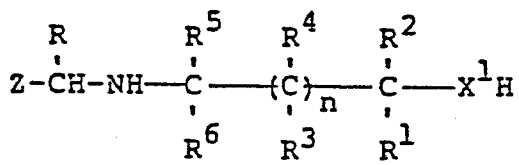

(II)

,

in der n, $R^1$, $R^2$, $R^3$, $R^4$, $R^5$, $R^6$, R, $X^1$ und Z die im Vorstehenden angegebenen Bedeutungen haben,

mit den Verbindungen der Formel (III)

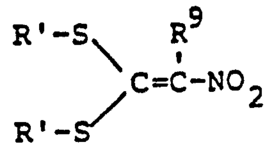

(III)

umgesetzt werden, in der R' eine Niederalkyl- oder Benzyl-Gruppe bezeichnet oder die beiden R' zusammen mit den zwei Schwefel-Atomen, an die sie gebunden sind, einen Ring bilden können, und $R^9$ die gleiche Bedeutung hat, wie sie oben angegeben ist, gegebenenfalls in Anwesenheit inerter Lösungsmittel,

(b) die Verbindungen der vorstehenden Formel (II) mit Verbindungen der Formel (IV)

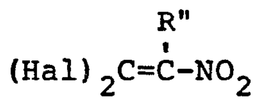

(IV)

umgesetzt werden, in der Hal ein Halogen-Atom bezeichnet und R" für ein Wasserstoff-Atom, ein Halogen-Atom oder eine Niederalkyl-Gruppe steht, gegebenenfalls in Anwesenheit inerter Lösungsmittel und in Anwesenheit von Säure-Acceptoren, oder

(c) die Verbindungen der vorstehenden Formel (II) mit Verbindungen der Formel (V)

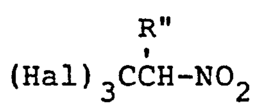

(V)

umgesetzt werden, in der Hal und R" die gleichen Bedeutungen haben, wie sie oben angegeben sind, gegebenenfalls in Anwesenheit inerter Lösungsmittel und in Anwesenheit von Säure-Acceptoren.

6. Verfahren zur Herstellung neuer heterocyclischer Verbindungen der Formel (Ib)

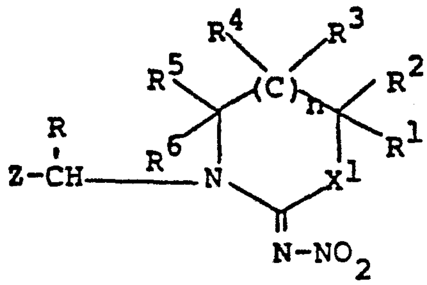

(Ib)

in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

$X^1$ ein Schwefel-Atom, ein Sauerstoff-Atom oder die folgende Gruppen $-\overset{|}{N}-R^{10}$ bezeichnet, worin

$R^{10}$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch

eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Phenylthio-Gruppe, die durch eine Alkyl-Gruppe und/oder ein Halogen-Atom substituiert sein kann, eine Alkylcarbonylmethyl-Gruppe, eine Phenacyl-Gruppe, die durch ein Halogen-Atom und/oder eine Alkyl-Gruppe substituiert sein kann, die folgende Gruppe $-CH_2-W$, worin W eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen bestehenden Klasse, darstellt,

R ein Wasserstoff-Atom oder eine Alkyl-Gruppe darstellt und

Z eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen, einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer Thioxo-Gruppe, Halogenoalkenylthio-Gruppen, Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die gegebenenfalls durch einen der gleichen Substituenten substituiert sind, wie sie für die Aryl-Gruppen angegeben ist, bestehenden Klasse,

dadurch gekennzeichnet, daß
die Verbindungen der Formel (II)

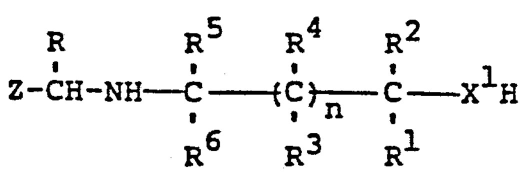 (II)

,

in der n, $R^1$, $R^2$, $R^3$, $R^4$, $R^5$, $R^6$, R, $X^1$ und Z die im Vorstehenden angegebenen Bedeutungen haben,
mit Nitroguanidin der folgenden Formel

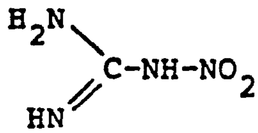

umgesetzt werden, gegebenfalls in Anwesenheit inerter Lösungsmittel.

7. Verfahren zur Herstellung neuer heterocyclischer Verbindungen der Formel (Ic)

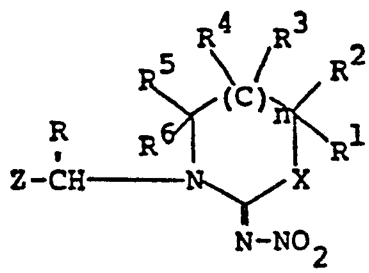 (Ic)

,

in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

X ein Schwefel-Atom, ein Sauerstoff-Atom oder eine der folgenden Gruppen $-\overset{|}{N}-R^7$ oder $-\overset{|}{C}H-R^8$ bezeichnet, worin

$R^7$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Alkenylcarbonyl-Gruppe, eine Alkylcarbonyl-Gruppe, die durch wenigstens einen Substituenten substituiert sein kann, der ausgewählt ist aus der aus Alkoxy-Gruppen, einer Phenoxy-Gruppe, Alkylthio-Gruppen und Halogen-Atomen bestehenden Klasse, eine Benzoyl-Gruppe, die durch wenigstens einen Substituenten substituiert sein kann, der ausgewählt ist aus der aus Halogen-Atomen, Alkyl-Gruppen, Halogenomethyl-Gruppen, Alkoxy-Gruppen, Halogenoalkoxy-Gruppen und einer Nitro-Gruppe bestehenden Klasse, eine Benzylcarbonyl-Gruppe, die durch eine Alkyl-Gruppe

und/oder ein Halogen-Atom substituiert sein kann, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus einer Methyl-Gruppe, einer Methoxy-Gruppe, Halogenomethyl-Gruppen, Halogenomethoxy-Gruppen, Halogen-Atomen und einer Nitro-Gruppe bestehenden Klasse, eine Phenylthiocarbonyl-Gruppe, die durch ein Halogen-Atom und/oder eine Alkyl-Gruppe substituiert sein kann, eine Benzyloxycarbonyl-Gruppe, eine Monoalkyl- oder Dialkylaminocarbonyl-Gruppe, eine Phenylaminocarbonyl-Gruppe, die durch wenigstens einen Substituenten substituiert sein kann, der ausgewählt ist aus der aus Alkyl-Gruppen, Halogenoalkyl-Gruppen und Halogen-Atomen bestehenden Klasse, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert sein kann, eine Phenylthio-Gruppe, die durch eine Alkyl-Gruppe und/oder ein Halogen-Atom substituiert sein kann, eine Alkylsulfonyl-Gruppe, die durch ein Halogen-Atom substituiert sein kann, eine Phenylsulfonyl-Gruppe, die durch wenigstens einen Substituenten substituiert sein kann, der ausgewählt ist aus der aus Alkyl-Gruppen, Halogen-Atomen und einer Nitro-Gruppe bestehenden

Klasse, eine Alkylcarbonylmethyl-Gruppe, eine Phenacyl-Gruppe, die durch ein Halogen-Atom und/oder eine Alkyl-Gruppe substituiert sein kann, eine Organophosphono-Gruppe, eine Organothiophosphono-Gruppe, die folgenden Gruppen $-CH_2-W$ oder $-CO-W$,

worin W eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen, darstellt,

$R^8$ ein Wasserstoff-Atom, eine Alkyl-Gruppe, eine Aryl-Gruppe oder eine Benzyl-Gruppe darstellt,

R ein Wasserstoff-Atom oder eine Alkyl-Gruppe darstellt und

Z eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen,

einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer Thioxo-Gruppe, Halogenoalkenylthio-Gruppen, Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die gegebenenfalls durch einen der gleichen Substituenten substituiert sind, wie sie für die Aryl-Gruppen angegeben ist, bestehenden Klasse,

dadurch gekennzeichnet, daß die Verbindungen der Formel (VI)

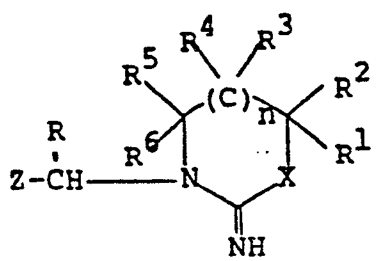

(VI)

,

in der

n, $R^1$, $R^2$, $R^3$, $R^4$, $R^5$, $R^6$, X, R und Z die im Vorstehenden angegebenen Bedeutungen haben, mit rauchender Salpetersäure umgesetzt werden, gegebenenfalls in Anwesenheit inerter Lösungsmittel.

8. Verfahren zur Herstellung neuer heterocyclischer Verbindungen der Formel (I)

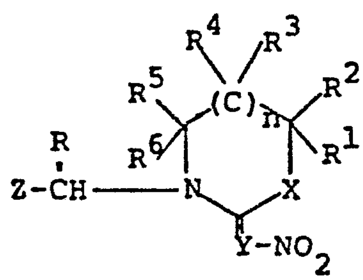

(I)

,

in der

n 0 oder 1 bezeichnet,

$R^1$, $R^2$, $R^5$ und $R^6$ unabhängig voneinander ein Wasserstoff-Atom oder eine Alkyl-Gruppe bezeichnen,

$R^3$ und $R^4$ unabhängig voneinander ein Wasserstoff-Atom, eine Hydroxy-Gruppe oder eine Alkyl-Gruppe bezeichnen,

wenn n 1 bezeichnet, $R^2$ eine Einfachbindung zusammen mit $R^5$ zu bilden vermag,

X ein Schwefel-Atom, ein Sauerstoff-Atom oder eine der folgenden Gruppen $-\dot{N}-R^7$ oder $-\dot{C}H-R^8$ bezeichnet, worin

$R^7$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus

Alkoxy-Gruppen, Alkylthio-Gruppen, einer Cyano-Gruppe, Halogen-Atomen, Dialkylamino-Gruppen und Trialkylsilyl bestehenden Klasse, eine Alkenyl-Gruppe, die durch ein Halogen-Atom substituiert sein kann, eine Alkinyl-Gruppe, die durch ein Halogen-Atom substituiert sein kann, eine Phenyl-Gruppe, die durch eine Alkyl-Gruppe und/oder ein Halogen-Atom substituiert sein kann, eine Benzyl-Gruppe, die durch wenigstens einen Substituenten substituiert sein kann, der ausgewählt ist aus der aus einer Methyl-Gruppe, einer Methoxy-Gruppe, Halogen-Atomen, Halogenomethyl-Gruppen, Halogenomethoxy-Gruppen und einer Nitro-Gruppe bestehenden Klasse, eine Formyl-Gruppe, eine Alkenylcarbonyl-Gruppe, eine Alkylcarbonyl-Gruppe, die durch wenigstens einen Substituenten substituiert sein kann, der ausgewählt ist aus der aus Alkoxy-Gruppen, einer Phenoxy-Gruppe, Alkylthio-Gruppen und Halogen-Atomen bestehenden Klasse, eine Benzoyl-Gruppe, die durch wenigstens einen Substituenten substituiert sein kann, der ausgewählt ist aus der aus Halogen-Atomen, Alkyl-Gruppen, Halogenomethyl-Gruppen, Alkoxy-Gruppen, Halogenoalkoxy-Gruppen und einer Nitro-Gruppe bestehenden Klasse, eine Benzylcarbonyl-Gruppe, die durch eine Alkyl-Gruppe und/oder ein Halogen-Atom substituiert sein kann, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus einer Methyl-Gruppe, einer Methoxy-Gruppe, Halogenomethyl-Gruppen, Halogenomethoxy-Gruppen, Halogen-Atomen und einer Nitro-Gruppe bestehenden Klasse, eine Phenylthiocarbonyl-Gruppe, die durch ein Halogen-Atom und/oder eine Alkyl-Gruppe substituiert sein kann, eine Benzyloxycarbonyl-Gruppe, eine Monoalkyl- oder Dialkylaminocarbonyl-Gruppe, eine Phenylaminocarbonyl-Gruppe, die durch wenigstens einen Substituenten substituiert sein kann, der ausgewählt ist aus der aus Alkyl-Gruppen, Halogenoalkyl-Gruppen und Halogen-Atomen bestehenden Klasse, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert sein kann, eine Phenylthio-Gruppe, die durch eine Alkyl-Gruppe und/oder ein Halogen-Atom substituiert sein kann, eine Alkylsulfonyl-Gruppe, die durch ein Halogen-Atom substituiert sein kann, eine Phenylsulfonyl-Gruppe, die durch wenigstens einen Substituenten substituiert sein kann, der ausgewählt ist aus der aus Alkyl-Gruppen, Halogen-Atomen und einer Nitro-Gruppe bestehenden Klasse, eine Alkylcarbonylmethyl-Gruppe, eine Phenacyl-Gruppe, die durch ein Halogen-Atom und/oder eine Alkyl-Gruppe substituiert sein kann, eine Organophosphono-Gruppe, eine Organothiophosphono-Gruppe, die folgenden Gruppen $-CH_2-W$ oder $-CO-W$,

worin W eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen und Halogenoalkyl-Gruppen, darstellt,

$R^8$ ein Wasserstoff-Atom, eine Alkyl-Gruppe, eine Aryl-Gruppe oder eine Benzyl-Gruppe darstellt,

Y ein Stickstoff-Atom oder die nachstehende Gruppe $=\overset{|}{C}-R^9$ bezeichnet, worin

$R^9$ ein Wasserstoff-Atom, ein Halogen-Atom, eine Hydroxy-Gruppe, eine Alkoxy-Gruppe, eine Benzyloxy-Gruppe, eine Alkyl-Gruppe, die durch wenigstens einen Substituenten substituiert sein kann, der ausgewählt ist aus der aus Halogen-Atomen, einer Hydroxy-Gruppe, Alkoxy-Gruppen, Alkylthio-Gruppen, einer Cyano-Gruppe, Mono- oder Dialkylamino-Gruppen, Alkylcarbonyl-Gruppen, Alkoxycarbonyl-Gruppen und Phenoxycarbonyl-Gruppen bestehenden Klasse, eine Alkenyl-Gruppe, die durch ein Halogen-Atom substituiert sein kann, eine Alkinyl-Gruppe, eine Phenyl-Gruppe, die durch eine Alkyl-Gruppe und/oder ein Halogen-Atom substituiert sein kann, eine Alkylcarbonyl-Gruppe, die durch ein Halogen-Atom substituiert sein kann, eine Alkenylcarbonyl-Gruppe, eine Benzoyl-Gruppe, die durch wenigstens einen Substituenten substituiert sein kann, der ausgewählt ist aus der aus Halogen-Atomen,

Alkyl-Gruppen und Alkoxy-Gruppen bestehenden Klasse, eine Alkoxycarbonyl-Gruppe, die durch ein Halogen-Atom substituiert sein kann, eine Alkylthiocarbonyl-Gruppe, eine Phenoxycarbonyl-Gruppe, die durch wenigstens einen Substituenten substituiert sein kann, der ausgewählt ist aus der aus Halogen-Atomen, Alkyl-Gruppen, Alkoxy-Gruppen und einer Nitro-Gruppe bestehenden Klasse, eine Phenylthiocarbonyl-Gruppe, die durch eine Alkyl-Gruppe und/oder ein Halogen-Atom substituiert sein kann, eine Benzyloxycarbonyl-Gruppe, eine Benzoylaminocarbonyl-Gruppe, die durch eine Alkyl-Gruppe und/oder ein Halogen-Atom substituiert sein kann, eine Phenylsulfonylaminocarbonyl-Gruppe, die durch eine Alkyl-Gruppe und/oder ein Halogen-Atom substituiert sein kann, eine Alkylsulfonylaminocarbonyl-Gruppe, eine Alkylthio-Gruppe, eine Alkylsulfonyl-Gruppe, die durch ein Halogen-Atom substituiert sein kann, eine Phenylthio-Gruppe, die durch eine Alkyl-Gruppe und/oder ein Halogen-Atom substituiert sein kann, eine Phenylsulfonyl-Gruppe, die durch eine Alkyl-Gruppe und/oder ein Halogen-Atom substituiert sein kann, und außerdem

$R^9$ eine Bis-Form der Formel (I) über eine Methylen-Gruppe zu bilden vermag,

R ein Wasserstoff-Atom oder eine Alkyl-Gruppe darstellt und

Z eine 5- bis 6-gliedrige heterocyclische Gruppe bezeichnet, die wenigstens ein aus Sauerstoff-Atomen, Schwefel-Atomen und Stickstoff-Atomen ausgewähltes Hetero-Atom enthält und durch wenigstens einen Substituenten substituiert sein kann, der ausgewählt ist aus der aus Halogen-Atomen, Alkyl-Gruppen, Halogenoalkyl-Gruppen, einer Nitro-Gruppe, einer Cyano-Gruppe, Alkoxy-Gruppen, Alkylthio-Gruppen, Alkylsulfinyl-Gruppen, Alkylsulfonyl-Gruppen, Alkenyl-Gruppen, Halogenoalkoxy-Gruppen, Halogenoalkylthio-Gruppen, Halogenoalkenyl-Gruppen, Acylamino-Gruppen, Halogenoacylamino-Gruppen, Alkoxycarbonyl-Gruppen, einer Thiocyanato-Gruppe, Alkinyl-Gruppen, einer Amino-Gruppe, Alkylamino-Gruppen, Dialkylamino-Gruppen, einer Carboxy-Gruppe, einer Hydroxy-Gruppe, einer Mercapto-Gruppe, Cycloalkyl-Gruppen, einer Oxo-Gruppe, einer Thioxo-Gruppe, Halogenoalkenylthio-Gruppen, Alkoxyalkyl-Gruppen, Alkoxycarbonylamino-Gruppen, einer Carbamoyl-Gruppe, Acyl-Gruppen, Alkylaminocarbonyl-Gruppen, Dialkylaminocarbonyl-Gruppen, einer Formyl-Gruppe, Aryl-Gruppen, die gegebenenfalls durch einen aus der aus Halogen-Atomen, Alkyl-Gruppen, Halogenoalkyl-Gruppen, Alkoxy-Gruppen, einer Nitro-Gruppe und einer Cyano-Gruppe bestehenden Klasse ausgewählten Substituenten substituiert sind, Aryloxy-Gruppen, die gegebenenfalls durch einen Substituenten substituiert sind, wie er für die vorstehenden Aryl-Gruppen angegeben ist, und Aralkyl-Gruppen, die gegebenenfalls durch einen der gleichen Substituenten substituiert sind, wie sie für die Aryl-Gruppen angegeben ist, bestehenden Klasse, mit der Maßgabe, daß, wenn

$R^1$, $R^2$, $R^3$, $R^4$, $R^5$ und $R^6$ gleichzeitig Wasserstoff-Atome darstellen,

X -NH bezeichnet und

Y =CH bezeichnet, dann

Z nicht für die Pyridyl-Gruppe stehen darf,

dadurch gekennzeichnet, daß die Verbindungen der Formel (VII)

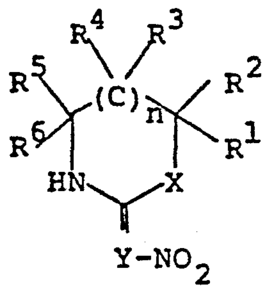

(VII) ,

in der

n, $R^1$, $R^2$, $R^3$, $R^4$, $R^5$, $R^6$, X und Y die im Vorstehenden angegebenen Bedeutungen haben, mit den Verbindungen der Formel (VIII)

$$\begin{matrix} R \\ | \\ Z\text{-}CH\text{-}M \end{matrix} \qquad (VIII)$$

umgesetzt werden, in der R und Z die im Vorstehenden angegebenen Bedeutungen haben, M ein Halogen-Atom oder die folgende Gruppe $-OSO_2T$ bezeichnet und T für eine Niederalkyl-Gruppe, eine Phenyl-Gruppe oder eine Tolyl-Gruppe steht, gegebenfalls in Anwesenheit inerter Lösungsmittel und in Anwesenheit von Säure-Acceptoren.

9. Insektizide Mittel, dadurch gekennzeichnet, daß sie wenigstens eine heterocyclische Verbindung der Formel (I) nach Ansprüchen 1 bis 4 enthalten.

10. Verfahren zur Bekämpfung von Schadinsekten, dadurch gekennzeichnet, daß man neue heterocyclische Verbindungen der Formel (I) nach Ansprüchen 1 bis 4 auf die schädlichen Insekten und/oder deren Lebensraum einwirken läßt.

11. Verwendung neuer heterocyclischer Verbindungen der Formel (I) nach Ansprüchen 1 bis 4 zur Bekämpfung von Schadinsekten.

12. Verfahren zur Herstellung von insektiziden Mitteln, dadurch gekennzeichnet, daß neue heterocyclische Verbindungen der Formel (I) nach Ansprüchen 1 bis 4 mit Streckmitteln und/oder grenzflächenaktiven Mitteln vermischt werden.

Europäisches Patentamt

**EUROPÄISCHER RECHERCHENBERICHT**

0192060
Nummer der Anmeldung
EP 86100708.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | EP - A2 - 0 092 158 (MITSUBISHI CHEMICAL ..)<br>* Formel I *<br>-- | 1 | C 07 D 401/06<br>C 07 D 401/14<br>C 07 D 403/06<br>C 07 D 403/14<br>C 07 D 413/06<br>C 07 D 413/14<br>C 07 D 417/06<br>C 07 D 417/14<br>C 07 D 405/06<br>C 07 D 409/06<br>C 07 D 419/06<br>A 01 N 43/50<br>A 01 N 43/76<br>A 01 N 43/86<br>A 01 N 43/54<br>A 01 N 43/78<br>A 01 N 43/64 |
| A | DE - A1 - 2 824 690 (BAYER)<br>* Formel I *<br>-- | 1 | |
| A | EP - A1 - 0 029 742 (PFIZER)<br>* Formel I *<br>-- | 1 | |
| A | EP - A3 - 0 060 730 (JCJ)<br>* Zusammenfassung *<br>-- | 1 | RECHERCHIERTE SACHGEBIETE (Int Cl 4) |
| A | DE - A1 - 3 409 801 (RICHTER)<br>* Zusammenfassung *<br>---- | 1,5-8 | C 07 D 401/00<br>C 07 D 403/00<br>C 07 D 413/00<br>C 07 D 417/00<br>C 07 D 405/00<br>C 07 D 409/00<br>C 07 D 419/00 |

Der vorliegende Recherchenbericht wurde für alle Patentansprüche erstellt.

| Recherchenort | Abschlußdatum der Recherche | Prüfer |
|---|---|---|
| WIEN | 23-04-1986 | HAMMER |

KATEGORIE DER GENANNTEN DOKUMENTEN
X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze
E : älteres Patentdokument, das jedoch erst am oder nach dem Anmeldedatum veröffentlicht worden ist
D in der Anmeldung angeführtes Dokument
L aus andern Gründen angeführtes Dokument
&: Mitglied der gleichen Patentfamilie, übereinstimmendes Dokument

EPA Form 1503 03 82